# EUROPEAN PATENT SPECIFICATION

(11) **EP 1 730 116 B1**
(45) Date of publication and mention of the grant of the patent: **05.09.2007**
(21) Application number: 05729380.5
(22) Date of filing: 22.03.2005
(51) Int. Cl.: C07D 231/38, C07D 403/04, C07D 409/04, C07D 405/12, C07D 403/12, C07D 403/10, A61K 31/415, A61P 31/12

(54) **4-CARBOX PYRAZOLE DERIVATES USEFUL AS ANTI-VIRAL AGENTS**
ALS ANTIVIRALE MITTEL GEEIGNETE 4-CARBOXYPYRAZOLDERIVATE
DÉRIVÉS DE 4-CARBOX PYRAZOLE UTILES EN TANT QU'AGENTS ANTIVIRAUX

(30) Priority: 26.03.2004 GB 0406916; 26.03.2004 GB 0406918; 15.10.2004 GB 0423000; 15.10.2004 GB 0423001
(43) Date of publication of application: 13.12.2006
(73) Proprietor: SMITHKLINE BEECHAM CORPORATION, Philadelphia, PA 19101 (US)
(72) Inventor: BRAVI, Gianpaolo, GlaxoSmithKline, Stevenage Hertfordshire SG1 2NY (GB); CORFIELD, John Andrew, GlaxoSmithKline, Stevenage Hertfordshire SG1 2NY (GB); GRIMES, Richard Martin, GlaxoSmithKline, Stevenage Hertfordshire SG1 2NY (GB); GUIDETTI, Rossella, Care of GlaxoSmithKline, Stevenage Hertfordshire SG1 2NY (GB); LOVEGROVE, Victoria Lucy Helen, Care of GlaxoSmithKline, Stevenage Hertfordshire SG1 2NY (GB); MORDAUNT, Jacqueline Elizabeth, GlaxoSmithKline, Stevenage Hertfordshire SG1 2NY (GB); SHAH, Pritom, GlaxoSmithKline, Stevenage Hertfordshire SG1 2NY (GB); SLATER, Martin John, GlaxoSmithKline, Stevenage Hertfordshire SG1 2NY (GB)
(74) Representative: McKinnell, Denise
(86) International application number: PCT/GB2005/001071
(87) International publication number: WO 2005/092863

(56) References cited:
- WO-A-03/037893
- WO-A-03/037895
- WO-A-20/04076415
- WO-A-20/04096210

## Description

### FIELD OF THE INVENTION

The present invention relates to novel 4-carboxy pyrazole derivatives useful as anti-viral agents. Specifically, the present invention involves novel inhibitors of Hepatitis C Virus (HCV) replication.

### BACKGROUND OF THE INVENTION

Infection with HCV is a major cause of human liver disease throughout the world. In the US, an estimated 4.5 million Americans are chronically infected with HCV. Although only 30% of acute infections are symptomatic, greater than 85% of infected individuals develop chronic, persistent infection. Treatment costs for HCV infection have been estimated at $5.46 billion for the US in 1997. Worldwide over 200 million people are estimated to be infected chronically. HCV infection is responsible for 40-60% of all chronic liver disease and 30% of all liver transplants. Chronic HCV infection accounts for 30% of all cirrhosis, end-stage liver disease, and liver cancer in the U.S. The CDC estimates that the number of deaths due to HCV will minimally increase to 38,000/year by the year 2010.

Due to the high degree of variability in the viral surface antigens, existence of multiple viral genotypes, and demonstrated specificity of immunity, the development of a successful vaccine in the near future is unlikely. Alpha-interferon (alone or in combination with ribavirin) has been widely used since its approval for treatment of chronic HCV infection. However, adverse side effects are commonly associated with this treatment: flu-like symptoms, leukopenia, thrombocytopenia, depression from interferon, as well as anemia induced by ribavirin (Lindsay, K.L. (1997) Hepatology 26 (suppl 1): 71S-77S). This therapy remains less effective against infections caused by HCV genotype 1 (which constitutes ~75% of all HCV infections in the developed markets) compared to infections caused by the other 5 major HCV genotypes. Unfortunately, only ∼50-80% of the patients respond to this treatment (measured by a reduction in serum HCV RNA levels and normalization of liver enzymes) and, of responders, 50-70% relapse within 6 months of cessation of treatment. Recently, with the introduction of pegylated interferon (Peg-IFN), both initial and sustained response rates have improved substantially, and combination treatment of Peg-IFN with ribavirin constitutes the gold standard for therapy. However, the side effects associated with combination therapy and the impaired response in patients with genotype 1 present opportunities for improvement in the management of this disease.

First identified by molecular cloning in 1989 (Choo, Q-L et al (1989) Science 244:359-362), HCV is now widely accepted as the most common causative agent of post-transfusion non A, non-B hepatitis (NANBH) (Kuo, G et al (1989) Science 244:362-364). Due to its genome structure and sequence homology, this virus was assigned as a new genus in the *Flaviviridae* family. Like the other members of the *Flaviviridae,* such as flaviviruses (e.g. yellow fever virus and Dengue virus types 1-4) and pestiviruses (e.g. bovine viral diarrhea virus, border disease virus, and classic swine fever virus) (Choo, Q-L et al (1989) Science 244:359-362; Miller, R.H. and R.H. Purcell (1990) Proc. Natl. Acad. Sci. USA 87:2057-2061), HCV is an enveloped virus containing a single strand RNA molecule of positive polarity. The HCV genome is approximately 9.6 kilobases (kb) with a long, highly conserved, noncapped 5' nontranslated region (NTR) of approximately 340 bases which functions as an internal ribosome entry site (IRES) (Wang CY et al 'An RNA pseudoknot is an essential structural element of the internal ribosome entry site located within the hepatitis C virus 5' noncoding region' RNA- A Publication of the RNA Society. 1(5): 526-537, 1995 Jul.). This element is followed by a region which encodes a single long open reading frame (ORF) encoding a polypeptide of ∼3000 amino acids comprising both the structural and nonstructural viral proteins.

Upon entry into the cytoplasm of the cell, this RNA is directly translated into a polypeptide of ~3000 amino acids comprising both the structural and nonstructural viral proteins. This large polypeptide is subsequently processed into the individual structural and nonstructural proteins by a combination of host and virally-encoded proteinases (Rice, C.M. (1996) in B.N. Fields, D.M.Knipe and P.M. Howley (eds) Virology 2nd Edition, p931-960; Raven Press, N.Y.). Following the termination codon at the end of the long ORF, there is a 3' NTR which roughly consists of three regions: an - 40 base region which is poorly conserved among various genotypes, a variable length poly(U)/polypyrimidine tract, and a highly conserved 98 base element also called the "3' X-tail" (Kolykhalov, A. et al (1996) J. Virology 70:3363-3371; Tanaka, T. et al (1995) Biochem Biophys. Res. Commun. 215:744-749; Tanaka, T. et al (1996) J. Virology 70:3307-3312; Yamada, N. et al (1996) Virology 223:255-261). The 3' NTR is predicted to form a stable secondary structure which is essential for HCV growth in chimps and is believed to function in the initiation and regulation of viral RNA replication.

The NS5B protein (591 amino acids, 65 kDa) of HCV (Behrens, S.E. et al (1996) EMBO J. 15:12-22), encodes an RNA-dependent RNA polymerase (RdRp) activity and contains canonical motifs present in other RNA viral polymerases. The NS5B protein is fairly well conserved both intra-typically (-95-98% amino acid (aa) identity across 1b isolates) and inter-typically (~85% aa identity between genotype 1a and 1b isolates). The essentiality of the HCV NS5B RdRp activity for the generation of infectious progeny virions has been formally proven in chimpanzees (A. A. Kolykhalov et al.. (2000) Journal of Virology, 74(4): 2046-2051). Thus, inhibition of NS5B RdRp activity (inhibition of RNA replication) is predicted to be useful to treat HCV infection.

Based on the foregoing, there exists a significant need to identify synthetic or biological compounds for their ability to inhibit HCV.

WO0102385 discloses certain 4-pyrazolyl quinoline derivatives having plant fungicidal activity.

WO0066562 discloses certain pyrazole derivatives having cyclooxygenase-2 inhibiting activity.

WO9600218 discloses certain pyrazole derivatives having PDE IV inhibiting activity.

### SUMMARY OF THE INVENTION

The present invention involves novel 4-carboxy pyrazole compounds represented hereinbelow, pharmaceutical compositions comprising such compounds and use of the compounds in treating viral infection, especially HCV infection.

### DETAILED DESCRIPTION OF THE INVENTION

The present invention provides at least one chemical entity chosen from compounds of Formula (I) : 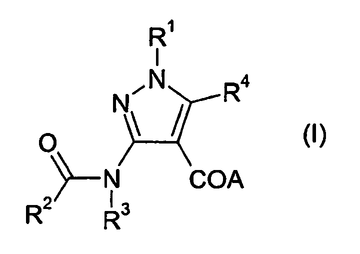 wherein:
A represents hydroxy;
R¹ represents aryl, heteroaryl bonded through a ring carbon atom, or heterocyclyl bonded through a ring carbon atom, each of which may be optionally substituted by one or more substituents selected from -C₁-₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, -CF₃, -OCF₃, NR^{E}SO₂R^{D}, phenyl and heterocyclyl, wherein the -C₁₋₆alkyl substituent itself may be optionally substituted by one or more substituents selected from -C₅₋₉cycloalkyl, halo, -NR^{B}R^{C}, -C(O)NR^{B}R^{C}, -NR^{E}C(O)R^{D}, -SR^{A}, -SO₂R^{D}, -OR^{A}, oxo, phenyl, heteroaryl or heterocyclyl; or R¹ represents -C₁₋₆alkyl or -C₅₋₉cycloalkyl;
R² represents phenyl substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, and heterocyclyl; or R² represents -(CH₂)ₙC₅₋₇cycloalkyl optionally substituted on the cycloalkyl by one or more substitutents selected from -C₁₋₆alkyl, =CH(CH₂)ₜH, -OR^{A}, -SR^{A}, -C(O)NR^{B}R^{C}, -C(O)R^{D}, -CO₂H, -CO₂R^{D},-NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo, and heterocyclyl, or wherein two substituents may together form a C₁₋₂alkylene bridge substituent;
t represents 0, 1, 2, 3 or 4;
n represents 0 or 1;
R³ represents heterocyclyl or heteroaryl; or phenyl optionally substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, and heterocyclyl; or R³ represents -C₁₋₆alkyl optionally substituted by one or more substituents selected from -C₁₋₆alkyl, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo, phenyl, heteroaryl and heterocyclyl;
R⁴ represents hydrogen;
R^{A} represents hydrogen, -C₁₋₆alkyl, arylalkyl, heteroarylalkyl, aryl, heterocyclyl or heteroaryl;
R^{B} and R^{C} independently represent hydrogen, -C₁₋₆alkyl, aryl, heterocyclyl or heteroaryl; or R^{B} and R^{C} together with the nitrogen atom to which they are attached form a 5 or 6 membered saturated cyclic group;
R^{D} is selected from the group consisting of -C₁₋₆alkyl, aryl, heterocyclyl, heteroaryl, arylalkyl, and heteroarylalkyl;
R^{E} represents hydrogen or -C₁₋₆alkyl;
R^{F} and R^{G} are independently selected from the group consisting of hydrogen, -C₁₋₆alkyl, aryl, heteroaryl, arylalkyl, and heteroarylalkyl; or R^{F} and R^{G} together with the nitrogen atom to which they are attached form a 5 or 6 membered saturated cyclic group;
and salts, solvates and esters thereof.

There is provided as a further aspect of the present invention at least one chemical entity chosen from compounds of Formula (I) and physiologically acceptable salts, solvates and esters thereof for use in human or veterinary medical therapy, particularly in the treatment or prophylaxis of viral infection, particularly HCV infection.

It will be appreciated that reference herein to therapy and/or treatment includes, but is not limited to prevention, retardation, prophylaxis, therapy and cure of the disease. It will further be appreciated that references herein to treatment or prophylaxis of HCV infection includes treatment or prophylaxis of HCV-associated disease such as liver fibrosis, cirrhosis and hepatocellular carcinoma.

According to another aspect of the invention, there is provided the use of at least one chemical entity chosen from compounds of Formula (I) and physiologically acceptable salts, solvates and esters thereof in the manufacture of a medicament for the treatment and/or prophylaxis of viral infection, particularly HCV infection.

It will be appreciated that the chemical entities of the present invention may contain one or more asymmetric carbon atoms and may exist in racemic, diastereoisomeric, and optically active forms. All of these racemic compounds, enantiomers and diastereoisomers are contemplated to be within the scope of the present invention.

In one aspect, the present invention provides at least one chemical entity chosen from compounds of Formula (Ia) : 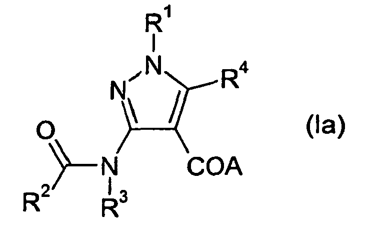 wherein:
A represents hydroxy;
R¹ represents aryl, heteroaryl bonded through a ring carbon atom, heterocyclyl bonded through a ring carbon atom, each optionally substituted by Q-R⁵; or R¹ represents -CH=CHR⁶, -C₁₋₆alkyl, or -C₅₋₉cycloalkyl;
R² represents phenyl substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, oxo, and heterocyclyl; or R² represents -(CH₂)ₙC₅₋₇cycloalkyl optionally substituted on the cycloalkyl by one or more substitutents selected from -C₁₋₆alkyl, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo, and heterocyclyl, or wherein two substituents may together form a C₁₋₂alkylene bridge substituent;
n represents 0 or 1;
R³ represents heterocyclyl or heteroaryl; or phenyl optionally substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, oxo, and heterocyclyl; or R³ represents -C₁₋₆alkyl optionally substituted by one or more substituents selected from -C₁₋₆alkyl, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo, phenyl, heteroaryl and heterocyclyl;
R⁴ represents hydrogen;
R⁵ represents aryl, heteroaryl or heterocyclyl;
R⁶ represents methylpropyl, *n*-butyl or cyclohexyl, or phenyl optionally substituted by fluoro;
R⁷ represents C₁₋₃alkyl, aryl, heteroaryl or heterocyclyl;
Q represents -C₂₋₄alkenylene-, -C₁₋₄alkylene-, -O-C₁₋₄alkylene- or -C₁₋₄alkylene-O- wherein each -C₂₋₄alkenyl- and -C₁₋₄alkylene- may be optionally substituted by -S(O)ₘR⁷;
m represents 0, 1 or 2;
R^{A} represents hydrogen, -C₁₋₆alkyl, arylalkyl, heteroarylalkyl, aryl or heteroaryl;
R^{B} and R^{C} independently represent hydrogen, -C₁₋₆alkyl, aryl or heteroaryl; or R^{B} and R^{C} together with the nitrogen atom to which they are attached form a 5 or 6 membered saturated cyclic group;
R^{D} is selected from the group consisting of -C₁₋₆alkyl, aryl, heteroaryl, arylalkyl, and heteroarylalkyl;
R^{E} represents hydrogen or -C₁₋₆alkyl;
R^{F} and R^{G} are independently selected from the group consisting of hydrogen, -C₁₋₆alkyl, aryl, heteroaryl, arylalkyl, and heteroarylalkyl; or R^{F} and R^{G} together with the nitrogen atom to which they are attached form a 5 or 6 membered saturated cyclic group;
and salts, solvates and esters thereof.

In a further aspect, the present invention provides at least one chemical entity chosen from compounds of Formula (Ib): 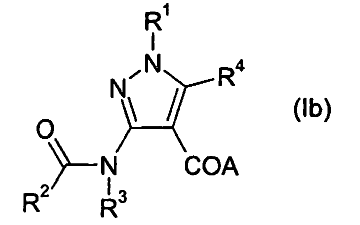 wherein:
A represents hydroxy;
R¹ represents aryl, heteroaryl bonded through a ring carbon atom, heterocyclyl bonded through a ring carbon atom, or C₁₋₆alkyl;
R² represents phenyl substituted by one or more substituents selected from C₁₋₆alkyl, halo, OR^{A}, SR^{A}, C(O)NR^{B}R^{C}, C(O)R^{D}, CO₂H, CO₂R^{D}, NR^{B}R^{C}, NR^{E}C(O)R^{D}, NR^{E}CO₂R^{D}, NR^{E}C(O)NR^{F}R^{G}, SO₂NR^{F}R^{G}, SO₂R^{D}, nitro, cyano, oxo, and heterocyclyl; or R² represents -(CH₂)ₙC₅₋₇cycloalkyl optionally substituted on the cycloalkyl by one or more substitutents selected from C₁₋₆alkyl, OR^{A}, SR^{A}, C(O)NR^{B}R^{C}, C(O)R^{D}, CO₂H, CO₂R^{D}, NR^{B}R^{C}, NR^{E}C(O)R^{D}, NR^{E}CO₂R^{D}, NR^{E}C(O)NR^{F}R^{G}, SO₂NR^{F}R^{G}, SO₂R^{D}, fluoro, nitro, cyano, oxo, and heterocyclyl, or wherein two substituents may together form a C₁₋₂alkylene bridge;
n represents 0 or 1;
R³ represents heterocyclyl; or phenyl optionally substituted by one or more substituents selected from C₁₋₆alkyl, halo, OR^{A}, SR^{A}, C(O)NR^{B}R^{C}, C(O)R^{D}, CO₂H, CO₂R^{D}, NR^{B}R^{C}, NR^{E}C(O)R^{D}, NR^{E}CO₂R^{D}, NR^{E}C(O)NR^{F}R^{G}, SO₂NR^{F}R^{G}. SO₂R^{D}, nitro, cyano, oxo, and heterocyclyl; or R³ represents C₁₋₆alkyl optionally substituted by one or more substituents selected from C₁₋₆alkyl, OR^{A}, SR^{A}, C(O)NR^{B}R^{C}, C(O)R^{D}, CO₂H, CO₂R^{D}, NR^{B}R^{C}, NR^{E}C(O)R^{D}, NR^{E}CO₂R^{D}, NR^{E}C(O)NR^{F}R^{G}, SO₂NR^{F}R^{G}, SO₂R^{D}, fluoro, nitro, cyano, oxo, phenyl, heteroaryl and heterocyclyl;
R⁴ represents hydrogen;
R^{A} represents hydrogen, C₁₋₆alkyl, arylalkyl, heteroarylalkyl, aryl or heteroaryl;
R^{B} and R^{C} independently represent hydrogen, C₁₋₆alkyl, aryl or heteroaryl; or R^{B} and R^{C} together with the nitrogen atom to which they are attached form a 5 or 6 membered saturated cyclic group;
R^{D} is selected from the group consisting of C₁₋₆alkyl, aryl, heteroaryl, arylalkyl, and heteroarylalkyl;
R^{E} represents hydrogen or C₁₋₆alkyl;
R^{F} and R^{G} are independently selected from the group consisting of hydrogen, C₁₋₆alkyl, aryl, heteroaryl, arylalkyl, and heteroarylalkyl; or R^{F} and R^{G} together with the nitrogen atom to which they are attached form a 5 or 6 membered saturated cyclic group;
and salts, solvates and esters thereof.

In a further aspect, the present invention provides at least one chemical entity chosen from compounds of Formula (Ic) : 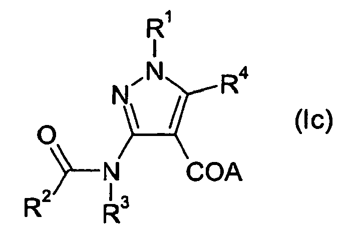 wherein:
A represents hydroxy;
R¹ represents aryl, heteroaryl bonded through a ring carbon atom, heterocyclyl bonded through a ring carbon atom, each optionally substituted by Q-R⁵; or R¹ represents -C₅₋₇cycloalkyl or -CH=CHR⁶;
R² represents -(CH₂)ₙC₅₋₇cycloalkyl optionally substituted on the cycloalkyl by one or more substitutents selected from -C₁₋₆alkyl, -OR^{A}, -SR^{A}, -C(O)NR^{B}R^{C}, -C(O)R^{D}, -CO₂H, -CO₂R^{D}, -NR^{B}R^{C}, -SO₂R^{D}, fluoro, cyano, and oxo; or R² represents phenyl optionally substituted by one or more substituents selected from -C₁₋₆alkyl and halo;
n represents 0 or 1;
R³ represents heterocyclyl; or phenyl optionally substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -SO₂R^{D}, nitro, cyano, oxo, and heterocyclyl; or R³ represents -C₁₋₆alkyl optionally substituted by one or more substituents selected from -C₁₋₆alkyl, -OR^{A}, -SR^{A}, -C(O)NR^{B}R^{C}, -C(O)R^{D}, -CO₂H, -CO₂R^{D} , -NR^{B}R^{C}, -SO₂R^{D}, fluoro, cyano, oxo, phenyl, heteroaryl and heterocyclyl;
R⁴ represents hydrogen;
R⁵ represents aryl, heteroaryl or heterocyclyl;
R⁶ represents 2-methylpropyl, n-butyl or cyclohexyl, or phenyl optionally substituted by fluoro;
R⁷ represents C₁₋₃alkyl, aryl, heteroaryl or heterocyclyl;
Q represents -C₂₋₄alkenylene-, -C₁₋₄alkylene-, -O-C₁₋₄alkylene- or -C₁₋₄alkylene-O- wherein each -C₂₋₄alkenyl- and -C₁₋₄alkylene- may be optionally substituted by -S(O)ₘR⁷;
m represents 0, 1 or 2;
R^{A} represents hydrogen, -C₁₋₆alkyl, arylalkyl, heteroarylalkyl, aryl or heteroaryl;
R^{B} and R^{C} independently represent hydrogen, -C₁₋₆alkyl, aryl or heteroaryl; or R^{B} and R^{C} together with the nitrogen atom to which they are attached form a 5 or 6 membered saturated cyclic group;
R^{D} is selected from the group consisting of C₁₋₆alkyl, aryl, heteroaryl, arylalkyl, and heteroarylalkyl;
and salts, solvates and esters thereof.

In a further aspect, the present invention provides at least one chemical entity chosen from compounds of Formula (Id) : 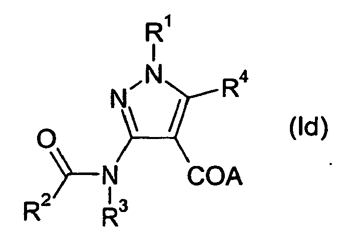 wherein:
A represents hydroxy;
R¹ represents aryl, heteroaryl bonded through a ring carbon atom, or heterocyclyl bonded through a ring carbon atom;
R² represents -(CH₂)ₙC₅₋₇cycloalkyl optionally substituted on the cycloalkyl by one or more substitutents selected from C₁₋₆alkyl, OR^{A}, SR^{A}, C(O)NR^{B}R^{C}, C(O)R^{D}, CO₂H, CO₂R^{D}, NR^{B}R^{C}, SO₂R^{D}, fluoro, cyano, and oxo;
n represents 0 or 1;
R³ represents heterocyclyl; or phenyl optionally substituted by one or more substituents selected from C₁₋₆alkyl, halo, OR^{A}, SR^{A}, C(O)NR^{B}R^{C}, C(O)R^{D}, CO₂H, CO₂R^{D}, NR^{B}R^{C}, SO₂R^{D}, nitro, cyano, oxo, and heterocyclyl; or R³ represents C₁₋₆alkyl optionally substituted by one or more substituents selected from C₁₋₆alkyl, OR^{A}, SR^{A}, C(O)NR^{B}R^{C}, C(O)R^{D}, CO₂H, CO₂R^{D}, NR^{B}R^{C}, SO₂R^{D}, fluoro, cyano, oxo, phenyl, heteroaryl and heterocyclyl;
R⁴ represents hydrogen;
R^{A} represents hydrogen, C₁₋₆alkyl, arylalkyl, heteroarylalkyl, aryl or heteroaryl;
R^{B} and R^{C} independently represent hydrogen, C₁₋₆alkyl, aryl or heteroaryl; or R^{B} and R^{C} together with the nitrogen atom to which they are attached form a 5 or 6 membered saturated cyclic group;
R^{D} is selected from the group consisting of C₁₋₆alkyl, aryl, heteroaryl, arylalkyl, and heteroarylalkyl;
and salts, solvates and esters thereof.

It will be appreciated that the following aspects may apply, where appropriate to compounds of Formula (I), (Ia), (Ib), (Ic) and (Id).

In one aspect, R¹ represents phenyl, heteroaryl bonded through a ring carbon atom, or heterocyclyl bonded through a ring carbon atom, each of which may be optionally substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -SO₂R^{D}, cyano, -CF₃, -OCF₃, NR^{E}SO₂R^{D}, phenyl and heterocyclyl, wherein the -C₁₋₆alkyl substituent itself may be optionally substituted by one or more substituents selected from -C₅₋₉cycloalkyl, halo, -NR^{B}R^{C}, -C(O)NR^{B}R^{C}, -NR^{E}C(O)R^{D}, -SR^{A}, -SO₂R^{D}, OR^{A}, phenyl, heteroaryl or heterocyclyl; or R¹ represents -C₁₋₆alkyl or -C₅₋₉cycloalkyl (in another aspect optionally substituted cyclohexenyl).

In a further aspect, R¹ represents a substituent selected from phenyl, 3-hydroxyphenyl, 4-hydroxyphenyl, 3-(trifluoromethyl)phenyl, 4-(trifluoromethyl)phenyl, 4-acetylphenyl, 4-(acetylamino)phenyl, 4-aminophenyl, 4-(dimethylamino)phenyl, 3-methoxyphenyl, 4-methoxyphenyl, 3-(trifluoromethoxy)phenyl, 4-(trifluoromethoxy)phenyl, 3-cyanophenyl, 4-cyanophenyl, 4-[(dimethylamino)carbonyl]phenyl, 3,5-dimethylphenyl, 3-chloro-5-fluorophenyl, 3-chloro-4-benzyloxyphenyl, 3,5-bis-(trifluoromethyl)phenyl, 1,3-benzodioxol-5-yl, 2,3-dihydro-1-benzofuran-5-yl, 2,3-dihydro-1,4-benzodioxin-6-yl, 1H-indol-5-yl, indol-6-yl, benzofuran-6-yl, 3,4,5-trifluorophenyl, 3-chlorophenyl, 4-chlorophenyl, 2-fluorophenyl, 3-fluorophenyl, 4-fluorophenyl, 4-methylsulfonylphenyl, 4-(phenylthiomethyl)phenyl, 4-[(phenylsulfonyl)amino]phenyl, 3-methylphenyl, 4-methylphenyl, 4-chloro-3-fluoro-phenyl, 4-chloro-3-methylphenyl, 3-amino-4-methylphenyl, 3-fluoro-4-methylphenyl, 3,4-di-fluorophenyl, 4-biphenylyl, 4-(phenylsulfonylmethyl)phenyl, 4-[(phenoxy)methyl]phenyl, 4-(phenoxy)phenyl, 4-[(1,3-thiazol-4-ylmethyl)oxy]phenyl, 4-[(E/Z)-2-phenylethenyl]phenyl, 4-(2-phenylethyl)phenyl, 4-[(1,3-thiazol-4-yl)ethyl]phenyl, 4-[(1,3-thiazol-2-yl)ethyl]phenyl, 4-[2-(1 H-pyrazol-3-yl)ethyl]phenyl, (E)-2-*tert*-butylethenyl, (E)-hexen-1-yl, (E)-2-cyclohexylethenyl, cyclohexyl, 4-(2-cyclohexylethyl)phenyl, cyclohexen-1-yl, cyclohepten-1-yl, 4-methyl-1-cyclohexen-1-yl, 4-trifluoromethylcyclohexen-1-yl, 4-benzyloxycyclohexen-1-yl, (4,4-dimethyl)cydohexen-1-yl, (E)-4-methyl-1-penten-1-yl, (E)-2-phenylethenyl, 4-[(E)-2-(cyclohexyl)ethenyl]phenyl, 4-[(E)-2-phenylethenyl]phenyl, 4-[(Z)-2-phenylethenyl]phenyl, 4-[(E)-phenyl-2-methylethenyl]phenyl, 4-[(E)-2-(3-pyrazolyl)-ethenyl]phenyl, 4-[(Z)-2-(3-pyrazolyl)ethenyl]phenyl, 4-[(E)-(pyridine-4-yl)ethenyl]phenyl, 4-[2-(pyridin-2-yl)ethenyl]-phenyl, 4-[2-(pyridin-2-yl)ethyl]phenyl, 4-[(E)-2-(tetrahydro-2H-pyran-4-yl)ethenyl]phenyl, 4-[(E/Z)-(1,3-thiazol-2-yl)ethenyl]phenyl, 4-[(E)-(1,3-thiazol-4-yl)ethenyl]phenyl, 4-[(Z)-(1,3-thiazol-4-yl)ethenyl]phenyl, 4-[(E)-(2-methyl-1,3-thiazol-4-yl)etheny]phenyl, 4-[(E)-(furan-2-yl)ethenyl]phenyl, (E)-2-(4-fluorophenyl)ethenyl, 4-ethenylphenyl, 4-(hydroxymethyl)phenyl, 4-ethylphenyl, 4-(1-methylethyl)phenyl, 3-thienyl, 5-acetyl-2-thienyl, 5-chloro-2-thienyl, 5-methyl-2-thienyl, 5-phenyl-2-thienyl, 1-(methylsulfonyl)-1,2,3,6-tetrahydro-4-pyridinyl, 4-[(phenylamino)carbonyl]phenyl, 4-{[(4-fluorophenyl)amino]carbonyl}phenyl, 4-[(phenylcarbonyl)amino]phenyl, 4-[(3-methylphenylcarbonyl)amino]phenyl, 4-[(3-chlorophenylcarbonyl)amino]phenyl, 4-[(4-fluorophenylcarbonyl)amino]phenyl, and 4-[(cyclohexylcarbonyl)-amino]phenyl.

In a further aspect, R¹ represents a substituent selected from phenyl, 3-hydroxyphenyl, 4-hydroxyphenyl, 3-(trifluoromethyl)phenyl, 4-(trifluoromethyl)phenyl, 4-acetylphenyl, 4-(acetylamino)phenyl, 4-aminophenyl, 4-(dimethylamino)phenyl, 3-methoxyphenyl, 4-methoxyphenyl, 3-(trifluoromethoxy)phenyl, 4-(trifluoromethoxy)phenyl, 3-cyanophenyl, 4-cyanophenyl, 4-[(dimethylamino)carbonyl]phenyl, 3,5-dimethylphenyl, 3-chloro-5-fluorophenyl, 3,5-bis-(trifluoromethyl)phenyl, 1,3-benzodioxol-5-yl, 2,3-dihydro-1-benzofuran-5-yl, 2,3-dihydro-1,4-benzodioxan-6-yl, 1 H-indol-5-yl, 3,4,5-trifluorophenyl, 3-chlorophenyl, 4-chlorophenyl, 2-fluorophenyl, 3-fluorophenyl, 4-methylsulfonylphenyl, 3-methylphenyl, 4-methylphenyl, 3-fluoro-4-chlorophenyl, 3-methyl-4-chlorophenyl, 3-amino-4-methylphenyl, 3-fluoro-4-methylphenyl, 3,4-di-fluorophenyl, 4-biphenylyl, 4-[(phenylmethyl)oxy]phenyl, 4-[(E/Z)-2-phenylethenyl]phenyl, 4-(2-phenylethyl)phenyl, (E)-hexen-1-yl, (E)-2-cyclohexylethenyl, cyclohexyl, cyclohexen-1-yl, 4-methyl-1-cyclohexen-1-yl, (E)-4-methyl-1-penten-1-yl, (E)-2-phenylethenyl, (E)-2-(4-fluorophenyl)ethenyl, 4-ethenylphenyl, 4-(hydroxymethyl)phenyl, 4-ethylphenyl, 4-isopropylphenyl, 3-thienyl, 5-acetyl-2-thienyl, 5-chloro-2-thienyl, 5-methyl-2-thienyl, 5-phenyl-2-thienyl, and 1-(methylsulfonyl)-1,2,3,6-tetrahydro-4-pyridinyl.

In a further aspect, R¹ represents phenyl optionally substituted by one or more substituents selected from -C₁₋₆alkyl, halo, -OR^{A}, -C(O)NR^{B}R^{C}, -C(O)R^{D}, -NR^{B}R^{C}, -NR^{E}C(O)R^{D}, -SO₂R^{D}, cyano, -CF₃, -OCF₃, NR^{E}SO₂R^{D}, phenyl and heterocyclyl, wherein the -C₁₋₆alkyl substituent itself may be optionally substituted by -C(O)NR^{B}R^{C}, -NR^{E}C(O)R^{D}, -SO₂R^{D}, OR^{A}, phenyl, heteroaryl or heterocyclyl; or R¹ represents -C₅₋₉cycloalkyl.

In another aspect, R¹ is other than optionally substituted quinolin-4-yl.

In another aspect, R¹ is other than 4-(S(O)₂R^{D})-phenyl or 4-(SR^{A})-phenyl each of which is further substituted on the phenyl ring by at least one other substituent.

In another aspect, R¹ is other than a group which comprises phenyl substituted in each of the 3 and 4 positions by a substituent, which may be the same of different, selected from -OR^{A} and -SR^{A}.

In one aspect, R² represents optionally substituted C₅₋₇cycloalkyl, especially C₅₋₇cycloalkyl substituted by C₁₋₄alkyl. In a further aspect, R² represents *trans*-4-methylcyclohexyl, cyclohexylmethyl, 4-methylphenyl, 4-bromo-2-chlorophenyl, 2-hydroxy-*trans*-4-methylcyclohexyl, 4-methylidenecyclohexyl, In another aspect, R² represents optionally substituted -C₅₋₆cycloalkyl. In yet another aspect R² represents -C₆cycloalkyl substituted by -C₁₋₄alkyl, especially *trans*-4-methylcyclohexyl.

In one aspect, R³ represents unsubstituted -C₁₋₄alkyl, phenyl, [2-(dimethylamino)-2-oxoethyl], 4-piperidinyl, {1-[(methyloxy)carbonyl]-4-piperidinyl}, [1-(methylsulfonyl)-4-piperidinyl], 1-methyl-4-piperidinyl, {1-[(ethylamino)carbonyl]-4-piperidinyl, {1-[(tert-butyloxy)carbonyl]-4-piperidinyl}, 2-pyrazinylmethyl, phenylmethyl, cyclopentyl, tetrahydro-2H-pyran-4-yl, tetrahydro-3-furanyl, [1-acetyl-4-piperidinyl]. In a further aspect, R³ represents unsubstituted -C₁₋₄alkyl, phenyl, [2-(dimethylamino)-2-oxoethyl], {1-[(methyloxy)carbonyl]-4-piperidinyl}, [1-(methylsulfonyl)-4-piperidinyl], 1-methyl-4-piperidinyl, {1-[(ethylamino)carbonyl]-4-piperidinyl, 2-pyrazinylmethyl, phenylmethyl, cyclopentyl, tetrahydro-2H-pyran-4-yl, tetrahydro-3-furanyl, [1-acetyl-4-piperidinyl]. In another aspect R³ represents unsubstituted C₁₋₄alkyl. In a further aspect, R³ represents 1-methylethyl, tetrahydro-2H-pyran-4-yl, tetrahydro-3-furanyl or [1-(methylsulfonyl)-4-piperidinyl].

In one aspect, R⁴ represents hydrogen.

It is to be understood that the present invention covers all combinations of aspects, suitable, convenient and preferred groups described herein.

As used herein, "acetyl" refers to -C(O)CH₃.

As used herein, "acetylamino" refers to -N(H)C(O)CH₃.

As used herein unless otherwise specified, "alkyl" refers to an optionally substituted hydrocarbon group. The alkyl hydrocarbon group may be linear, branched or cyclic, saturated or unsaturated. Where the alkyl group is linear or branched, examples of such groups include methyl, ethyl, n-propyl, 1-methylethyl (isopropyl), n-butyl, isobutyl, sec-butyl, tert-butyl, n-pentyl, isopentyl, neopentyl or hexyl and the like. Where the alkyl hydrocarbon group is unsaturated, it will be understood that there will be a minimum of 2 carbon atoms in the group, for example an alkenyl or alkynyl group. Where the alkyl hydrocarbon group is cyclic, it will be understood that there will be a minimum of 3 carbon atoms in the group. In one aspect, alkyl moieties are -C₁₋₄alkyl. Unless otherwise stated, optional substituents include -C₁₋₆alkyl (unsubstituted), =CH(CH₂)ₜH, fluoro, -CF₃, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, oxo, aryl, heteroaryl and heterocyclyl.

As used herein, the term "alkenyl" refers to a linear or branched hydrocarbon group containing one or more carbon-carbon double bonds. In one aspect the alkenyl group has from 2 to 6 carbon atoms. Examples of such groups include ethenyl, propenyl, butenyl, pentenyl or hexenyl and the like.

As used herein, the term "alkynyl" refers to a linear or branched hydrocarbon group containing one or more carbon-carbon triple bonds. In one aspect the alkynyl group has from 2 to 6 carbon atoms. Examples of such groups include ethynyl, propynyl, butynyl, pentynyl or hexynyl and the like.

As used herein unless otherwise specified, "cycloalkyl" refers to an optionally substituted, cyclic hydrocarbon group. The hydrocarbon group may be saturated or unsaturated, monocyclic or bridged bicyclic. Where the cycloalkyl group is saturated, examples of such groups include cyclopropyl, cyclobutyl, cyclopentyl, cyclohexyl, cycloheptyl or cyclooctyl and the like. Where the cycloalkyl group is unsaturated, examples of such groups include cyclobutenyl, cyclopentenyl, cyclohexenyl, cycloheptenyl or cyclooctenyl and the like. In one aspect, the cycloalkyl group has from 5 to 7 carbon atoms. In one aspect, cycloalkyl moieties are cyclohexenyl, cyclopentenyl and cyclohexyl. Unless otherwise stated, the cycloalkyl group may be substituted by one or more optional substituents including -C₁₋₆alkyl (unsubstituted), =CH(CH₂)ₜH, fluoro, -CF₃, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, oxo, phenyl and heterocyclyl.

As used herein, the term " alkoxy" refers to an -O-alkyl group wherein alkyl is as defined herein. Examples of such groups include methoxy, ethoxy, propoxy, butoxy, pentoxy or hexoxy and the like.

As used herein, "aryl" refers to an optionally substituted aromatic group with at least one ring having a conjugated pi-electron system, containing up to two conjugated or fused ring systems. "Aryl" includes carbocyclic aryl and biaryl groups, all of which may be optionally substituted. In one aspect, "aryl" moieties contain 6-10 carbon atoms. In one aspect, "aryl" moieties are unsubstituted, monosubstituted, disubstituted or trisubstituted phenyl. In one aspect, unless otherwise stated, "aryl" substituents are selected from the group consisting of -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, heterocyclyl, -CF₃, -OCF₃ and phenyl.

As used herein, "arylalkyl" refers to an aryl group attched to the parent molecular moiety through an alkyl group.

As used herein, "carbonyl" refers to -C(O)-.

As used herein, "cyano" refers to -CN.

As used herein, "halogen" or "halo" refer to a fluorine, chlorine, bromine or iodine atom. References to "fluoro", "chloro", "bromo" or "iodo" should be construed accordingly.

As used herein, "heteroaryl" refers to an optionally substituted, 5, 6, 8, 9 or 10 membered, aromatic group comprising one to four heteroatoms selected from N, O and S, with at least one ring having a conjugated pi-electron system, containing up to two conjugated or fused ring systems. In one aspect, "heteroaryl" moieties are unsubstituted, monosubstituted, disubstituted or trisubstituted (where applicable) pyridyl, pyrazinyl, thiazolyl, thienyl, benzodioxolyl, benzofuranyl, benzodioxinyl and indolyl. In one aspect, unless otherwise stated, "heteroaryl" substituents are selected from the group consisting of -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, heterocyclyl, -CF₃ and phenyl.

As used herein, "heteroarylalkyl" refers to a heteroaryl group attched to the parent molecular moiety through an alkyl group.

As used herein, "heterocyclic" and "heterocyclyl" refer to an optionally substituted, 5 or 6 membered, saturated or partially saturated, cyclic group containing 1 or 2 heteroatoms selected from N, optionally substituted by hydrogen, -C₁₋₆alkyl, -C(O)R^{D}, -C(O)NR^{B}R^{C}, -C(O)OR⁴, -SO₂R^{D}, aryl or heteroaryl; O; and S, optionally substituted by one or two oxygen atoms. Ring carbon atoms may be optionally substituted by -C₁₋₆alkyl, -C(O)R^{D}, or -SO₂R^{D}. In one aspect, unless otherwise stated, "heterocyclic" moieties are unsubstituted or monosubstituted tetrahydro-2H-pyran-4-yl, piperidinyl and 1,2,3,6-tetrahydro-4-pyridinyl.

As used herein, "nitro" refers to -NO₂.

As used herein, "oxo" refers to =O.

As used herein, "Ac" refers to "acetyl", "Et" refers to "ethyl", "iPr" refers to "isopropyl", "Me" refers to "methyl", "OBn" refers to "benzyloxy", and "Ph" refers to "phenyl".

In one aspect, chemical entities useful in the present invention may be chosen from compounds of Formula (I) selected from the group consisting of:
3-[[(*trans*-4-Methylcyclohexyl)carbonyl](1-methylethyl)amino]-1-phenyl-1*H*-pyrazole-4-carboxylic acid;
3-[[(*trans*-4-Methylcyclohexyl)carbonyl](1-methylethyl)amino]-1-(4-methylphenyl)-1H-pyrazole-4-carboxylic acid;
1-(1-Cyclohexen-1-yl)-3-[[(*trans*-4-methylcyclohexyl)carbonyl](-methylethyl)amino]-1H-pyrazole-4-carboxylic acid;
1-(4-Chloro-3-methylphenyl)-3-[[(*trans*-4-methylcyclohexyl)carbonyl](1-methylethyl)amino]-1H-pyrazole-4-carboxylic acid;
1-(4-Fluorophenyl)-3-[[(*trans*-4-methylcyclohexyl)carbonyl](1-methylethyl)amino]-1H-pyrazole-4-carboxylic acid;
1-(6-Indolyl)-3-[[(*trans*-4-methylcyclohexyl)carbonyl](1-methylethyl)amino]-1H-pyrazole-4-carboxylic acid;
1-(4-Hydroxyphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methytethyl)amino]-1-[4-(trifluoromethyl)phenyl]-1H-pyrazole-4-carboxylic acid;
1-[4-(Acetylamino)phenyl]-3-[[(*trans*-4-methylcyclohexyl)carbonyl](1-methylethyl)amino]-1H-pyrazole-4-carboxylic acid;
1-(4-Biphenylyl)-3-[[(*trans*-4-methylcyclohexyl)carbonyl](1-methylethyl)amino]-1H-pyrazole-4-carboxylic acid;
1-[4-(Dimethylamino)phenyl]-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(methyloxy)phenyl]-1H-pyrazole-4-carboxylic acid;
1-(4-Acetylphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{4-[(trifluoromethyl)oxy]phenyl}-1H-pyrazole-4-carboxylic acid;
1-(4-Cyanophenyl)-3-[[(*trans*-4-methylcyclohexyl)carbonyl](-methylethyl)amino]-1H-pyrazole-4-carboxylic acid;
1-{4-[(Dimethylamino)carbonyl]phenyl}-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3-thienyl)-1H-pyrazole-4-carboxylic acid;
3-[[(*trans*-4-Methylcyclohexyl)carbonyl](1-methylethyl)amino]-1-[3-(trifluoromethyl)phenyl]-1H-pyrazole-4-carboxylic acid;
1-(3,5-Dimethylphenyl)-3-[[(*trans-*4-methylcyclohexyl)carbonyl](1-methylethyl)amino]-1H-pyrazole-4-carboxylic acid;
1-(3-Chloro-5-fluorophenyl)-3-[[(*trans*-4-methylcyclohexyl)carbonyl](1 -methylethyl)amino]-1H-pyrazole-4-carboxylic acid;
1-[3,5-Bis(trifluoromethyl)phenyl]-3-[[(*trans-*4-methylcyclohexyl)carbonyl](1-methylethyl)amino]-1H-pyrazole-4-carboxylic acid;
1-(1,3-Benzodioxol-5-yl)-3-[[(*trans-*4-methylcyclohexyl)carbonyl](1-methylethyl)amino]-1H-pyrazole-4-carboxylic acid;
1-(2,3-Dihydro-1-benzofuran-5-yl)3-[[(*trans*-4-methylcyclohexyl)carbonyl](1-methylethyl)amino]-1H-pyrazole-4-carboxylic acid;
1-(2,3-Dihydro-1-,4-benzodioxin-6-yl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3,4,5-trifluorophenyl)-1H-pyrazole-4-carboxylic acid;
1-(4-Chlorophenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methytethyl)amino]-1-[3-(methyloxy)phenyl]-1H-pyrazole-4-carboxylic acid;
3-[[(*trans*-4-Methylcyclohexyl)carbonyl](1-methylethyl)amino]-1-[4-(methylsulfonyl)phenyl]-1H-pyrazole-4-carboxylic acid;
1-(2-Fluorophenyl)-3-[[(*trans*-4-methylcyclohexyl)carbonyl](1-methylethyl)amino]-1H-pyrazole-4-carboxylic acid;
1-(3-Hydroxyphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3-methylphenyl)-1H-pyrazole-4-carboxylic acid;
1-(3-Fluorophenyl)-3-[[(*trans*-4-methylcyclohexyl)carbonyl](1-methylethyl)amino]-1H-pyrazole-4-carboxylic acid;
1-(4-Aminophenyl)-3-[[(*trans*-4-methylcyclohexyl)carbonyl](1-methylethyl)amino]-1H-pyrazole-4-carboxylic acid;
1-(3-Chlorophenyl)-3-[[(trans-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{3-[(trifluoromethyl)oxy]phenyl}-1H-pyrazole-4-carboxylic acid;
1-(4-Chloro-3-fluorophenyl)-3-[[(*trans*-4-methylcyclohexyl)carbonyl](1-methylethyl)amino]-1H-pyrazole-4-carboxylic acid;
1-(3-Amino-4-methylphenyl)-3-[[(*trans*-4-methylcyclohexyl)carbonyl](1-methylethyl)amino]-1H-pyrazole-4-carboxylic acid;
1-(3-Fluoro-4-methylphenyl)-3-[[(*trans*-4-methylcyclohexyl)carbonyl](1-methylethyl)amino]-1H-pyrazole-4-carboxylic acid;
1-(3,4-Difluorophenyl)-3-[[(*trans*-4-methylcyclohexyl)carbonyl](1-methylethyl)amino]-1H-pyrazole-4-carboxylic acid;
1-[(E)-1-Hexen-1-yl]-3-[[(*trans*-4-methylcyclohexyl)carbonyl](1-methylethyl)amino]-1H-pyrazole-4-carboxylic acid;
1-[(E)-2-Cyclohexylethenyl]-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[(E)-4-methyl-1-penten-1-yl]-1 H-pyrazole-4-carboxylic acid;
1-[(E)-2-(4-Fluorophenyl)ethenyl]-3-[[(*trans*-4-methylcyclohexyl)carbonyl](1-methylethyl)amino]-1H-pyrazole-4-carboxylic acid;
1-(4-Ethenylphenyl)-3-[[(*trans*-4-methylcyclohexyl)carbonyl](1-methylethyl)amino]-1H-pyrazole-4-carboxylic acid;
1-[4-(Hydroxymethyl)phenyl]-3-[[(*trans*-4-methylcyclohexyl)carbonyl](1-methylethyl)amino]-1H-pyrazole-4-carboxylic acid;
1-(4-Ethylphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(1-methylethyl)phenyl]-1H-pyrazole-4-carboxylic acid;
1-(5-Acetyl-2-thienyl)-3-[[(*trans*-4-methylcyclohexyl)carbonyl](1-methylethyl)amino]-1H-pyrazole-4-carboxylic acid;
1-(5-Chloro-2-thienyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(5-methyl-2-thienyl)-1H-pyrazole-4-carboxylic acid;
3-[[(*trans*-4-Methylcyclohexyl)carbonyl](1-methylethyl)amino]-1-(5-phenyl-2-thienyl)-1H-pyrazole-4-carboxylic acid;
1-((4-Methyl)cyclohexen-1-yl)-3-[[(*trans*-4-methylcyclohexyl)carbonyl](tetrahydro-2H-pyran-4-yl)amino]-1H-pyrazole-4-carboxylic acid;
1-(6-Benzofuranyl)-3-[[(*trans*-4-methylcyclohexyl)carbonyl](1-methylethyl)amino]-1H-pyrazole-4-carboxylic acid;
1-(Cyclohepten-1-yl)-3-[[(*trans*-4-methylcyclohexyl)carbonyl](tetrahydro-2H-pyran-4-yl)amino]-1H-pyrazole-4-carboxylic acid;
1-((4-Methyl)cyclohexen-1-yl)-3-[[(*trans*-4-methylcyclohexyl)carbonyl](1-(methylsuffonyl)-4-piperidinyl]amino]-1H-pyrazole-4-carboxylic acid;
1-((4,4-Dimethyl)cyclohexen-1-yl)-3-[[(*trans*-4-methylcyclohexyl)carbonyl](tetrahydro-2H-pyran-4-yl)amino]-1H-pyrazole-4-carboxylic acid;
1-(3-Chloro-4-benzyloxyphenyl)-3-[[(*trans*-4-methylcyclohexyl)carbonyl](1-methylethyl)amino]-1H-pyrazole-4-carboxylic acid;
1-(4-Benzyloxy-cyclohexen-1-yl)-3-[[(*trans*-4-methylcyclohexyl)carbonyl](1-methylethyl)amino]-1)-1H-pyrazole-4-carboxylic acid;
1-(4,4-Dimethyl)cyclohexen-1-yl)-3-{[(*trans*-4-methylcyclohexyl)carbonyl](1-methylethyl)amino}-1H-pyrazole-4-carboxylic acid;
3-[[(*trans*-4-Methylcyclohexyl)carbonyl](tetrahydro-2H-pyran-4-yl)amino]-1-{4-[(E)-2-phenylethenyl]phenyl}-1H-pyrazole-4-carboxylic acid;
3-[[(*trans*-4-Methylcyclohexyl)carbonyl](tetrahydro-2H-pyran-4-yl)amino]-1-{4-[(Z)-2-phenylethenyl]phenyl}-1H-pyrazole-4-carboxylic acid;
3-[[(*trans*-4-Methylcyclohexyl)carbonyl](1-methylethyl)amino]-1-{4[(Z)-2-(3-pyrazolyl)-ethenyl]phenyl}-1H-pyrazole-4-carboxylic acid;
3-[[(*trans*-4-Methylcyclohexyl)carbonyl](1-methylethyl)amino]-1-{4[(E)-2-(3-pyrazolyl)-ethenyl]phenyl}-1H-pyrazole-4-carboxylic acid;
3-[[(*trans*-4-Methylcyclohexyl)carbonyl](1-methylethyl)amino]-1-{4[(E)-2-(tetrahydro-2H-pyran-4-yl)-ethenyl]phenyl}-1H-pyrazole-4-carboxylic acid;
3-[[(*trans*-4-Methylcyclohexyl)carbonyl](tetrahydro-2H-pyran-4-yl)amino]-1-{4-[(E)-2-(4-thiazolyl)-ethenyl]phenyl}-1H-pyrazole-4-carboxylic acid;
3-[[(*trans*-4-Methylcyclohexyl)carbonyl](tetrahydro-2H-pyran-4-yl)amino]-1-{4-[(Z)-2-(4-thiazolyl)-ethenyl]phenyl}-1H-pyrazole-4-carboxylic acid;
1-((E)-2-*tert*-Butyl-ethenyl)-3-[[(*trans*-4-methylcyclohexyl)carbonyl)(1-methylethyl)amino]-1H-pyrazole-4-carboxylic acid;
1-((E)-2-Phenyl-ethenyl)-3-[[(*trans*-4-methylcyclohexyl)carbonyl)(1-methylethyl)amino]-1H-pyrazole-4-carboxylic acid;
1-(4-Methyl-1-cyclohexen-1-yl)-3-[[(*trans-*4-methylcyclohexyl)carbonyl](1-methylethyl)amino]-1H-pyrazole-4-carboxylic acid;
1-(3-Cyanophenyl)-3-[[(*trans*-4-methylcyclohexyl)carbonyl](1-methylethyl)amino]-1H-pyrazole-4-carboxylic acid;
3-{(1-Methylethyl)[(4-methylidenecyclohexyl)carbonyl]amino}-1-phenyl-1H-pyrazole-4-carboxylic acid;
1-(4-Trifluoromethyl-cyclohexen-1-yl)-3-[[(*trans*-4-methylcyclohexyl)carbonyl)(1-methylethyl)amino]-1H-pyrazole-4-carboxylic acid;
3-[[(*trans*-4-Methylcyclohexyl)carbonyl](1-methylethyl)amino]-1-{4-[(phenyloxy)methyl] phenyl}-1*H*-pyrazole-4-carboxylic acid;
1-[4-(Phenylsulfonylmethyl)phenyl]-3-[[(*trans*-4-methylcyclohexyl)carbonyl](1-methylethyl)amino]-1H-pyrazole-4-carboxylic acid;
1-[4-(Phenylthiomethyl)phenyl]-3-[[(*trans*-4-methylcyclohexyl)carbonyl](1-methylethyl)amino]-1H-pyrazole-4-carboxylic acid;
1-[4-(Phenoxy)phenyl]-3-[[(*trans*-4-methylcyclohexyl)carbonyl](1-methylethyl)amino]-1H-pyrazole-4-carboxylic acid;
1-[4-{(1,3-Thiazol-4-ylmethyl)oxy}phenyl]-3-[[(*trans*-4-methylcyclohexyl)carbonyl](1-methylethyl)amino]-1H-pyrazole-4-carboxylic acid;
1-[4-([E]-Phenylethenyl)phenyl]-3-[[(*trans*-4-methylcyclohexyl)carbonyl](1-methylethyl)amino]-1H-pyrazole-4-carboxylic acid;
1-[4-[Z]-Phenylethenyl))phenyl]-3-[[(*trans*-4-methylcyclohexyl)carbonyl](1-methylethyl)amino]-1H-pyrazole-4-carboxylic acid;
1-[4-([E,Z]-(1,3-Thiazol-2-yl)ethenyl)phenyl]-3-[[(*trans*-4-methylcyclohexyl)carbonyl](1-methylethyl)amino]-1H-pyrazole-4-carboxylic acid;
1-[4-([E]-Phenyl-2-methylethenyl)phenyl]-3-[[(*trans*-4-methylcyclohexyl)carbonyl](1-methylethyl)amino]-1H-pyrazole-4-carboxylic acid;
1-[4-[E]-(Pyridin-4-yl)ethenyl))phenyl]-3-[[(*trans*-4-methylcyclohexyl)carbonyl](1-methylethyl)amino]-1 H-pyrazole-4-carboxylic acid;
1-[4-([E]-(1,3-Thiazol-4-yl)ethenyl)phenyl]-3-[[(*trans*-4-methylcyclohexyl)carbonyl](1-methylethyl)amino]-1H-pyrazole-4-carboxylic acid;
1-[4-([E]-(Furan-2-yl)ethenyl))phenyl]-3-[[(*trans*-4-methylcyclohexyl)carbonyl(1-methylethyl)amino]-1H-pyrazole-4-carboxylic acid;
1-[4-([E]-(2-Methyl-1,3-thiazol-4-yl)ethenyl)phenyl]-3-[[(*trans*-4-methylcyclohexyl)carbonyl](1-methylethyl)amino]-1H-pyrazole-4-carboxylic acid;
3-[(Cyclohexylacetyl)(1-methylethyl)amino]-1-phenyl-1H-pyrazole-4-carboxylic acid;
3-{(1-Methylethyl)[(4-methylphenyl)carbonyl]amino}-1-phenyl-1H-pyrazole-4-carboxylic acid;
3-[[(4-Bromo-2-chlorophenyl)carbonyl](1-methylethyl)amino]-1-phenyl-1H-pyrazole-4-carboxylic acid;
3-[[(*trans*-4-Methylcyclohexyl)carbonyl](phenyl)amino]-1-phenyl-1H-pyrazole-4-carboxylic acid;
3-([2-(Dimethylamino)-2-oxoethyl][(*trans*-4-methylcyclohexyl)carbonyl]amino)-1-phenyl-1H-pyrazole-4-carboxylic acid;
3-([(*trans*-4-Methylcyclohexyl)carbonyl]{1-[(methyloxy)carbonyl]-4-piperidinyl}amino)-1-phenyl-1H-pyrazole-4-carboxylic acid;
3-{[(*trans*-4-Methylcyclohexyl)carbonyl][1-(methylsulfonyl)-4-piperidinyl]amino}-1-phenyl-1H-pyrazole-4-carboxylic acid;
3-[[(*trans*-4-Methylcyclohexyl)carbonyl](1-methyl-4-piperidinyl)amino]-1-phenyl-1H-pyrazole-4-carboxylic acid;
3-{{1-[(Ethylamino)carbonyl]-4-piperidinyl}[(*trans*-4-methylcyclohexyl)carbonyl]amino}-1-phenyl-1H-pyrazole-4-carboxylic acid;
3-[[(*trans*-4-Methylcyclohexyl)carbonyl](2-pyrazinylmethyl)amino]-1-phenyl-1H-pyrazole-4-carboxylic acid;
*rel*-3-[{[(1S,2R,4S)-2-Hydroxy-4-methylcyclohexy)]carbonyl}(1-methylethyl)amino]-1-phenyl-1H-pyrazole-4-carboxylic acid;
3-[[(*trans*-4-Methylcyclohexyl)carbonyl](1-methylethyl)amino]-1-{4-[(3-methoxyphenylcarbonyl)amino]phenyl}-1H-pyrazole-4-carboxylic acid;
3-[[(*trans*-4-Methylcyclohexyl)carbonyl](1-methylethyl)amino]-1-{4-[(phenylmethyl)oxy]phenyl}-1H-pyrazole-4-carboxylic acid;
1-(1H-Indol-5-yl)-3-[[(*trans*-4-methylcyclohexyl)carbonyl](1-methylethyl)amino]-1H-pyrazole-4-carboxylic acid;
3-[[(*trans-*4-Methylcyclohexyl)carbonyl](1-methylethyl)amino]-1-{4-[(E/Z)-2-phenylethenyl]phenyl}-1H-pyrazole-4-carboxylic acid;
3-[[(*trans*-4-Methylcyclohexyl)carbonyl](1-methytethyl)amino]-1-[4-(2-phenylethyl)phenyl]-1H-pyrazole-4-carboxylic acid;
3-[[(*trans*-4-Methylcyclohexyl)carbonyl](tetrahydro-2H-pyran-4-yl)amino]-1-{4-[2-phenylethyl]phenyl}-1H-pyrazole-4-carboxylic acid;
3-[[(*trans*-4-Methylcyclohexyl)carbonyl](1-methylethyl)amino]-1-[4-[(1,3-thiazol-4-yl)-ethyl]phenyl]-1H-pyrazole-4-carboxylic acid;
3-[[(*trans*-4-Methylcyclohexyl)carbonyl](tetrahydro-2H-pyran-4-yl)amino]-1-{4-[(1,3-thiazol-4-yl)-ethyl]phenyl}-1H-pyrazole-4-carboxylic acid;
1-Cyclohexyl-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[1-(methylsulfonyl)-1,2,3,6-tetrahydro-4-pyridinyl]-1H-pyrazole-4-carboxylic acid;
3-[[(*trans*-4-Methylcyclohexyl)carbonyl](phenylmethyl)amino]-1-phenyl-1H-pyrazole-4-carboxylic acid;
3-{Cyclopentyl[(*trans*-4-methylcyclohexyl)carbonyl]amino}-1-phenyl-1H-pyrazole-4-carboxylic acid;
3-[[(*trans*-4-Methylcyclohexyl)carbonyl](tetrahydro-2H-pyran-4-yl)amino]-1-phenyl-1H-pyrazole-4-carboxylic acid;
3-{(1-Acetyl-4-piperidinyl)[(*trans*-4-methylcyclohexyl)carbonyl]amino}-1-phenyl-1H-pyrazole-4-carboxylic acid;
3-[[(*trans*-4-Methylcyclohexyl)carbonyl](4-piperidinyl)amino]-1-phenyl-1*H*-pyrazole-4-carboxylic acid;
3-[[(*trans*-4-Methylcyclohexyl)carbonyl](1-methylethyl)aminol-1-{4-[(E)-2-cyclohexylethenyl]phenyl}-1H-pyrazole-4-carboxylic acid;
1-[4-(2-Cyclohexylethyl)phenyl]-3-[[(*trans*-4-methylcyclohexyl)carbonyl](1-methylethyl)amino]-1*H*-pyrazole-4-carboxylic acid;
3-[[(*trans*-4-Methylcyclohexyl)carbonyl](1-methylethyl)amino]-1-{4-[2-pyridinylethenyl]phenyl}-1H-pyrazole-4-carboxylic acid;
3-[[(*trans*-4-Methylcyclohexyl)carbonyl](1-methylethyl)amino]-1-{4-[2-pyridinylethyl]phenyl}-1H-pyrazole-4-carboxylic acid;
3-[[(*trans*-4-Methylcyclohexyl)carbonyl](1-methylethyl)amino]-1-{4-[1,3-thiazol-2-ylethyl]phenyl}-1H-pyrazole-4-carboxylic acid;
3-[[(*trans*-4-Methylcyclohexyl)carbonyl](1-methylethyl)amino]-1-{4-[2-(1*H*-pyrazol-3-yl)ethyl]phenyl}-1*H*-pyrazole-4-carboxylic acid;
3-[[(*trans*-4-Methylcyclohexyl)carbonyl](1-methylethyl)amino]-1-{4-[(phenylamino)carbonyl]phenyl}-1*H*-pyrazole-4-carboxylic acid;
3-[[(*trans*-4-Methylcyclohexyl)carbonyl](1-methylethyl)amino]-1-{4-[(phenylcarbonyl)amino]phenyl}-1*H*-pyrazole-4-carboxylic acid;
3-[[(*trans*-4-Methylcyclohexyl)carbonyl](1-methylethyl)amino]-1-{4-[(3-methylphenylcarbonyl)amino]phenyl}-1*H*-pyrazole-4-carboxylic acid;
3-([(*trans*-4-Methylcyclohexyl)carbonyl]{1-[(*tert*-butyloxy)carbonyl]-4-piperidinyl}amino)-1-phenyl-1H-pyrazole-4-carboxylic acid;
3-[[(*trans*-4-Methylcyclohexyl)carbony](1-methylethyl)amino]-1-{4-[(4-fluorophenylcarbonyl)amino]phenyl}-1*H*-pyrazole-4-carboxylic acid;
3-[[(*trans*-4-Methylcyclohexyl)carbonyl](1-methylethyl)amino]-1-{4-[(cyclohexylcarbonyl)amino]phenyl}-1H-pyrazole-4-carboxylic acid;
1-(4-{[(4-Fluorophenyl)amino]carbonyl}phenyl)-3-[[(*trans*-4-methylcyclohexyl)carbonyl](1-methylethyl)amino]-1H pyrazole-4-carboxylic acid;
3-[[(trans-4-Methylcyclohexyl)carbonyl](1-methylethyl)amino]-1-{3-[(chlorophenylcarbonyl)amino]phenyl}-1*H*-pyrazole-4-carboxylic acid;
3-[[(*trans*-4-Methylcyclohexyl)carbonyl](1-methylethyl)amino]-1-{4-[(phenylsulfonyl)amino]phenyl}-1H-pyrazole-4-carboxylic acid;
1-(4-Methyl-1-cyclohexen-1-yl)-3-[[(*trans*-4-methylcyclohexyl)carbonyl](1-methylethyl)amino]-1H-pyrazole-4-carboxylic acid;
1-(4,4-Dimethyl-1-cyclohexen-1-yl)-3-[[(*trans*-4-methylcyclohexyl)carbonyl](tetrahydro-3-furanyl)amino]-1*H*-pyrazole-4-carboxylic acid;
and salts, solvates and esters, and individual enantiomers thereof where appropriate.

In one aspect, chemical entities useful in the present invention may be chosen from compounds of Formula (I) selected from the group consisting of:
3-[[(*trans*-4-Methylcyclohexyl)carbonyl](1-methylethyl)amino]-1-phenyl-1*H*-pyrazole-4-carboxylic acid;
3-[[(*trans*-4-Methylcyclohexyl)carbonyl](1-methylethyl)amino]-1-(4-methylphenyl)-1H-pyrazole-4-carboxylic acid;
1-(4-Hydroxyphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(trifluoromethyl)phenyl]-1H-pyrazole-4-carboxylic acid;
1-[4-(Acetylamino)phenyl]-3-[[(*trans-*4-methylcyclohexyl)carbonyl](1-methylethyl)amino]-1H-pyrazole-4-carboxylic acid;
1-(4-Biphenylyl)-3-[[(*trans*-4-methylcyclohexyl)carbonyl](1-methylethyl)amino]-1H-pyrazole-4-carboxylic acid;
1-[4-(Dimethylamino)phenyl]-3-[[(*trans-*4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(methyloxy)phenyl]-1H-pyrazole-4-carboxylic acid;
1-(4-Acetylphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{4-[(trifluoromethyl)oxy] phenyl}-1H-pyrazole-4-carboxylic acid;
1-(4-Cyanophenyl)-3-[[(*trans*-4-methylcyclohexyl)carbonyl](1-methylethyl)amino]-1H-pyrazole-4-carboxylic acid;
1-{4-[(Dimethylamino)carbonyl]phenyl}-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3-thienyl)-1H-pyrazole-4-carboxylic acid;
3-[[(*trans*-4-Methylcyclohexyl)carbonyl](1-methylethyl)amino]-1-[3-(trifluoromethyl)phenyl]-1H-pyrazole-4-carboxylic acid;
1-(3,5-Dimethylphenyl)3-[[(*trans*-4-methylcyclohexyl)carbonyl](1-methylethyl)amino]-1H-pyrazole-4-carboxylic acid;
1-(3-Chloro-5-fluorophenyl)-3-[[(*trans*-4-methylcyclohexyl)carbonyl](1-methylethyl)amino]-1H-pyrazole-4-carboxylic acid;
1-[3,5-Bis(trifluoromethyl)phenyl]-3-[[(*trans*-4-methylcyclohexyl)carbonyl](1-methylethyl)amino]-1H-pyrazole-4-carboxylic acid;
1-(1,3-Benzodioxol-5-yl)-3-[[(*trans*-4-methylcyclohexyl)carbonyl](1-methylethyl)amino]-1H-pyrazole-4-carboxylic acid;
1-(2,3-Dihydro-1-benzofuran-5-yl)-3-[[(*trans*-4-methylcyclohexyl)carbonyl](1-methylethyl) amino]-1H-pyrazole-4-carboxylic acid;
1-(2,3-Dihydro-1,4-benzodioxin-6-yl)-3-[[(*trans*-4-methylcyclohexyl)carbonyl](1-methylethyl) amino]-1H-pyrazole-4-carboxylic acid;
3-[[(*trans*-4-Methylcyclohexyl)carbonyl](1-methylethyl)amino]-1-(3,4,5-trifluorophenyl)-1H-pyrazole-4-carboxylic acid;
1-(4-Chlorophenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[3-(methyloxy)phenyl]-1H-pyrazole-4-carboxylic acid;
3-[[(*trans*-4-Methylcyclohexyl)carbonyl](1-methylethyl)amino]-1-[4-(methylsulfonyl)phenyl]-1H-pyrazole-4-carboxylic acid;
1-(2-Fluorophenyl)-3-[[(*trans-*4-methylcyclohexyl)carbonyl](1-methylethyl)amino]-1H-pyrazole-4-carboxylic acid;
1-(3-Hydroxyphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3-methylphenyl)-1H-pyrazole-4-carboxylic acid;
1-(3-Fluorophenyl)-3-[[(*trans*-4-methylcyclohexyl)carbonyl](1-methylethyl)amino]-1H-pyrazole-4-carboxylic acid;
1-(4-Aminophenyl)-3-[[(*trans*-4-methylcyclohexyl)carbonyl](1-methylethyl)amino]-1H-pyrazole-4-carboxylic acid;
1-(3-Chlorophenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{3-[(trifluoromethyl)oxy] phenyl}-1H-pyrazole-4-carboxylic acid;
1-(4-Chloro-3-fluorophenyl)-3-[[(*trans*-4-methylcyclohexyl)carbonyl](1-methylethyl)amino]-1H-pyrazole-4-carboxylic acid;
1-(4-Chloro-3-methylphenyl)-3-[[(*trans*-4-methycyclohexyl)carbonyl](1-methylethyl)amino]-1H-pyrazole-4-carboxylic acid;
1-(3-Amino-4-methylphenyl)-3-[[(*trans*-4-methylcyclohexyl)carbonyl](1-methylethyl)amino]-1H-pyrazole-4-carboxylic acid;
1-(3-Fluoro-4-methylphenyl)-3-[[(*trans*-4-methylcyclohexyl)carbonyl](1-methylethyl)amino]-1 H-pyrazole-4-carboxylic acid;
1-(3,4-Difluorophenyl)-3-[[(*trans*-4-methylcyclohexyl)carbonyl](1-methylethyl)amino]-1H-pyrazole-4-carboxylic acid;
1-[(1E)-1-Hexen-1-yl]-3-[[(*trans*-4-methylcyclohexyl)carbonyl](1-methylethyl)amino]-1H-pyrazole-4-carboxylic acid;
1-[(E)-2-Cyclohexylethenyl]-3-[[(*tran*s-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[(1E)-4-methyl-1-penten-1-yl]-1H-pyrazole-4-carboxylic acid;
1-[(E)-2-(4-Fluorophenyl)ethenyl]-3-[[(*trans*-4-methylcyclohexyl)carbonyl](1-methylethyl) amino]-1H-pyrazole-4-carboxylic acid;
1-(4-Ethenylphenyl)-3-[[(*trans*-4-methylcyclohexyl)carbonyl](1-methylethyl)amino]-1H-pyrazole-4-carboxylic acid;
1-[4-(Hydroxymethyl)phenyl]-3-[[(*trans*-4-methylcyclohexyl)carbonyl](1-methylethyl)amino]-1H-pyrazole-4-carboxylic acid;
1-(4-Ethylphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(1-methylethyl)phenyl]-1H-pyrazole-4-carboxylic acid;
1-(5-Acetyl-2-thienyl)-3-[[(*trans*-4-methylcyclohexyl)carbonyl](1-methylethyl)amino]-1H-pyrazole-4-carboxylic acid;
1-(5-Chloro-2-thienyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(5-methyl-2-thienyl)-1H-pyrazole-4-carboxylic acid;
3-[[(*trans*-4-Methycyclohexyl)carbonyn(1-methylethyl)amino]-1-(5-phenyl-2-thienyl)-1H-pyrazole-4-carboxylic acid;
1-(3-Cyanophenyl)-3-[[(*trans*-4-methylcyclohexyl)carbonyl](1-methylethyl)amino]-1H-pyrazole-4-carboxylic acid;
3-[(Cyclohexylacetyl)(1-methylethyl)amino]-1-phenyl-1H-pyrazole-4-carboxylic acid;
3-{(1-Methylethyl) [(4-methylphenyl)carbonyl] amino}-1-phenyl-1H-pyrazole-4-carboxylic acid;
3-[[(4-Bromo-2-chlorophenyl)carbonyl](1-methylethyl)aminol-1-phenyl-1H-pyrazole-4-carboxylic acid;
3-[[(*trans*-4-Methylcyclohexyl)carbonyl](phenyl)amino]-1-phenyl-1H-pyrazole-4-carboxylic acid;
3-{[2-(Dimethylamino)-2-oxoethyl][(*trans*-4-methylcyclohexyl)carbonyl]amino}-1-phenyl-1H-pyrazole-4-carboxylic acid;
3-([(*tran*s-4-Methylcyclohexyl)carbonyl]{1-[(methyloxy)carbonyl]-4-piperidinyl}amino)-1-phenyl-1H-pyrazole-4-carboxylic acid;
3-{[(*trans*-4-Methylcyclohexyl)carbonyl][1-(methylsulfonyl)-4-piperidinyl]amino}-1-phenyl-1H-pyrazole-4-carboxylic acid;
3-[[(*trans-*4-Methylcyclohexyl)carbonyl(1-methyl-4-piperidinyl)amino)-1-phenyl-1H-pyrazole-4-carboxylic acid;
3-{{1-[(Ethylamino)carbonyl]-4-piperidinyl}[(*trans*-4-methylcyclohexyl)carbonyl]amino}-1-phenyl-1H-pyrazole-4-carboxylic acid;
3-[[(*trans*-4-Methylcyclohexyl)carbonyl](2-pyrazinylmethyl)amino]-1-phenyl-1H-pyrazole-4-carboxylic acid;
3-[{[(1S,2R,4S)-2-Hydroxy-4-methylcyclohexyl]carbonyl}(1-methylethyl)amino]-1-phenyl-1H-pyrazole-4-cartioxylic acid;
3-{(1-Isopropyl)[(4-methylidenecyclohexyl)carbonyl]amino}-1-phenyl-1*H*-pyrazole-4-carboxylic acid;
3-[[(*trans*-4-Methylcyclohexyl)carbonyl](1-methylethyl)amino]-1-{4-[(phenylmethyl)oxy] phenyl}-1H-pyrazole-4-carboxylic acid;
1-(1H-Indol-5-yl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{4-[(E/Z)-2-phenylethenyl] phenyl}-1H-pyrazole-4-carboxylic acid;
3-[[(*trans*-4-Methylcyclohexyl)carbonyl](1-methylethyl)amino]-1-[4-(2-phenylethyl)phenyl]-1H-pyrazole-4-carboxylic acid;
1-(1-Cyclohexen-1-yl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[(E)-2-phenylethenyl]-1H-pyrazole-4-carboxylic acid;
1-(4-Methyl-1-cyclohexen-1-yl)-3-[[(*trans*-4-methylcyclohexyl)carbonyl](1-methylethyl)amino]-1H-pyrazole-4-carboxylic acid;
1-Cyclohexyl-3-[[(*trans*-4-methylcyclohexyl)carbonyl](1-methylethyl)amino]-1H-pyrazole-4-carboxylic acid;
3-[[(*tran*s-4-Methylcyclohexyl)carbonyl](1-methylethyl)amino]-1-[1-(methylsulfonyl)-1,2,3,6-tetrahydro-4-pyridinyl]-1H-pyrazole-4-carboxylic acid;
3-[[(*trans*-4-Methylcyclohexyl)carbonyl](phenylmethyl)amino]-1-phenyl-1H-pyrazole-4-carboxylic acid;
3-{Cyclopentyl[(*trans*-4-methylcyclohexyl)carbonyl]amino}-1-phenyl-1H-pyrazole-4-carboxylic acid;
3-[[(trans-4-Methylcyclohexyl)carbonyl](tetrahydro-2H-pyran-4-yl)amino]-1-phenyl-1H-pyrazole-4-carboxylic acid;
3-{(1-Acetyl-4-piperidinyl)[(*trans*-4-methylcyclohexyl)carbonyl]amino}-1-phenyl-1H-pyrazole-4-carboxylic acid;
and salts, solvates and esters, and individual enantiomers thereof where appropriate.

Also included in the present invention are pharmaceutically acceptable salt complexes. The present invention also covers the physiologically acceptable salts of the compounds of Formula (I). Suitable physiologically acceptable salts of the compounds of Formula (I) include acid salts, for example sodium, potassium, calcium, magnesium and tetraalkylammonium and the like, or mono- or di- basic salts with the appropriate acid for example organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids and the like.

The present invention also relates to solvates of the compounds of Formula (I), for example hydrates.

The present invention also relates to pharmaceutically acceptable esters of the compounds of Formula (I), for example carboxylic acid esters -COOR, in which R is selected from straight or branched chain alkyl, for example n-propyl, n-butyl, alkoxyalkyl (e.g. methoxymethyl), aralkyl (e.g. benzyl), aryloxyalkyl (e.g. phenoxymethyl), aryl (e.g. phenyl optionally substituted by halogen, -C₁₋₄alkyl or -C₁₋₄alkoxy or amino); or for example - CH₂OC(O)R' or -CH₂OCO₂R' in which R' is alkyl (e.g. R' is *t*-butyl). Unless otherwise specified, any alkyl moiety present in such esters preferably contains 1 to 18 carbon atoms, particularly 1 to 4 carbon atoms. Any aryl moiety present in such esters preferably comprises a phenyl group.

It will further be appreciated that certain compounds of the present invention may exist in different tautomeric forms. All tautomers are contemplated to be within the scope of the present invention.

### PROCESSES

Compounds of Formula (I) in which A is hydroxy may be prepared from a compound of Formula (II) 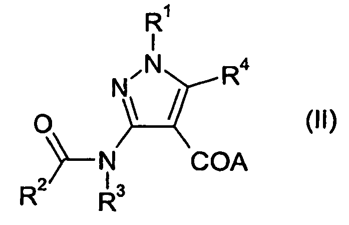 in which A is a protected hydroxy group, for example an alkoxy, benzyloxy or silyloxy group and R¹, R², R³ and R⁴ are as defined above for Formula (I). For example when A is methoxy or ethoxy, and R¹, R², R³ and R⁴ are as defined above for Formula (I), by treatment with an appropriate base, for example aqueous sodium hydroxide or lithium hydroxide, optionally in a solvent such as methanol, tetrahydrofuran or combinations thereof. Suitably, the temperature is in the range 25 to 100°C, more preferably 50 to 100°C. Alternatively, when A is methoxy or ethoxy and R¹, R², R³ and R⁴ are as defined above for Formula (I), by treatment with lithium iodide in a suitable solvent such as pyridine, lutidine or collidine, suitably in the temperature range 100-170°C.
For example when A is *tert*-butoxy, and R¹, R², R³ and R⁴ are as defined above for Formula (I), by treatment with an appropriate acid, for example trifluoroacetic acid. Suitably, the reaction is carried out in a solvent, for example dichloromethane. Suitably, the temperature is in the range 0 to 50°C, more preferably 15 to 30°C.
For example when A is silyloxy, and R¹, R², R³ and R⁴ are as defined above for Formula (I), by treatment with a suitable fluoride source for example tetrabutylammonium fluoride. The reaction is carried out in a suitable solvent, for example tetrahydrofuran. Suitably, the temperature is in the range 0 to 50°C, more preferably 15 to 30°C.

Compounds of Formula (I) in which A is hydroxy, or (II) in which A is an alkoxy, benzyloxy or silyloxy group may be prepared by reaction of a compound of Formula (III) 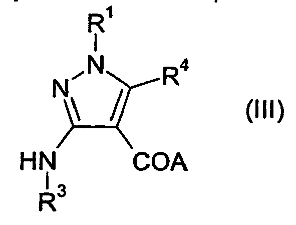 in which A is hydroxy or an alkoxy, benzyloxy or silyloxy group, and R¹, R³ and R⁴ are as defined above for Formula (I); with a suitable acylating agent, for example R²-C(O)-Y,
wherein Y is a halo atom, for example chloro or bromo, and R² is as defined above for Formula (I). The reaction may be carried out in a suitable solvent, for example dichloromethane, in the presence of a suitable base, for example pyridine or triethylamine and thereafter removing any protecting group if desired. Where R² represents an aliphatic group, a phosphine such as triphenylphosphine may optionally be used. Suitable protecting groups can be found, but are not restricted to, those found in T W Greene and P G M Wuts 'Protective Groups in Organic Synthesis', 3rd Ed (1999), J Wiley and Sons.

In a further aspect, a compound of Formula (II) may be prepared by appropriate manipulation of another compound of Formula (II). For example, a compound of Formula (II) in which any substituent comprises -C₂₋₄alkenyl may be prepared from a suitable aldehyde or ketone substituent and a phosphorous ylid generated from a phosphonium salt in the presence of a suitable base, such as potassium *tert-*butoxide, in a suitable solvent such as THF. Optionally, the *trans* and *cis* isomers may be separated by standard techniques known in the art
For example, a compound of Formula (II) in which any substituent comprises -C₂₋₄alkyl may be prepared by reduction of an alkenyl substituent, for example using hydrogen, optionally under pressure, in the presence of a suitable catalyst such as palladium on carbon, in a suitable solvent such as ethanol.

For example, a compound of Formula (II) in which any substituent comprises -C(O)NR^{A}R^{B} may be prepared by reacting a suitable acid substituent with an amine (HNR^{A}R^{B}) in the presence of a coupling agent such as HATU, in the presence of a suitable base such as triethylamine, in a suitable solvent such as DMF. Alternatively, a compound of Formula (II) in which any substituent comprises -C(O)NR^{A}R^{B} may be prepared by reacting a suitable acid chloride substituent with an amine (HNR^{A}R^{B}) in the presence of a suitable base such as triethylamine, in a suitable solvent such as dichloromethane.

For example, a compound of Formula (II) in which any substituent comprises -NR^{E}C(O)R^{D} may be prepared by reacting a suitable amine substituent with a carboxylic acid (R^{D}CO₂H) in the presence of a coupling agent such as HATU, in the presence of a suitable base such as triethylamine, in a suitable solvent such as DMF. Alternatively, a compound of Formula (II) in which any substituent comprises -NR^{E}C(O)^{D} may be prepared by reacting a suitable amine substituent with an acid chloride in the presence of a suitable base such as triethylamine in a suitable solvent such as dichloromethane.

For example, a compound of Formula (II) in which any substituent comprises -NR^{E}SO₂R^{D} may be prepared by reacting an amine substituent with a suitable sulfonyl chloride in the presence of a suitable base such as triethylamine, in a suitable solvent such as dichloromethane.

For example, a compound of Formula (II) in which any substituent comprises -SO₂NR^{F}R^{G} may be prepared by reacting a sulfonyl chloride substituent with a suitable amine (HNR^{F}R^{G}) in the presence of a suitable base such as triethylamine, in a suitable solvent such as dichloromethane.

For example, a compound of Formula (II) in which any substituent comprises -(CH₂)ₙSR^{A}, wherein n=0, 1, 2, 3 or 4 may be prepared by reacting a thiol -(CH₂)ₙSH substituent or a thiolate salt (for example sodium thiomethoxide) substituent with an alkyl halide R^{A}X wherein X is a halo atom such as bromo, in a suitable solvent such as DMF, in the presence of a suitable base such as triethylamine.

For example, a compound of Formula (II) in which any substituent comprises -SO₂R^{A} may be prepared by oxidation of a compound in which a substituent represents -SR^{A}, using for example oxone, sodium periodate, 3-chloro perbenzoic acid, or hydrogen peroxide. For example, a compound of Formula (II) in which any substituent comprises -(CH₂)ₙOR^{A} may be prepared by reacting an alcohol -(CH₂)ₙOH substituent, wherein n=0, 1, 2, 3, or 4, with an alkyl halide R^{A}X wherein X is a halo atom such as bromo in the presence of a suitable base such as triethylamine or sodium hydride, or a compound of Formula (II) in which any substituent comprises -(CH₂)ₚOR^{A} may prepared by reacting an alkyl halide -(CH₂)ₚX substituent, where p=1, 2 or 3 and X is a halo atom such as bromo, with an alcohol R^{A}OH, optionally in the presence of a base such as triethylamine or sodium hydride, or with an alkoxide (for example sodium methoxide) in a suitable solvent such as DMF.

For example, a compound of Formula (II) in which any substituent comprises -(CH₂)ₙNR^{A}R^{B}, where n=0,1, 2, 3, or 4, may be prepared by reacting an amine -(CH₂)ₙNHR^{B} substituent with an alkyl halide R^{A}X wherein X is a halo atom such as bromo, in the presence of a suitable base such as triethylamine or sodium hydride, or a compound of Formula (II) in which any substituent comprises -(CH₂)ₙNR^{A}R^{B} wherein n=0,1, 2, 3, or 4, may be prepared by reacting an aldehyde substituent with an amine, in the presence of a reducing agent such as sodium triacetoxyborohydride, in a suitable solvent such as dichloromethane.

Compounds of Formula (III) in which A is an alkoxy, benzyloxy or silyloxy group may be prepared from compounds of Formula (IV) 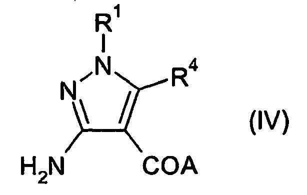 in which A is an alkoxy, benzyloxy or silyloxy, and R¹ and R⁴ are as defined above for Formula (I), by treatment with a suitable vinyl ether, or a suitable aldehyde or a suitable ketone in the presence of a suitable acid, such as acetic acid, and a suitable reducing agent such as sodium triacetoxyborohydride, in a suitable solvent such as dichloromethane. Alternatively, compounds of Formula (III) in which A is an alkoxy, benzyloxy or silyloxy group may be prepared from compounds of Formula (IV) in which A is an alkoxy, benzyloxy or silyloxy, and R¹ and R⁴ are as defined above for Formula (I), by treatment with a suitable alkylating agent R³-X where X is a halo group such as chloride, bromide or iodide, or X is a sulphonate ester such as methanesulfonate, in suitable solvent such as dimethylformamide in the presence of a suitable base such as triethylamine.

Compounds of Formula (III) in which A is hydroxy may be prepared from compounds of Formula (III) in which A is an alkoxy, benyloxy or silyloxy group, for example by treatment with an appropriate base, acid or fluoride source as described in relation to the preparation of compounds of Formula (I) from compounds of Formula (II).

Compounds of Formula (IV) may be prepared by reaction of compounds of Formula (V)

R¹-NHNH₂ (V)

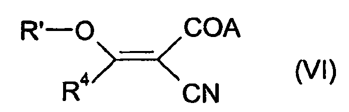

in which R¹ is as defined above for Formula (I) with compounds of Formula (VI) in which R⁴ and A are as defined above for Formula (II) and R' is -C₁₋₄alkyl (such as ethyl) in a suitable solvent such as ethanol or xylene, preferably in the temperature range 50-75°C.

Compounds of Formula (IV) may also be prepared by reaction of compounds of Formula (VII)

R¹-NH-N=P (VII)

in which R¹ is as defined above for Formula (I) and P is a suitable nitrogen protecting group such as benzylidine, with compounds of Formula (VI) in a suitable solvent such as ethanol or xylene, preferably in the temperature range 50-75°C. This is followed by treatment with a suitable acid, such as hydrochloric acid, in a suitable solvent, such as ethanol to effect removal of the protecting group and cyclisation.

Compounds of Formula (V), (VI) and (VII) are commercially available or well known in the art.

Compounds of Formula (II) may also be prepared by reaction of a compound of Formula (VIII) 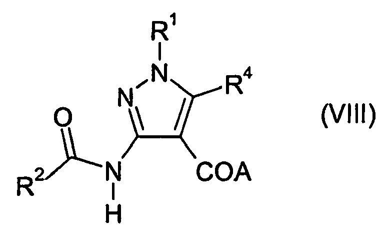 in which A is an alkoxy, benzyloxy or silyloxy group, and R¹, R² and R⁴ are as defined above for Formula (I); with a suitable alkylating agent R³-X in which X is a halo atom such as chloro, bromo or iodo, or X is a sulphonate ester such as methanesulfonate, in a suitable solvent such as dimethylformamide, in the presence of a suitable base such as triethylamine and sodium hydride.

Compounds of Formula (VIII) may be prepared from compounds of Formula (IV) by reaction with a suitable acylating agent, for example R²-C(O)-Y, in which Y is a halo atom, preferably chloro or bromo, and R² is as defined above for Formula (I). Suitably, the reaction is carried out in a suitable solvent, for example dichloromethane, in the presence of a suitable base, for example pyridine or triethylamine. For example, where R² represents an aliphatic group, a phosphine such as triphenylphosphine may optionally be used in place of an amine base.

Compounds of Formula (II) may also be prepared by reaction of a compound of Formula (IX) 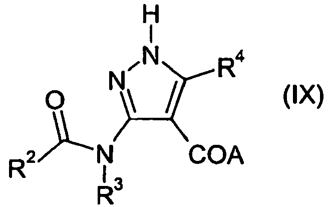 in which A is an alkoxy, benzyloxy or silyloxy group, and R², R³ and R⁴ are as defined above for Formula (I); by treatment with an aryl, heteroaryl, cycloalkenyl or alkenyl boronic acid in the presence of a copper catalyst such as copper (II) acetate. Suitably, the reaction is carried out in the presence of a base, such as pyridine, in air, and in a suitable solvent such as dichloromethane or THF, and at a temperature in the range 10-30°C. Alternatively, compounds of Formula (II) in which R¹ represents aryl or heteroaryl may be prepared by reaction of compounds of Formula (IX) with an aryl or heteroaryl halide or triflate in the presence of a copper catalyst such as copper (I) iodide. Suitably, the reaction is carried out in the presence of a base such as potassium carbonate or *trans*-1,2-diaminocyclohexane or a combination thereof in a suitable solvent such as dioxan or pyridine or a combination thereof, and at a temperature in the range 90-110°C.

Compounds of Formula (II) in which R¹ represents heteroaryl (for example 2-pyridyl, 2-pyrimidyl, 2-pyrazinyl, 2-pyridazinyl), may also be prepared by reaction of a compound of Formula (IX) 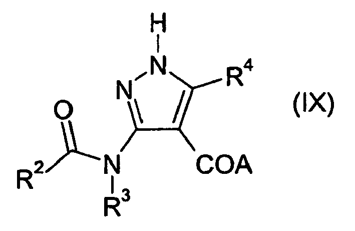 in which A is an alkoxy, benzyloxy or silyloxy group, and R², R³ and R⁴ are as defined above for Formula (I); by treatment with a 2-halo substituted heteroaryl compound in the presence of a suitable base such as sodium hydride, in a suitable solvent such as dimethylformamide.

Compounds of Formula (IX) in which A is an alkoxy, benzyloxy or silyloxy group may be prepared by deprotection of a compound of Formula (X) 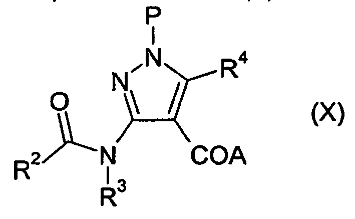 in which A is an alkoxy, benzyloxy or silyloxy group, and R², R³ and R⁴ are as defined above for Formula (I); and P is a suitable protecting group. Suitable protecting groups include, but are not restricted to, benzyl and tert-butyloxycarbonyl . Benzyl groups can be removed by hydrogenation using hydrogen gas with a catalyst such as palladium on carbon in a suitable solvent such as ethanol, optionally in the presence of a suitable acid, for example hydrochloric acid, and optionally conducting the reaction under pressure. *tert-*Butyloxycarbonyl groups may be removed using an acid such as hydrochloric acid or trifluoroacetic acid.

It will be understood by those skilled in the art that compounds of Formula (X) may be prepared from compounds of Formulae (III), (IV) or (VIII) in which the group R¹ is a protecting group instead of a group as defined for Formula (I), by application of the synthetic routes described above in relation to the synthesis of compounds of Formula (II).

Esters of compounds of Formula (I), in which A is -OR where R is selected from straight or branched chain alkyl, aralkyl, aryloxyalkyl, or aryl, may also be prepared by esterification of a compound of Formula (I) in which A is hydroxy by standard literature procedures for esterification.

It will be appreciated that compounds of Formula (I), (II), (III), (IV), (VIII), (IX) and (X) which exist as diastereoisomers may optionally be separated by techniques well known in the art, for example by column chromatography or recrystallisation. For example, the formation of an ester using a chiral alcohol, separation of the resulting diastereoisomers, and subsequent hydrolysis of the ester to yield the individual enantiomeric acid of Formula (I) (II), (III), (IV), (VIII), (IX) and (X).

It will be appreciated that racemic compounds of Formula (I), (II), (III), (IV), (VIII), (IX) and (X) may be optionally resolved into their individual enantiomers. Such resolutions may conveniently be accomplished by standard methods known in the art. For example, a racemic compound of Formula (I), (II), (III), (IV), (VIII), (IX) and (X) may be resolved by chiral preparative HPLC. Alternatively, racemic compounds of Formula (I), (II), (III), (IV), (VIII), (IX) and (X) which contain an appropriate acidic or basic group, such as a carboxylic acid group or amine group may be resolved by standard diastereoisomeric salt formation with a chiral base or acid reagent respectively as appropriate. Such techniques are well established in the art. For example, a racemic compound may be resolved by treatment with a chiral acid such as (R)-(-)-1,1'-binaphthyl-2,2'-diyl-hydrogen phosphate or (-)-di-O,O'-p-tolyl-L-tartaric acid, in a suitable solvent, for example isopropanol. Alternatively, racemic acid compounds may be resolved using a chiral base, for example (S)-alpha methylbenzylamine, (S)-alpha phenylethylamine, (1S, 2S)-(+)-2-amino-1-phenyl-1,3-propane-diol, (-) ephidrine, quinine, brucine. Individual enantiomers of Formula (II), (III), (IV), (VIII), (IX) and/or (X) may then be progressed to an enantiomeric compound of Formula (I) by the chemistry described above in respect of racemic compounds.

With appropriate manipulation and protection of any chemical functionality, synthesis of compounds of Formula (I) is accomplished by methods analogous to those above and to those described in the Experimental section. Suitable protecting groups can be found, but are not restricted to, those found in T W Greene and P G M Wuts 'Protective Groups in Organic Synthesis', 3rd Ed (1999), J Wiley and Sons.

### EXAMPLES

### ABBREVIATIONS

- STRATA cartridge: Dual action SPE cartridge available from Phenomenex
- SPE: solid phase extraction column
- TFA: trifluoroacetic acid
- HPLC: high pressure liquid chromatography
- DCM: dichloromethane
- DMF: N,N-dimethylformamide
- THF: tetrahydrofuran
- EtOAc: ethyl acetate
- AcOH: acetic acid
- DME: 1,2-dimethoxyethane
- OASIS HLB cartridge: Sample extraction cartridge available from Waters
- hrs: hours
- HATU: *O*-(7-Azabenzotriazol-1-yl)-N,N,N',N'-tetramethyluronium-hexafluorophosphate
- TBDMS: *tert*-butyldimethylsilyl
- HCl: hydrochloric acid
- MDAP HPLC: reverse phase HPLC on a C₁₈ column using a two-solvent gradient elution with (A) water containing formic acid (0.1%) and (B) acetonitrile-water (95:5 v/v) containing formic acid (0.05%) as the eluents, and analysis of the fractions by electrospray mass spectroscopy.
- ISCO Companion: Automated flash chromatography equipment with fraction analysis by UV absorption available from Presearch.

All mass spectroscopy was performed using electrospray as the method of ionisation.

### Intermediate 1

### Ethyl 3-[(1-methylethyl)amino]-1-phenyl-1H-pyrazole-4-carboxylate

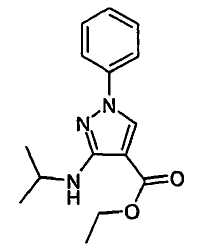

Ethyl 3-amino-1-phenyl-1H-pyrazole-4-carboxylate hydrogen chloride¹ (5 g) was partitioned between ethyl acetate (100 mL) and a half saturated solution of sodium bicarbonate (100 mL), the organic layer was separated, dried (Na₂SO₄) and concentrated to dryness. The residue (4.21 g) was dissolved in dichloromethane (100 mL), the solution treated with 2-methoxypropene (6.97 mL, 4 eq), acetic acid (4.17 mL, 4 eq), sodium triacetoxyborohydride (7.72 g, 2 eq) and stirred at room temperature under nitrogen overnight. The reaction mixture was concentrated to dryness, diluted with water (100 mL), adjusted to pH7 with the addition of sodium bicarbonate and extracted with ethyl acetate (2 X 100 mL). The combined extracts were washed with brine, dried (Na₂SO₄), concentrated to dryness and purified by silica gel chromatography, eluting with ethyl acetate/cyclohexane (1: 20) to give the title compound.
MS calcd for (C₁₅H₁₉N₃O₂ + H)⁺: 274
MS found (electrospray): (M+H)⁺ = 274
1. Massa, Silvio; Mai, Antonello; Artico, Marino; Corelli, Federico; J. Heterocycl.Chem.; 27; 6; 1990; p1805-1808

### Intermediate 2

### Ethyl 3-[[(trans-4-methylcyclohexyl)carbonyl](1-methylethyl)aminol-1-phenyl-1H-pyrazole-4-carboxylate

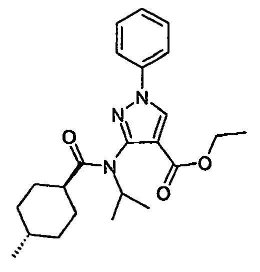

A solution of Intermediate 1 (158 mg) in dichloromethane (2 mL) was treated with *trans-4-*methylcydohexanecarbonyl chloride² (185 mg), triphenyl phosphine (258 mg) and stirred at 45°C overnight. The reaction mixture was diluted with dichloromethane (3 mL), washed with sodium bicarbonate (6 mL), dried (via a hydrophobic frit) and concentrated to dryness. The residue was purified by silica gel chromatography, eluting with ethyl acetate/cyclohexane (0:100, 5:95, 10:90, 15:85 to 100:0) to give the title compound.
MS calcd for (C₂₃H₃₁N₃O₃ + H)⁺: 398
MS found (electrospray): (M+H)⁺= 398
2. WO 2004/052885

### Intermediate 3

### Ethyl 3-[(1-methylethyl)amino]-1-(phenylmethyl)-1H-pyrazole-4-carboxylate

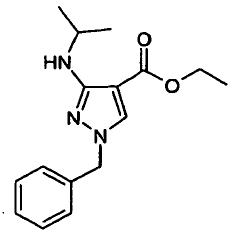

Prepared by a similar method to that described for Intermediate 1 from ethyl 3-amino-1-(phenylmethyl)-1H-pyrazole-4-carboxylate³.
MS calcd for (C₁₆H₂₁N₃O₂ + H)⁺: 288
MS found (electrospray): (M+H)⁺= 288
*3.* Chem.Pharm.Bull 1972, 20(2), 391-398

### Intermediate 4

### Ethyl 3-[[(trans-4-methylcyclohexyl)carbonyl](1-methylethyl)amino]-1-(phenylmethyl)-1H-pyrazole-4-carboxylate

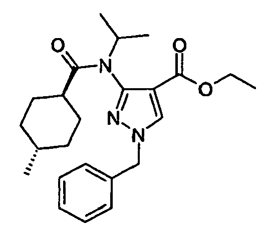

Prepared by a similar method to that described for Intermediate 2 replacing Intermediate 1 with Intermediate 3 to give the title compound.
MS calcd for (C₂₄H₃₃N₃O₃ + H)⁺: 412
MS found (electrospray): (M+H)⁺= 412

### Intermediate 5

### Ethyl 3-[[(trans-4-methylcyclohexyl)carbonyl](1-methylethyl)amino]-1H-pyrazole-4-carboxylate

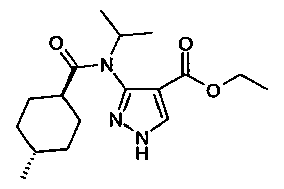

Intermediate 4 (1.52 g, 3.7 mmol) in ethanol (60 mL) and c.hydrochloric acid (0.6 mL) was subjected to atmospheric pressure hydrogenation with 10% palladium on carbon (0.45 g wet) for 16hrs. The reaction was filtered through a Celite pad, and the filter cake washed with ethanol. The combined organics were concentrated to give a gum. This was partitioned between DCM and saturated sodium bicarbonate solution, passed through a hydrophobic frit and the organics concentrated to give the title compound.
MS calcd for (C₁₇H₂₇N₃O₃ + H)⁺: 322
MS found (electrospray): (M+H)⁺ = 322

### Intermediate 6

### Ethyl 3-[[(trans-4-methylcyclohexyl)carbonyl](1-methylethyl)amino]-1-(4. methylphenyl)-1H-pyrazole-4-carboxylate

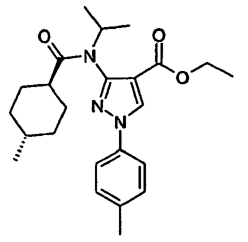

To p-tolylboronic acid (84 mg, 0.62 mmol) was added copper (II) acetate (85 mg, 0.47 mmol), Intermediate 5 (100 mg, 0.31 mmol) and pyridine (50 uL, 0.62 mmol). The reaction was stirred at room temperature in air for 16 hrs. The solvent removed and the residue purified by MDAP HPLC to give the title compound.
MS calcd for (C₂₄H₃₃N₃O₃ + H)⁺: 412
MS found (electrospray): (M+H)⁺= 412

The following compounds were made from the corresponding commercially available boronic acids by a similar procedure to that described for Intermediate 6:

### Intermediate 7

### Ethyl 1-(4-hydroxyphenyl)-3-[[(trans-4-methylcyclohexyl)carbonyl](1-methylethyl)amino]-1H-pyrazole-4-carboxylate

### Intermediate 8

### Ethyl 3-[[(trans-4-methylcyclohexyl)carbonyl](1-methylethyl)amino]-1-[4-(trifluoromethyl)phenyl]-1H-pyrazole-4-carboxylate

### Intermediate 9

### Ethyl 1-[4-(acetylamino)phenyl]-3-[[(trans-4-methylcyclohexyl)carbonyl](1-methylethyl)amino]-1H-pyrazole-4-carboxylate

### Intermediate 10

### Ethyl 1-(4-biphenylyl)-3-[[(trans-4-methylcyclohexyl)carbonyl](1-methylethyl)amino]-1H-pyrazole-4-carboxylate

### Intermediate 11

### Ethyl 1-[4-(dimethylamino)phenyl]-3-[[(trans-4-methylcyclohexyl)carbonyl](1-methylethyl)amino]-1H-pyrazole-4-carboxylate

### Intermediate 12

### Ethyl 3-[[(trans-4-methylcyclohexyl)carbonyl](1-methylethyl)amino]-1-[4-(methyloxy)phenyl]-1H-pyrazole-4-carboxylate

### Intermediate 13

### Ethyl 1-(4-acetylphenyl)-3-[[(trans-4-methylcyclohexyl)carbonyl](1-methylethyl)amino]-1H-pyrazole-4-carboxylate

### Intermediate 14

### Ethyl 3-[[(trans-4-methylcyclohexyl)carbonyl](1-methylethyl)amino]-1-{4-[(trifluoromethyl)oxy]phenyl}-1H-pyrazole-4-carboxylate

### Intermediate 15

### Ethyl 1-(4-cyanophenyl)-3-[[(trans-4-methylcyclohexyl)carbonyl](1-methylethyl)amino]-1H-pyrazole-4-carboxylate

### Intermediate 16

### Ethyl 1-{4-[(dimethylamino)carbonyl]phenyl}-3-[[(trans-4-methylcyclohexyl)carbonyl](1-methylethyl)amino]-1H-pyrazole-4-carboxylate

### Intermediate 17

### Ethyl 3-[[(frans-4-methylcyclohexyl)carbonyl](1-methylethyl)amino]-1-(3-thienyl)-1 H-pyrazole-4-carboxylate

### Intermediate 18

### Ethyl 3-[[(trans-4-methylcyclohexyl)carbonyl](1-methylethyl)amino]-1-[3-(trifluoromethyl)phenyl]-1H-pyrazole-4-carboxylate

### Intermediate 19

### Ethyl 1-(3,5-dimethylphenyl)-3-[[(trans-4-methylcyclohexyl)carbonyl](1-methylethyl)amino]-1 H-pyrazole-4-carboxylate

### Intermediate 20

### Ethyl 1-(3-chloro-5-fluorophenyl)-3-[[(trans-4-methylcyclohexyl)carbonyl](1-methylethyl)amino]-1H-pyrazole-4-carboxylate

### Intermediate 21

### Ethyl 1-[3,5-bis(trifluoromethyl)phenyll-3-[[(trans-4-methylcyclohexyl)carbonyl](1-methylethyl)amino]-1H-pyrazole-4-carboxylate

### Intermediate 22

### Ethyl 1-(1,3-benzodioxol-5-yl)-3-[[(trans-4-methylcyclohexyl)carbonyl](1-methylethyl)amino]-1H-pyrazole-4-carboxylate

### Intermediate 23

### Ethyl 1-(2,3-dihydro-1-benzofuran-5-yl)-3-[[trans-4-methylcyclohexyl)carbonyl](1-methylethyl)amino]-1H-pyrazole-4-carboxylate

### Intermediate 24

### Ethyl 1-(2,3-dihydro-1,4-benzodioxin-6-yl)-3-[[(trans-4-methylcyclohexyl)carbonyl](1-methylethyl)amino]-1H-pyrazole-4-carboxylate

### Intermediate 25

### Ethyl 3-[[(trans-4-methylcyclohexyl)carbonyl](1-methylethyl)amino]-1-(3,4,5-trifluorophenyl)-1H-pyrazole-4-carboxylate

### Intermediate 26

### Ethyl 1-(4-chlorophenyl)-3-[[(trans-4-methylcyclohexyl)carbonyl](1-methylethyl)amino]-1H-pyrazole-4-carboxylate

### Intermediate 27

### Ethyl 3-[[(trans-4-methylcyclohexyl)carbonyl](1-methylethyl)amino]-1-[3-(methyloxy)phenyl]-1H-pyrazole-4-carboxylate

### Intermediate 28

### Ethyl 3-[[(trans-4-methylcyclohexyl)carbonyl](1-methylethyl)amino]-1-[4-(methylsulfonyl)phenyl]-1H-pyrazole-4-carboxylate

### Intermediate 29

### Ethyl 1-(2-fluorophenyl)-3-[[(trans-4-methylcyclohexyl)carbonyl](1-methylethyl)amino]-1H-pyrazole-4-carboxylate

### Intermediate 30

### Ethyl 1-(3-hydroxyphenyl)-3-[[(trans-4-methylcyclohexyl)carbonyl](1-methylethyl)amino]-1H-pyrazole-4-carboxylate

### Intermediate 31

### Ethyl 3-[[(trans-4-methylcyclohexyl)carbonyl](1-methylethyl)amino]-1-(3-methylphenyl)-1H-pyrazole-4-carboxylate

### Intermediate 32

### Ethyl 1-(3-fluorophenyl)-3-[[(trans-4-methylcyclohexyl)carbonyl](1-methylethyl)amino]-1H-pyrazole-4-carboxylate

### Intermediate 33

### Ethyl 1-(4-aminophenyl)-3-[[(trans-4-methylcyclohexyl)carbonyl](1-methylethyl)amino]-1H-pyrazole-4-carboxylate

### Intermediate 34

### Ethyl1-(3-chlorophenyl)-3-[[trans-4-methylcyclohexyl)carbonyl](1-methylethyl)amino]-1H-pyrazole-4-carboxylate

### Intermediate 35

### Ethyl 3-[[(trans-4-methylcyclohexyl)carbonyl](1-methylethyl)amino]-1-{3-[(trifluoromethyl)oxy]phenyl}-1H-pyrazole-4-carboxylate

### Intermediate 36

### Ethyl 1-(4-chloro-3-fluorophenyl)-3-[[(trans-4-methylcyclohexyl)carbonyl](1-methylethyl)amino]-1H-pyrazole-4-carboxylate

### Intermediate 37

### Ethyl 1-(4-chloro-3-methylphenyl)-3-[[(trans-4-methylcyclohexyl)carbonyl(1-methylethyl)amino]-1H-pyrazole-4-carboxylate

### Intermediate 38

### Ethyl 1-(3-amino-4-methylphenyl)-3-[[(trans-4-methylcyclohexyl)carbonyl](1-methylethyl)amino]-1H-pyrazole-4-carboxylate

### Intermediate 39

### Ethyl 1-(3-fluoro-4-methylphenyl)-3-[[(trans-4-methylcyclohexyl)carbonyl](1-methylethyl)amino]-1H-pyrazole-4-carboxylate

### Intermediate 40

### Ethyl 1-(3,4-difluorophenyl)-3-[[(trans-4-methylcyclohexyl)carbonyl](1-methylethyl)amino]-1H-pyrazole-4-carboxylate

### Intermediate 41

### Ethyl 1-[(E)-1-hexen-1-yl]-3-[[(trans-4-methylcyclohexyl)carbonyl](1-methylethyl)amino]-1H-pyrazole-4-carboxylate

### Intermediate 42

### Ethyl 1-[(E)-2-cyclohexylethenyl]-3-[[(trans-4-methylcyclohexyl)carbonyl](1-methylethyl)amino]-1H-pyrazole-4-carboxylate

### Intermediate 43

### Ethyl 3-[[(trans-4-methylcyclohexyl)carbonyl](1-methylethyl)amino]-1-[(E)-4-methyl-1-penten-1-yl]-1H-pyrazole-4-carboxylate

### Intermediate 44

### Ethyl 1-[(E)-2-(4-fluorophenyl)ethenyl]-3-[[(trans-4-methylcyclohexyl)carbonyl(1-methylethyl)amino]-1H-pyrazole-4-carboxylate

### Intermediate 45

### Ethyl 1-(4-ethenylphenyl)-3-[[(trans-4-methylcyclohexyl)carbonyl](1-methylethyl)amino]-1H-pyrazole-4-carboxylate

### Intermediate 46

### Ethyl 1-[4-(hydroxymethyl)phenyl]-3-[[(trans-4-methylcyclohexyl)carbonyl](1-methylethyl)amino]-1H-pyrazole-4-carboxylate

### Intermediate 47

### Ethyl 1-(4-ethylphenyl)-3-[[(trans-4-methylcyclohexyl)carbonyl](1-methylethyl)amino]-1H-pyrazole-4-carboxylate

### Intermediate 48

### Ethyl 3-[[(trans-4-methylcyclohexyl)carbonyl](1-methylethyl)amino]-1-[4-(1-methylethyl)phenyl]-1H-pyrazole-4-carboxylate

### Intermediate 49

### Ethyl 3-[[(trans-4-methylcyclohexyl)carbonyl] (1-methylethyl)amino]-1-{4-[(phenylmethyl)oxy]phenyl}-1 H-pyrazole-4-carboxylate

### Intermediate 50

### Ethyl 1-(1H-indol-5-yl)-3-[[(trans-4-methylcyclohexyl)carbonyl](1-methylethyl)amino]-1 H-pyrazole-4-carboxylate

### Intermediate 51

### Ethyl 1-(4-formylphenyl)-3-[[(trans-4-methylcyclohexyl)carbonyl](1-methylethyl)amino]-1H-pyrazole-4-carboxylate

This compound was purified by Biotage silica flash column, eluting with ethyl acetate:cyclohexane (20:80) to give the title compound.

### Intermediate 52

### Ethyl 1-(1-cyclohexen-1-yl)-3-[[(trans-4-methylcyclohexyl)carbonyl](1-methylethyl)amino]-1H-pyrazole-4-carboxylate

This compound was purified by partition between DCM and saturated sodium bicarbonate, the organics washed with citric acid solution, passed through a hydrophobic frit and concentrated. The residue was purified by chromatography on a 10 g silica SPE, eluting with a gradient of ethyl acetate in DCM until the title compound was eluted.

### Intermediate 53

### Ethyl 3-[[(trans-4-methylcyclohexyl)carbonyl](1-methylethyl)amino]-1-[(E)-2-phenylethenyl]-1H-pyrazole-4-carboxylate

This compound was purified by partition between DCM and saturated sodium bicarbonate, the organics washed with citric acid solution, passed through a hydrophobic frit and concentrated. The residue was purified by chromatography on a 10g silica SPE, eluting with an ethyl acetate in DCM gradient until title compound was isolated.

### Intermediate 54

### Ethyl 1-(4-phenoxyphenyl)-3-[[(trans-4-methylcyclohexyl)carbonyl](1-methylethyl)amino]-1H-pyrazole-4-carboxylate

### Intermediate 55

### Ethyl 1-(3-chloro-4-benzyloxyphenyl)-3-[[(trans-4-methylcyclohexyl)carbonyl](1-methylethyl)amino]-1H-pyrazole-4-carboxylate

### Intermediate 56

### Ethyl 1-(4-tert-butytoxycarbonylaminophenyl)-3-[[(trans-4-methylcyclohexyl)carbonyl](1-methylethyl)amino]-1H-pyrazole-4-carboxylate

### Intermediate 57

### Ethyl 1-(4-tert-butyloxycarbonylphenyl)-3-[[(trans-4-methylcyclohexyl)carbonyl](1-methylethyl)amino]-1H-pyrazole-4-carboxylate

### Intermediate 58

### Ethyl 1-((E)-2-tert-butyl-ethenyl)-3-[[(trans-4-methylcyclohexyl)carbonyl](1-methylethyl)amino]-1H-pyrazole-4-carboxylate

### Intermediate 59

### Ethyl 1-(4-fluorophenyl)-3-[[(trans-4-methylcyclohexyl)carbonyl](1-methylethyl)amino]-1H-pyrazole-4-carboxylate

### Intermediate 60

### Ethyl1-(4-(phenylaminocarbonyl)phenyl)-3-[[(trans-4-methylcyclohexyl)carbonyl](1-methylethyl)amino]-1H-pyrazole-4-carboxylate

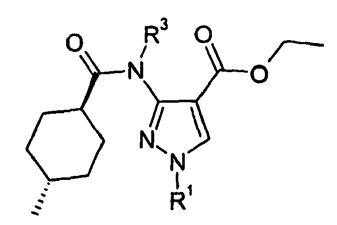

| Intermediate Number | Pre-Cursor Intermediate Number | Structure | | Molecular Formula | Mass Spectrometry | |
|---|---|---|---|---|---|---|
| | | R¹ | R³ | | (M+H)⁺ Calcd | (M+H)⁺ Found |
| **7** | 5 | 4-OH-Ph | iPr | C₂₃H₃₁N₃O₄ | 414 | 414 |
| **8** | 5 | 4-CF₃-Ph | iPr | C₂₄H₃₀F₃N₃O₃ | 466 | 466 |
| **9** | 5 | 4-NHAc-Ph | iPr | C₂₅H₃₄N₄O₄ | 455 | 455 |
| **10** | 5 | 4-Ph-Ph | iPr | C₂₉H₃₅N₃O₃ | 474 | 474 |
| **11** | 5 | 4-NMe₂-Ph | iPr | C₂₅H₃₆N₄O₃ | 441 | 441 |
| **12** | 5 | 4-OMe-Ph | iPr | C₂₄H₃₃N₃O₄ | 428 | 428 |
| **13** | 5 | 4-Ac-Ph | iPr | C₂₅H₃₃N₃O₄ | 440 | 440 |
| **14** | 5 | 4-OCF₃-Ph | iPr | C₂₄H₃₀F₃N₃O₄ | 482 | 482 |
| **15** | 5 | 4-CN-Ph | iPr | C₂₄H₃₀N₄O₃ | 423 | 423 |
| **16** | 5 | 4-CONMe₂-Ph | iPr | C₂₆H₃₆N₄O₄ | 469 | 469 |
| **17** | 5 | 3-thienyl | iPr | C₂₁H₂₉N₃O₃S | 404 | 404 |
| **18** | 5 | 3-CF₃-Ph | iPr | C₂₄H₃₀F₃N₃O₃ | 466 | 466 |
| **19** | 5 | 3,5-di-Me-Ph | iPr | C₂₅H₃₅N₃O₃ | 426 | 426 |
| **20** | 5 | 3-Cl-5-F-Ph | iPr | C₂₃H₂₉ClFN₃O₃ | 450 | 450 |
| **21** | 5 | 3,5-di-CF₃-Ph | iPr | C₂₅H₂₉F₆N₃O₃ | 534 | 534 |
| **22** | 5 | 3,4-benzodioxol-5-yl | iPr | C₂₄H₃₁N₃O₅ | 442 | 442 |
| **23** | 5 | 2,3-dihydro-1- | iPr | C₂₅H₃₃N₃O₄ | 440 | 440 |
| | | benzofuran-5-yl | | | | |
| **24** | 5 | 2,3-dihydro-1,4- | iPr | C₂₅H₃₃N₃O₅ | 456 | 456 |
| **25** | 5 | benzodioxin-6-yl 3,4,5-tri-F-Ph | iPr | C₂₃H₂₈F₃N₃O₃ | 452 | 452 |
| **26** | 5 | 4-Cl-Ph | iPr | C₂₃H₃₀CIN₃O₃ | 432/4 | 432/4 |
| **27** | 5 | 3-OMe-Ph | iPr | C₂₄H₃₃N₃O₄ | 428 | 428 |
| **28** | 5 | 4-MeSO₂-Ph | iPr | C₂₄H₃₃N₃O₅S | 476 | 476 |
| **29** | 5 | 2-F-Ph | iPr | C₂₃H₃₀FN₃O₃ | 412 | 412 |
| **30** | 5 | 3-OH-Ph | iPr | C₂₃H₃₁N₃O₄ | 414 | 414 |
| **31** | 5 | 3-Me-Ph | iPr | C₂₄H₃₃N₃O₃ | 412 | 412 |
| **32** | 5 | 3-F-Ph | iPr | C₂₃H₃₀FN₃O₃ | 416 | 416 |
| **33** | 5 | 4-NH₂-Ph | iPr | C₂₃H₃₂N₄O₃ | 413 | 413 |
| **34** | 5 | 3-Cl-Ph | iPr | C₂₃H₃₀CIN₃O₃ | 432/4 | 432/4 |
| **35** | 5 | 3-OCF₃-Ph | iPr | C₂₄H₃₀F₃N₃O₄ | 482 | 482 |
| **36** | 5 | 3-F-4-Cl-Ph | iPr | C₂₃H₂₉ClFN₃O₃ | 450/2 | 450/2 |
| **37** | 5 | 3-Me-4-Cl-Ph | iPr | C₂₄H₃₂ClN₃O₃ | 446/8 | 446/8 |
| **38** | 5 | 3-NH₂-4-Me-Ph | iPr | C₂₄H₃₄N₄O₃ | 427 | 427 |
| **39** | 5 | 3-F-4-Me-Ph | iPr | C₂₄H₃₂FN₃O₃ | 430 | 430 |
| **40** | 5 | 3,4-di-F-Ph | iPr | C₂₃H₂₉F₂N₃O₃ | 434 | 434 |
| **41** | 5 | (E)-1-hexen-1-yl | iPr | C₂₃H₃₇N₃O₃ | 404 | 404 |
| **42** | 5 | (E)-2- | iPr | C₂₅H₃₉N₃O₃ | 430 | 430 |
| | | cyclohexylethenyl | | | | |
| **43** | 5 | (E)-4-methyl-1- | iPr | C₂₃H₃₇N₃O₃ | 404 | 404 |
| | | penten-1-yl | | | | |
| **44** | 5 | (E)-2-(a- | iPr | C₂₅H₃₂FN₃O₃ | 442 | 442 |
| | | fluorophenyl)ethenyl | | | | |
| **45** | 5 | 4-ethenyl-phenyl | iPr | C₂₅H₃₃N₃O₃ | 424 | 424 |
| **46** | 5 | 4-CH₂OH-Ph | iPr | C₂₄H₃₃N₃O₄ | 428 | 428 |
| **47** | 5 | 4-Et-Ph | iPr | C₂₅H₃₅N₃O₃ | 426 | 426 |
| **48** | 5 | 4-iPr-Ph | iPr | C₂₆H₃₇N₃O₃ | 440 | 440 |
| **49** | 5 | 4-BnO-Ph | iPr | C₃₀H₃₇N₃O₄ | 504 | 504 |
| **50** | 5 | 5-indolyl | iPr | C₂₅H₃₂N₄O₃ | 437 | 437 |
| **51** | 5 | 4-formyl-Ph | iPr | C₂₄H₃₁N₃O₄ | 426 | 426 |
| **52** | 5 | 1-cyclohexen-1-yl | iPr | C₂₃H₃₅N₃O₃ | 402 | 402 |
| **53** | 5 | (E)-2-phenylethenyl | iPr | C₂₅H₃₃N₃O₃ | 424 | 424 |
| **54** | 5 | 4-PhO-Ph | iPr | C₂₉H₃₅N₃O₄ | 490 | 490 |
| **55** | 5 | 3-Chloro-4-BnO-Ph | iPr | C₃₀H₃₈CIN₃O₄ | 539/41 | 539/41 |
| **56** | 5 | 4- tBuOCO NH-Ph | iPr | C₂₈H₄₀N₄O₅ | 513 | 513 |
| **57** | 5 | 4-tBuOCO-Ph | iPr | C₂₈H₃₉N₃O₅ | 498 | 498 |
| **58** | 5 | (E)-2-tert- | iPr | C₂₅H₃₃N₃O₃ | 376 | 376 |
| | | butylethenyl | | | | |
| **59** | 5 | 4-F-Ph | iPr | C₂₃H₃₀FN₃O₃ | 416 | 416 |
| **60** | 5 | 4-PhNHCO-Ph | iPr | C₃₀H₃₆N₄O₃ | 517 | 517 |

### Intermediate 61

### Ethyl 1-(5-acetyl-2-thienyl)-3-[[(trans-4-methylcyclohexyl)carbonyl](1-methylethyl)amino]-1H-pyrazole-4-carboxylate

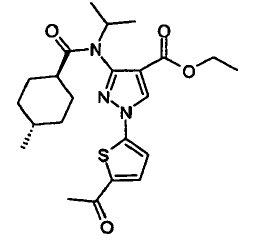

To Intermediate 5 (100 mg, 0.31 mmol) in dry 1,4-dioxane (0.3 mL) was added 2-bromo-5-acetylthiophene (51 mg, 0.25 mmol), potassium carbonate (115 mg, 0.54 mmol), copper (I) iodide (4.9 mg, 0.02 mmol) and trans-1,2-diaminocyclohexane (5.8 mg, 0.05 mmol). The reaction was stirred at 110°C overnight, cooled, partitioned between DCM and 2N hydrochloric acid, passed through a hydrophobic frit and the organics concentrated. The crude product was purified by MDAP HPLC to give the title compound.
MS calcd for (C₂₃H₃₁N₃O₃S + H)⁺: 446
MS found (electrospray): (M+H)⁺= 446

The following compounds were made from the corresponding aryl halides by a similar procedure to that described for Intermediate 61:

### Intermediate 62

### Ethyl 1-(5-chloro-2-thienyl)-3-[[(trans-4-methylcyclohexyl)carbonyl](1-methylethyl)amino]-1H-pyrazole-4-carboxylate

### Intermediate 63

### Ethyl 3-[[(trans-4-methylcyclohexyl)carbonyl](1-methylethyl)amino]-1-(5-methyl-2-thienyl)-1H-pyrazole-4-carboxylate

### Intermediate 64

### Ethyl 3-[[(trans-4-methylcyclohexyl)carbonyl](1-methylethyl)amino]-1-(5-phenyl-2-thienyl)-1H-pyrazole-4-carboxylate

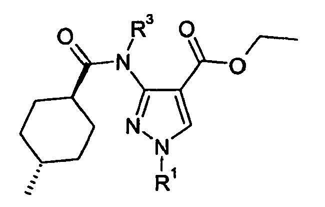

| Intermediate Number | Pre-Cursor Intermediate Number | Structure | | Molecular Formula | Mass Spectrometry | |
|---|---|---|---|---|---|---|
| | | R¹ | R³ | | (M+H)⁺ Calcd | (M+H)⁺ Found |
| **62** | 5 | 5-Cl-2-thienyl | iPr | C₂₁H₂₈CIN₃O₃S | 438 | 438 |
| **63** | 5 | 5-Me-2-thienyl | iPr | C₂₂H₃₁N₃O₃S | 418 | 418 |
| **64** | 5 | 5-Ph-2-thienyl | iPr | C₂₇H₃₃N₃O₃S | 480 | 480 |

### Intermediate 65

### 3-[[(trans-4-Methylcyclohexyl)carbonyl](1-methylethyl)amino]-1-(phenylmethyl)-1H-pyrazole-4-carboxylic acid

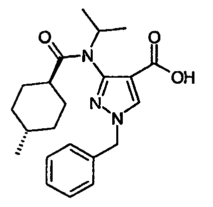

To Intermediate 4 (0.47 g, 1.14 mmol) was added THF (5 ml), ethanol (5 ml) and 2M lithium hydroxide (5 mL). The reaction was stirred at room temperature for 16 hours. The reaction mixture was partitioned between ethyl acetate and 2N hydrochloric acid. The organic layer was separated and washed with brine, dried over sodium sulphate and concentrated to give the title compound.
MS calcd for (C₂₂H₂₉N₃O₃ + H)⁺: 384
MS found (electrospray): (M+H)⁺ = 384

### Intermediate 66

### 3-[[(trans-4-Methylcyclohexyl)carbonyl](1-methylethyl)amino]-1H-pyrazole-4-carboxylic acid

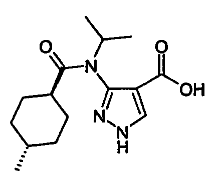

Intermediate 65 (0.44 g, 1.13 mmol) in ethanol (40 mL) and c.hydrochloric acid (0.4 mL) was subjected to atmospheric pressure hydrogenation with 10% palladium on carbon (0.12 g, 30 wt% wet) for 16 hrs. The reaction was filtered through a Celite pad, the pad was washed with ethanol and the filtrate concentrated to give the title compound.
MS calcd for (C₁₅H₂₃N₃O₃ + H)⁺: 294
MS found (electrospray): (M+H)⁺= 294

### Intermediate 67

### Ethyl 3-[(cyclohexylacetyl)(1-methylethyl)amino]-1-phenyl-1H-pyrazole-4-carboxylate

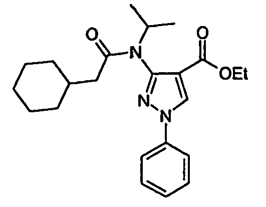

To a mixture of Intermediate 1 (200 mg, 0.86 mmol) in dry DCM (2 mL) was added triphenylphosphine (338 mg, 1.30 mmol) and cyclohexaneacetyl chloride (209 mg, 1.30 mmol). The reaction was stirred overnight at 45°C, then further cyclohexaneacetyl chloride (69 mg, 0.43 mmol) was added and heating continued for 16 hrs. The reaction was cooled, partitioned between DCM and saturated sodium bicarbonate, passed through a hydrophobic frit and organics concentrated. This was purified by 20 g silica SPE cartridge, eluted with 100% cyclohexane then 5% ethyl acetate increments in cyclohexane until the title compound was isolated.
MS calcd for (C₂₃H₃₁N₃O₃ + H)⁺: 398
MS found (electrospray): (M+H)⁺ = 398

### Intermediate 68

### Ethyl 3-{(1-methylethyl)[(4-methylphenyl)carbonyl]amino}-1-phenyl-1H-pyrazole-4-carboxylate

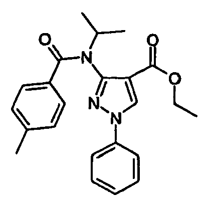

To a mixture of Intermediate 1 (200 mg, 0.86 mmol) in dry pyridine (2 mL) was added 4-methylbenzoyl chloride (201 mg, 1.30 mmol). The reaction was stirred overnight at room temperature, then further 4-methylbenzoyl chloride (67 mg, 0.43 mmol) added and the reaction refluxed overnight. The reaction was cooled, partitioned between DCM and 2N hydrochloric acid, passed through a hydrophobic frit and organics concentrated. This was purified by 10 g silica SPE cartridge, eluted with 100% cyclohexane then 5% ethyl acetate increments in cyclohexane until the title compound was isolated.
MS calcd for (C₂₃H₂₅N₃O₃ + H)⁺: 392
MS found (electrospray): (M+H)⁺= 392

### Intermediate 69

### Ethyl 3-[[(4-bromo-2-chlorophenyl)carbonyl](1-methylethyl)amino]-1-phenyl-1H-pyrazole-4-carboxylate

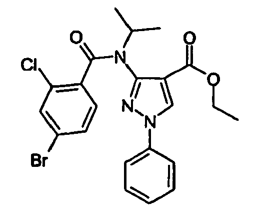

Prepared by a similar method to that described for Intermediate 68 replacing 4 methylbenzoyl chloride with 4-bromo-2-chlorobenzoyl chloride to give the title compound.
MS calcd for (C₂₂H₂₁BrClN₃O₃ + H)⁺: 490/492
MS found (electrospray): (M+H)⁺= 490/492

### Intermediate 70

### Ethyl 1-phenyl-3-(phenylamino)-1H-pyrazole-4-carboxylate

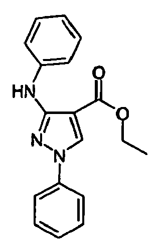

To ethyl 3-amino-1-phenyl-1*H*-pyrazole-4-carboxylate (1 g, 4.32 mmol) in dry DCM (50 mL) was added phenyl boronic acid (1.05 g, 8.64 mmol), pyridine (683 mg, 8.64 mmol) and copper (II) acetate (1.18 g, 6.43 mmol). The reaction was stirred at room temperature over 60 hours. The solvent removed and the residue purified by ISCO companion silica chromatography, eluted with a gradient of ethyl acetate in cyclohexane, to give the title compound.
MS calcd for (C₁₈H₁₇N₃O₂ + H)⁺: 308
MS found (electrospray): (M+H)⁺ = 308

### Intermediate 71

### Ethyl 3-[[(trans-4-methylcyclohexyl)carbonyl](phenyl)amino]-1-phenyl-1H-pyrazole-4-carboxylate

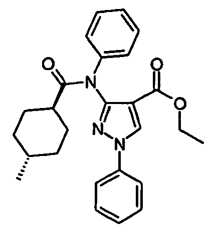

Prepared by a similar method to that described for Intermediate 2 replacing Intermediate 1 with Intermediate 70 to give the title compound.
MS calcd for (C₂₆H₂₉N₃O₃ + H)⁺: 432
MS found (electrospray): (M+H)⁺ = 432

### Intermediate 72

### Ethyl 3-([(trans-4-methylcyclohexyl)carbonyl]amino)-1-phenyl-1H-pyrazole-4-carboxylate

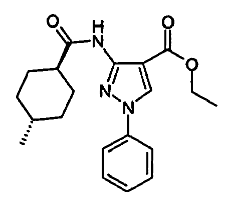

To ethyl 3-amino-1-phenyl-1*H*-pyrazole-4-carboxylate (500 mg, 2.16 mmol) was added dry DCM (10 mL), *trans*-4-methylcyclohexanecarbonyl chloride (0.37 g, 0.24 mmol) and triethylamine (0.45 mL, 0.33 mmol). The reaction was stirred at reflux overnight. The reaction was quenched with saturated sodium bicarbonate, extracted with DCM, organics dried over sodium sulphate and concentrated. Purified by Biotage silica chromatography, eluted with a gradient of ethyl acetate in DCM to give the title compound.
MS calcd for (C₂₀H₂₅N₃O₃ + H)⁺: 356
MS found (electrospray): (M+H)⁺ = 356

### Intermediate 73

### Ethyl 3-{[2-(dimethylamino)-2-oxoethyl][(trans-4-methylcyclohexyl)carbonyl]amino}-1-phenyl-1H-pyrazole-4-carboxylate

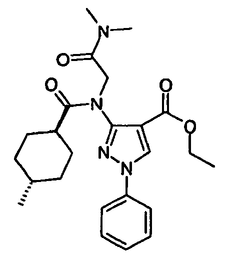

To Intermediate 72 (200 mg, 0.56 mmol) was added dry DMF (3 mL) then sodium hydride, [60% in oil (20 mg, 0.84 mmol)] and stirred for 30 minutes. N,N-dimethylbromoacetamide (155 mg, 0.94 mmol) added and the reaction stirred at room temperature overnight. The solvent was removed, partitioned between DCM and water, passed through a hydrophobic frit and the organic layer concentrated. Purified by ISCO companion silica chromatography using a gradient of ethyl acetate in cyclohexane to give the title compound.
MS calcd for (C₂₄H₃₂N₄O₄ + H)⁺: 441
MS found (electrospray): (M+H)⁺ = 441

### Intermediate 74

### 1,1-Dimethylethyl 4-({4-[(ethytoxy)carbonyl]-1-phenyl-1H-pyrazol-3-yl}amino)-1 - piperidinecarboxylate

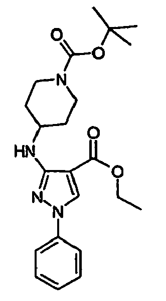

Ethyl 3-amino-1-phenyl-1*H*-pyrazole-4-carboxylate (2.5 g, 10.8 mmol) was dissolved in DCM (60 mL), then 1,1-dimethylethyl 4-oxo-1-piperidinecarboxylate (4.3 g, 21.6 mmol), acetic acid (1.9 g, 32.4 mmol) and then sodium triacetoxyborohydride (4.58 g, 21.6 mmol) added. The reaction was stirred at room temperature over 64 hours, partitioned between DCM and saturated sodium bicarbonate, passed through a hydrophobic frit and the organic layer concentrated. Purified by 120 g silica ISCO companion flash column eluting with a gradient of ethyl acetate in cyclohexane to give the title compound.
MS calcd for (C₂₂H₃₀N₄O₄ + H)⁺: 415
MS found (electrospray): (M+H)⁺= 415

### Intermediate 75

### 1,1-Dimethylethyl 4-({4-[(ethyloxy)carbonyl]-1-phenylmethyl-1H-pyrazol-3-yl}amino)-1-piperidinecarboxylate

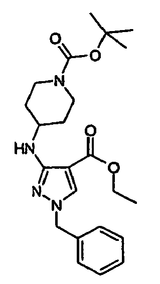

Prepared by a similar method to that described for Intermediate 74 replacing ethyl 3-amino-1-phenyl-1H pyrazole-4-carboxylate with ethyl 3-amino-1-(phenylmethyl)-1*H*-pyrazole-4-carboxylate to give the title compound.
MS calcd for (C₂₃H₃₂N₄O₄ + H)⁺: 429
MS found (electrospray): (M+H)⁺ = 429

### Intermediate 76

### 1,1-Dimethylethyl 4-{{4-[(ethyloxy)carbonyl]-1-phenyl-1H-pyrazol-3-yl}[(trans-4-methylcyclohexyl)carbonyl]amino}-1-piperidinecarboxylate

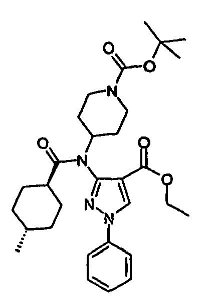

To Intermediate 74 (4.2 g, 0.01 mol) was added dry DCM (70 mL), triethylamine (2.7 mL, 0.02 mol) and *trans*-4-methylcyclohexanecarbonyl chloride (3.2 g, 0.02 mol). The reaction was stirred at 45°C for 7 hours then further *trans*-4-methylcyclohexanecarbonyl chloride (1.6 g, 0.01 mol) added and heated for a further 2 hrs. The reaction was cooled, partitioned between DCM and saturated sodium bicarbonate, passed through a hydrophobic frit and organics concentrated. Purified by ISCO companion silica chromatography using a gradient of ethyl acetate in cyclohexane to give the title compound.
MS calcd for (C₃₀H₄₂N₄O₅ + H)⁺: 539
MS found (electrospray): (M+H)⁺ = 539

### Intermediate 77

### 1,1-Dimethylethyl 4-{{4-[(ethyloxy)carbonyl]-1-phenylmethyl-1H-pyrazol-3-yl}[(trans-4-methylcyclohexyl)carbonyl]amino}-1-piperidinecarboxylate

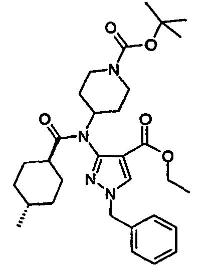

Prepared by a similar method to that described for Intermediate 76 replacing Intermediate 74 with intermediate 75 to give the title compound.
MS calcd for (C₃₁H₄₄N₄O₅ + H)⁺: 553
MS found (electrospray): (M+H)⁺= 553

### Intermediate 78

### Ethyl 3-[[(trans-4-methylcyclohexyl)carbonyl](4-piperidinyl)amino]-1-phenyl-1H-pyrazole-4-carboxylate

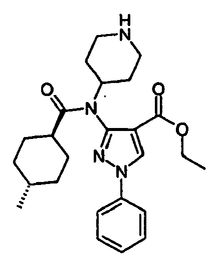

Intermediate 76 (2.7 g, 5.01 mmol) was dissolved in DCM (20 mL) and trifluoroacetic acid (20 mL). The reaction was stirred overnight, solvent removed, partitioned between DCM and saturated sodium bicarbonate and organics concentrated to give the title compound.
MS calcd for (C₂₅H₃₄N₄O₃ + H)⁺: 439
MS found (electrospray): (M+H)⁺= 439

### Intermediate 79

### Ethyl 3-[[(trans-4-methylcyclohexyl)carbonyl](4-piperidinyl)amino]-1-phenylmethyl-1H-pyrazole-4-carboxylate

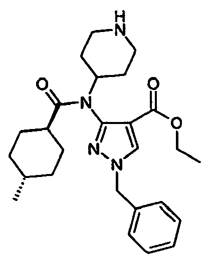

Prepared by a similar method to that described for Intermediate 78 replacing Intermediate 76 with Intermediate 77 to give the title compound.
MS calcd for (C₂₆H₆N₄O₃ + H)⁺: 453
MS found (electrospray): (M+H)⁺ = 453

### Intermediate 80

### Methyl 4-{{4-[(ethyloxy)carbonyl]-1-phenyl-1H-pyrazol-3-yl}[(trans-4-methylcyclohexyl)carbonyl]amino}-1-piperidinecarboxylate

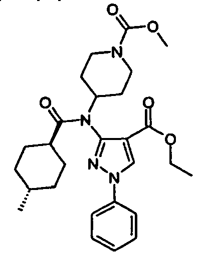

To Intermediate 78 (200 mg, 0.46 mmol) was added DCM (2 mL), triethylamine (0.076 mL, 0.55 mmol) and methyl chloroformate (52 mg, 0.52 mmol). The reaction was stirred at room temperature and then partitioned between DCM and 2N hydrochloric acid, passed through a hydrophobic frit and the organic layer concentrated. Purified by ISCO companion silica chromatography with a gradient of ethyl acetate in cyclohexane to give the title compound.
MS calcd for (C₂₇H₃₆N₄O₅ + H)⁺: 497
MS found (electrospray): (M+H)⁺ = 497

### Intermediate 81

### Ethyl 3-{[(trans-4-methylcyclohexyl)carbonyl][1-(methylsulfonyl)-4-piperidinyl]amino}-1-phenyl-1H-pyrazole-4-carboxylate

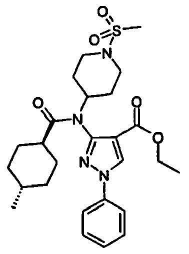

To Intermediate 78 (200 mg, 0.46 mmol) was added DCM (2 mL), triethylamine (0.076 mL, 0.55 mmol) and methanesulfonyl chloride (63 mg, 0.52 mmol). The reaction was stirred at room temperature until complete then partitioned between DCM and 2N hydrochloric acid, passed through a hydrophobic frit and the organic layer concentrated. Purified by ISCO companion silica chromatography with a gradient of ethyl acetate in cyclohexane to give the title compound.
MS calcd for (C₂₆H₃₆N₄O₅S + H)⁺: 517
MS found (electrospray): (M+H)⁺ = 517

### Intermediate 82

### Ethyl 3-{[(trans-4-methylcyclohexyl)carbonyl][1-(methylsulfonyl)-4-piperidinyl]amino}-1-phenylmethyl-1H-pyrazole-4-carboxylate

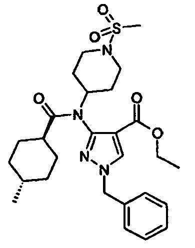

Prepared by a similar method to that described for Intermediate 81 replacing Intermediate 78 with Intermediate 79 to give the title compound.
MS calcd for (C₂₇H₃₈N₄O₅S + H)⁺: 531
MS found (electrospray): (M+H)⁺ = 531

### Intermediate 83

### Ethyl 3-[[(trans-4-methylcyclohexyl)carbonyl](1-methyl-4-piperidinyl)amino]-1-phenyl-1H-pyrazole-4-carboxylate

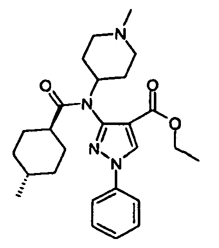

To Intermediate 78 (171 mg, 0.39 mmol) was added methanol (1.2 mL), formic acid (72 mg, 1.56 mmol) and 37% aqueous formaldehyde (0.056 mL, 0.78 mmol). The reaction was heated at 90°C in a reactivial overnight and the solvent removed to give the title compound.
MS calcd for (C₂₆H₃₆N₄O₃ + H)⁺: 453
MS found (electrospray): (M+H)⁺= 453

### Intermediate 84

### Ethyl 3-{{1-[(ethylamino)carbonyl]-4-piperidinyl}[(trans-4-methylcyclohexyl)carbonyl]amino}-1-phenyl-1H-pyrazole-4-carboxylate

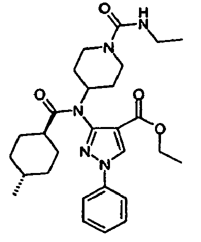

To Intermediate 78 (100 mg, 0.46 mmol) was added DCM (2 mL) and ethyl isocyanate (39 mg, 0.552 mmol). The reaction was stirred at room temperature overnight, partitioned with water, passed through a hydrophobic frit and the organic layer concentrated. Purified by MDAP HPLC to give the title compound.
MS calcd for (C₂₈H₃₉N₅O₄ + H)⁺: 510
MS found (electrospray): (M+H)⁺= 510

### Intermediate 85

### Ethyl 1-phenyl-3-[(2-pyrazinylmethyl)amino]-1H-pyrazole-4-carboxylate

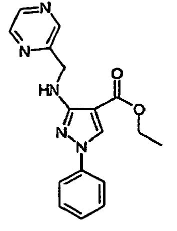

To ethyl 3-amino-1-phenyl-1*H*-pyrazole-4-carboxylate (250 mg, 1.08 mmol) was added DCM (6 mL), pyrazine-2-carboxaldehyde (233 mg, 2.16 mmol), acetic acid (0.18 mL, 3.24 mmol) and sodium triacetoxyborohydride (0.46 g, 2.16 mmol). The reaction was stirred overnight at room temperature, partitioned between DCM and water, passed through a hydrophobic frit and the organic layer concentrated. Purified by MDAP HPLC to give the title compound.
MS calcd for (C₁₇H₁₇N₅O₂ + H)⁺: 324
MS found (electrospray): (M+H)⁺ = 324

### Intermediate 86

### Ethyl 3-[[(trans-4-methylcyclohexyl)carbonyl](2-pyrazinylmethyl)amino]-1-phenyl-1H-pyrazole-4-carboxylate

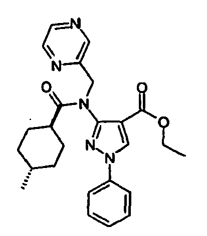

Prepared by a similar method to that described for Intermediate 76 replacing Intermediate 74 with Intermediate 85 to give the title compound.
MS calcd for (C₂₅H₂₉N₅O₃ + H)⁺: 448
MS found (electrospray): (M+H)⁺ = 448

### Intermediate 87

### Rel-Ethyl 3-[{[(1S,2R,4S)-2-(acetyloxy)-4-methylcyclohexyl]carbonyl}(1-methylethyl)amino]-1-phenyl-1H-pyrazole-4-carboxylate

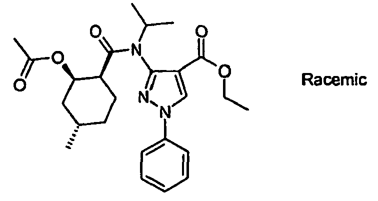

Prepared similarly to the procedure described for Intermediate 2 from Intermediate 1 and *rel-*(1R, 2S, 4R)-2-acetoxy-4-methyl-cyclohexane carboxylic acid chloride² to give the title compound.
MS calcd for (C₂₅H₃₃N₃O₅ + H)⁺: 456
MS found (electrospray): (M+H)⁺ = 456
2. WO2004/052885

### Intermediate 88

### 4-Oxocyclohexanecarbonyl chloride

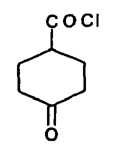

4-0xocyclohexane carboxylic acid (2.4 g, 16 mmol) was dissolved in DCM (30 mL) and treated with oxalyl chloride (2.95 mL, 33.8 mmol). Diethylformamide (1 drop) was added and the mixture stirred at room temperature for 18 hrs. The reaction mixture was concentrated *in vacuo* to give the title compound as an oil. This was used in the next step without further purification.

### Intermediate 89

### Ethyl 3-{(1-methylethyl)[(4-oxocyclohexyl)carbonyl]amino}-1-phenyl-1H-pyrazole-4-carboxylate

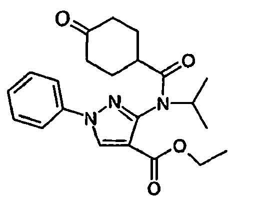

Prepared similarly to the procedure described for Intermediate 2 using Intermediate 1 and Intermediate 88 to give the title compound.
MS calcd for (C₂₂H₂₇N₃O₄ + H)⁺: 398
MS found (electrospray): (M+H)⁺= 398

### Intermediate 90

### Ethyl 3-{(1-methylethyl)[(4-methylidenecyclohexyl)carbonyl]amino}-1-phenyl-1H-pyrazole-4-carboxylate

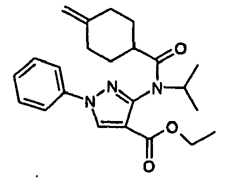

Methyltriphenylphosphonium bromide (540 mg) was dissolved in THF (6 mL) under nitrogen and treated with potassium *tert*-butoxide (1M in THF, 1.35 mL). After 15 minutes a solution of Intermediate 89 (300 mg) in THF (4 mL) was added and the mixture stirred at room temperature for 2 hrs. The reaction was quenched with water and extracted with ethyl acetate. The combined organic extracts were washed with brine, dried (Na₂SO₄) and concentrated to give a gum. This was purified on a silica column eluting with ethyl acetate : cyclohexane (stepped gradient 10:90 to 20:80) to give the title compound.
MS calcd for (C₂₃H₂₉N₃O₃ + H)⁺: 396
MS found (electrospray): (M+H)⁺= 396

### Intermediate 91

### Ethyl 3-[[(trans-4-methylcyclohexyl)carbonyl](1-methylethyl)amino]-1-{4-[(E/Z)-2-phenylethenyl]phenyl}-1H-pyrazole-4-carboxylate

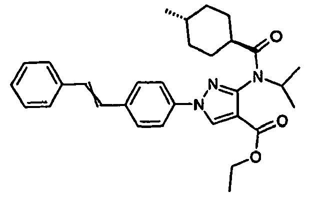

To benzyl triphenylphosphonium bromide (549 mg, 1.4 mmol) was added THF (6 mL) and 1M potassium t-butoxide in THF (1.26 mL, 1.26 mmol). The reaction was stirred for 10 mins then Intermediate 51 (300 mg, 0.7 mmol) in THF (3 mL) added and stirred at room temperature for 2 hrs. Saturated ammonium chloride was added and the mixture extracted with ethyl acetate. The organic layer was washed with brine, dried over sodium sulphate and concentrated. Purified by 20 g silica SPE, loaded in DCM, eluted with ethyl acetate:cyclohexane (1:9) until the title compound was isolated as a mixture of *cis* and *trans* isomers.
MS calcd for (C₃₁H₃₇N₃O₃ + H)⁺: 500
MS found (electrospray): (M+H)⁺ = 500

### Intermediate 92

### Ethyl 3-[[(trans-4-methylcyclohexyl)carbonyl](1-methylethyl)amino]-1-{4-[(E/Z)-2-cyclohexylethenyl]phenyl}-1H-pyrazole-4-carboxylate

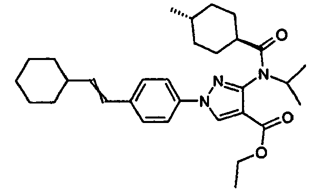

To cyclohexylmethyl triphenylphosphonium chloride (383 mg, 0.96 mmol) in THF (1.5 mL) under nitrogen at 0 °C was added n-BuLi (0.63 mL, 1.6 M solution in hexane) and the mixture stirred for one minute when a solution was obtained. A solution of Intermediate 51 (408 mg, 0.96 mmol) in THF (1.5 mL) was added and the mixture stirred for one hour at 0 C. Saturated ammonium chloride was added and the mixture extracted with ethyl acetate. The organic layer was washed with brine, dried over sodium sulphate and concentrated. Purified by 50 g silica SPE, eluted with cyclohexane, then a stepwise gradient of ethyl acetate:cyclohexane (1:9, 2:8, 3;7, etc) until the title compound was isolated as a mixture of *cis* and *trans* isomers.
MS calcd for (C₃₁H₄₃N₃O₃ + H)⁺: 506
MS found (electrospray): (M+H)⁺ = 506

### Intermediate 93

### Ethyl 3-[[(trans-4-methylcyclohexyl)carbonyl](1-methylethyl)amino]-1-(4-[(E/Z)-2-thiazole-ethenyl]phenyl}-1H-pyrazole-4-carboxylate

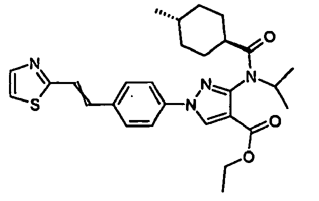

Similarly prepared by the procedure described for Intermediate 91 using 2-thiazolemethyl triphenylphosphonium chloride, and purified using a silica SPE cartridge, eluted with cyclohexane, then a stepwise gradient of ethyl acetate:cyclohexane (1:9, 2:8, 3;7, etc) until the title compound was isolated as a mixture of cis and *trans* isomers.
MS calcd for (C₂₈H₃₄N₄O₃S + H)⁺: 507
MS found (electrospray): (M+H)⁺= 507

### Intermediate 94

### Ethyl 3-[[(trans-4-methylcyclohexyl)carbonyl](1-methylethyl)amino]-1-{4-[(E/Z)-2-phenyl-2-methyl-ethenyl]phenyl}-1H-pyrazole-4-carboxylate

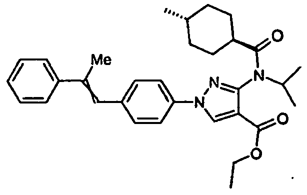

Similarly prepared by the procedure described for Intermediate 91 using diethyl 1-phenethyl phosphonate. Purification was by ISCO Companion silica chromatography, eluted with ethyl acetate:cyclohexane (1:9 then 2:8), to give the title compound as a mixture of *cis* and *trans* isomers.
MS calcd for (C₃₂H₃₉N₃O₃ + H)⁺: 514
MS found (electrospray): (M+H)⁺= 514

### Intermediate 95

### Ethyl 3-[[(trans-4-methylcyclohexyl)carbonyl](1-methylethyl)amino]-1-{4-[(E/Z)-2-(4-pyridyl)-ethenyl]phenyl}-1H-pyrazole-4-carboxylate

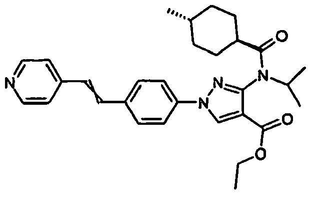

Similarly prepared by the procedure described for Intermediate 91 using 4-pyridylmethyl triphenylphosphonium chloride. Purification was by MDAP HPLC, to give the title compound as a mixture of *cis* and *trans* isomers.
MS calcd for (C₃₀H₃₆N₄O₃ + H)⁺: 501
MS found (electrospray): (M+H)⁺ = 501

### Intermediate 96

### Ethyl 3-[[(trans-4-methylcyclohexyl)carbonyl](1-methylethyl)amino]-1-{4-[(E/Z)-2-(4-thiazolyl)-ethenyl]phenyl}-1H-pyrazole-4-carboxylate

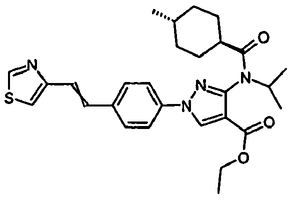

Similarly prepared by the procedure described for Intermediate 91 using 4-thiazolylmethyl triphenylphosphonium chloride. Purification was by SPE silica cartridge, eluted with ethyl acetate:hexane (20:80), to give the title compound as a mixture of *cis* and *trans* isomers.
MS calcd for (C₂₈H₃₄N₄O₃S + H)⁺: 507
MS found (electrospray): (M+H)⁺ = 507

### Intermediate 97

### Ethyl 3-[[(trans-4-methylcyclohexyl)carbonyl](1-methylethyl)amino]-1-{4-[(E/Z)-2-(2-pyridyl)-ethenyl]phenyl}-1H-pyrazole-4-carboxylate

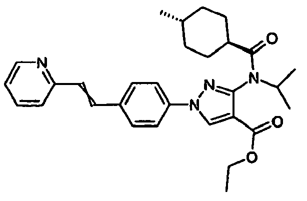

Similarly prepared by the procedure described for Intermediate 91 using 2-pyridylmethyl triphenylphosphonium chloride (hydrochloride salt). Purification was by ISCO Companion silica chromatography, eluted with ethyl acetate:cyclohexane (1:9 then 2:8), to give the title compound as a mixture of *cis* and *trans* isomers.
MS calcd for (C₃₀H₃₈N₄O₃ + H)⁺: 501
MS found (electrospray): (M+H)⁺ = 501

### Intermediate 98

### Ethyl 3-[[(trans-4-methylcyclohexyl)carbonyl](1-methylethyl)amino]-1-{4-[(E/Z)-2-(2-methyl-4-thiazolyl)-ethenyl]phenyl}-1H-pyrazole-4-carboxylate

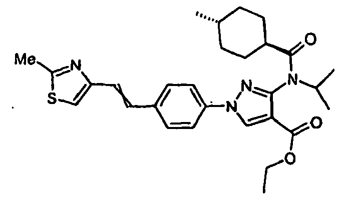

Similarly prepared by the procedure described for Intermediate 91 using 2-methyl-4-thiazolemethyl triphenylphosphonium chloride. Purification was by SPE silica cartridge, eluted with a gradient of ethyl acetate:cyclohexane, to give the title compound as a mixture of *cis* and *trans* isomers (ratio ∼ 1:4). Data for the *trans* isomer given.
¹H NMR (CDCl₃) δ 8.55 (1H, s), 7.71 (2H, d), 4.68 (1H, m), 7.65 (2H, d), 7.48 (1H, d), 7.10 (1H, d), 7.09 (1H, s), 4.95 (1H, m), 4.30 (2H, m), 2.18 (2H, br), 2.80 (3H, s), 1.95 (1H, m), 1.9-0.5 (21H, m).

### Intermediate 99

### Ethyl 3-[[(trans-4-methylcyclohexyl)carbonyl](tetrahydro-2H-pyran-4-yl)amino]-1-{4-[(E/Z)-2-phenylethenyl]phenyl}-1H-pyrazole-4-carboxylate

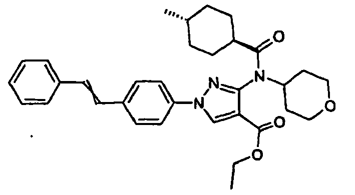

Similarly prepared by the procedure described for Intermediate 91 using benzyl triphenylphosphonium chloride and Intermediate 145. Purification was by ISCO Companion silica chromatography, eluted with a gradient of ethyl acetate:cyclohexane (0% to 50%), to give the title compound as a mixture of cis and *trans* isomers.
MS calcd for (C₃₃H₃₉N₃O₄ + H)⁺: 542
MS found (electrospray): (M+H)⁺ = 542

### Intermediate 100

### Ethyl 3-[[(trans-4-methylcyclohexyl)carbonyl](tetrahydro-2H-pyran-4-yl)amino]-1-{4-[(E/Z)-2-(4-thiazolyl)-ethenyl]phenyl}-1H-pyrazole-4-carboxylate

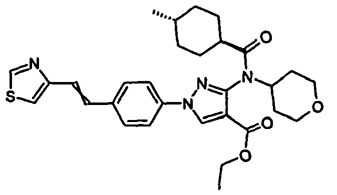

Similarly prepared by the procedure described for Intermediate 91 using 4-thiazolemethyl triphenylphosphonium chloride and Intermediate 145. Purification was by ISCO Companion silica chromatography, eluted with a gradient of ethyl acetate:cyclohexane (5% to 50%), to give the title compound as a mixture of *cis* and *trans* isomers.
MS calcd for (C₃₀H₃₆N₄O₄S + H)⁺: 549
MS found (electrospray): (M+H)⁺= 549

### Intermediate 101

### Ethyl 3-[[(trans-4-methylcyclohexyl)carbonyl](1-methylethyl)amino]-1-{4-hydroxymethyl-phenyl}-1H-pyrazole-4-carboxylate

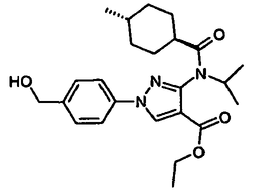

To a solution of Intermediate 51 (534 mg, 1.26 mmol) in ethanol (10 mL) was added sodium borohydride (72 mg) and the mixture stirred at room temperature for 30 minutes. The reaction was quenched with dilute aqueous hydrochloric acid and the solvent removed in vacuo. The residue was partitioned between ethyl acetate and dilute HCl (2N), the organic layer washed with water, dried (Na₂SO₄) and concentrated to give the title compound.
MS calcd for (C₂₄H₃₃N₃O₄ + H)⁺: 428
MS found (electrospray): (M+H)⁺ = 428

### Intermediate 102

### Ethyl 3-[[(trans-4-methylcyclohexyl)carbonyl](1-methylethyl)amino]-1-{4-iodomethyl-phenyl}-1H-pyrazole-4-carboxylate

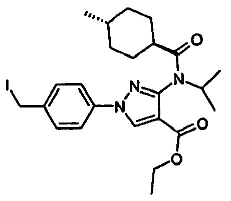

To a solution of Intermediate 101 (515 mg, 1.2 mmol) in THF (10 mL) was added triphenyl phosphine (316 mg, 1.2 mmol) and imidazole (106 mg, 1.56 mmol), followed by iodine (305 mg, 1.2 mmol) and the mixture stirred at room temperature for 2 hours. The reaction was diluted with water and extracted with ethyl acetate. The organic extracts were washed with brine, dried (Na₂SO₄) and concentrated. This was purified by SPE (silica) eluted with ethyl acetate:cyclohexane (15:85) to give the title compound.
MS calcd for (C₂₄H₃₂IN₃O₃ + H)⁺: 538
MS found (electrospray): (M+H)⁺ = 538

### Intermediate 103

### Ethyl 3-[[(trans-4-methylcyclohexyl)carbonyl](1-methylethyl)amino]-1-{4-triphenylphosphoniummethyl-phenyl}-1H-pyrazole-4-carboxylate

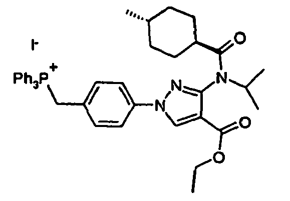

To a solution of Intermediate 102 (230 mg, 0.43 mmol) in THF (3 mL) was added triphenyl phosphine (146 mg, 0.57 mmol) and the mixture heated at 100 °C for 18 hours. The solvent was removed in vacuo to give the title compound.
MS calcd for ylid (C₄₂H₄₆N₃O₃P ⁺ H)⁺: 672
MS found (electrospray): (M+H)⁺= 672

### Intermediate 104

### Ethyl 3-[[(trans-4-methylcyclohexyl)carbonyl](1-methylethyl)amino]-1-{4[(E/Z)-2-(3-furanyl)-ethenyl]phenyl}-1H-pyrazole-4-carboxylate

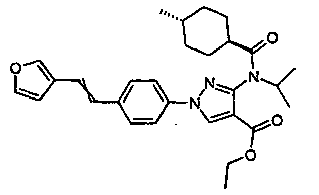

To a solution of Intermediate 103 (200 mg, 0.25 mmol) in THF (2 mL) was added a solution of potassium *tert*-butoxide in THF (1M, 0.25 mL). After stirring for 3 minutes under nitrogen, a solution of furan-3-carboxaldehyde (36 mg, 0.37 mmol) was added and the mixture stirred at room temperature for 2 hours. Saturated ammonium chloride solution was added and the mixture extracted with ethyl acetate. The organic extracts were washed with brine, dried (Na₂SO₄) and concentrated. This was purified by chromatography (silica) eluted with ethyl acetate:cyclohexane (20:80) to give the title compound as a mixture of cis and *trans* isomers. MS calcd for (C₂₉H₃₅N₃O₃ + H)⁺: 490
MS found (electrospray): (M+H)⁺ = 490

### Intermediate 105

### Ethyl 3-[[(trans-4-methylcyclohexyl)carbonyl](1-methylethyl)amino]-1-{4[(E/Z)-2-(3-pyrazolyl)-ethenyl]phenyl}-1H-pyrazole-4-carboxylate

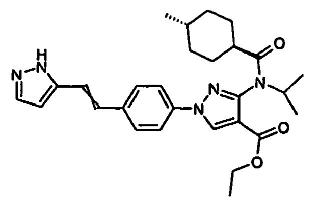

Similarly prepared by the procedure described for Intermediate 104 using 3-pyrazole carboxaldehyde and Intermediate 103. Purification was by ISCO Companion silica chromatography, eluted with a gradient of ethyl acetate:cyclohexane (12% to 75%), to give the title compound as the pure cis isomer and a mixture of *cis* and *trans* isomers. *cis* and *trans* isomers
MS calcd for (C₂₈H₃₅N₅O₃ + H)⁺: 490
MS found (electrospray): (M+H)⁺ = 490

### Intermediate 106

### Ethyl 3-[[(trans-4-methylcyclohexyl)carbonyl](1-methylethyl)amino]-1-{4[(Z)-2-(3-pyrazolyl)-ethenyl]phenyl}-1H-pyrazole-4-carboxylate

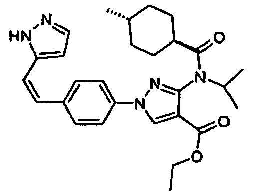

*Cis* isomer from Intermediate 105.
MS calcd for (C₂₈H₃₅N₅O₃ + H)⁺: 490
MS found (electrospray): (M+H)⁺= 490

### Intermediate 107

### Ethyl 3-[[(trans-4-methylcyclohexyl)carbonyl](1-methylethyl)amino]-1-{4[(E)-2-( tetrahydro-2H-pyran-4-yl)-ethenyl]phenyl}-1H-pyrazole-4-carboxylate

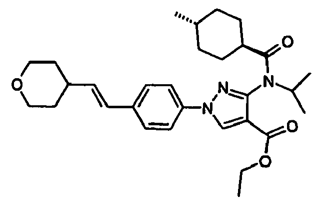

Similarly prepared by the procedure described for Intermediate 104 using 4-(tetrahydro-2H-pyran)carboxaldehyde. Purification was by ISCO Companion silica chromatography, eluted with ethyl acetate:cyclohexane (15:85), to give the title compound as the *trans* isomer.
MS calcd for (C₃₀H₄₁N₃O₄ + H)⁺: 508
MS found (electrospray): (M+H)⁺= 508

### Intermediate 108

### (1-{[(1,1-Dimethylethyl)oxy]carbonyl}-1,2,3,6-tetrahydro-4-pyridinyl)boronic acid

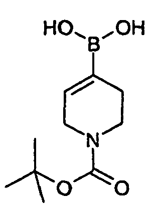

Sodium periodate (2.08 g, 9.75 mmol) was added in portions to a mixture of 1,1-dimethylethyl 4-(4,4,5,5-tetramethyl-1,3,2-dioxaborolan-2-yl)-3,6-dihydro-1(2H)-pyridine-carboxylate (1.0 g, 3.25 mmol, TDC Research Inc) and ammonium acetate (750 mg, 9.74 mmol) in acetone (40 mL) and water (40 mL) at room temperature, under nitrogen. The reaction was stirred overnight, filtered through a Celite pad, washed with acetone and the organic solvent removed. Brine (50 mL) was added and the solution was extracted three times with ethyl acetate. The combined organic layers were dried over sodium sulphate and concentrated to give the title compound.
MS calcd for (C₁₀H₁₈NO₄ + H)⁺: 228
MS found (electrospray): (M+H)⁺= 228

### Intermediate 109

### 1,1-Dimethylethyl 4-{4-[(ethyloxy)carbonyl]-3-[[(trans-4-methylcyclohexyl)carbonyl](1-methylethyl)amino]-1H-pyrazol-1-yl}-3,6-dihydro-1(2H)-pyridinecarboxylate

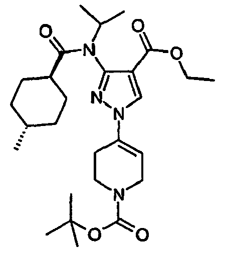

Copper (II) acetate (210 mg, 1.17 mmol) was added to a DCM (10 mL) solution of Intermediate 5 (250 mg, 0.778 mmol) and Intermediate 108 (353 mg, 1.56 mmol). The reaction was stirred at room temperature, in air for 24 hrs, filtered through Celite, partitioned between DCM and saturated sodium bicarbonate, passed through a hydrophobic frit and the organic layer concentrated. Purified by 20 g silica SPE eluted DCM in cyclohexane (0-100%) then ethyl acetate:cyclohexane (1:4) to give the title compound.
MS calcd for (C₂₇H₄₂N₄O₅ + H)⁺ : 503
MS found (electrospray): (M+H)⁺ = 503

### Intermediate 110

### Ethyl 3-[[(trans-4-methylcyclohexyl)carbonyl](1-methylethyl)amino]-1-(1,2,3,6-tetrahydro-4-pyridinyl)-1H-pyrazole-4-carboxylate

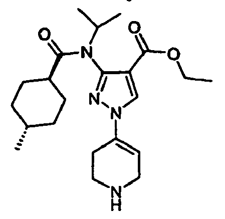

Hydrogen chloride in 1,4-dioxane (4 mL, 4N) was added to a solution of Intermediate 109 (330 mg, 0.66 mmol) in 1,4-dioxane, then stirred at room temperature, under nitrogen for 2 hrs. The solvent was removed and the residue co-evaporated with diethyl ether. The residue was dissolved in methanol (5 mL) and loaded onto a 10 g SCX-2 SPE cartridge. The column was washed with methanol (3 column volumes) then eluted with 10% 0.88 ammonia in methanol to give the title compound.
MS calcd for (C₂₂H₃₄N₄O₃ + H)⁺: 403
MS found (electrospray): (M+H)⁺= 403

### Intermediate 111

### Ethyl 3-[[(trans-4-methylcyclohexyl)carbonyl](1-methylethyl)amino]-1-[1-(methylsulfonyl)-1,2,3,6-tetrahydro-4-pyridinyl]-1H-pyrazole-4-carboxylate

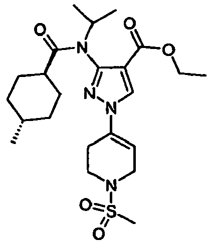

Triethylamine (0.028 mL, 0.2 mmol) followed by methanesulfonyl chloride (0.015 mL, 0.2 mmol) was added to a DCM (3 mL) solution of Intermediate 110 (68.5 mg, 0.17 mmol). The reaction was stirred for 1 hour at room temperature, under nitrogen, then loaded onto a 2 g STRATA cartridge and eluted with DCM (3 column volumes) then methanol (3 column volumes) until the title compound was isolated.
MS calcd for (C₂₃H₃₆N₄O₅S + H)⁺: 481
MS found (electrospray): (M+H)⁺= 481

### Intermediate 112

### 4-Methyl-1-cyclohexen-1-yl trifluoromethanesulfonate

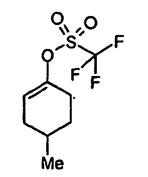

Triflic anhydride (1.57 mL, 9.37 mmol) was added dropwise to a solution of 4-methylcyclohexanone (1 g, 8.92 mmol) and 2,6-di-*tert*-butyl-4-methylpyridine (2 g, 9.8 mmol) in dry DCM (50 mL). The reaction was stirred at room temperature, under nitrogen overnight. The solution was filtered and concentrated to 10 mL, applied to a 20 g silica SPE and eluted with DCM to give the title compound.
Thin layer chromatography (silica) 1:9 ethyl acetate:cyclohexane R_{f} = 0.9 (UV detection).

### Intermediate 113

### 4,4-Dimethyl-1-cyclohexen-1-yl trifluoromethanesulfonate

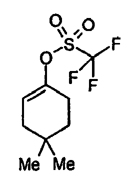

Prepared by a similar method to that described for Intermediate 112 using 4,4-dimethylcyclohexanone⁴, to give the title compound.
Thin layer chromatography (silica) 1:9 ethyl acetate:cyclohexane R_{f} = 0.9 (detection with KMnO₄).
4. Tetrahedron (1994), 50 (4) 973-978.

### Intermediate 114

### 4-tert-Butyldimethylsilyloxy-1-cyclohexen-1-yl trifluoromethanesulfonate

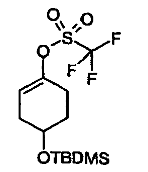

Prepared by a similar method to that described for Intermediate 112 using 4-*tert* butyldimethylsilylcyclohexanone, to give the title compound.
Thin layer chromatography (silica) 1:9 ethyl acetate:cyclohexane R_{f} = 0.95 (detection with KMnO₄).

### Intermediate 115

### 4-Trifluoromethyl-1-cyclohexen-1-yl trifluoromethanesulfonate

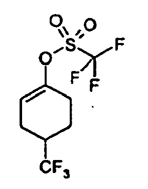

Prepared by a similar method to that described for Intermediate 112 using 4-trifluoromethylcyclohexanone, to give the title compound.
¹H NMR (CDCl₃): δ 5.79 (1H, d), 2.57-2.12 (6H, m), 1.78 (1H, m).

### Intermediate 116

### 4,4,5,5-Tetramethyl-2-(4-methyl-1-cyclohexen-1-yl)-1,3,2-dioxaborolane

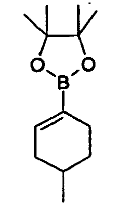

[1,1'-Bis(diphenylphosphino)ferrocene]dichtoropalladium (II) (167 mg, 0.20 mmol) was added to a solution of Intermediate 112 (1 g, 4.09 mmol) and bis(pinacolato)diboron (3.11 g, 12.27 mmol) and potassium acetate (1.8 g, 18.4 mmol) in dry DMF (5 mL). The reaction was stirred at 80°C, under nitrogen, for 24 hours. The reaction was cooled, solvent removed, partitioned between diethyl ether and water, organics washed twice further with water, dried over sodium sulphate and concentrated. Purified by 50 g silica SPE, loaded in DCM, eluted with a gradient of DCM in cyclohexane to give the title compound which was used without further purification.
Thin layer chromatography (silica) 1:9 ethyl acetate:cyclohexane R_{f} = 0.8

### Intermediate 117

### 4,4,5,5-Tetramethyl-2-(4,4-dimethyl-1-cyclohexen-1-yl)-1,3,2-dioxaborolane

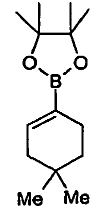

Prepared by a similar method to that described for Intermediate 116 using Intermediate 113, to give the title compound.
Thin layer chromatography (silica) cyclohexane R_{f} = 0.2 (uv detection).

### Intermediate 118

### (1,1-Dimethylethyl)(dimethyl){[4-(4,4,5,5-tetramethyl-1,3,2-dioxaborolan-2-yl)-3-cyclohexen-1-yl]oxy}silane

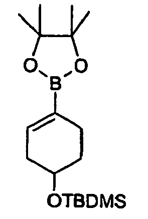

Prepared by a similar method to that described for Intermediate 116 using Intermediate 114, to give the title compound.
Thin layer chromatography (silica) 5:95 ethyl acetate:cyclohexane R_{f} = 0.9 (uv detection).

### Intermediate 119

### 4,4,5,5 Tetramethyl-2-[4-(trifluoromethyl)-1-cyclohexen-1-yl]-1,3,2-dioxaborolane

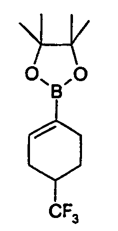

Prepared by a similar method to that described for Intermediate 116 using Intermediate 115, to give the title compound.
¹H NMR (CDCl₃): δ 6.53 (1H, d), 2.4-1.97 (5H, m), 1.45 (1H, m), 1.27 (12H, s), 1.22 (1H, m).

### Intermediate 120

### (4-Methyl-1-cyclohexen-1-yl)boronic acid

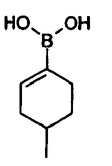

Prepared by a similar method to that described for Intermediate 108 replacing 1,1-dimethylethyl 4-(4,4,5,5-tetramethyl-1,3,2-dioxaborolan-2-yl)-3,6-dihydro-1(2H)-pyridinecarboxylate with Intermediate 116 to give the crude title compound used without further purification.
MS calcd for (C₇H₁₃BO₂ + H)⁺: 141
MS found (electrospray): (M+H)⁺= 141

### Intermediate 121

### (4,4-Dimethyl-1-cyclohexen-1-yl)boronic acid

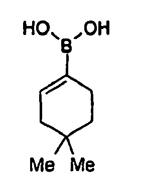

Prepared by a similar method to that described for Intermediate 108 using Intermediate 117, to give the title compound used directly in the next step.
¹H NMR (CDCl₃): δ 6.9 for characteristic alkene proton.

### Intermediate 122

### (4-{[(1,1-dimethylethyl)(dimethyl)silyl]oxy}-1-cyclohexen-1-yl)boronic acid

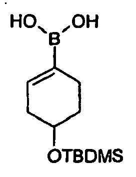

Prepared by a similar method to that described for Intermediate 108 using Intermediate 118, to give the title compound, used directly in the next step.
¹H NMR (CDCl₃): δ 6.8 for characteristic alkene proton.
Thin layer chromatography (silica) 5:95 ethyl acetate:cyclohexane R_{f} = 0.8 (uv detection).

### Intermediate 123

### [4-(Trifluoromethyl)-1-cyclohexen-1-yl]boronic acid

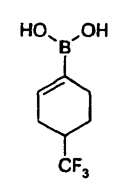

Prepared by a similar method to that described for Intermediate 108 using Intermediate 119, to give the title compound, used directly in the next step.
¹H NMR (CDCl₃): δ 7.28 (1H, d), 2.52-1.98 (7H, m), 1.5 (2H, m).

### Intermediate 124

### 6-Indoleboronic acid

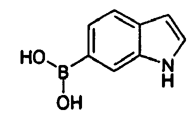

Prepared by a similar method to that described for Intermediate 108 using 6-(4,4,5,5-tetramethyl-1,3,2-dioxaborolan-2-yl)-1*H*-indole to give the title compound.
MS calcd for (C₈H₈BNO₂- H)⁻: 161
MS found (electrospray): (M-H)⁻= 161

### Intermediate 125

### 5-Benzofuranboronic acid

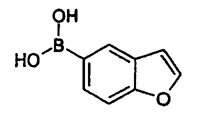

Prepared by a similar method to that described for Intermediate 108 using 5-(4,4,5,5-tetramethyl-1,3,2-dioxaborolan-2-yl)-1-benzofuran to give the title compound.
MS calcd for (C₈H₇BO₃ + H)⁺: 163
MS found (electrospray): (M+H)⁺= 163

### Intermediate 126

### Ethyl 1-(4-methyl-1-cyclohexen-1-yl)-3-[[(trans-4-methylcyclohexyl)carbonyl](1-methylethyl)amino]-1H-pyrazole-4-carboxylate

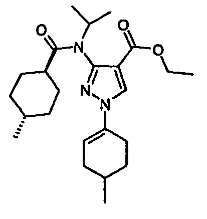

Prepared by a similar method to that described for Intermediate 52 using Intermediate 120 to give the title compound.
MS calcd for (C₂₄H₃₇N₃O₃ + H)⁺: 416
MS found (electrospray): (M+H)⁺ = 416

### Intermediate 127

### Ethyl 3-[[(trans-4-methylcyclohexyl)carbonyl](phenylmethyl)amino]-1-phenyl-1H-pyrazole-4-carboxylate

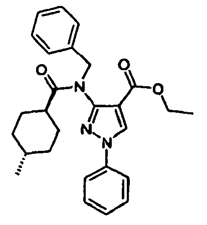

Prepared by a similar method to that described for intermediate 73, replacing N,N-dimethylbromoacetamide with benzyl bromide and purified by MDAP HPLC to give the title compound.
MS calcd for (C₂₇H₃₁N₃O₃ + H)⁺: 446
MS found (electrospray): (M+H)⁺= 446

### Intermediate 128

### Ethyl 3-(cyclopentylamino)-1-phenyl-1H-pyrazole-4-carboxylate

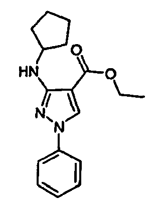

Prepared by a similar method to that described for Intermediate 85, replacing pyrazine-2-carboxaldehyde with cyclopentanone and purified by ISCO Companion silica chromatography (12 g cartridge), eluted with 0-80% ethyl acetate in cyclohexane to give the title compound.
¹H NMR (CDCl₃): δ 8.39 (1H, s), 7.61 (2H, d), 7.43 (2H, t), 7.22 (1H, t), 5.41 (1H, br), 4.30 (2H, m), 4.14 (1H, m), 2.11 (2H, m), 1.78-1.70 (2H, br), 1.69-1.53 (4H, br), 1.36 (3H, t)

### Intermediate 129

### Ethyl 3-{cyclopentyl[(trans-4-methylcyclohexyl)carbonyl]amino}-1-phenyl-1H-pyrazole-4-carboxylate

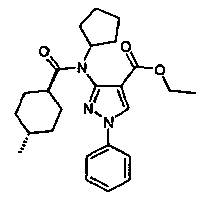

Prepared by a similar method to that described for Intermediate 76, replacing Intermediate 74 with Intermediate 128 and purified by silica Biotage flash column, eluted with 5% acetonitrile in DCM to give the title compound.
¹H NMR (CDCl₃): δ 8.50 (1 H, s), 7.72 (2H, d), 7.53 (2H, t), 7.41 (1H, t), 4.86 (1H, m), 4.30 (2H, m), 2.16-1.75 (5H, br), 1.73-1.43 (7H, br), 1.40-1.25 (5H, br), 1.18 (1H, m), 0.75 (3H, d), 0.70-0.53 (2H, br)

### Intermediate 130

### Ethyl1-phenyl-3-(tetrahydro-2H-pyran-4-ylamino)-1H-pyrazole-4-carboxylate

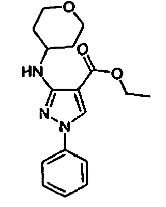

Prepared by a similar method to that described for intermediate 85, replacing pyrazine-2 carboxaldehyde with tetrahydro-4H-pyran-4-one and purified by ISCO Companion silica chromatography (12 g cartridge), eluted with 0-80% ethyl acetate in cyclohexane to give the title compound.
¹H NMR (CDCl₃): δ 8.15 (1H, s), 7.64 (2H, d), 7.44 (2H, t), 7.28 (1H, hidden under solvent peak), 5.46 (1H, d), 4.30 (2H, q), 4.02 (2H, d), 3.88 (1H, m), 3.55 (2H, t), 2.15 (2H, d), 1.62 (2H, m), 1.36 (3H, t).

### Intermediate 131

### Ethyl 1-phenylmethyl-3-(tetrahydro-2H-pyran-4-ylamino)-1H-pyrazole-4-carboxylate

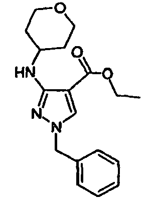

Prepared by a similar method to that described for Intermediate 85, replacing pyrazine-2-carboxaldehyde with tetrahydro-4H-pyran-4-one and ethyl 3-amino-1-phenyl-1*H*-pyrazole-4-carboxylate with ethyl 3-amino-1-phenylmethyl-1*H*-pyrazole-4-carboxylate.³ Purification was by ISCO Companion silica chromatography (330 g cartridge), eluted with 5-60% ethyl acetate in cyclohexane to give the title compound.
MS calcd for (C₁₈H₂₃N₃O₃ + H)⁺: 330
MS found (electrospray): (M+H)⁺ = 330

### Intermediate 132

### Ethyl 3-[[(trans-4-methylcyclohexyl)carbonyl](tetrahydro-2H-pyran-4-yl)amino]-1-phenyl-1H-pyrazole-4-carboxylate

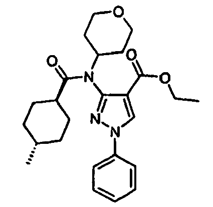

Prepared by a similar method to that described for Intermediate 76, replacing Intermediate 74 with Intermediate 130 and purified by ISCO companion silica flash column, eluted with 0-70% ethyl acetate in cyclohexane to give the title compound.
¹H NMR (CDCl₃): δ 8.51 (1H, s), 7.71 (2H, d), 7.53 (2H, t), 7.41 (1H, t), 4.83 (1H, m), 4.03 (2H, m), 3.93 (2H, br), 3.50 (2H, q), 2.05 (1H, s), 2.0-1.55 (8H, br), 1.50-1.25 (6H, br), 0.78 (3H, d), 0.76-0.60 (2H, br).

### Intermediate 133

### Ethyl 3-[[(trans-4-methylcyclohexyl)carbonyl](tetrahydro-2H-pyran-4-yl)amino]-1-phenylmethyl-1H-pyrazole-4-carboxylate

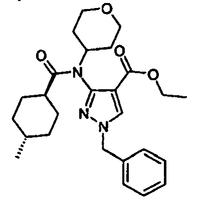

Prepared by a similar method to that described for Intermediate 2, replacing Intermediate 1 with Intermediate 131 and purified by ISCO companion silica flash column, eluted with 10% ethyl acetate in cyclohexane, then a gradient of 10-50% ethyl acetate in cyclohexane to give the title compound.
MS calcd for (C₂₆H₃₅N₃O₄ + H)⁺: 454
MS found (electrospray): (M+H)⁺ = 454

### Intermediate 134

### Ethyl 3-[[(trans-4-methylcyclohexyl)carbonyl](tetrahydro-2H-pyran-4-yl)amino]-1H-pyrazole-4-carboxylate

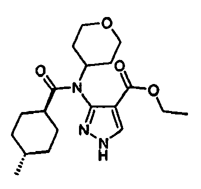

A solution of Intermediate 133 (5.1 g, 11.3 mmol) in ethanol (200 mL) and concentrated hydrochloric acid (2 mL) was subjected to atmospheric hydrogenation using 10% palladium on carbon catalyst (1.35 g) for 16 hours. The mixture was filtered through a Celite pad, the pad washed with ethanol and the combined organics evaporated. The residue was partitioned between DCM and saturated sodium bicarbonate solution, passed through a hydrophobic frit and the organic layer evaporated to give the title compound.
MS calcd for (C₁₉H₂₉N₃O₄ + H)⁺: 364
MS found (electrospray): (M+H)⁺= 364.

### Intermediate 135

### Ethyl 3-{[(trans-4-methylcyclohexyl)carbonyl][1-(methylsulfonyl)-4-piperidinyl]amino}-1H-pyrazole-4-carboxylate

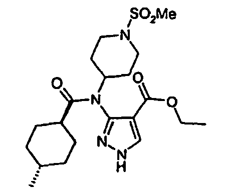

Prepared by a similar method to that described for Intermediate 134, replacing Intermediate 133 with Intermediate 82 to give the title compound.
MS calcd for (C₂₀H₃₂N₄O₅S + H)⁺: 441
MS found (electrospray): (M+H)⁺ = 441

### Intermediate 136

### Ethyl 3-{(1-acetyl-4-piperidinyl)[(trans-4-methylcyclohexyl)carbonyl]amino}-1-phenyl-1H-pyrazole-4-carboxylate

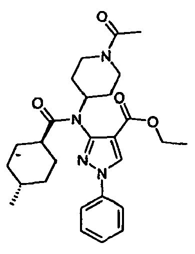

To Intermediate 78 (50 mg, 0.114 mmol) was added DCM (3 mL), acetyl chloride (0.016 mL, 0.23 mmol) and triethylamine (0.23 mmol). The reaction was stirred overnight at room temperature, partitioned between DCM and saturated sodium bicarbonate, the aqueous layer separated and the organic layer concentrated to give the title compound.
MS calcd for (C₂₇H₃₆N₄O₄ + H)⁺: 481
MS found (electrospray): (M+H)⁺= 481

The following compounds were prepared by coupling the appropriate boronic acid derivative to pyrazoles using a similar method to that described for Intermediate 6.

### Intermediate 137

### Ethyl 1-(4-(6-indolyl)phenyl)-3-[[(trans-4-methylcyclohexyl)carbonyl](1-methylethyl)amino]-1H-pyrazole-4-carboxylate

### Intermediate 138

### Ethyl 1-(4-(5-benzofuranyl)phenyl)-3-[[(trans-4-methylcyclohexyl)carbonyl](1-methylethyl)amino]-1H-pyrazole-4-carboxylate

### Intermediate 139

### Ethyl 1-(4-(trifluoromethyl)cyclohexen-1-yl)-3-[[(trans-4-methylcyclohexyl)carbonyl](1-methylethyl)amlno]-1H-pyrazole-4-carboxylate

### Intermediate 140

### Ethyl 1-(4-(tert-butyldimethylsilyloxy)cyclohexen-1-yl)-3-[[(trans-4-methylcyclohexyl)carbonyl](1-methylethyl)amino]-1H-pyrazole-4-carboxylate

### Intermediate 141

### Ethyl 1-(4,4-dimethyl)cyclohexen-1-yl)-3-{[(trans-4-methylcyclohexyl)carbonyl](1-methylethyl)amino}-1H-pyrazole-4-carboxylate

### Intermediate 142

### Ethyl1-((4,4-dimethyl)cyclohexen-1-yl)-3-[[(trans-4-methylcyclohexyl)-carbonyl] (tetrahydro-2H-pyran-4-yl)amino]-1H-pyrazole-4-carboxylate

This compound was purified by ISCO Companion silica chromatography (12 g cartridge), eluted with 0-95% ethyl acetate in cyclohexane to give the title compound.

### Intermediate 143

### Ethyl 1-(cyclohepten-1-yl)-3-[[(trans-4-methylcyclohexyl)carbonyl](tetrahydro-2H-pyran-4-yl)amino]-1H-pyrazole-4-carboxylate

### Intermediate 144

### Ethyl 1-((4-methyl)cyclohexen-1-yl)-3-[[(trans-4-methylcyclohexyl)carbonyl] (tetrahydro-2H-pyran-4-yl)amino]-1H-pyrazole-4-carboxylate

This compound was purified by ISCO Companion silica chromatography (12 g cartridge), eluted with 0-50% ethyl acetate in cyclohexane to give the title compound.

### Intermediate 145

### Ethyl 1-((4-formyl)phenyl)-3-[[(trans-4-methylcyclohexyl)carbonyl](tetrahydro-2H-pyran-4-yl)amino]-1H-pyrazole-4-carboxylate

### Intermediate 146

### Ethyl 1-((4-methyl)cyclohexen-1-yl)-3-[[(trans-4-methylcyclohexyl)carbonyl](1-(methylsulfonyl)-4-piperidinyl]amino]-1H-pyrazole-4-carboxylate

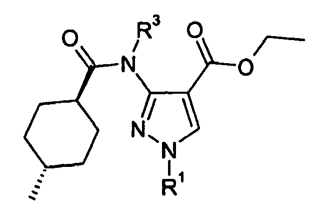

| Intermediate Number | Pre-Cursor Intermediate Numbers | Structure | | Molecular Formula | Mass Spectrometry | |
|---|---|---|---|---|---|---|
| | | R¹ | R³ | | (M+H)⁺ Calcd | (M⁺H)⁺ Found |
| **137** | 5, 124 | 6-indolyl | iPr | C₂₅H₃₂N₄O₃ | 437 | 437 |
| **138** | 5, 125 | 5-benzofuranyl | iPr | C₂₅H₃₁N₃O₄ | 438 | 438 |
| **139** | 5, 123 | 4-(Trifluoromethyl)-1-cyclohexen-1-yl | iPr | C₂₄H₃₄F₃N₃O₃ | 470 | 470 |
| **140** | 5, 122 | 4-(TBDMSO)-1- | iPr | C₂₉H₄₉N₃O₄Si | 532 | 532 |
| | | cyclohexen-1-yl | | | | |
| **141** | 5, 121 | 4,4-(Dimethyl)-1- | iPr | C₂₉H₃₉N₃O₃ | 430 | 430 |
| | | cyclohexen-1-yl | | | | |
| **142** | 134, 121 | 4,4-(Dimethyl)-1- | pyran-4-yl | C₂₇H₄₁N₃O₄ | 472 | 472 |
| | | cyclohexen-1-yl | | | | |
| **143** | 134 | 1-Cyclohepten-1-yl | pyran-4-yl | C₂₆H₃₉N₃O₄ | 458 | 458 |
| **144** | 120, 134 | 4-Methyl-1- | pyran-4-yl | C₂₆H₃₉N₃O₄ | 458 | 458 |
| | | cyclohexen-1-yl | | | | |
| **145** | 134 | 4-Formyl Ph | pyran-4-yl | C₂₆H₃₃N₃O₅ | 468 | 468 |
| **146** | 120, 135 | 4-Methyl-1- | 1- | C₂₇H₄₂N₄O₅S | 535 | 535 |
| | | cydohexen-1-yf | Methylsulfonyl- | | | |
| | | | 4-piperidinyl | | | |

| | | | | | | |
|---|---|---|---|---|---|---|
| Cyclohepten-1-yl boronic acid and 4-formylphenyl boronic acid are commercially available. | | | | | | |

### Intermediate 147

### Ethyl 3-[[(trans-4-methylcyclohexyl)carbonyl](1-methylethyl)amino]-1-{4-[(phenylthio)methyl]phenyl}-1H-pyrazole-4-carboxylate

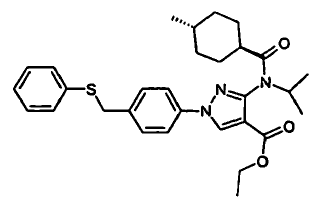

To a solution of Intermediate 102 (137 mg, 0.26 mmol) in ethanol (2 mL) was added sodium thiophenolate (67 mg, 0.51 mmol) and the mixture stirred at room temperature for 2 hours. The ethanol was removed *in vacuo,* the residue partitioned between ethyl acetate and saturated sodium bicarbonate solution, the organic extract dried (Na₂SO₄) and concentrated. This was purified by silica column chromatography, eluted with ethyl acetate:cyclohexane (10:90) to give the title compound.
MS calcd for (C₃₀H₃₇N₃O₃S + H)⁺: 520
MS found (electrospray): (M+H)⁺ = 520

### Intermediate 148

### Ethyl 3-[[(trans-4-methylcyclohexyl)carbonyl](1-methylethyl)amino]-1-{4-[(phenylsulfonyl)methyl]phenyl}-1H-pyrazole-4-carboxylate

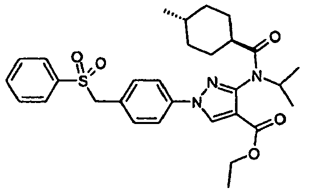

To a solution of Intermediate 147 (119 mg, 0.23 mmol) in ethanol (10 mL) was added a solution of oxone (423 mg, 0.69 mmol) in water (5 mL) and the mixture stirred at room temperature for 18 hours. The solvents were removed *in vacuo,* the residue partitioned between DCM and water, the organic extract passed through a hydrophobic frit and concentrated. This was purified by silica column chromatography, eluted with ethyl acetate:cyclohexane (10:90, then 20:80, then 30:70, and finally 40:60) to give the title compound.
MS calcd for (C₃₀H₃₇N₃O₅S + H)⁺: 552
MS found (electrospray): (M+H)⁺ = 552

### Intermediate 149

### Ethyl 3-[[(trans-4-methylcyclohexyl)carbonyl](1-methylethyl)amino]-1-{4-[(phenyloxy)methyl]phenyl}-1H-pyrazole-4-carboxylate

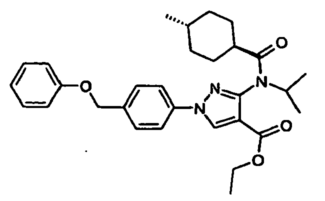

To a solution of Intermediate 102 (137 mg, 0.26 mmol) in acetone (5 mL) was added potassium carbonate (90 mg) and phenol (50 mg) and the mixture stirred at room temperature for 2 days. The solvent was removed *in vacuo,* the residue partitioned between DCM and water, the organic extract passed through a hydrophobic frit and concentrated. This was purified by SPE (silica), eluted with a gradient of ethyl acetate:cyclohexane (20-50%) to give the title compound.
MS calcd for (C₃₀H₃₇N₃O₄ + H)⁺: 504
MS found (electrospray): (M+H)⁺ = 504

### Intermediate 150

### Ethyl 3-[[(trans-4-methylcyclohexyl)carbonyl](1-methylethyl)amino]-1-{4-[(1,3-thiazol-4-ylmethyl)oxy]phenyl}-1H-pyrazole-4-carboxylate

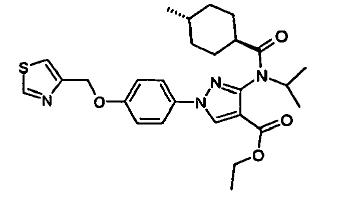

To a solution of Intermediate 7 (230 mg, 0.55 mmol) and potassium carbamate (450 mg, 3.3 mmol) in acetone (10 mL) was added 4-(chloromethyl)-1,3-thiazole (230 mg, 1.37 mmol) and the mixture heated under reflux for 16 hours under nitrogen. The solvent was removed *in vacuo,* the residue partitioned between DCM and water, the organic extract passed through a hydrophobic frit and concentrated. This was purified by SPE (silica), eluted with a gradient of ethyl acetate:cyclohexane (20-30%) to give the title compound.
MS calcd for (C₂₇H₃₄N₄O₄S + H)⁺: 511
MS found (electrospray): (M+H)⁺ = 511

### Intermediate 151

### Ethyl 1-(4-hydroxy-1-cyclohexen-1-yl)-3-[[(trans-4-methylcyclohexyl)carbonyl](1-methylethyl)amino]-1H-pyrazole-4-carboxylate

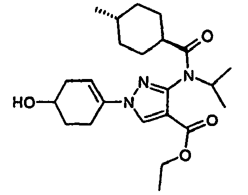

A solution of tetrabutyl ammonium fluoride in THF (1M, 1 mL, 1 mmol) was added a solution of Intermediate 140 (133 mg, 0.25 mmol) in THF (3 ml) at 0 °C under nitrogen. After 15 minutes, the mixture was allowed to attain room temperature and stirred for one hour. The mixture was poured into sodium bicarbonate solution (30 mL, 8% solution) and extracted with ethyl acetate (3 x 20 mL). The organic extract was dried (Na₂SO₄) and concentrated to give the title compound.
MS calcd for (C₂₃H₃₅N₃O₄ + H)⁺: 418
MS found (electrospray): (M+H)⁺ = 418

### Intermediate 152

### Ethyl 1-(4-benzyloxy-1-cyclohexen-1-yl)-3-[[(trans-4-methylcyclohexyl)carbonyl](1-methylethyl)amino]-1H-pyrazole-4-carboxylate

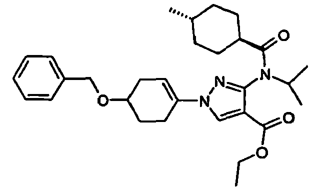

To a solution of Intermediate 151 (100mg, 0.24 mmol) in DMF (2 mL) under nitrogen was added sodium hydride (11 mg, 0.26 mmol, 60% dispersion in mineral oil). After effervescence ceased, benzyl bromide (28 ul, 0.24 mmol) was added dropwise. The mixture was stirred at room temperature for 24 hours, quenched by the addition of a few drops of water and ammonia, and the solvents evaporated *in vacuo.* The residue was partitioned between ethyl acetate and brine and the aqueous layer further extracted with ethyl acetate. The organic extracts were dried (Na₂SO₄), concentrated and purified by SPE (silica), eluted with cyclohexane, then cyclohexane:DCM (3:1, then 1:1, then 1:3), then DCM and finally DCM/ethyl acetate to give the title compound.
MS calcd for (C₂₃H₃₅N₃O₄ + H)⁺: 508
MS found (electrospray): (M+H)⁺= 508

### Intermediate 153

### Ethyl 3-[[(trans-4-methylcyclohexyl)carbonyl](1-methylethyl)amino]-1-{4-aminophenyl}-1H-pyrazole-4-carboxylate

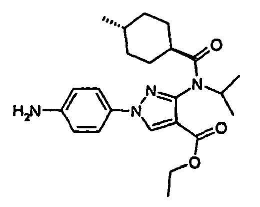

To a solution of Intermediate 56 (1.14g, 2.22 mmol) in dioxane (5 mL) was slowly added a solution of HCl in dioxane (4N, 10 mL) and the reaction stirred at room temperature for 16 hours under nitrogen. A further portion of HCl in dioxane was added (10 mL) and stirring continued for 2 days. The solvents were evaporated and the mixture was partitioned between sodium bicarbonate solution (30 mL, 8% solution) and ethyl acetate and the aqueous layer further extracted with ethyl acetate (3 x 20 mL). The organic extract was dried (Na₂SO₄), evaporated, and purified by SPE (silica), eluted with DCM, then DCM:ethyl acetate (9:1, then 8:2) to give the title compound.
MS calcd for (C₂₃H₃₂N₄O₃ + H)⁺: 413
MS found (electrospray): (M+H)⁺= 413

### Intermediate 154

### Ethyl 3-[[(trans-4-methylcyclohexyl)carbonyl](1-methylethyl)amino]-1-{4-[(phenylcarbonyl)amino]phenyl}-1H-pyrazole-4-carboxylate

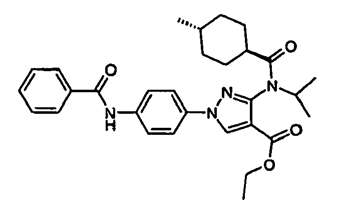

To a solution of Intermediate 153 (70 mg, 0.17 mmol) and ethyl diisopropylamine (30 ul, 0.17 mmol) in DCM (2 mL) under nitrogen, was added benzoyl chloride (20 ul, 0.17 mmol). After 2 hours, an 8% solution of sodium bicarbonate was added and the layers separated. The organic fraction was concentrated to give the title compound.
MS calcd for (C₃₀H₃₆N₄O₄ + H)⁺: 517
MS found (electrospray): (M+H)⁺= 517

### Intermediate 155

### Ethyl 3-[[(trans-4-methylcyclohexyl)carbonyl](1-methylethyl)amino]-1-{4-[(3-chlorophenylcarbonyl)amino]phenyl}-1H-pyrazole-4-carboxylate

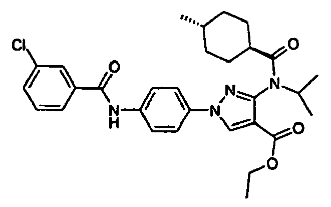

Prepared by a similar method to that described for Intermediate 154, replacing benzoyl chloride with 3-chloro benzoyl chloride. Purified by SPE (silica), eluted with DCM:cyclohexane (1:1), then DCM, then DCM:ethyl acetate (3:1), then then DCM:ethyl acetate (1:1) to give the title compound.
MS calcd for (C₃₀H₃₅ClN₄O₄ + H)⁺: 551/553
MS found (electrospray): (M+H)⁺= 551/553

### Intermediate 156

### Ethyl 3-[[(trans-4-methylcyclohexyl)carbonyl](1-methylethyl)amino]-1-{4-[(3-methylphenylcarbonyl)amino]phenyl}-1H-pyrazole-4-carboxylate

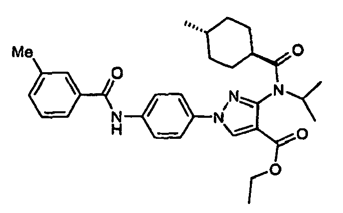

Prepared by a similar method to that described for Intermediate 154, replacing benzoyl chloride with 3-methyl benzoyl chloride. Purified by SPE (silica), eluted with DCM:cyclohexane (1:1), then DCM, then DCM:ethyl acetate (3:1), then DCM:ethyl acetate (1:1) to give the title compound.
MS calcd for (C₃₁H₃₈N₄O₄ ⁺ H)⁺: 531
MS found (electrospray): (M+H)⁺ = 531

### Intermediate 157

### Ethyl 3-[[(trans-4-methylcyclohexyl)carbonyl](1-methylethyl)amino]-1-{4-[(4-cyclohexylcarbonyl)amino]phenyl}-1H-pyrazole-4-carboxylate

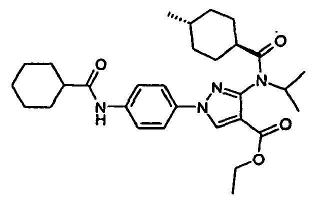

Prepared by a similar method to that described for Intermediate 154, replacing benzoyl chloride with cyclohexyl carbonyl chloride to give the title compound.
MS calcd for (C₃₀H₄₂N₄O₄ + H)⁺: 523
MS found (electrospray): (M+H)⁺ = 523

### Intermediate 158

### Ethyl 3-[[(trans-4-methylcyclohexyl)carbonyl](1-methylethyl)amino]-1-{4-[(4-fluorophenylcarbonyl)amino]phenyl}-1H-pyrazole-4-carboxylate

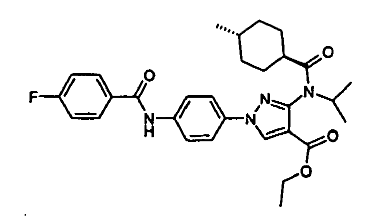

Prepared by a similar method to that described for Intermediate 154, replacing benzoyl chloride with 4-fluorobenzoyl chloride to give the title compound.
MS calcd for (C₃₀H₃₅FN₄O₄ ⁺ H)⁺: 535
MS found (electrospray): (M+H)⁺ = 535

### Intermediate 159

### Ethyl 3-[[(trans-4-methylcyclohexyl)carbonyl](1-methylethyl)amino]-1-{4-[(phenylsulfonyl)amino]phenyl}-1H-pyrazole-4-carboxylate

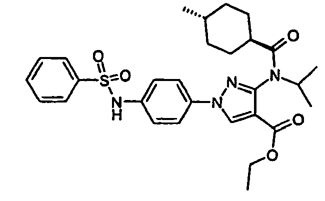

Prepared by a similar method to that described for Intermediate 154, replacing benzoyl chloride with phenyl sulfonyl chloride to give the crude title compound containing the di-sulfonylated product.
MS calcd for (C₂₉H₃₆N₄O₅S + H)⁺: 553
MS found (electrospray): (M+H)⁺ = 553

### Intermediate 160

### Ethyl 3-[[(trans-4-methylcyclohexyl)carbonyl](1-methylethyl)amino]-1-{4-[(4-carboxy phenyl}-1H-pyrazole-4-carboxylate

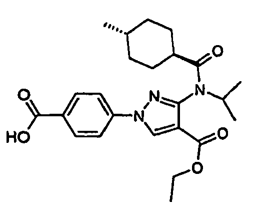

To a solution of Intermediate 57 in DCM (3 mL) was added TFA (3 mL) and the mixture stirred at room temperature for 16 hours. The solvent was evaporated *in vacuo* and the product purified by SPE (silica), eluted with cyclohexane, then DCM, then acetone, then methanol to give the title compound.
MS calcd for (C₂₄H₃₁N₃O₅ + H)⁺: 442
MS found (electrospray): (M+H)⁺ = check

### Intermediate 161

### Ethyl 3-[[(trans-4-methylcyclohexyl)carbonyl](1-methylethyl)amino]-1-{4-[(4-fluorophenylamino)carbonyl]phenyl}-1H-pyrazole-4-carboxylate

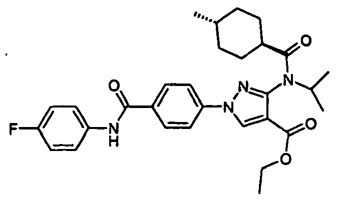

A solution of Intermediate 160 (74 mg, 0.17 mmol), HATU (76 mg, 0.20 mmol), and diisopropylethylamine (73 ul, 0.42 mmol) was stirred in dry DMF for 10 minutes. 4-Fluoroaniline (19 ul, 0.20 mmol) was added and the mixture stirred for 16 hours. The solvent was evaporated *in vacuo,* the residue dissolved in DCM and applied to a STRATA cartridge, eluted with DCM then methanol. Pooling and evaporation of the appropriate fractions afforded the title compound.
MS calcd for (C₃₀H₃₅FN₄O₄ + H)⁺: 535
MS found (electrospray): (M+H)⁺= 535

### Intermediate 162

### Ethyl 3-[[(trans-4-Methylcyclohexyl)carbonyl](1-methylethyl)amino]-1-{4-[(3-methoxyphenylcarbonyl)amino]phenyl}-1H-pyrazole-4-carboxylate

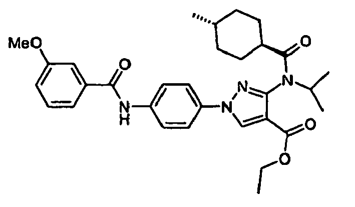

Prepared by a similar method to that described for Intermediate 154, replacing benzoyl chloride with 3-methoxy benzoyl chloride, to give the title compound.
MS calcd for (C₃₁H₃₈N₄O₅ + H)⁺: 547
MS found (electrospray): (M+H)⁺ = 547

### Intermediate 163

### Ethyl 1-(phenylmethyl)-3-[(tetrahydro-3-furanyl)amino]-1H-pyrazole-4-carboxylate.

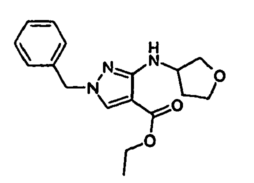

To a solution of ethyl 3-amino-1-(phenylmethyl)-1*H*-pyrazole-4-carboxylate (7.88 g, 32 mmol) and dihydro-3(2*H*)-furanone (1.60 g, 18.6 mmol) in DCM (100 mL) was added sodium triacetoxyborohydride (7.88 g, 37 mmol) and acetic acid (3.2 mL). The mixture was stirred for 16 hours, partitioned between DCM and saturated sodium bicarbonate solution and applied through a hydrophobic frit. The organic fraction was evaporated, the residue purified using a 330 g ISCO Flash column, eluting with a gradient of ethyl acetate in cyclohexane (5-60%) to give the title compound.
MS calcd for (C₁₇H₂₁N₃O₃+ H)⁺: 316
MS found (electrospray): (M+H)⁺= 316

### Intermediate 164

### Ethyl 3-[[(trans-4-methylcyclohexyl)carbonyl](tetrahydro-3-furanyl)amino]-1-(phenylmethyl)-1H-pyrazole-4-carboxylate

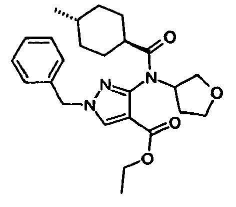

To a solution of intermediate 163 (3.73 g, 11.8 mmol) in DCM (35 mL) was added *trans-4-*methylcyclohexanecarbonyl chloride (2.28 g, 14 mmol), followed by triethylamine (2.5 mL). The reaction was heated at 45 °C for 16 hours under nitrogen, whereupon further aliquots of triethylamine (2 mL) and *trans*-4-methylcyclohexanecarbonyl chloride (1.5 g) were added. Heating was continued for a further 16 hours. After cooling, the mixture was diluted with DCM, washed with hydrochloric acid solution (1M), then water, and then saturated sodium bicarbonate solution. The organic layer was passed through a hydrophobic frit, evaporated, and purified using 120 g ISCO Flash column, eluting with a gradient of ethyl acetate in cyclohexane (5-100%) to give the title compound.
MS calcd for (C₂₅H₃₃N₃O₄+ H)⁺: 440
MS found (electrospray): (M+H)⁺ = 440

### Intermediate 165

### Ethyl 3-[[(trans-4-methylcyclohexyl)carbonyl](tetrahydro-3-furanyl)amino]-1H-pyrazole-4-carboxylate

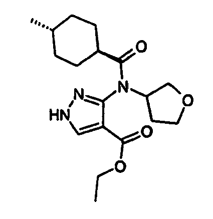

A mixture of Intermediate 164 (3.90 g, 8.88 mmol), 10% palladium on carbon (1.14 g, wet) and concentrated hydrochloric acid (2.5 mL) in ethanol (160 mL) was hydrogenated for 20 hours. The mixture was filtered through Celite, the solvent evaporated, and the residue purified using a 50 g SPE column, eluting with cyclohexane, then cyclohexane/ethyl acetate (3:1, 2:1, 1;1, 1:2, 1:3), then ethyl acetate. The crude product was dissolved in DCM and washed with sodium bicarbonate solution. The organic layer was passed through a hydrophobic frit and evaporated to give the title compound.
MS calcd for (C₁₈H₂₇N₃O₄+ H)⁺: 350
MS found (electrospray): (M+H)⁺= 350

### Intermediate 166

### Ethyl 1-(4,4-dimethyl-1-cyclohexen-1-yl)-3-[[(trans-4-methylcyclohexyl)carbonyl] (tetrahydro-3-furanyl)amino]-1H-pyrazole-4-carboxylate

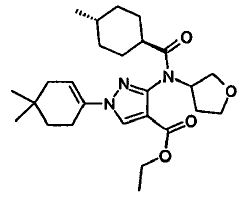

A mixture of Intermediate 165 (122 mg, 0.35 mmol), (4,4-dimethyl-1-cyclohexen-1-yl)boronic acid (107 mg, 0.7 mmol), copper (II) acetate (95 mg, 0.53 mmol) and pyridine (0.057 mL, 0.7 umol) in DCM (3 mL) was stirred in air for 18 hours. The mixture was filtered and the filtrate purified using Biotage silica column chromatography, eluting with ethyl acetate/cyclohexane (1:5), to give the title compound.
MS calcd for (C₂₆H₃₉N₃O₄⁺ H)⁺: 458
MS found (electrospray): (M+H)⁺ = 458

### Example 1

### 3-[[(trans-4-Methylcyclohexyl)carbonyl](1-methylethyl)amino]-1-(4-methylphenyl)-1H-pyrazole-4-carboxylic acid

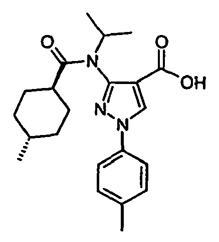

To Intermediate 6 (50 mg; 0.12 mmol) was added THF (1 mL), ethanol (1 mL) and 2M lithium hydroxide (1 mL; 2 mmol). The reaction was stirred at room temperature for 16 hrs.
The residue was purified by being partitioned between DCM and 2N hydrochloric acid, passed through a hydrophobic frit and the organic layer was concentrated to give the title compound. This purification protocol is purification Method A. When the product is not soluble in DCM, then ethyl acetate is employed as the organic solvent, the organic layer separated, dried (Na₂SO₄), and evaporated.
MS calcd for (C₂₂H₂₉N₃O₃ + H)⁺: 384
MS found (electrospray): (M+H)⁺ = 384
¹H NMR (CD₃OD) δ 8.79 (1H, s), 7.69 (2H, d), 7.35 (2H, d), 4.82 (1H, m), 2.40 (3H, s), 2.06 (1H, m), 1.79 (2H, d), 1.70-1.52 (3H, br), 1.43-1.18 (5H, br), 0.98 (3H, d), 0.78 (3H, d), 0.78-0.55 (2H, br) carboxylic acid proton not seen.

### Example 2

### 3-[[(trans-4-Methylcyclohexyl)carbonyl)(1-methylethyl)amino)-1-phenyl-1H-pyrazole-4-carboxylic acid

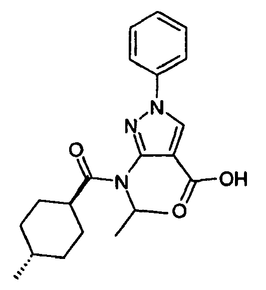

A solution of Intermediate 2 (143 mg; 0.39 mmol) in tetrahydrofuran (1.5 mL), ethanol (1 mL) and water (0.5 mL) was treated with lithium hydroxide monohydrate (101 mg; 2.40 mmol) and stirred at room temperature overnight. The reaction mixture was concentrated to dryness and the residue partitioned between 2M HCl and dichloromethane. The organic layer was separated and dried (via a hydrophobic frit), and concentrated to dryness.
The residue was purified by reverse phase HPLC on a C18 column using a two-solvent gradient elution with (A) water containing formic acid (0.1%) and (B) acetonitrile-water (95:5 v/v) containing formic acid (0.05%), as the eluents and the detection method was mass spectroscopy using electrospray as the ionization method. Pooling and evaporation of the appropriate fractions gave the title compound. This purification protocol is purification Method B (MDAP HPLC).
MS calcd for (C₂₁H₂₇N₃O₃ ⁺ H)⁺: 370
MS found (electrospray): (M+H)⁺= 370
¹H NMR (CDCl₃): δ 8.57 (1H, s), 7.75 (2H, d), 7.54 (2H, t), 7.42 (1H, t), 4.98 (1H, m), 2.00 (1H, m), 1.71 (5H, m), 1.45 (1H, m), 1.30 (5H, m), 1.00 (2H, d), 0.77 (4H, d), 0.62 (1H, m) Carboxylic acid proton not seen.

### Example 3

### 1-(1-Cyclohexen-1-yl)-3-[[(trans-4-methylcyclohexyl)carbonyl](1-methylethyl)amino]-1H-pyrazole-4-carboxylic acid

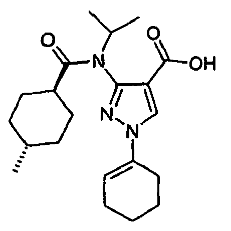

To Intermediate 52 (100 mg, 0.25 mmol) was added THF (2 mL), methanol (2 mL) and 2M lithium hydroxide (0.67 mL, 1.5 mmol). The reaction was stirred at room temperature overnight and the solvent removed.
The residue was purified by being acidified with 2N hydrochloric acid. The suspension added to a 1 g OASIS HLB cartridge, washed with water (3 column volumes) then eluted with methanol to give the title compound. This purification protocol is purification Method C. MS calcd for (C₂₁H₃₁N₃O₃ + H)⁺: 374
MS found (electrospray): (M+H)⁺= 374
¹H NMR (d₆-DMSO) δ 8.49 (1 H, s), 6.28 (1 H, br), 4.68 (1H. m), 2.18 (2H, br), 1.88 (1 H, br), 1.77 (2H, br), 1.67-1.38 (7H, br), 1.29-0.97 (5H,br), 0.82 (3H, d), 0.76 (3H, d), 0.68-0.43 (2H, br) plus 2 protons hidden under DMSO peak and carboxylic acid proton not seen.

### Example 4

### 1-(4-Chloro-3-methylphenyl)-3-[[(trans-4-methylcyclohexyl)carbonyl](1-methylethyl)amino]-1H-pyrazole-4-carboxylic acid

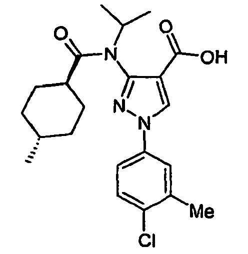

Similarly prepared by the procedure described for Example 1 from Intermediate 37.

Subsequent to purification by Method A, further purification was achieved by dissolving the compound in dioxane, applying to an SPE column (NH₂) and eluting with dioxane then dioxane:acetic acid (9:1). This purification protocol is **purification Method D**.
MS calcd for (C₂₂H₂₈ClN₃O₃ + H)⁺: 418/20
MS found (electrospray): (M+H)⁺ = 418/20

### Example 5

### 1-(4-Fluorophenyl)-3-[[(trans-4-methylcyclohexyl)carbonyl](1-methylethyl)amino]-1H-pyrazole-4-carboxylic acid

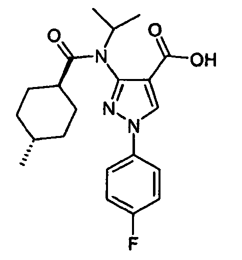

Similarly prepared by the procedure described for Example 1 from Intermediate 59.
The crude product was purified using SPE (silica) column, eluted with DCM, then DCM:MeOH (6:1, then 4:1, then, 2:1, 1:1 and finally MeOH) to give the title compound. This purification protocol is **purification Method E**.
MS calcd for (C₂₃H₃₀FN₃O₃ + H)⁺: 416
MS found (electrospray): (M+H)⁺= 416

### Example 6

### 1-(6-Indolyl)-3-[[(trans-4-methylcyclohexyl)carbonyl](1-methylethyl)amino]-1H-pyrazole-4-carboxylic acid

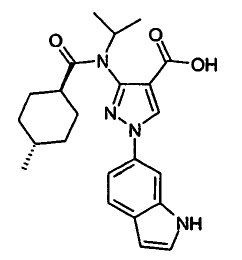

Similarly prepared by the procedure described for Example 1 from Intermediate 137. The crude product was purified using ISCO Companion chromatography over silica, eluted with DCM, then DCM:MeOH (6:1, then 4:1, then 2:1 and finally 1:1) to give the title compound. This purification protocol is **purification Method F**.
MS calcd for (C₂₅H₃₂N₄O₃ + H)⁺: 437
MS found (electrospray): (M+H)⁺ = 437

The following compounds were made from the corresponding esters by a similar procedure to that described for Example 1, using an excess of lithium hydroxide. The method of purification (A, B, C, D, E or F) is given in the following Table.

### Example 7

### 1-(4-Hydroxyphenyl)-3-[[(trans-4-methylcyclohexyl)carbonyl](1-methylethyl)amino]-1H-pyrazole-4-carboxylic acid

### Example 8

### 3-[[(trans-4-Methylcyclohexyl)carbonyl](1-methylethyl)amino]-1-[4-(trifluoromethyl)phenyl]-1H-pyrazole-4-carboxylic acid

### Example 9

### 1-[4-(Acetylamino)phenyl]-3-[[(trans-4-methylcyclohexyl)carbonyl](1-methylethyl)amino]-1H-pyrazole-4-carboxylic acid

### Example 10

### 1-(4-Biphenylyl)-3-[[(trans-4-methylcyclohexyl)carbonyl](1-methylethyl)amino]-1H-pyrazole-4-carboxylic acid

### Example 11

### 1-[4-(Dimethylamino)phenyl]-3-[[(trans-4-methylcyclohexyl)carbonyl](1-methylethyl)amino]-1H-pyrazole-4-carboxylic acid

### Example 12

### 3-[[(trans-4-Methylcyclohexyl)carbonyl](1-methylethyl)amino]-1-[4-(methyloxy)phenyl]-1H-pyrazole-4-carboxylic acid

¹H NMR (CD₃OD) δ 8.70 (1H, s), 7.70 (2H, d), 7.05 (2H, d), 4.82 (1H, m), 3.86 (3H,s), 2.06 (1H, m), 1.79 (2H, d), 1.71-1.52 (3H, br), 1.42-1.15 (5H, br), 0.98 (3H, d), 0.78 (3H, d), 0.78-0.52 (2H, br) carboxylic acid not seen

### Example 13

### 1-(4-Acetylphenyl)-3-[[(trans-4-methylcyclohexyl)carbonyl](1-methylethyl)amino]-1H-pyrazole-4-carboxylic acid

### Example 14

### 3-[[(trans-4-Methylcyclohexyl)carbonyl](1-methylethyl)amino]-1-{4-[(trifluoromethyl)oxy]phenyl}-1H-pyrazole-4-carboxylic acid

### Example 15

### 1-(4-Cyanophenyl)-3-[[(trans-4-methylcyclohexyl)carbonyl](1-methylethyl)amino]-1H-pyrazole-4-carboxylic acid

### Example 16

### 1-{4-[(Dimethylamino)carbonyl]phenyl}-3-[[(trans-4-methylcyclohexyl)carbonyl](1-methylethyl)amino]-1 H-pyrazole-4-carboxylic acid

### Example 17

### 3-[[(trans-4-Methylcyclohexyl)carbonyl](1-methylethyl)amino]-1-(3-thlenyl)-1 H-pyrazole-4-carboxylic acid

### Example 18

### 3-[[(trans-4-Methylcyclohexyl)carbonyl](1-methylethyl)amino]-1-[3-(trifluoromethyl)phenyl]-1H-pyrazole-4-carboxylic acid

### Example 19

### 1-(3,5-Dimethylphenyl)-3-[[(trans-4-methylcyclohexyl)carbonyl](1-methylethyl)amino]-1H-pyrazole-4-carboxylic acid

### Example 20

### 1-(3-Chloro-5-fluorophenyl)-3-[[(trans-4-methylcyclohexyl)carbonyl](1-methylethyl)amino]-1H-pyrazole-4-carboxylic acid

### Example 21

### 1-[3,5-Bis(trifluoromethyl)phenyl]-3-[[(trans-4-methylcyclohexyl)carbonyl](1-methylethyl)amino]-1H-pyrazole-4-carboxylic acid

### Example 22

### 1-(1,3-Benzodioxol-5-yl)-3-[[(trans-4-methylcyclohexyl)carbonyl](1-methylethyl)amino]-1H-pyrazole-4-carboxylic acid

### Example 23

### 1-(2,3-Dihydro-1-benzofuran-5-yl)-3-[[(trans-4-methylcyclohexyl)carbonyl](1-methylethyl)amino]-1H-pyrazole-4-carboxylic acid

### Example 24

### 1-(2,3-Dihydro-1,4-benzodioxin-6-yl)-3-[[(trans-4-methylcyclohexyl)carbonyl](1-methylethyl)amino]-1H-pyrazole-4-carboxylic acid

### Example 25

### 3-[[(trans-4-Methylcyclohexyl)carbonyl](1-methylethyl)amino]-1-(3,4,5-trifluorophenyl)-1H-pyrazole-4-carboxylic acid

### Example 26

### 1-(4-Chlorophenyl)-3-[[(trans-4-methylcyclohexyl)carbonyl](1-methylethyl)amino]-1H-pyrazole-4-carboxylic acid

¹H NMR (CD₃OD) δ 8.86 (1H, s), 7.82 (2H, d), 7.55 (2H, d), 4.81 (1H, m), 2.05 (1H, m), 1.78 (2H, br.d), 1.71-1.51 (3H, br), 1.40-1.18 (5H, br), 1.03-0.90 (3H, br.d), 0.80-0.75 (3H, d), 0.75-0.60 (2H, br) carboxylic acid proton not seen

### Example 27

### 3-[[(trans-4-Methylcyclohexyl)carbonyl](1-methylethyl)amino]-1-[3-(methyloxy)phenyl]-1H-pyrazole-4-carboxylic acid

### Example 28

### 3-[[(trans-4-Methylcyclohexyl)carbonyl](1-methylethyl)amino]-1-[4-(methylsulfonyl)phenyl]-1H-pyrazole-4-carboxylic acid

### Example 29

### 1-(2-Fluorophenyl)-3-[[(trans-4-methylcyclohexyl)carbonyl](1-methylethyl)amino]-1H-pyrazole-4-carboxylic acid

### Example 30

### 1-(3-Hydroxyphenyl)-3-[[(trans-4-methylcyclohexyl)carbonyl](1-methylethyl)amino]-1H-pyrazole-4-carboxylic acid

### Example 31

### 3-[[(trans-4-Methylcyclohexyl)carbonyl](1-methylethyl)amino]-1-(3-methylphenyl)-1H-pyrazole-4-carboxylic acid

### Example 32

### 1-(3-Fluorophenyl)-3-[[(trans-4-methylcyclohexyl)carbonyl](1-methylethyl)amino]-1H-pyrazole-4-carboxylic acid

¹H NMR (CD₃OD) δ 8.89 (1H, s), 7.68 (2H, m), 7.58 (1H, m), 7.15 (1H, t), 4.82 (1H, m), 2.05 (1H, m), 1.78 (2H, d), 1.62 (3H, br), 1.41-1.14 (5H, br), 0.99 (3H, br), 0.78 (3H, d), 0.78-0.62 (2H, br) carboxylic acid proton not seen

### Example 33

### 1-(4-Aminophenyl)-3-[[(trans-4-methylcyclohexyl)carbonyl](1-methylethyl)amino]-1H-pyrazole-4-carboxylic acid

### Example 34

### 1-(3-Chlorophenyl)-3-[[(trans-4-methylcyclohexyl)carbonyl](1-methylethyl)amino]-1H-pyrazole-4-carboxylic acid

### Example 35

### 3-[[(trans-4-Methylcyclohexyl)carbonyl](1-methylethyl)amino]-1-(3-[(trifluoromethyl)oxy]phenyl}-1H-pyrazole-4-carboxylic acid

### Example 36

### 1-(4-Chloro-3-fluorophenyl)-3-[[(trans-4-methylcyclohexyl)carbonyl](1-methylethyl)amino]-1H-pyrazole-4-carboxylic acid

### Example 37

### 1-(3-Amino-4-methylphenyl)-3-[[(trans-4-methylcyclohexyl)carbonyl](1-methylethyl)amino]-1H-pyrazole-4-carboxylic acid

### Example 38

### 1-(3-Fluoro-4-methylphenyl)-3-[[(trans-4-methylcyclohexyl)carbonyl](1-methylethyl)amino]-1H-pyrazole-4-carboxylic acid

¹H NMR (CD₃OD) δ 8.85 (1H, s), 7.56 (2H, d), 7.39 (1H. t), 4.82 (1H, m), 2.05 (1H, m), 1.78 (2H, d), 1.70-1.51 (3H, br), 1.40-1.15 (5H, br), 0.98 (3H, d), 0.77 (3H, d), 0.77-0.52 (2H, br) carboxylic acid not seen

### Example 39

### 1-(3,4-Difluorophenyl)-3-[[(trans-4-methylcyclohexyl)carbonyl](1-methylethyl)amino]-1H-pyrazole-4-carboxylic acid

### Example 40

### 1-[(E)-1-Hexen-1-yl]-3-[[(trans-4-methylcyclohexyl)carbonyl](1-methylethyl)amino]-1H-pyrazole-4-carboxylic acid

### Example 41

### 1-[(E)-2-Cyclohexylethenyl]-3-[[(trans-4-methylcyclohexyl)carbonyl](1-methylethyl)amino]-1H-pyrazote-4-carboxylic acid

### Example 42

### 3-[[(trans-4-Methylcyclohexyl)carbonyl](1-methylethyl)amino]-1-[(E)-4-methyl-1-penten-1-yl]-1H-pyrazole-4-carboxylic acid

### Example 43

### 1-[(E)-2-(4-Fluorophenyl)ethenyl]-3-[[(trans-4-methylcyclohexyl)carbonyl](1-methylethyl)amino]-1H-pyrazole-4-carboxylic acid

### Example 44

### 1-(4-Ethenylphenyl)-3-[[(trans-4-methylcyclohexyl)carbonyl](1-methylethyl)amino]-1H-pyrazole-4-carboxylic acid

### Example 45

### 1-[4-(Hydroxymethyl)phenyl]-3-[[(trans-4-methylcyclohexyl)carbonyl](1-methylethyl)amino]-1H-pyrazole-4-carboxylic acid

### Example 46

### 1-(4-Ethylphenyl)-3-[[(trans-4-methylcyclohexyl)carbonyl](1-methylethyl)amino]-1H-pyrazole-4-carboxylic acid

¹H NMR (CD₃OD) δ 8.77 (1H, s), 7.69 (2H, d), 7.35 (2H, d), 4.79 (1H, m), 2.70 (2H, q), 2.05 (1H, m), 1.79 (2H, d), 1.68-1.51 (3H, br), 1.40-1.16 (8H, br), 0.98 (3H, d), 0.78 (3H, d), 0.78-0.52 (2H, br) carboxylic acid not seen

### Example 47

### 3-[[(trans-4-Methylcyclohexyl)carbonyl](1-methylethyl)amino]-1-[4-(1-methylethyl)phenyl]-1H-pyrazole-4-carboxylic acid

### Example 48

### 1-(5-Acetyl-2-thienyl)-3-[[(trans-4-methylcyclohexyl)carbonyl](1-methylethyl)amino]-1H-pyrazole-4-carboxylic acid

### Example 49

### 1-(5-Chloro-2-thienyl)-3-[[(trans-4-methylcyclohexyl)carbonyl](1-methylethyl)amino]-1H-pyrazole-4-carboxylic acid

### Example 50

### 3-[[(trans-4-Methylcyclohexyl)carbonyl](1-methylethyl)amino]-1-(5-methyl-2-thienyl)-1H-pyrazole-4-carboxylic acid

### Example 51

### 3-[[(trans-4-Methylcyclohexyl)carbonyl](1-methylethyl)amino]-1-(5-phenyl-2-thienyl)-1H-pyrazole-4-carboxylic acid

### Example 52

### 1-((4-Methyl)cyclohexen-1-yl)-3-[[(trans-4-methylcyclohexyl)carbonyl](tetrahydro-2H-pyran-4-yl)amino]-1H-pyrazole-4-carboxylic acid

¹H NMR (CD₃OD): δ 8.40 (1H, s), 6.28 (1H, m), 4.63 (1 H, m), 3.94-3.81 (2H, m), 3.47 (2H, m), 2.74-2.48 (2H, m), 2.35 (1H, m), 2.01-1.12 (16H, m), 1.05 (3H, d), 0.79 (3H, d), 0.75-0.53 (2H, m) Carboxylic acid proton is assumed to be exchanged with solvent.

### Example 53

### 1-(6-Benzofuranyl)-3-[[(trans-4-methylcyclohexyl)carbonyl](1-methylethyl)amino]-1H-pyrazole-4-carboxylic acid

### Example 54

### 1-(Cyclohepten-1-yl)-3-[[(trans-4-methylcyclohexyl)carbonyl](tetrahydro-2H-pyran-4-yl)amino]-1H-pyrazole-4-carboxylic acid

### Example 55

### 1-((4-Methyl)cyclohexen-1-yl)-3-[[(trans-4-methylcyclohexyl)carbonyl](1-(methylsulfonyl)-4-piperidinyl]amino]-1H-pyrazole-4-carboxylic acid

¹H NMR (CD₃OD): δ 8.39 (1H, s), 6.28 (1 H, m), 4.52 (1 H, m), 3.74-3.61 (2H, m), 2.86-2.74 (2H, m), 2.79 (3H, s), 2.73-2.50 (2H, m), 2.35 (1H. m), 2.03-1.14 (16H excess, m), 1.05 (3H, d), 0.80 (3H, d), 0.76-0.52 (2H, m) Carboxylic acid proton is assumed to be exchanged with solvent.

### Example 56

### 1-((4,4-Dimethyl)cyclohexen-1-yl)-3-[[(trans-4-methylcyclohexyl)carbonyl](tetrahydro-2H-pyran-4-yl)amino]-1H-pyrazole-4-carboxylic acid

¹H NMR (CD₃OD): δ 8.40 (1H, s), 6.24 (1H, m), 4.64 (1 H, m), 3.96-3.81 (2H, m), 3.45 (2H, m), 2.59 (2H, m), 2.07 (2H, m), 1.97 (1H, m), 1.90-1.04 (14H, m), 1.01 (6H, s), 0.79 (3H, d), 0.75-0.54 (2H, m) Carboxylic acid proton is assumed to be exchanged with solvent

### Example 57

### 1-(3-Chloro-4-benzyloxyphenyl)-3-[[(trans-4-methylcyclohexyl)carbonyl](1-methylethyl)amino]-1H-pyrazole-4-carboxylic acid

¹H NMR (CD₃OD): δ 8.77 (1H, s), 7.90 (1H, d), 7.71 (1H, dd), 7.50 (2H, d), 7.40 (2H, t), 7.33 (1H. t), 7.29 (1H, d), 5.25 (2H, s), 4.82 (1H, quintet), 2.05 (1H, m), 1.78 (2H, br d), 1.63 (2H, br m), 1.58 (1H, br m) 1.33(2H, m), 1.24 (3H, d), 0.98 (3H, d), 0.79 (3H, d), 0.66 (2H, br m) Carboxylic acid proton is assumed to be exchanged with solvent.

### Example 58

### 1-(4-Benzyloxy-cyclohexen-1-yl)-3-[[(trans-4-methylcyclohexyl)carbonyl](1-methylethyl)amino]-1)-1H-pyrazole-4-carboxylic acid

### Example 59

### 1-(4,4-Dimethyl)cyclohexen-1-yl)-3-{[(trans-4-methylcyclohexyl)carbonyl](1-methylethyl)amino}-1H-pyrazole-4-carboxylic acid

### Example 60

### 3-[[(trans-4-Methylcyclohexyl)carbonyl](tetrahydro-2H-pyran-4-yl)amino]-1-{4-[(E)-2-phenylethenyl]phenyl}-1H-pyrazole-4-carboxylic acid

### Example 61

### 3-[[(trans-4-Methylcyclohexyl)carbonyl](tetrahydro-2H-pyran-4-yl)amino]-1-{4-[(Z)-2-phenylethenyl]phenyl}-1H-pyrazole-4-carboxylic acid

### Example 62

### 3-[[(trans-4-Methylcyclohexyl)carbonyl](1-methylethyl)amino]-1-{4[(Z)-2-(3-pyrazolyl)-ethenyl]phenyl}-1H-pyrazole-4-carboxylic acid

### Example 63

### 3-[[(trans-4-Methylcyclohexyl)carbonyl](1-methylethyl)amino]-1-{4[(E)-2-(3-pyrazolyl)-ethenyl]phenyl}-1H-pyrazole-4-carboxylic acid

### Example 64

### 3-[[(trans-4-Methylcyclohexyl)carbonyl](1-methylethyl)amino]-1-{4[(E)-2-(tetrahydro-2H-pyran-4-yl)-ethenyl]phenyl}-1H-pyrazole-4-carboxylic acid

### Example 65

### 3-[[(trans-4-Methylcyclohexyl)carbonyl](tetrahydro-2H-pyran-4-yl)amino]-1-{4-[(E)-2-(4-thiazolyl)-ethenyl]phenyl}-1H-pyrazole-4-carboxylic acid

¹H NMR (CD₃OD): δ 9.03 (1H, d), 8.88 (1H, s), 7.85 (2H, d), 7.74 (2H, d), 7.59 (1H, d), 7.50 (1H. d), 7.34 (1H, d), 4.68 (1H, m), 3.98-3.84 (2H, m), 3.49 (2H, m), 2.06 (1H. m), 1.94-1.23 (11 H, m), 0.79 (3H, d), 0.75-0.55 (2H, m) Carboxylic acid proton is assumed to be exchanged with solvent.

### Example 66

### 3-[[(trans-4-Methylcyclohexyl)carbonyl](tetrahydro-2H-pyran-4-yl)aminol-1-{4-[(Z)-2-(4-thiazolyl)-ethenyl]phenyl}-1H-pyrazole-4-carboxylic acid

¹H NMR (CD₃0D): δ 8.95 (1 H, d), 8.81 (1H, s), 7.77 (2H, d), 7.51 (2H, d), 7.32 (1 H, d), 6.81 (1H, d), 6.76 (1H, d), 4.69 (1H, m), 3.91 (2H, m), 3.49 (2H, br. q), 2.08 (1 H, m), 1.90-1.72 (5H, m), 1.70-1.51 (3H, m), 1.42-1.23 (3H, m), 0.79 (3H, d), 0.77-0.54 (2H, m). Carboxylic acid proton is assumed to be exchanged with solvent.

### Example 67

### 1-((E)-2-tert-Butyl-ethenyl)-3-[[(trans-4-methylcyclohexyl)carbonyl](1-methylethyl)amino]-1H-pyrazole-4-carboxylic acid

### Example 68

### 1-((E)-2-Phenyl-ethenyl)-3-[[(trans-4-methylcyclohexyl)carbonyl](1-methylethyl)amino]-1H-pyrazole-4-carboxylic acid

### Example 69

### 1-(4-Methyl-1-cyclohexen-1-yl)-3-[[(trans-4-methylcyclohexyl)carbonyl](1-methylethyl)amino)-1H-pyrazole-4-carboxylic acid

¹H NMR (d₆-DMSO) δ 8.00 (1H, s), 6.08 (1H, br), 4.60 (1H, m), 2.52 (2H, br.q), 2.25 (1H, br), 1.99 (1H, br), 1.90-1.45 (7H, br), 1.42-1.29 (2H, br), 1.29-1.12 (2H,br), 1.12-0.88 (9H, br), 0.76 (3H, d), 0.65-0.38 (2H, br) carboxylic acid proton not seen.

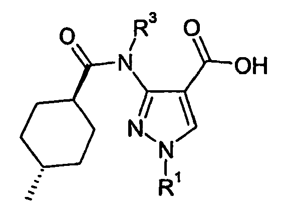

| Ex. No. | Precursor Intermediate Number | Purification method | Structure | | Molecular Formula | Mass Spectrometry | |
|---|---|---|---|---|---|---|---|
| | | | R¹ | R³ | | (M+H)⁺ Calcd | (M+H)⁺ Found |
| **7** | 7 | A | -4-OH-Ph | iPr | C₂₁H₂₇N₃O₄ | 386 | 386 |
| **8** | 8 | A | -4-CF₃-Ph | iPr | C₂₂H₂₆F₃N₃O₃ | 438 | 438 |
| **9** | 9 | A | -4-NHAc-Ph | iPr | C₂₃H₃₀N₄O₄ | 427 | 427 |
| **10** | 10 | A | -4-Ph-Ph | iPr | C₂₇H₃₁N₃O₃ | 446 | 446 |
| **11** | 11 | A | -4-NMe₂-Ph | iPr | C₂₃H₃₂N₄O₃ | 413 | 413 |
| **12** | 12 | A | -4-OMe-Ph | iPr | C₂₂H₂₉N₃O₄ | 400 | 400 |
| **13** | 13 | A | 4-Ac-Ph | iPr | C₂₃H₂₉N₃O₄ | 412 | 412 |
| **14** | 14 | A | -4-OCF₃-Ph | iPr | C₂₂H₂₆F₃N₃O₄ | 454 | 454 |
| **15** | 15 | A | 4-CN-Ph | iPr | C₂₂H₂₆N₄O₃ | 395 | 395 |
| **16** | 16 | B | 4-CONMe₂-Ph | iPr | C₂₄H₃₂N₄O₄ | 441 | 441 |
| **17** | 17 | C | 3-thienyl | iPr | C₁₉H₂₅N₃O₃S | 376 | 376 |
| **18** | 18 | A | 3-CF₃-Ph | iPr | C₂₂H₂₆F₃N₃O₃ | 438 | 438 |
| **19** | 19 | D | 3,5-di-Me-Ph | iPr | C₂₃H₃₁N₃O₃ | 398 | 398 |
| **20** | 20 | D | 3-Cl-5-F-Ph | iPr | C₂₁H₂₅ClFN₃O₃ | 422 | 422 |
| **21** | 21 | D | 3,5-di-CF₃-Ph | iPr | C₂₃H₂₅F₆N₃O₃ | 506 | 506 |
| **22** | 22 | D | 1,3-benzodioxol-5-yl | iPr | C₂₂H₂₇N₃O₅ | 414 | 414 |
| **23** | 23 | D | 2,3-dihydro-1-benzoturan-5-yl | iPr | C₂₃H₂₉N₃O₄ | 412 | 412 |
| **24** | 24 | D | 2,3-dihydro-1,4-benzodioxin-6-yl | iPr | C₂₃H₂₉N₃O₅ | 428 | 428 |
| **25** | 25 | D | 3,4,5-tri-F-Ph | iPr | C₂₁H₂₄F₃N₃O₃ | 424 | 424 |
| **26** | 26 | A | 4-Cl-Ph | iPr | C₂₁H₂₆CIN₃O₃ | 402/04 | 402/04 |
| **27** | 27 | A | 3-OMe-Ph | iPr | C₂₂H₂₉N₃O₄ | 400 | 400 |
| **28** | 28 | A | 4-MeSO₂-Ph | iPr | C₂₂H₂₆N₃O₅S | 448 | 448 |
| **29** | 29 | A | 2-F-Ph | iPr | C₂₁H₂₆FN₃O₃ | 388 | 388 |
| **30** | 30 | A | 3-OH-Ph | iPr | C₂₁H₂₇N₃O₄ | 386 | 386 |
| **31** | 31 | A | 3-Me-Ph | iPr | C₂₂H₂₉N₃O₄ | 384 | 384 |
| **32** | 32 | A | 3-F-Ph | iPr | C₂₁H₂₆FN₃O₃ | 388 | 388 |
| **33** | 33 | A | 4-NH₂-Ph | iPr | C₂₁H₂₈N₄O₃ | 385 | 385 |
| **34** | 34 | A | 3-Cl-Ph | iPr | C₂₁H₂₆CIN₃O₃ | 402/04 | 402/04 |
| **35** | 35 | A | 3-OCF₃-Ph | iPr | C₂₂H₂₆F₃N₃O₄ | 454 | 454 |
| **36** | 36 | A | 3-F-4-Cl-Ph | iPr | C₂₂H₂₈ClN₃O₃ | 418/420 | 418/420 |
| **37** | 38 | A | 3-NH₂-4-Me-Ph | iPr | C₂₂H₃₀N₄O₃ | 399 | 399 |
| **38** | 39 | A | 3-F-4-Me-Ph | iPr | C₂₂H₂₈FN₃O₃ | 402 | 402 |
| **39** | 40 | B | 3,4-di-F-Ph | iPr | C₂₁H₂₅F₂N₃O₃ | 406 | 406 |
| **40** | 41 | A | (E)-1-hexen-1-yl | iPr | C₂₁H₃₃N₃O₃ | 376 | 376 |
| **41** | 42 | A | (E)-2-cyclohexylethenyl | iPr | C₂₃H₃₅N₃O₃ | 402 | 402 |
| **42** | 43 | A | (E)-4-methyl-1-penten-1-yl | iPr | C₂₁H₃₃N₃O₃ | 376 | 376 |
| **43** | 44 | A | (E)-2-(4-fluorophenyl)ethenyl | iPr | C₂₃H₂₈FN₃O₃ | 414 | 414 |
| **44** | 45 | A | 4-ethenylphenyl | iPr | C₂₃H₂₉N₃O₃ | 396 | 396 |
| **45** | 46 | A | 4-CH₂OH-Ph | iPr | C₂₂H₂₉N₃O₃ | 400 | 400 |
| **46** | 47 | D | 4-Et-Ph | iPr | C₂₃H₃₁N₃O₃ | 398 | 398 |
| **47** | 48 | D | 4-iPr-Ph | iPr | C₂₄H₃₃N₃O₃ | 412 | 412 |
| **48** | 61 | D | 5-Ac-2-thienyl | iPr | C₂₁H₂₇N₃O₄S | 418 | 418 |
| **49** | 62 | D | 5-Cl-2-thienyl | iPr | C₁₉H₂₄CIN₃O₃S | 410/12 | 410/12 |
| **50** | 63 | D | 5-Me-2-thienyl | iPr | C₂₀H₂₇N₃O₃S | 390 | 390 |
| **51** | 64 | D | 5-Ph-2-thienyl | iPr | C₂₅H₂₉N₃O₃S | 452 | 452 |
| **52** | 144 | A | 4-methyl-1-cyclohexen-1-yl | pyran-4-yl | C₂₄H₃₅N₃O₄ | 430 | 430 |
| **53** | 138 | A | 6-benzofuranyl | iPr | C₂₃H₂₇N₃O₄ | 410 | 410 |
| **54** | 143 | A | cyGohepten-1-yl | pyran-4-yl | C₂₃H₂₇N₃O₄ | 430 | 430 |
| **55** | 146 | A | 4-methyl -1-cydohexen-1-yl | 1-Methyl sulfonyl-4-piperidinyl | C₂₅H₃₈N₄O₅S | 507 | 507 |
| **56** | 142 | A | 4,4-dimethyl-cyclohexen-1-yl | pyran-4-yl | C₂₅H₃₇N₃O₄ | 444 | 444 |
| | | | | | | | |
| **57** | 55 | A | 3-Cl-4-BnO-Ph | iPr | C₂₈H₃₂CIN₃O₄ | 510/512 | 510/512 |
| **58** | 152 | E | 4-benzyloxy -1-cyclohexen-1-yl | iPr | C₂₈H₃₇N₃O₄ | 480 | 480 |
| **59** | 141 | C | 4,4-dimethyl-cyclohexen-1-yl | iPr | C₂₃H₃₅N₃O₃ | 402 | 402 |
| **60** | 99 | B | 4-([E]-Ph-ethenyl)Ph | pyran-4-yl | C₃₁H₃₅N₃O₄ | 514 | 514 |
| **61** | 99 | B | 4-([Z]-Ph-ethenyl)Ph | pyran-4-yl | C₃₁H₃₅N₃O₄ | 514 | 514 |
| **62** | 106 | A | 4-([Z]-(pyrazol-3-yl)ethenyl)Ph | iPr | C₂₆O₃₀N₅O₄ | 462 | 462 |
| **63** | 105 | A | 4-([E]-(pyrazol-3-yl)ethenyl)Ph | iPr | C₂₆H₃₀N₅O₄ | 462 | 462 |
| **64** | 107 | A | 4-([E]-(pyran-4-yl)ethenyl)Ph | iPr | C₂₈H₃₇N₃O₄ | 480 | 480 |
| **65** | 100 | B | 4-([E]-(1,3-thiazol-4-yl)ethenyl)Ph | pyran-4-yl | C₂₈H₃₂N₄O₄S | 521 | 521 |
| **66** | 100 | B | 4-([Z]-(1,3-thiazol-4-yl)ethenyl)Ph | pyran-4-yl | C₂₈H₃₂N₄O₅S | 521 | 521 |
| **67** | 58 | D | (E)-2-tert-butylethenyl | iPr | C₂₁H₃₃O₃ | 376 | 376 |
| **68** | 53 | C | (E)-2-phenylethenyl | iPr | C₂₃H₂₉N₃O₃ | 396 | 396 |
| **69** | 126 | C | 4-Methyl-1-cyclohexen-1-yl | iPr | C₂₂H₃₃N₃O₃ | 387 | 387 |
| | | | | | | | |

### Example 70

### 1-(3-Cyanophenyl)-3-[[(trans-4-methylcyclohexyl)carbonyl](1-methylethyl)amino]-1H-pyrazole-4-carboxylic acid

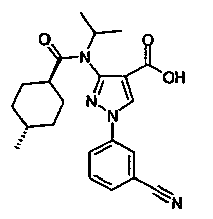

To 3-cyanophenylboronic acid (91 mg, 0.62 mmol) was added copper (II) acetate (85 mg, 0.55 mmol), Intermediate 66 (100 mg, 0.31 mmol) in dry THF (2 mL) and pyridine (50 uL, 0.62 mmol). The reaction was stirred at room temperature, in air for 16 hours. The solvent was removed and the residue purified by MDAP HPLC (purification method B) to give the title compound.
MS calcd for (C₂₂H₂₆N₄O₃ + H)⁺: 395
MS found (electrospray): (M+H)⁺ = 395

### Example 71

### 3-{(1-Methylethyl)[(4-methylidenecyclohexyl)carbonyl]amino}-1-phenyl-1H-pyrazole-4-carboxylic acid

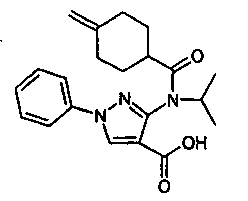

Intermediate 90 (122 mg, 0.31 mmol) was dissolved in THF (2 mL) and ethanol (2 mL). 2N sodium hydroxide solution (0.93 mL, 1.8 mmol) was added and the mixture stirred at room temperature for 2 days.
The residue was purified according to purification method A. Thus, the volatiles were removed *in vacuo* and the residue partitioned between 2N aqueous hydrochloric acid and ethyl acetate. The organic phase was separated, washed with brine, dried (Na₂SO₄) and concentrated. The residue was triturated with ether to give the title compound.
MS calcd for (C₂₁H₂₅N₃O₃ + H)⁺: 368
MS found (electrospray): (M+H)⁺ = 368

### Example 72

### 1-(4-Trifluoromethyl-cyclohexen-1-yl)-3-[[(trans-4-methylcyclohexyl)carbonyl](1-methylethyl)amino]-1H-pyrazole-4-carboxylic acid

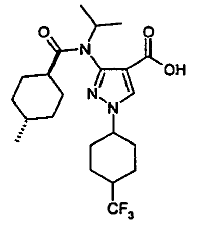

Similarly prepared by the procedure described for Example 71 from Intermediate 139.
The residue was purified according to purification method F. Thus, the volatiles were removed *in vacuo* and the residue partitioned between 2N aqueous hydrochloric acid and ethyl acetate. The organic phase was separated, washed with brine, dried (Na₂SO₄) and concentrated. The residue was purified by SPE (silica) column, eluted with a gradient of ethyl acetate in cyclohexane (20% to 60%) to give the title compound.
MS calcd for (C₂₂H₃₄F₃N₃O₃ + H)⁺: 442
MS found (electrospray): (M+H)⁺= 442

The following compounds were made from the corresponding esters by a similar procedure to that described for Example 71. The method of purification (A or B) is given in the following Table.

### Example 73

### 3-[[(trans-4-Methylcyclohexyl)carbonyl](1-methylethyl)amino]-1-{4-[(phenyloxy)methyl] phenyl}-1H-pyrazole-4-carboxylic acid

¹H NMR (CDCl₃): 8.58 (1H, s), 7.76 (2H, d), 7.61 (2H, d), 7.32 (2H, m), 6.95 (3H, m), 5.15 (2H, s), 4.93 (1H, m), 2.00 (1H, m), 1.56-1.85 (5H, m), 1.24-1.48. (5H, m), 0.99 (3H, d), 0.78 (3H, d), 0.55-0.75 (2H, m). Carboxylic acid proton is assumed to be exchanged with moisture in the solvent.

### Example 74

### 1-[4-(Phenylsulfonylmethyl)phenyl]-3-[[(trans-4-methylcyclohexyl)carbonyl](1-methylethyl)amino]-1H-pyrazole-4-carboxylic acid

### Example 75

### 1-[4-(Phenylthiomethyl)phenyl]-3-[[(trans-4-methylcyclohexyl)carbonyl](1-methylethyl)amino]-1H-pyrazole-4-carboxylic acid

### Example 76

### 1-[4-(Phenoxy)phenyl]-3-[[(trans-4-methylcyclohexyl)carbonyl](1-methylethyl)amino]-1H-pyrazole-4-carboxylic acid

### Example 77

### 1-[4-{(1,3-Thiazol-4-ylmethyl)oxy}phenyl]-3-[[(trans-4-methylcyclohexyl)carbonyl](1-methylethyl)amino]-1H-pyrazole-4-carboxylic acid

### Example 78

### 1-[4-([E]-Phenylethenyl)phenyl]-3-[[(trans-4-methylcyclohexyl)carbonyl](1-methylethyl)amino]-1H-pyrazole-4-carboxylic acid

### Example 79

### 1-[4-[Z]-Phenylethenyl))phenyl]-3-[[(trans-4-methylcyclohexyl)carbonyl](1-methylethyl)amino]-1H-pyrazole-4-carboxylic acid

### Example 80

### 1-[4-([E,Z]-(1,3-Thiazol-2-yl)ethenyl)phenyl]-3-[[(trans-4-methylcyclohexyl)carbonyl](1-methylethyl)amino]-1H-pyrazole-4-carboxylic acid

### Example 81

### 1-[4-([E]-Phenyl-2-methylethenyl)phenyl]-3-[[(trans-4-methylcyclohexyl)carbonyl](1-methylethyl)amino]-1H-pyrazole-4-carboxylic acid

### Example 82

### 1-[4-[E]-(Pyridin-4-yl)ethenyl))phenyl]-3-[[(trans-4-methylcyclohexyl)carbonyl](1-methylethyl)amino]-1H-pyrazole-4-carboxylic acid

¹ H NMR (CD₃OD): δ 8.56 (1H, s), 8.46 (2H, d), 7.75 (2H, d), 7.67 (2H, d), 7.43 (2H, d), 7.35 (1H, d), 7.06 (1H, d), 4.81 (1H, s), 2.02-1.93 (1H, m), 1.80-1.65 (2H, m), 1.64-1.47 (3H, m), 1.40-1.11 (5H, m), 1.02-0.85 (3H, m), 0.72 (3H, d), 0.70-0.50 (2H, m) Carboxylic acid proton is assumed to be exchanged with solvent.

### Example 83

### 1-[4-([E)-(1,3-Thiazol-4-yl)ethenyl)phenyl]-3-[[(trans-4-methylcyclohexyl)carbonyl](1-methylethyl)amino]-1H-pyrazole-4-carboxylic acid

¹H NMR (CDCl₃): 8.93 (1H, d), 8.58 (1H, s), 7.76 (2H, d), 7.68 (2H, d), 7.56 (1H, d), 7.33 (1H, d), 7.23 (2H, d), 4.97 (1H, m), 2.00 (1H, m), 1.87-1.56 (4H, m), 1.24-1.50. (6H, m), 0.87-1.05 (3H, m), 0.78 (3H, d), 0.75-0.55 (2H, m). Carboxylic acid proton is assumed to be exchanged with moisture in the solvent.

### Example 84

### 1-[4-([E]-(Furan-2-yl)ethenyl))phenyl]-3-[[(trans-4-methylcyclohexyl)carbonyl](1-methylethyl)amino]-1H-pyrazole-4-carboxylic acid

### Example 85

### 1-[4-([E]-(2-Methyl-1,3-thiazol-4-yl)ethenyl)phenyl]-3-[[(trans-4-methylcyclohexyl)carbonyl](1-methylethyl)amino]-1H-pyrazole-4-carboxylic acid

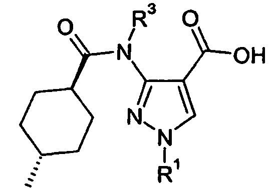

| Example Number | Precursor | Purifi cation | Structure | | Molecular Formula | Mass Spectrometry | |
|---|---|---|---|---|---|---|---|
| | Intermediate Number | meth od | R¹ | R³ | | (M+H)⁺ Calcd | (M+H)⁺ Found |
| **73** | 149 | B | 4-PhOCH₂-Ph | iPr | C₂₈H₃₃N₃O₄ | 476 | 476 |
| **74** | 148 | A | 4-Ph-SO₂CH₂-Ph | iPr | C₂₈H₃₃N₃O₅S | 524 | 524 |
| **75** | 147 | B | 4-Ph-SCH₂-Ph | iPr | C₂₈H₃₃N₃O₃S | 492 | 492 |
| **76** | 54 | A | 4-PhO-Ph | iPr | C₂₇H₃₁N₃O₄ | 462 | 462 |
| **77** | 150 | B | 4-[(1,3-thiazol-4-ylmethyl)oxy]Ph | iPr | C₂₅H₃₀N₄O₄S | 483 | 483 |
| **78** | 91 | B | 4-([E]-Ph-ethenyl)Ph | iPr | C₂₉H₃₃N₃O₃ | 472 | 472 |
| **79** | 91 | B | 4-([Z]-Ph-ethenyl)Ph | iPr | C₂₉H₃₃N₃O₃ | 472 | 472 |
| **80** | 93 | A | 4-([E,Z]-(1,3-thiazo)-2-yl)ethenyl)Ph | iPr | C₂₆H₃₀N₄O₃S | 479 | 479 |
| **81** | 94 | B | 4-([E]-Ph-2-methylethenyl)Ph | iPr | C₃₀H₃₅N₃O₃ | 486 | 486 |
| **82** | 95 | B | 4-([E]-(pyridin-4-yl)ethenyl)Ph | iPr | C₂₈H₃₂N₄O₃ | 473 | 473 |
| **83** | 96 | B | 4-([E]-(1,3-thiazol-4-yl)ethenyl)Ph | iPr | C₂₆H₃₀N₄O₃S | 479 | 479 |
| **84** | 104 | B | 4-([E]-(furan-3-yl)ethenyl)Ph | iPr | C₂₇H₃₁N₃O₄ | 462 | 462 |
| **85** | 98 | B | 4-([E]-(2-methyl-1,3-thiazol-4-yl)ethenyl)Ph | iPr | C₂₇H₃₂N₄O₃S | 493 | 493 |

### Example 86

### 3-[(Cyclohexylacetyl)(1-methylethyl)amino]-1-phenyl-1H-pyrazole-4-carboxylic acid

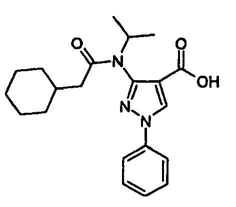

Prepared by a similar method to that described for Example 2 replacing Intermediate 2 with Intermediate 67. Purified by MDAP HPLC (purification method B) to give the title compound. MS calcd for (C₂₁H₂₇N₃O₃ + H)⁺: 370
MS found (electrospray): (M+H)⁺ = 370
¹H NMR (CDCl₃): δ 8.58 (1H, s), 7.75 (2H, d), 7.54 (2H, t), 7.42 (1H, t), 5.01 (1H, m), 2.00 (2H, bm), 1.88 (1H, bm), 1.69 (2H, bm), 1.60 (2H, bm), 1.26 (5H, bm), 1.03 (5H, bm), 0.78 (2H, bm). Carboxylic acid proton not seen.

### Example 87

### 3-{(1-Methylethyl)[(4-methylphenyl)carbonyl]amino}-1-phenyl-1H-pyrazole-4-carboxylic acid

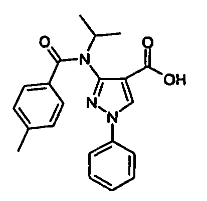

Prepared by a similar method to that described for Example 2 replacing Intermediate 2 with Intermediate 68 to give the title compound.
MS calcd for (C₂₁H₂₁N₃O₃ + H)⁺: 364
MS found (electrospray): (M+H)⁺= 364
¹H NMR (CDCl₃): δ 8.58 (1H, s), 7.79 (2H, d), 7.52 (2H, t), 7.38 (1H, t), 7.25 (2H, d), 7.00 (2H, d), 4.82 (1H, m), 2.22 (3H; s), 1.25 (6H, d). Carboxylic acid proton not seen.

### Example 88

### 3-[[(4-Bromo-2-chlorophenyl)carbonyl](1-methylethyl)amino]-1-phenyl-1H-pyrazole-4-carboxylic acid

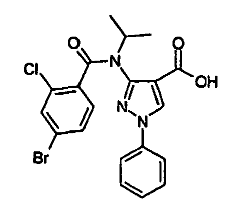

Prepared by a similar method to that described for Example 1 replacing Intermediate 6 with Intermediate 69 to give the title compound.
MS calcd for (C₂₀H₁₇BrClN₃O₃ + H)⁺: 460/2
MS found (electrospray): (M+H)⁺ = 460/2

### Example 89

### 3-[[(trans-4-Methylcyclohexyl)carbonyl)(phenyl)amino)-1-phenyl-1H-pyrazole-4-carboxylic acid

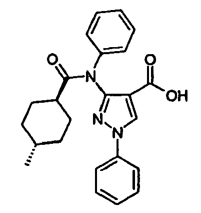

Prepared by a similar method to that described for Example 1 replacing Intermediate 6 with Intermediate 71 to give the title compound.
MS calcd for (C₂₄H₂₅N₃O₃ + H)⁺: 404
MS found (electrospray): (M+H)⁺= 404

### Example 90

### 3-([2-(Dimethylamino)-2-oxoethyl][(trans-4-methylcyclohexyl)carbonyl]amino)-1-phenyl-1H-pyrazole-4-carboxylic acid

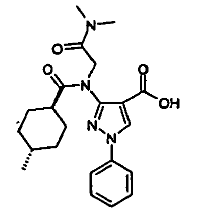

Prepared by a similar method to that described for Example 1 replacing Intermediate 6 with Intermediate 73 to give the title compound.
MS calcd for (C₂₂H₂₈N₄O₄ + H)⁺: 413
MS found (electrospray): (M+H)⁺= 413

### Example 91

### 3-([(trans-4-Methylcyclohexyl)carbonyl]{1-[(methyloxy)carbonyl]-4-piperidinyl}amino)-1-phenyl-1H-pyrazole-4-carboxylic acid

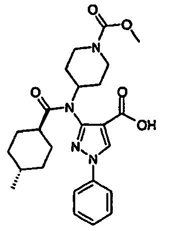

Prepared by a similar method to that described for Example 1 replacing Intermediate 6 with Intermediate 80 to give the title compound.
MS calcd for (C₂₅H₃₂N₄O₅ + H)⁺: 469
MS found (electrospray): (M+H)⁺= 469

### Example 92

### 3-{[(trans-4-Methylcyclohexyl)carbonyl][1-(methylsulfonyl)-4-piperidinyl]amino}-1-phenyl-1H-pyrazole-4-carboxylic acid

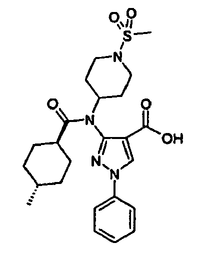

Prepared by a similar method to that described for Example 1 replacing intermediate 6 with Intermediate 81 to give the title compound.
MS calcd for (C₂₄H₃₂N₄O₅S + H)⁺: 489
MS found (electrospray): (M+H)⁺ = 489

### Example 93

### 3-[[(trans-4-Methylcyclohexyl)carbonyl](1-methyl-4-piperldinyl)amino]-1-phenyl-1H-pyrazole-4-carboxylic acid

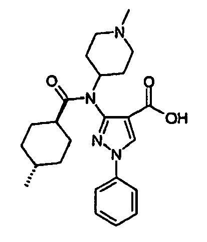

Prepared by a similar method to that described for Example 1 replacing Intermediate 6 with Intermediate 83 to give the title compound.
MS calcd for (C₂₄H₃₂N₄O₃ + H)⁺: 425
MS found (electrospray): (M+H)⁺ = 425
¹H NMR (CD₃OD) δ 8.91 (1H, s), 7.84 (2H, d), 7.55 (2H, t), 7.45 (1H, m), 4.75 (1H, m), 3.50 (2H, br), 3.17 (2H, br.t), 2.78 (3H, s), 2.22 (1H, br), 2.15-2.02 (3H, br), 1.77 (2H, br), 1.70-1.50 (4H, br), 1.40-1.25 (2H, br), 0.80 (3H, d), 0.80-0.55 (2H, br) carboxylic acid proton not seen.

### Example 94

### 3-{{1-[(Ethylamino)carbonyl]-4-piperidinyl}[(trans-4-methylcyclohexyl)carbonyl]amino}-1-phenyl-1H-pyrazole-4-carboxylic acid

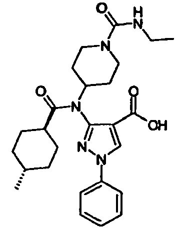

Prepared by a similar method to that described for Example 1 replacing Intermediate 6 with Intermediate 84 to give the title compound.
MS calcd for (C₂₆H₃₅N₅O₄ + H)⁺: 482
MS found (electrospray): (M+H)⁺ = 482

### Example 95

### 3-[[(trans-4-Methylcyclohexyl)carbonyl](2-pyrazinylmethyl)amino]-1-phenyl-1H-pyrazole-4-carboxylic acid

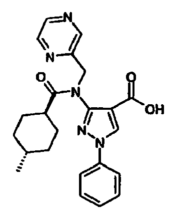

Prepared by a similar method to that described for Example 1 replacing Intermediate 6 with Intermediate 86 to give the title compound.
MS calcd for (C₂₃H₂₅N₅O₃ + H)⁺: 420
MS found (electrospray): (M+H)⁺= 420

### Example 96

### rel-3-[{[(1S,2R,4S)-2-Hydroxy-4-methylcyclohexyl]carbonyl}(1-methylethyl)amino]-1-phenyl-1H-pyrazole-4-carboxylic acid

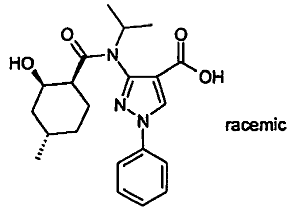

Prepared by a similar method to that described for Example 1 replacing Intermediate 6 with Intermediate 87 to give the title compound.
MS calcd for (C₂₁H₂₇N₃O₄ ⁺ H)⁺: 386
MS found (electrospray): (M+H)⁺= 386

### Example 97

### 3-[[(trans-4-Methylcyclohexyl)carbonyl](1-rnethylethyl)amino]-1-{4-[(3-methoxyphenylcarbonyl)amino]phenyl}-1H-pyrazole-4-carboxylic acid

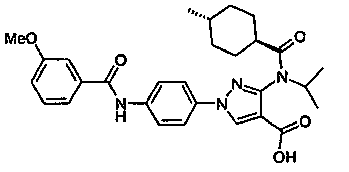

Prepared by a similar method to that described for Example 3 replacing intermediate 52 with intermediate 162 to give the title compound.
MS calcd for (C₂₉H₃₄N₄O₅ + H)⁺: 519
MS found (electrospray): (M+H)⁺= 519

### Example 98

### 3-[[(trans-4-Methylcyclohexyl)carbonyl](1-methylethyl)amino]-1-{4-[(phenylmethyl)oxy]phenyl}-1H-pyrazole-4-carboxylic acid

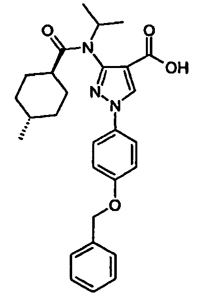

Prepared by a similar method to that described for Example 71 replacing Intermediate 90 with Intermediate 49, and purified by MDAP HPLC to give the title compound.
MS calcd for (C₂₈H₃₃N₃O₄ + H)⁺: 476
MS found (electrospray): (M+H)⁺= 476

### Example 99

### 1-(1H-Indol-5-yl)-3-[[(trans-4-methylcyclohexyl)carbonyl](1-methylethyl)amino]-1H-pyrazole-4-carboxylic acid

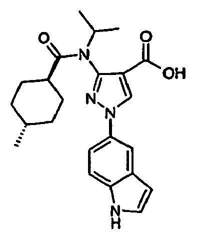

Prepared by a similar method to that described for Example 71 replacing Intermediate 90 with Intermediate 50 and purfied by method B to give the title compound.
MS calcd for (C₂₃H₃₂N₄O₃ + H)⁺: 409
MS found (electrospray): (M+H)⁺ = 409
¹H NMR (CDCl₃) δ 8.63 (1H, br.s), 8.53 (1H, s), 7.95 (1H, s), 7.53 (2H, br.s), 7.36 (1H, t), 6.67 (1H, m), 4.99 (1H, m), 2.11-2.01 (1H, m), 1.92-1.56 (6H, m), 1.37-1.24 (5H, m), 1.06-0.98 (3H, m), 0.81-0.74 (4H, m) carboxylic acid proton not seen.

### Example 100

### 3-[[(trans-4-Methylcyclohexyl)carbonyl](1-methylethyl)amino]-1-{4-[(E/Z)-2-phenylethenyl]phenyl}-1H-pyrazole-4-carboxylic acid

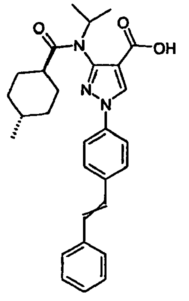

Prepared by a similar method to that described for Example 71 replacing Intermediate 90 with Intermediate 91 to give the title compound.
MS calcd for (C₂₉H₃₃N₃O₃ + H)⁺: 472
MS found (electrospray): (M+H)⁺= 472
¹H NMR (CDCl₃) δ 8.58 (1H, s), 8.51 (1H, s), 7.74 (2H, d), 7.67 (2H, d), 7.61-7.53 (4H, m), 7.43-7.37 (4H, m), 7.34-7.24 (6H, m), 7.19 (1H, d), 7.14 (1H, d), 6.72 (1H, d), 6.61 (1H, d), 4.98 (2H, m), 2.05-1.93 (2H, m), 1.88-0.56 (36H, m) carboxylic acid proton not seen.

### Example 101

### 3-[[(trans-4-Methylcyclohexyl)carbonyl](1-methylethyl)amino]-1-[4-(2-phenylethyl)phenyl]-1H-pyrazole-4-carboxylic acid

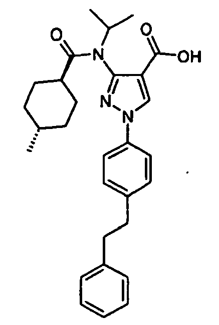

Example 100 (266 mg) in ethanol (10 mL) was subjected to atmospheric pressure hydrogenation with 10% palladium on carbon (60 mg, wet) for 3 hours. The reaction was filtered through Celite and concentrated.
The product was purified by silica flash column (20 g), eluted with a gradient of ethyl acetate in cyclohexane to give the title compound.
MS calcd for (C₂₉H₃₅N₃O₃ + H)⁺: 474
MS found (electrospray): (M+H)⁺= 474
¹H NMR (CDCl₃) δ 8.55 (1H, s), 7.69-7.59 (2H, m), 7.39-7.16 (7H, m), 4.99 (1H, m), 3.09-2.90 (4H, m), 2.09-1.95 (1H, m), 1.90-0.55 (18H, m) carboxylic acid proton not seen.

The following compounds were prepared by a similar procedure to that described for Example 101.

### Example 102

### 3-[[(trans-4-Methylcyclohexyl)carbonyl](tetrahydro-2H-pyran-4-yl)amino]-1-{4-[2-phenylethyl]phenyl}-1H-pyrazole-4-carboxylic acid

### Example 103

### 3-[[(trans-4-Methylcyclohexyl)carbonyl](1-methylethyl)amino]-1-[4-[(1,3-thiazol-4-yl)-ethyl]phenyl]-1H-pyrazole-4-carboxylic acid

¹H NMR (CDCl₃): 8.85 (1H, d), 8.52 (1H, s), 7.66 (2H, d), 7.45-7.25 (4H, m), 6.95 (1H, d), 4.97 (1H, m), 3.25-3.10 (4H, m), 2.00 (1H, m), 1.20-1.87 (7H, m), 0.87-1.10 (6H, m), 0.76 (3H, d), 0.75-0.55 (2H, m). Carboxylic acid proton is assumed to be exchanged with moisture in the solvent.

### Example 104

### 3-[[(trans-4-Methylcyclohexyl)carbonyl](tetrahydro-2H-pyran-4-yl)amino]-1-(4-[(1,3-thiazol-4-yl)-ethyl]phenyl}-1H-pyrazole-4-carboxylic acid

¹H NMR (CD₃OD): δ 8.96 (1H, s), 8.41 (1H, s), 7.64 (2H, d), 7.30 (2H, d), 7.16 (1H, d), 4.65 (1H, m), 3.97-3.81 (2H, m), 3.47 (2H, m), 3.18-3.05 (4H, m), 2.15 (1H, m), 2.0-1.21 (11H excess, m), 0.77 (3H, d), 0.74-0.52 (2H, m) Carboxylic acid proton is assumed to be exchanged with solvent

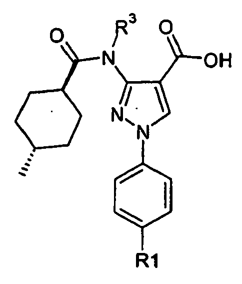

| Example Number | Precursor | Structure | | Molecular Formula | Mass Spectrometry | |
|---|---|---|---|---|---|---|
| | Example Number | R¹ | R³ | | (M+H)⁺ Calcd | (M+H)⁺ Found |
| **102** | Ex 61 | PhCH2CH2 | pyran-4-yl | C₃₁H₃₇N₃O₄ | 516 | 516 |
| **103** | Ex 83 | (1,3-thiazol-4-yl)- | iPr | C₂₆H₃₂N₄O₃S | 481 | 481 |
| | | CH2CH2 | | | | |
| **104** | Ex 66 | (1,3-thiazol-4-yl)- | pyran-4-yl | C₂₈H₃₄N₄O₄S | 523 | 523 |
| | | CH2CH2 | | | | |

### Example 105

### 1-Cyclohexyl-3-[[(trans-4-methylcyclohexyl)carbonyl](1-methylethyl)amino]-1H-pyrazole-4-carboxylic acid

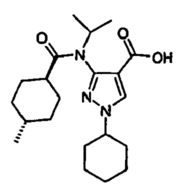

Example 3 (30 mg, 0.08 mmol) in ethanol (4 mL) was subjected to atmospheric pressure hydrogenation with 10% palladium on carbon, (wet, 10 mg) until hydrogen uptake had ceased. The reaction was filtered through Celite and concentrated to give the title compound.
MS calcd for (C₂₁H₃₃N₃O₃ + H)⁺: 376
MS found (electrospray): (M+H)⁺= 376

### Example 106

### 3-[[(trans-4-Methylcyclohexyl)carbonyl](1-methylethyl)amino]-1-[1-(methylsufonyl)-1,2,3,6-tetrahydro-4-pyridinyl]-1H-pyrazole-4-carboxylic acid

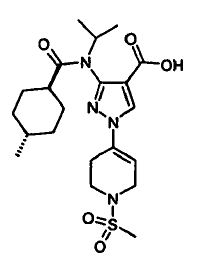

Prepared by a similar method to that described for Example 3 replacing Intermediate 52 with Intermediate 111 to give the title compound.
MS calcd for (C₂₃H₃₆N₄O₅S + H)⁺: 453
MS found (electrospray): (M+H)⁺ = 453

### Example 107

### 3-[[(trans-4-Methylcyclohexyl)carbonyl](phenylmethyl)amino]-1-phenyl-1H-pyrazole-4-carboxylic acid

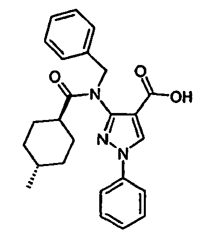

Prepared by a similar method to that described for Example 1 replacing Intermediate 6 with Intermediate 127 to give the title compound.
MS calcd for (C₂₅H₂₇N₃O₃ + H)⁺: 418
MS found (electrospray): (M+H)⁺= 418

### Example 108

### 3-{Cyclopentyl[(trans-4-methylcyclohexyl)carbonyl]amino}-1-phenyl-1H-pyrazole-4-carboxylic acid

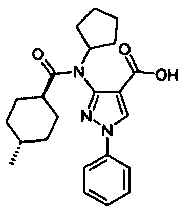

Prepared by a similar method to that described for Example 2 replacing Intermediate 2 with Intermediate 129 to give the title compound.
¹H NMR (CDCl₃) δ 8.56 (1H, s), 7.75 (2H, d), 7.55 (2H, t), 7.43 (1H, t), 4.91 (1H, m), 4.14 (1H, q), 2.11 (1H, br), 1.97 (1H, br), 1.85 (2H, br), 1.75-1.14 (11H, br), 0.77 (3H, d), 0.76-0.55 (2H, br) carboxylic acid proton not seen.

### Example 109

### 3-[[(trans-4-Methylcyclohexyl)carbonyl](tetrahydro-2H-pyran-4-yl)amino]-1-phenyl-1H-pyrazole-4-carboxylic acid

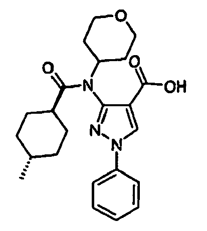

Prepared by a similar method to that described for Example 2 replacing Intermediate 2 with Intermediate 132 to give the title compound.
¹H NMR (CDCl₃) δ 8.57 (1H. s), 7.75 (2H, d), 7.55 (2H, t), 7.44 (1H, t), 4.88 (1H, m), 4.14 (2H, q), 4.02-3.87 (2H, br), 3.50 (2H, m), 2.05-1.55 (6H, br), 1.41-1.20 (5H, br), 0.78 (3H, d), 0.77-0.55 (2H, br) carboxylic acid proton not seen.

### Example 110

### 3-{(1-Acetyl-4-piperidinyl)[(trans-4-methylcyclohexyl)carbonyl]amino}-1-phenyl-1H-pyrazole-4-carboxylic acid

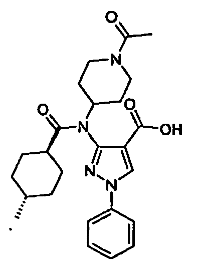

Prepared by a similar method to that described for Example 2 replacing Intermediate 2 with Intermediate 136 and purified by MDAP HPLC (purification method B) to give the title compound.
¹H NMR (CD₃OD) δ 8.80 (1H, s), 7.82 (2H, d), 7.55 (2H, t), 7.42 (1H, t), 4.71 (1H, br), 4.56 (1H, br), 3.94 (1H, br), 2.20 (1H, br), 2.65 (2H, br), 2.05-0.55 (19H, br) carboxylic acid proton not seen.

### Example 111

### 3-[[(trans-4-Methylcyclohexyl)carbonyl](4-piperidinyl)amino]-1-phenyl-1H-pyrazole-4-carboxylic acid

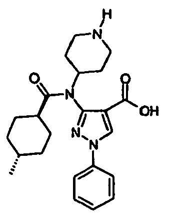

A solution of Example 121 (10 mg, 0.02 mmol, *supra*) in DCM (2 mL) and TFA (0.5 mL) was stirred at room temperature for 3 hours. The solvent was evaporated, and the residue purified by SPE (C18, reverse phase) to give the title compound.
¹H NMR (MeOD): δ 8.55 (1H, s), 7.80 (2H, m), 7.52 (2H, m), 7.38 (1H, m), 4.69 (1H, m), 3.35 (1H, m), 3.07 (2H, m), 12.18 (3H, m), 1.87 (3H, m), 1.25-1.64 (8H, m) 0.78 (3H, d), 0.56-0.74 (2H, m) Carboxylic acid proton is assumed to be exchanged with moisture in the solvent.

### Example 112

### 3-[[(trans-4-Methylcyclohexyl)carbonyl](1-methylethyl)amino]-1-{4-[(E)-2-cyclohexylethenyl]phenyl}-1H-pyrazole-4-carboxylic acid

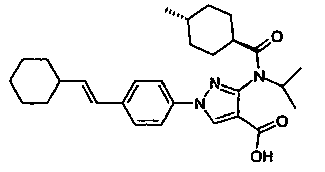

Intermediate 92 was treated with sodium hydroxide in a similar manner to that described in Example 71, and purified according to method A. A portion of this E/Z mixture (70 mg) was dissolved in dueterated chloroform (5 mL), a few crystals of iodine added and the mixture placed at room temperature for 2 days. DCM (20 mL) and sodium thiosulphate solution (20 mL) were added, the organic layer passed through a hydrophobic frit and evaporated to give the title compound.
MS calcd for (C₂₉H₃₉N₃O₃ + H)⁺: 478
MS found (electrospray): (M+H)⁺= 478

### Example 113

### 1-[4-(2-Cyclohexylethyl)phenyl]-3-[[(trans-4-methylcyclohexyl)carbonyl](1-methylethyl)amino]-1H-pyrazole-4-carboxylic acid

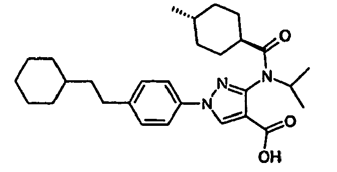

Intermediate 92 was treated with sodium hydroxide in a similar manner to that described in Example 71, and purified according to method A. A portion of this E/Z mixture was hydrogenated using a similar procedure to that described for Example 101 to give the title compound.
MS calcd for (C₂₉H₄₁N₃O₃ + H)⁺: 480
MS found (electrospray): (M+H)⁺ = 480

### Example 114

### 3-[[(trans-4-Methylcyclohexyl)carbonyl](1-methylethyl)amino]-1-(4-[2-pyridinylethenyl]phenyl)-1H-pyrazole-4-carboxylic acid

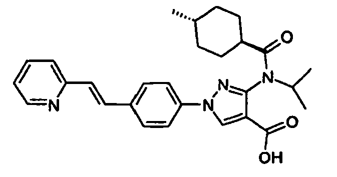

Intermediate 97 was treated with sodium hydroxide and methanol in a similar manner to that described in Example 71, and purified according to method B. This E/Z mixture (108 mg) was dissolved in chloroform (40 mL), a few crystals of iodine (17 mg) added and the mixture placed at room temperature for 4 days. Sodium thiosulphate solution (50 mL) were added, the organic layer passed through a hydrophobic frit and evaporated to give the title compound.
MS calcd for (C₂₈H₃₂N₄O₃ + H)⁺: 473
MS found (electrospray): (M+H)⁺ = 473
¹H NMR (CDCl₃): δ 8.81 (1H, d), 8.66 (1H, s), 7.85-7.73 (5H, m), 7.66 (1H, d), 7.50 (1H, d), 7.27-7.21 (2H, m), 4.99 (1H, m), 2.04 (1H, m), 1.89-1.55 (5H, m), 1.53-1.20 (5H, m), 1.01 (3H, d), 0.77 (3H, d), 0.77-0.57 (2H, m). Carboxylic acid proton is assumed to be exchanged with moisture in the solvent.

### Example 115

### 3-[[(trans-4-Methylcyclohexyl)carbonyl](1-methylethyl)amino]-1-{4-[2-pyridinylethyl]phenyl}-1H-pyrazole-4-carboxylic acid

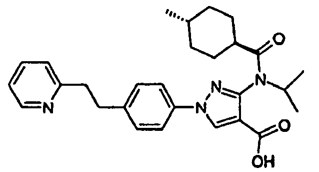

Intermediate 97 was treated with sodium hydroxide in a similar manner to that described in Example 71, and purified according to method B. A portion of this E/Z mixture was hydrogenated using a similar procedure to that described for Example 101 and purified by MDAP (purification method A) to give the title compound.
MS calcd for (C₂₈H₃₂N₄O₃ + H)⁺: 475
MS found (electrospray): (M+H)⁺= 475
¹H NMR (CDCl₃): δ 8.67 (1H, d), 8.51 (1H, s), 8.13 (1H, br), 7.74-7.64 (2H, m), 7.41 (1H, d), 7.25 (1H, d), 7.21 (1H. d), 4.96 (1H, m), 3.20-3.09 (4H, m), 2.06-1.98 (1H, m), 1.86-1.29 (6H, m), 1.26 (3H, d), 1.00 (3H, d), 0.76 (3H, d), 0.75-0.52 (3H, m).

### Example 116

### 3-[[(trans-4-Methylcyclohexyl)carbonyl](1-methylethyl)amino]-1-{4-[1,3-thiazol-2-ylethyl]phenyl}-1H-pyrazole-4-carboxylic acid

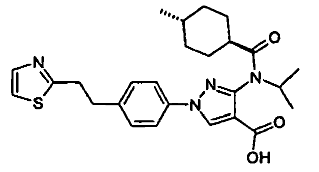

Intermediate 96 was treated with sodium hydroxide in a similar manner to that described in Example 71, and purified according to method B. A portion of this E/Z mixture was hydrogenated using a similar procedure to that described for Example 99 and purified by MDAP (purification method A) to give the title compound.
MS calcd for (C₂₆H₃₂N₄O₃S + H)⁺: 481
MS found (electrospray): (M+H)⁺ = 481

### Example 117

### 3-[[(trans-4-Methylcyclohexyl)carbonyl](1-methylethyl)amino]-1-{4-[2-(1H-pyrazol-3-yl)ethyl]phenyl}-1H-pyrazole-4-carboxylic acid

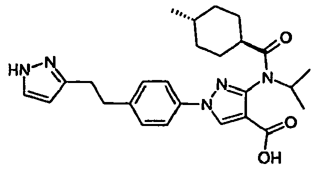

Intermediate 106 was treated with sodium hydroxide in a similar manner to that described in Example 71, and purified according to method A. A portion of this cis isomer was hydrogenated using a similar procedure to that described for Example 99, using a pressure of 25 psi, and purified by filtration through Celite to give the title compound.
MS calcd for (C₂₆H₃₃N₅O₃ + H)⁺: 464
MS found (electrospray): (M+H)⁺ = 464

### Example 118

### 3-[[(trans-4-Methylcyclohexyl)carbonyl](1-methylethyl)amino]-1-{4-[(phenylamino)carbonyl]phenyl}-1H-pyrazole-4-carboxylic acid

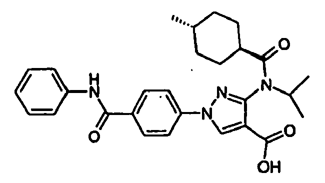

A solution of Intermediate 60 (35 mg, 0.07 mmol) and lithium iodide (46 mg, 0.35 mmol) in pyridine (1.5 mL) was heated at 120 °C for 2 days. A further portion of lithium iodide was added (46 mg) and heating continued for 3 days. The mixture was partitioned between ethyl acetate (20 mL) and dilute hydrochloric acid (10 mL, 2N), the aqueous layer extracted with ethyl actetate (2 x 10 mL), the combined organic fractions dried (Na₂SO₄) and evaporated. Purification was by SPE (silica), eluted with DCM, then EtOAc, then MeCN, then acetone, then acetone/MeOH, and finally MeOH to give the title compound.
MS calcd for (C₂₈H₃₂N₄O₄ + H)⁺: 489
MS found (electrospray): (M+H)⁺ = 489

### Example 119

### 3-[[(trans-4-Methylcyclohexyl)carbonyl](1-methylethyl)amino]-1-{4-[(phenylcarbonyl)amino]phenyl}-1H-pyrazole-4-carboxylic acid

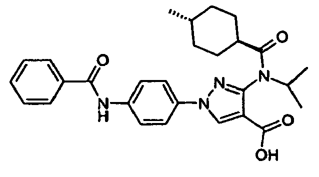

Prepared by a similar method to that described for Example 118 replacing Intermediate 60 with Intermediate 154 to give the title compound.
MS calcd for (C₂₈H₃₂N₄O₄ + H)⁺: 489
MS found (electrospray): (M+H)⁺ = 489
¹H NMR (DMSO): δ 12.8 (1H, br s), 10.35 (1H, s), 9.05 (1H, s), 7.88-8.0 (6H, m), 7.53-7.63 (3H, m), 4.73 (1H, m), 1.95 (1H, m), 1.20-1.80 (7H, m), 1.15 (3H, d), 0.88 (3H, d), 0.75 (3H, d), 0.45-0.70 (2H, m)

### Example 120

### 3-[[(trans-4-Methylcyclohexyl)carbonyl](1-methylethyl)amino]-1-{4-[(3-methylphenylcarbonyl)amino]phenyl}-1H-pyrazole-4-carboxylic acid

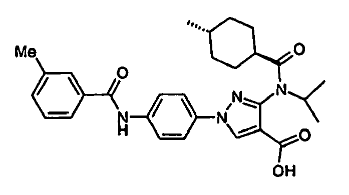

Prepared by a similar method to that described for Example 3 replacing Intermediate 52 with Intermediate 156, and using ethanol in place of methanol, to give the title compound.
MS calcd for (C₂₉H₃₄N₄O₄ + H)⁺: 503
MS found (electrospray): (M+H)⁺ = 503
¹H NMR (DMSO): δ 13.00 (1H, br s), 10.40 (1H, s), 9.05 (1H, s), 7.75-7.97 (6H, m), 7.40-7.45 (2H, m), 4.73 (1H, m), 2.42 (3H, s), 1.95 (1H, m), 1.15-1.70 (7H, m), 1.15 (3H, br d), 0.75 (3H, br), 0.50-0.70 (2H, m)

### Example 121

### 3-([(trans-4-Methylcyclohexyl)carbonyl](1-[(tert-butyloxy)carbonyl]-4-piperidinyl)amino)-1-phenyl-1H-pyrazole-4-carboxylic acid

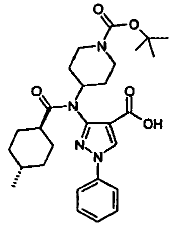

Prepared by a similar method to that described for Example 5 replacing Intermediate 59 with Intermediate 76 to give the title compound.
MS calcd for (C₂₈H₃₈N₄O₅ + H)⁺: 511
MS found (electrospray): (M+H)⁺ = 511

### Example 122

### 3-[[(trans-4-Methylcyclohexyl)carbonyl](1-methylethyl)amino]-1-{4-[(4-fluorophenylcarbonyl)amino]phenyl}-1H-pyrazole-4-carboxylic acid

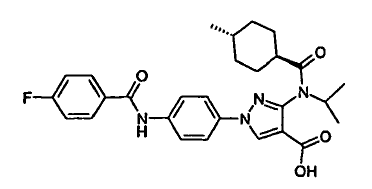

Prepared by a similar method to that described for Example 3 replacing Intermediate 52 with Intermediate 158, and using ethanol in place of methanol, and purified by MDAP to give the title compound.
MS calcd for (C₂₈H₃₁FN₄O₄ + H)⁺: 507
MS found (electrospray): (M+H)⁺= 507
¹H NMR (DMSO): δ 12.80 (1H, br s), 10.45 (1H, s), 9.03 (1H, s), 8.07 (2H, m), 7.92 (4H, m), 7.40 (2H, m), 4.73 (1 H, m), 1.95 (1 H, m), 1.05-1.70 (7H, m), 1.15 (3H, br d), 0.88 (3H, br d), 0.75 (3H, d), 0.45-0.70 (2H, m)

### Example 123

### 3-[[(trans-4-Methylcyclohexyl)carbonyl](1-methylethyl)amino]-1-{4-[(cyclohexylcarbonyl)amino]phenyl}-1H-pyrazole-4-carboxylic acid

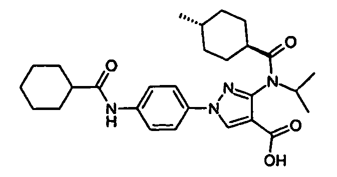

Prepared by a similar method to that described for Example 3 replacing Intermediate 52 with Intermediate 157 to give the title compound.
MS calcd for (C₃₀H₃₈N₄O₄ + H)⁺: 495
MS found (electrospray): (M+H)⁺ = 495

### Example 124

### 1-(4-{[(4-Fluorophenyl)amino]carbonyl}phenyl)-3-[[(trans-4-methylcyclohexyl)carbonyl](1-methylethyl)amino]-1H-pyrazole-4-carboxylic acid

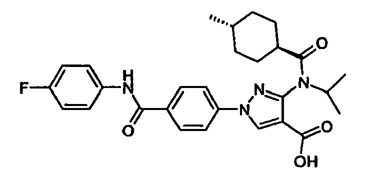

Prepared by a similar method to that described for Example 3 replacing Intermediate 52 with Intermediate 161 to give the title compound.
MS calcd for (C₂₈H₃₁FN₄O₄ + H)⁺: 507
MS found (electrospray): (M+H)⁺= 507
¹H NMR (d₆-DMSO): δ 12.95 (1H, s), 10.40 (1H, s), 9.26 (1H, s), 8.15 (2H, d), 8.08 (2H, t), 7.81 (2H, quart), 7.22 (2H, t), 4.75 (1H, quint), 1.92-1.97 (1H, m), 1.62-1.68 (2H, m), 1.45-1.52 (3H, m), 1.19-1.27 (2H, m), 1.17 (3H, d), 0.88 (3H, d), 0.74 (3H, d), 0.50-0.68 (2H, m).

### Example 125

### 3-[[(trans-4-Methylcyclohexyl)carbonyl](1-methylethyl)amino]-1-{3-[(chlorophenylcarbonyl)amino]phenyl}-1H-pyrazole-4-carboxylic acid

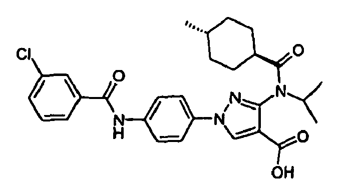

Prepared by a similar method to that described for Example 3 replacing Intermediate 52 with Intermediate 155 to give the title compound.
MS calcd for (C₂₈H₃₁ClN₄O₄ + H)⁺: 523/25
MS found (electrospray): (M+H)⁺= 523/25

### Example 126

### 3-[[(trans-4-Methylcyclohexyl)carbonyl](1-methylethyl)amino]-1-{4-[(phenylsulfonyl)amino]phenyl}-1H-pyrazole-4-carboxylic acid

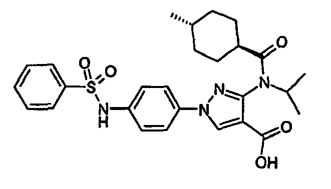

Prepared by a similar method to that described for Example 3 replacing Intermediate 52 with Intermediate 159 to give the title compound.
MS calcd for (C₂₇H₃₂N₄OS + H)⁺: 525
MS found (electrospray): (M+H)⁺: 525

### Example 127

### Enantiomer A of 1-(4-methyl-1-cyclohexen-1-yl)-3-[[(trans-4-methylcyclohexyl)carbonyl](1-methylethyl)amino]-1H-pyrazole-4-carboxylic acid

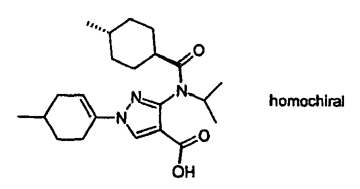

The enantiomers of Example 69 were separated using a chiralpak AD column, eluted with n-heptane:ethanol (95:5) containing 0.1% TFA. Enantiomer A eluted first with a retention time of 15.8 minutes.
¹H NMR (DMSO): δ 12.60 (1H, br), 8.00 (1H, s), 6.27 (1H, br m), 4.68 (1H, m), 2.60 (1H, m), 2.28 (1H, br d), 1.15-1.90 (13H, m), 1.09 (3H, d), 0.95 (3H, d), 0.81 (3H, d), 0.75 (3H, d), 0.45-0.70 (2H, m)
The ¹H NMR was identical with that of Example 128.
MS calcd for (C₂₂H₃₃N₃O₃+H)⁺: 388
MS found (electrospray): (M+H)⁺: 388

### Example 128

### Enantiomer B of 1-(4-methyl-1-cyctohexen-1-yl)-3-[[(trans-4-methylcyclohexyl)carbonyl](1-methylethyl)amino]-1H-pyrazole-4-carboxylic acid

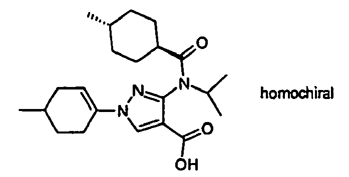

The enantiomers of Example 69 were separated using a chiralpak AD column, eluted with n-heptane:ethanol (95:5) containing 0.1% TFA. Enantiomer B eluted second with a retention time of 17.6 minutes.
The ¹H NMR was identical with that of Example 127.
MS calcd for (C₂₂H₃₃N₃O₃ + H)⁺: 388
MS found (electrospray): (M+H)⁺: 388

### Example 129

### Enantiomer A of 1-((4-Methyl)cyclohexen-1-yl)-3-[[(trans-4-methylcyclohexyl)carbonyl](tetrahydro-2H-pyran-4-yl)amino]-1H-pyrazole-4-carboxylic acid

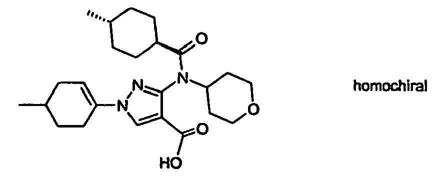

### Step 1:

The enantiomers of Intermediate 144 were separated using a 2 cm Chiralpak AD column, eluted with [n-heptane:IPA (95:5)], mixed fractions being re-processed as required. Enantiomer A eluted first. Pooling and evaporation of the fractions gave Enantiomer A of ethyl 1-((4-Methyl)cyclohexen-1-yl)-3-[[(*trans*-4-methylcyclohexyl)carbonyl](tetrahydro-2H-pyran-4-yl)amino]-1H-pyrazole-4-carboxylate.

Chiral purity >99% e.e. with a retention time of 40.3 min., Analytical Chiralpak AD-H column, eluted with [n-heptane:IPA (97:3)] flow rate 1ml/min.
MS calcd for (C₂₆H₃₉N₃O₄ + H)⁺: 458
MS found (electrospray): (M+H)⁺: 458

### Step 2:

Enantiomer A of ethyl 1-((4-Methyl)cyclohexen-1-yl)-3-[[(*trans*-4-methylcyclohexyl) carbonyl](tetrahydro-2H-pyran-4-yl)amino]-1H-pyrazole-4-carboxylate was deprotected using sodium hydroxide in a similar manner to that described in Example 71, to give the title compound.
¹H NMR (CDCl₃): δ 8.17 (1H, s), 6.26 (1H, m), 4.80 (1H, m), 4.01-3.83 (2H, m), 3.55-3.40 (2H, m), 2.69-2.44 (2H, m), 2.40-2.28 (1H, m), 2.00-1.17 (16H excess, m), 1.06 (3H, d), 0.79 (3H, d), 0.76-0.55 (2H, m) Carboxylic acid proton not seen
The ¹H NMR spectrum was identical with that of Example 130.
MS calcd for (C₂₄H₃₅N₃O₄ ⁺ H)⁺: 430
MS found (electrospray): (M+H)⁺: 430

### Example 130

### Enantiomer B of 1-((4-Methyl)cyclohexen-1-yl)-3-[[(trans-4-methylcyclohexyl)carbonyl](tetrahydro-2H-pyran-4-yl)amino]-1H-pyrazole-4-carboxylic acid

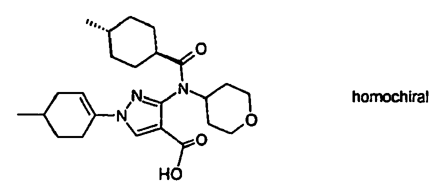

### Step 1:

The enantiomers of Intermediate 144 were separated using a 2 cm Chiralpak AD column, eluted with [n-heptane:IPA (95:5)], mixed fractions being re-processed as required. Enantiomer B eluted second. Pooling and evaporation of the fractions gave Enantiomer B of ethyl 1-((4-Methyl)cyctohexen-1-yl)-3-[[(*trans*-4-methylcyclohexyl)carbonyl](tetrahydro-2H-pyran-4-yl)amino]-1H-pyrazole-4-carboxylate.

Chiral purity >99% e.e. with a retention time of 41.7 min., Analytical Chiralpak AD-H column, eluted with [n-heptane:IPA (97:3)] flow rate 1ml/min.
MS calcd for (C₂₆H₃₉N₃O₄ + H)⁺: 458
MS found (electrospray): (M+H)⁺: 458

### Step 2:

Enantiomer B of ethyl 1-((4-Methyl)cyclohexen-1-yl)-3-[[(*trans*-4-methylcyclohexyl) carbonyl](tetrahydro-2H-pyran-4-yl)amino]-1H-pyrazole-4-carboxylate was deprotected using sodium hydroxide in a similar manner to that described in Example 71, to give the title compound.
¹H NMR (CDCl₃): δ 8.17 (1H, s), 6.26 (1H, m), 4.80 (1H, m), 4.01-3.83 (2H, m), 3.55-3.40 (2H, m), 2.69-2.44 (2H, m), 2.40-2.28 (1H, m), 2.00-1.17 (16H excess, m), 1.06 (3H, d), 0.79 (3H, d), 0.76-0.55 (2H, m) Carboxylic acid proton not seen
The ¹H NMR spectrum was identical with that of Example 129.
MS calcd for (C₂₄H₃₅N₃O₄ + H)⁺: 430
MS found (electrospray): (M+H)⁺: 430

### Example 131

### 1-(4,4-Dimethyl-1-cyclohexen-1-yl)-3-[[(trans-4-methylcyclohexyl) carbonyl](tetrahydro-3-furanyl)amino]-1H-pyrazole-4-carboxylic acid

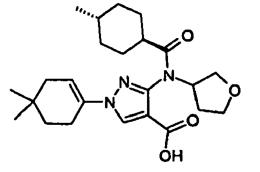

To a solution of Intermediate 166 (75 mg, 0.16 mmol) in THF (1.0 mL) and methanol (1.0 mL) was added sodium hydroxide solution (0.5 mL, 2N). The mixture was stirred for 24 hours, DCM added and the organic fraction washed with hydrochloric acid solution. The organic layer was passed through a hydrophobic frit and the solvent evaporated to give the title compound.
MS calcd for (C₂₄H₃₅N₃O₄+ H)⁺: 430
MS found (electrospray): (M+H)⁺ = 30
¹H NMR (CD₃OD): δ 8.35 (1H, s), 6.20 (1H, dd), 5.13-4.93 (1 H, m), 4.03-3.50 (4H, m), 2.55 (2H, br s), 2.25-1.25 (14H, m), 1.10 (6H, s), 0.75-0.52 (2H, m), 0.78 (3H, d) carboxylic acid proton not seen.

The chemical entities according to the invention may be formulated for administration in any convenient way, and the invention therefore also includes within its scope pharmaceutical compositions for use in therapy, comprising a compound of formula (I) or a physiologically acceptable salt or solvate thereof in admixture with one or more physiologically acceptable diluents or carriers.

The chemical entities of the present invention can be administered by different routes including intravenous, intraperitoneal, subcutaneous, intramuscular, oral, topical, transdermal, or transmucosal administration. For systemic administration, oral administration is preferred. For oral administration, for example, the chemical entities can be formulated into conventional oral dosage forms such as capsules, tablets and liquid preparations such as syrups, elixirs and concentrated drops.

Alternatively, injection (parenteral administration) may be used, e.g., intramuscular, intravenous, intraperitoneal, and subcutaneous. For injection, the chemical entities of the invention are formulated in liquid solutions, preferably, in physiologically compatible buffers or solutions, such as saline solution, Hank's solution, or Ringer's solution. In addition, the chemical entities may be formulated in solid form and redissolved or suspended immediately prior to use. Lyophilized forms can also be produced.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration, for example, may be through nasal sprays, rectal suppositories, or vaginal suppositories.

For topical administration, the compounds of the invention can be formulated into ointments, salves, gels, or creams, as is generally known in the art.

The amounts of various chemical entities to be administered can be determined by standard procedures taking into account factors such as the compound (IC₅₀) potency, (EC₅₀) efficacy, and the biological half-life (of the chemical entity), the age, size and weight of the patient, and the disease or disorder associated with the patient. The importance of these and other factors to be considered are known to those of ordinary skill in the art.

Amounts administered also depend on the routes of administration and the degree of oral bioavailability. For example, for chemical entities with low oral bioavailability, relatively higher doses will have to be administered. Oral administration is a preferred method of administration of the present chemical entities.

Preferably the composition is in unit dosage form. For oral application, for example, a tablet, or capsule may be administered, for nasal application, a metered aerosol dose may be administered, for transdermal application, a topical formulation or patch may be administered and for transmucosal delivery, a buccal patch may be administered. In each case, dosing is such that the patient may administer a single dose.

Each dosage unit for oral administration contains suitably from 0.01 to 500 mg/Kg, and preferably from 0.1 to 50 mg/Kg, of a compound of Formula (I) or a pharmaceutically acceptable salt thereof, calculated as the free base. The daily dosage for parenteral, nasal, oral inhalation, transmucosal or transdermal routes contains suitably from 0.01 mg to 100 mg/Kg, of a compound of Formula(I). A topical formulation contains suitably 0.01 to 5.0% of a compound of Formula (I). The active ingredient may be administered from 1 to 6 times per day, preferably once, sufficient to exhibit the desired activity, as is readily apparent to one skilled in the art.

Chemical entities of Formula (I) which are active when given orally can be formulated as syrups, tablets, capsules and lozenges. A syrup formulation will generally consist of a suspension or solution of the compound or salt in a liquid carrier for example, ethanol, peanut oil. olive oil, glycerine or water with a flavoring or coloring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

Typical parenteral compositions consist of a solution or suspension of a compound or salt in a sterile aqueous or non-aqueous carrier optionally containing a parenterally acceptable oil, for example polyethylene glycol, polyvinylpyrrolidone, lecithin, arachis oil or sesame oil.

Typical compositions for inhalation are in the form of a solution, suspension or emulsion that may be administered as a dry powder or in the form of an aerosol using a conventional non-CFC propellant such as 1,1,1,2-tetrafluoroethane or 1,1,1,2,3,3,3-heptafluoropropane.

A typical suppository formulation comprises a compound of Formula (I) or a pharmaceutically acceptable salt thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

No unacceptable toxicological effects are expected when compounds of the present invention are administered in accordance with the present invention.

### ASSAY

The potential for chemical entities of the invention to inhibit NS5B wildtype HCV polymerase activity may be demonstrated, for example, using the following *in vitro* assay:

### In Vitro Detection of inhibitors of HCV RNA-dependent RNA Polymerase Activity

Incorporation of [³³P]-GMP into RNA was followed by absorption of the biotin labelled RNA polymer by streptavidin containing SPA beads. A synthetic template consisting of biotinylated 13mer-oligoG hybridised to polyrC was used as a homopolymer substrate.

Reaction Conditions were 0.5 µM [³³P]-GTP (20 Ci/mMol), 1 mM Dithiothreitol, 20 mM MgCl₂, 5mM MnCl₂, 20 mM Tris-HCl, pH7.5, 1.6 µg/mL polyC/0.256 µM biotinylated oligoG13, 10% glycerol, 0.01% NP-40, 0.2 u/µL RNasin and 50 mM NaCl.

HCV RNA Polymerase (Recombinant full-length NS5B (Lohmann et al, J. Virol. 71 (11), 1997, 8416 Biochemical properties of hepatitis C virus NS5B RNA-dependent RNA polymerase and identification of amino acid sequence motifs essential for enzymatic activity') expressed in baculovirus and purified to homogeneity) was added to 4 nM final concentration.

5x concentrated assay buffer mix was prepared using 1 M MnCl₂ (0.25 mL), glycerol (2.5mL), 10% NP-40 (0.025 mL) and Water (7.225 mL), *Total* 10 mL.

2x concentrated enzyme buffer contained 1M-Tris-HCl, pH7.5 (0.4 mL), 5M NaCl (0.2 mL), 1M-MgCl₂ (0.4 mL), glycerol (1 mL), 10% NP-40 (10 µL), 1 M DTT (20 µL) and water (7.97 mL), *Total* 10 mL.

Substrate Mix was prepared using 5x Concentrated assay Buffer mix (4µL), [³³P]-GTP (10 µCi/µL, 0.02µL), 25 µM GTP (0.4 µL), 40 u/µL RNasin (0.1 µL), 20 µg/mL polyrC/biotinylated-oligorG (1.6 µL), and Water (3.94 µL), *Total* 10 µL.

Enzyme Mix was prepared by adding 1mg/ml full-length NS5B polymerase (1.5 µL) to 2.81 mL 2x-concentrated enzyme buffer.

The Assay was set up using compound (1µL), Substrate Mix (10 µL), and Enzyme Mix (added last to start reaction) (10 µL), *Total* 21 µL.

The reaction was performed in a U-bottomed, white, 96-well plate. The reaction was mixed on a plate-shaker, after addition of the Enzyme, and incubated for 1h at 22°C. After this time, the reaction was stopped by addition of 40 µL 1.875 mg/ml streptavidin SPA beads in 0.1 M EDTA. The beads were incubated with the reaction mixture for 1h at 22°C after which 120 µL 0.1 M EDTA in PBS was added. The plate was sealed, mixed centrifuged and incorporated radioactivity determined by counting in a Trilux (Wallac) or Topcount (Packard) Scintillation Counter.

After subtraction of background levels without enzyme, any reduction in the amount of radioactivity incorporated in the presence of a compound, compared to that in the absence, was taken as a measure of the level of inhibition. Ten concentrations of compounds were tested in three- or fivefold dilutions. From the counts, percentage of inhibition at highest concentration tested or IC₅₀s for the compounds were calculated using Grafit3, Grafit4 or Grafit5 software packages or a data evaluation macro for Excel based on XLFit software (IDBS).

The exemplified compounds had an IC₅₀ of <80µM in the above described assay. In one aspect, compounds have an IC₅₀ of <35µM. In another aspect, compounds have an IC₅₀ of <5µM; in yet another aspect, compounds have an IC₅₀ of <1µM. Accordingly, the compounds of the invention are of potential therapeutic benefit in the treatment and prophylaxis of HCV.

The pharmaceutical compositions according to the invention may also be used in combination with other therapeutic agents, for example immune therapies (eg. Interferon, such as Interferon alfa-2a (Roferon-A; Hoffmann-La Roche), inteferon alpha-2b (Intron-A; Schering-Plough), interferon alfacon-1 (Infergen; Intermune), peginterferon alpha-2b (Peg-Intron; Schering-Plough) or peginterferon alpha-2a (Pegasys; Hoffmann-La Roche)), therapeutic vaccines, antifibrotic agents, anti-inflammatory agents such as corticosteroids or NSAIDs, bronchodilators such as beta-2 adrenergic agonists and xanthines (e.g. theophylline), mucolytic agents, anti-muscarinics, anti-leukotrienes, inhibitors of cell adhesion (e.g. ICAM antagonists), anti-oxidants (eg N-acetylcysteine), cytokine agonists, cytokine antagonists, lung surfactants and/or antimicrobial and anti-viral agents (eg ribavirin and amantidine). The compositions according to the invention may also be used in combination with gene replacement therapy.

The invention thus provides, in a further aspect, a combination comprising at least one compound of formula (I) or a physiologically acceptable salt or solvate thereof together with at least one other therapeutically active agent, especially interferon and/or ribavirin.

The combinations referred to above may conveniently be presented for use in the form of a pharmaceutical formulation and thus pharmaceutical formulations comprising a combination as defined above together with a pharmaceutically acceptable carrier thereof represent a further aspect of the invention.

The individual components of such combinations may be administered either sequentially or simultaneously in separate or combined pharmaceutical formulations. Appropriate doses of known therapeutic agents will be readily appreciated by those skilled in the art.

## Claims

1. At least one chemical entity chosen from compounds of Formula (I) : 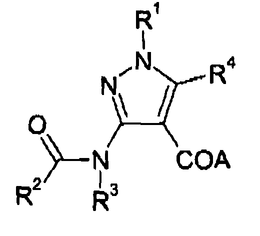 wherein:
A represents hydroxy;
R¹ represents aryl, heteroaryl bonded through a ring carbon atom, or heterocyclyl bonded through a ring carbon atom, each of which may be optionally substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, -CF₃, -OCF₃, NR^{E}SO₂R^{D}, phenyl and heterocyclyl, wherein the -C₁₋₆alkyl substituent itself may be optionally substituted by one or more substituents selected from -C₅₋₉cycloalkyl, halo, -NR^{B}R^{C}, -C(O)NR^{B}R^{C}, -NR^{E}C(O)R^{D}, -SR^{A}, -SO₂R^{D}, OR^{A}, oxo, phenyl, heteroaryl or heterocyclyl; or R¹ represents -C₁₋₆alkyl or -C₅₋₉cycloalkyl;
R² represents phenyl substituted by one or more substituents selected from -C₁₋₈alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, and heterocyclyl; or R² represents -(CH₂)ₙC₅₋₇cycloalkyl optionally substituted on the cycloalkyl by one or more substitutents selected from -C₁-₆alkyl, =CH(CH₂)ₜH, -OR^{A}, -SR^{A}, -C(O)NR^{B}R^{C}, -C(O)R^{D}, -CO₂H, -CO₂R^{D}, -NR^{B}R^{C}, -NR⁶C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo, and heterocyclyl, or wherein two substituents may together form a C₁₋₂alkylene bridge substituent;
t represents 0, 1, 2, 3 or 4;
n represents 0 or 1;
R³ represents heterocyclyl or heteroaryl; or phenyl optionally substituted by one or more substituents selected from -C₁₋₆-alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, and heterocyclyl; or R³ represents -C₁₋₆alkyl optionally substituted by one or more substituents selected from -C₁₋₆alkyl, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo, phenyl, heteroaryl and heterocyclyl;
R⁴ represents hydrogen;
R^{A} represents hydrogen, -C₁₋₆alkyl, arylalkyl, heteroarylalkyl, aryl, heterocyclyl or heteroaryl;
R^{B} and R^{C} independently represent hydrogen, -C₁₋₆alkyl, aryl, heterocyclyl or heteroaryl; or R^{B} and R^{C} together with the nitrogen atom to which they are attached form a 5 or 6 membered saturated cyclic group:
R^{D} is selected from the group consisting of -C₁₋₆alkyl, aryl, heterocyclyl, heteroaryl, arylalkyl, and heteroarylalkyl;
R^{E} represents hydrogen or -C₁₋₆alkyl;
R^{F} and R^{G} are independently selected from the group consisting of hydrogen, -C₁₋₆alkyl, aryl, heteroaryl, arylalkyl, and heteroarylalkyl; or R^{F} and R^{G} together with the nitrogen atom to which they are attached form a 5 or 6 membered saturated cyclic group;
and salts, solvates and esters thereof.

2. At least one chemical entity as claimed in claim 1 chosen from compounds of Formula (I) selected from the group consisting of:
3-[[(*trans*-4-Methylcyclohexyl)carbonyl](1-methylethyl)amino]-1-phenyl-1*H*-pyrazole-4-carboxylic acid;
3-[[(*trans*-4-Methylcyclohexyl)carbonyl](1-methylethyl)amino]-1-(4-methylphenyl)-1H-pyrazole-4-carboxylic acid;
1-(1-Cyclohexen-1-yl)-3-[[(*trans*-4-methylcyclohexyl)carbonyl](1-methylethyl)amino]-1H-pyrazole-4-carboxylic acid;
1-(4-Chloro-3-methylphenyl)-3-[[(*trans*-4-methylcyclohexyl)carbonyl](1-methylethyl)amino]-1H-pyrazole-4-carboxylic acid;
1-(4-Fluorophenyl)-3-[[(*trans-*4-methylcyclohexyl)carbonyl](1-methylethyl)amino]-1H-pyrazole-4-carboxylic acid;
1-(6-Indolyl)-3-[[(*trans*-4-methylcyclohexyl)carbonyl](1-methylethyl)amino]-1H-pyrazole-4-carboxylic acid;
1-(4-Hydroxyphenyl)-3-[[(*trans*-4-methyloyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(trifluoromethyl)phenyl]-1H-pyrazole-4-carboxylic acid;
1-[4-(Acetylamino)phenyl]-3-[[(*trans*-4-methylcyclohexyl)carbonyl](1-methylethyl)amino]-1H-pyrazole-4-carboxylic acid;
1-(4-Biphenylyl)-3-[[(*trans*-4-methylcyclohexy)carbonyl](1-methylethyl)amino]-1H-pyrazole-4-carboxylic acid;
1-[4-(Dimethylamino)phenyl]-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(methyloxy)phenyl]-1H-pyrazole-4-carboxylic acid;
1-(4-Acetylphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{4-[(trifluoromethyl)oxy]phenyl}-1H-pyrazole-4-carboxylic acid;
1-(4-Cyanophenyl)-3-[[(*trans*-4-methylcyclohexyl)carbonyl](1-methylethyl)amino]-1H-pyrazole-4-carboxylic acid;
1-{4-[(Dimethylamino)carbonyl]phenyl}-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3-thienyl)-1H-pyrazole-4-carboxylic acid;
3-[[(*trans*-4-Methylcyclohexyl)carbonyl](1-methylethyl)amino]-1-[3-(trifluoromethyl)phenyl]-1H-pyrazole-4-carboxylic acid;
1-(3,5-Dimethylphenyl)-3-[[(*trans*-4-methylcyclohexyl)carbonyl](1-methylethyl)amino]-1H-pyrazole-4-carboxylic acid;
1-(3-Chloro-5-fluorophenyl)-3-[[(*trans*-4-methylcyclohexyl)carbonyl](1-methylethyl)amino]-1H-pyrazole-4-carboxylic acid;
1-[3,5-Bis(trifluoromethyl)phenyl]-3-[[(*trans*-4-methylcyclohexyl)carbonyl](1-methylethyl)amino]-1H-pyrazole-4-carboxylic acid;
1-(1,3-Benzodioxol-5-yl)-3-[[(*trans*-4-methylcyclohexyl)carbonyl](1-methylethyl)amino]-1H-pyrazole-4-carboxylic acid;
1-(2,3-Dihydro-1-benzofuran-5-yl)-3-[[(*trans*-4-methylcyclohexyl)carbonyl](1-methylethyl)amino]-1H-pyrazole-4-carboxylic acid;
1-(2,3-Dihydro-1,4-benzodioxin-6-yl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3,4,5-trifluorophenyl)-1H-pyrazole-4-carboxylic acid;
1-(4-Chlorophenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[3-(methyloxy)phenyl)-1H-pyrazole-4-carboxylic acid;
3-[[(*trans*-4-Methylcyclohexyl)carboryl](1-methylethyl)amino]-1-[4-(methylsulfonyl)phenyl]-1H-pyrazole-4-carboxylic acid;
1-(2-Fluorophenyl)-3-[[(*trans*-4-methylcyclohexyl)carbonyl](1-methylethyl)amino]-1H-pyrazole-4-carboxylic acid:
1-(3-Hydroxyphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(3-methylphenyl)-1H-pyrazole-4-carboxylic acid;
1-(3-Fluorophenyl)-3-[[(*trans*-4-methylcyclohexyl)carbonyl](1-methylethyl)amino]-1H-pyrazole-4-carboxylic acid;
1-(4-Aminophenyl)-3-[[(*trans*-4-methylcyclohexyl)carbonyl](1-methylethyl)amino]-1H-pyrazole-4-carboxylic acid;
1-(3-Chlorophenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{3-[(trifluoromethyl)oxy]phenyl}-1H-pyrazole-4-carboxylic acid;
1-(4-Chloro-3-fluorophenyl)-3-[[(*trans*-4-methylcyclohexyl)carbonyl](1-methylethyl)amino]-1H-pyrazole-4-carboxylic acid;
1-(3-Amino-4-methylphenyl)-3-[[(*trans*-4-methylcyclohexyl)carbonyl](1-methylethyl)amino]-1H-pyrazole-4-carboxylic acid;
1-(3-Fluoro-4-methylphenyl)-3-[[(*trans*-4-methylcyclohexyl)carbonyl](1-methylethyl)amino]-1 H-pyrazole-4-carboxylic acid;
1-(3,4-Difluorophenyl),3-[[(*trans*-4-methylcyclohexyl)carbonyl](1-methylethyl)amino]-1H-pyrazole-4-carboxylic acid;
1-[(E)-1-Hexen-1-yl]-3-[[(*trans*-4-methylcyclohexyl)carbonyl](1-methylethyl)amino]-1H-pyrazole-4-carboxylic acid;
1-(E)-2-Cyclohexylethenyl]-3-[[(*trans*-4-methylcyclohexyl)carbonyl](1-methylethyl)amino)-1H-pyrazole-4-carboxylic acid;
3-[[(*trans*-4-Methylcyclohexyl)carbonyl](1-methylethyl)amino]-1-[(E)-4-methyl-1-penten-1-yl]-1H-pyrazole-4-carboxylic acid;
1-[(E)-2-(4-Fluorophenyl)ethenyl]-3-[[(*trans*-4-methylcyclohexyl)carbonyl](1-methylethyl)amino]-1H-pyrazole-4-carboxylic acid;
1-(4-Ethenylphenyl)-3-[[(*trans*-4-methylcyclohexyl)carbonyl](1-methylethyl)amino]-1H-pyrazole-4-carboxylic acid;
1-[4-(Hydroxymethyl)phenyl]-3-[[(*trans*-4-methylcyclohexyl)carbonyl](1-methylethyl)amino]-1H-pyrazole-4-carboxylic acid;
1-(4-Ethylphenyl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[4-(1-methylethyl)phenyl]-1H-pyrazole-4-carboxylic acid;
1-(5-Acetyl-2-thienyl)-3-[[(trans-4-methylcyclohexyl)carbonyl](1-methylethyl)amino]-1H-pyrazole-4-carboxylic acid;
1-(5-Chloro-2-thienyl)3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-(5-methyl-2-thienyl)-1H-pyrazole-4-carboxylic acid;
3-[[(*trans*-4-Methylcyclohexyl)carbonyl](1-methylethyl)amino]-1-(5-phenyl-2-thienyl)-1H-pyrazole-4-carboxylic acid;
1-((4-Methyl)cyclohexen-1-yl)-3-[[(*trans*-4-methylcyclohexyl)carbonyl](tetrahydro-2H-pyran-4-yl)amino]-1H-pyrazole-4-carboxylic acid:
1-(6-Benzofuranyl)-3-[[(*trans*-4-methylcyclohexyl)carbonyl](1-methylethyl)amino]-1H-pyrazole-4-carboxylic acid;
1-(Cyclohepten-1-yl)-3-[[(*trans*-4-methylcyclohexyl)carbonyl](tetrahydro-2H-pyran-4-yl)amino]-1H-pyrazole-4-carboxylic acid;
1-((4-Methyl)cyclohexen-1-yl)-3-[[(*trans*-4-methylcyclohexyl)carbonyl](1-(methylsulfonyl)-4-piperidinyl)amino]-1H-pyrazole-4-carboxylic acid;
1-((4,4-Dimethyl)cyclohexen-1-yl)-3-[[*trans*-4-methylcyclohexyl)carbonyl](tetrahydro-2H-pyran-4-yl)amino]-1H-pyrazole-4-carboxylic acid;
1-(3-Chloro-4-benzyloxyphenyl)-3-[[(*trans*-4-methylcyclohexyl)carbonyl](1-methylethyl)amino]-1H-pyrazoie-4-carboxylic acid;
1-(4-Benzyloxy-cyclohexen-1-yl)-3-[[(*trans*-4-methylcyclohexyl)carbonyl](1-methylethyl)amino]-1H-pyrazole-4-carboxylic acid;
1-((4,4-Dimethyl)cyclohexen-1-yl)-3-{[(*trans*-4-methylcyclohexyl)carbonyl](1-methylethyl)amino}-1H-pyrazole-4-carboxylic acid;
3-[[(*trans*-4-Methylcyclohexyl)carbonyl](tetrahydro-2H-pyran-4-yl)amino]-1-{4-[(E)-2-phenylethenyl]phenyl}-1H-pyrazole-4-carboxylic acid;
3-[[(*trans*-4-Methylcyclohexyl)carbonyl](tetrahydro-2H-pyran-4-yl)amino]-1-{4-[(Z)-2-phenylethenyl]phenyl}-1H-pyrazole-4-carboxylic acid;
3-[[(*trans*-4-Methylcyclohexyl)carbonyl](1-methylethyl)amino]-1-{4-[(Z)2-(3-pyrazolyl)-ethenyl]phenyl}-1H-pyrazole-4-carboxylic acid;
3-[[(*trans*-4-Methylcyclohexyl)carbonyl](1-methylethyl)amino]-1-{4-[(E)-2-(3-pyrazolyl)-ethenyl]phenyl}-1H-pyrazole-4-carboxylic acid;
3-[[(*trans*-4-Methylcyclohexyl)carbonyl](1-methylethyl)amino]-1-{4-[(E)-2-(tetrahydro-2H-pyran-4-yl)-ethenyl]phenyl}-1H-pyrazole-4-carboxylic acid;
3-[[(*trans*-4-Methylcyclohexyl)carbonyl](tetrahydro-2H-pyran-4-yl)amino]-1-{4-[(E)-2-(4-thiazolyl)-ethenyl]phenyl}-1H-pyrazole-4-carboxylic acid;
3-[[(*trans*-4-Methylcyclohexyl)carbonyl](tetrahydro-2H-pyran-4-yl)amino]-1-{4-[(Z)-2-(4-thiazolyl)-ethenyl]phenyl}-1H-pyrazole-4-carboxylic acid;
1-((E)-2-*tert*-Butyl-ethenyl)-3-[[(*trans*-4-methylcyclohexyl)carbonyl](1-methylethyl)amino]-1H-pyrazole-4-carboxylic acid;
1-((E)-2-Phenyl-ethenyl)-3-[[(*trans*-4-methylcyclohexyl)carbonyl](1-methylethyl)amino]-1H-pyrazole-4-carboxylic acid;
1-(4-Methyl-1-cyclohexen-1-yl)-3-[[(*trans*-4-methylcyclohexyl)carbonyl](1-methylethyl)amino]-1H-pyrazole-4-carboxylic acid;
1-(3-Cyanophenyl)-3-[[(*trans*-4-methylcyclohexyl)carbonyl](1-methylethyl)amino]-1H-pyrazole-4-carboxylic acid;
3-{(1-Methylethyl)[(4-methylidenecyclohexyl)carbonyl]amino}-1-phenyl-1H-pyrazole-4-carboxylic acid;
1-(4-Trifluoromethyl-cyclohexen-1-yl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{4-[(phenyloxy)methyl] phenyl}-1*H*-pyrazole-4-carboxylic acid;
1-[4-(Phenylsulfonylmethyl)phenyl]-3-[[(*trans*-4-methylcyclohexyl)carbonyl](1-methylethyl)amino]-1H-pyrazole-4-carboxylic acid;
1-[4-(Phenylthiomethyl)phenyl]-3-[[(*trans*-4-methylcyclohexyl)carbonyl](1-methylethyl)amino]-1H-pyrazole-4-carboxylic acid;
1-[4-(Phenoxy)phenyl]-3-[[(*trans*-4-methylcyclohexyl)carbonyl](1-methylethyl)amino]-1H-pyrazole-4-carboxylic acid;
1-[4-{(1,3-Thiazol-4-ylmethyl)oxy}phenyl]-3-[[(*trans*-4-methylcyclohexyl)carbonyl](1-methylethyl)amino]-1H-pyrazole-4-carboxylic acid;
1-[4-((E)-Phenylethenyl)phenyl]-3-[[(*trans*-4-methylcyclohexyl)carbonyl](1-methylethyl)amino]-1H-pyrazole-4-carboxylic acid;
1-[4-(Z)-Phenylethenyl))phenyl]-3-[[(*trans*-4-methylcyclohexyl)carbonyl](1-methylethyl)amino]-1H-pyrazole-4-carboxylic acid;
1-[4-((E,Z)-(1,3-Thiazol-2-yl)ethenyl)phenyl]-3-[[(*trans-*4-methylcyclohexyl)carbonyl](1-methylethyl)amino]-1H-pyrazole-4-carboxylic acid;
1-[4-((E)-Phenyl-2-methylethenyl)phenyl]-3-[[(*trans-*4-methylcyclohexyl)carbonyl](1-methylethyl)amino]-1H-pyrazole-4-carboxylic acid;
1-[4-(E)-(Pyridin-4-yl)ethenyl))phenyl]-3-[[(*trans*-4-methylcyclohexyl)carbonyl](1-methylethyl)amino]-1H-pyrazole-4-carboxylic acid;
1-[4-((E)-(1,3-Thiazol-4-yl)ethenyl)phenyl]-3-[[(*trans*-4-methylcyclohexyl)carbonyl](1-methylethyl)amino]-1H-pyrazole-4-carboxylic acid;
1-[4-((E)-(Furan-2-yl)ethenyl))phenyl]-3[[(*trans*-4-methylcyclohexyl)carbonyl](1-methylethyl)amino]-1H-pyrazole-4-carboxylic acid;
1-[4-((E)-(2-Methyl-1,3-thiazol-4-yl)ethenyl)phenyl]-3-[[(*trans*-4-methylcyclohexyl)carbonyl](1-methylethyl)amino]-1H-pyrazole-4-carboxylic acid;
3-[(Cyclohexylacetyl)(1-methylethyl)amino]-1-phenyl-1H-pyrazole-4-carboxylic acid; 3-{(1-Methylethyl)[(4-methylphenyl)carbonyl]amino}-1-phenyl-1H-pyrazole-4-carboxylic acid;
3-[[(4-Bromo-2-chlorophenyl)carbonyl](1-methylethyl)amino]-1-phenyl-1H-pyrazole-4-carboxylic acid;
3-[[(*trans*-4-Methylcyclohexyl)carbonyl](phenyl)amino]-1-phenyl-1H-pyrazole-4-carboxylic acid;
3-{[2-(Dimethylamino)-2-oxoethyl][(*trans-*4-methylcyclohexyl)carbonyl]amino}-1-phenyl-1H-pyrazole-4-carboxylic acid;
3-{[(*trans*-4-Methylcyclohexyl)carbonyl]{1-[(methyloxy)carbonyl]-4-piperidinyl}amino}-1-phenyl-1H-pyrazole-4-carboxylic acid;
3-{[{*trans*-4-Methylcyclohexyl)carbonyl][1-(methylsulfonyl)-4-piperidinyl]amino}-1-phenyl-1H-pyrazole-4-carboxylic acid;
3-[[(*trans*-4-Methylcyclohexyl)carbonyl](1-methyl-4-piperidinyl)amino]-1-phenyl-1H-pyrazole-4-carboxylic acid;
3-{{1-[(Ethylamino)carbonyl]-4-piperidinyl}[(*trans*-4-methylcyclohexyl)carbonyl]amino}-1-phenyl-1H-pyrazole-4-carboxylic acid;
3-[[(*trans*-4-Methylcyclohexyl)carbonyl](2-pyrazinylmethyl)amino]-1-phenyl-1H-pyrazole-4-carboxylic acid;
*r*e*l*-3-[{[(1S,2R,4S)-2-Hydroxy-4-methylcyclohexyl]carbony}(1-methylethyl)amino]-1-phenyl-1H-pyrazole-4-carboxylic acid;
3-[[(*trans*-4-Methylcyclohexyl)carbonyl](1-methylethyl)amino]-1-{4-[(3-methoxyphenylcarbonyl)amino]phenyl}-1*H*-pyrazole-4-carboxylic acid;
3-[[(*trans*-4-Methylcyclohexyl)carbonyl](1-methylethyl)amino]-1-{4-[(phenylmethyl)oxy]phenyl}-1H-pyrazole-4-carboxylic acid;
1-(1H-Indol-5-yl)-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{4-[(E/Z)-2-phenylethenyl]phenyl}-1H-pyrazole-4-carboxylic acid;
3-[[(*trans*-4-Methylcyclohexyl)carbonyl](1-methylethyl)amino]-1-[4-(2-phenylethyl)phenyl]-1H-pyrazole-4-carboxylic acid;
3-[[(*trans*-4-Methylcyclohexyl)carbonyl](tetrahydro-2H-pyran-4-yl)amino]-1-{4-[2-phenylethyl]phenyl}-1H-pyrazole-4-carboxylic acid;
3-[[(*trans*-4-Methylcyclohexyl)carbonyl](1-methylethyl)amino]-1-[4-[(1,3-thiazol-4-yl)-ethyl]phenyl]-1H-pyrazole-4-carboxylic acid;
3-[[(*trans*-4-Methylcyclohexyl)carbonyl](tetrahydro-2H-pyran-4-yl)amino]-1-{4-[(1,3-thiazol-4-yl)-ethyl]phenyl}-1H-pyrazole-4-carboxylic acid;
1-Cyclohexyl-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-[1-(methylsulfonyl)-1,2,3,6-tetrahydro-4-pyridinyl]-1H-pyrazole-4-carboxylic acid;
3-[[(*trans*-4-Methylcyclohexyl)carbonyl](phenylmethyl)amino]-1-phenyl-1H-pyrazole-4-carboxylic acid;
3-{Cyclopentyl[(*trans-*4-methylcyclohexyl)carbonyl]amino}-1-phenyl-1H-pyrazole-4-carboxylic acid;
3-[[(*trans-*4-Methylcyclohexyl)carbonyl](tetrahydro-2H-pyran-4-yl)amino]-1-phenyl-1H-pyrazote-4-carboxylic acid;
3-{(1-Acetyl-4-piperidinyl)[(*trans*-4-methylcyclohexyl)carbonyl]amino}-1-phenyl-1H-pyrazole-4-carboxylic acid;
3-[[(*trans*-4-Methylcyclohexyl)carbonyl](4-piperidinyl)amino]-1-phenyl-1H-pyrazole-4-carboxylic acid;
3-[[(*trans*-4-Methylcyclohexyl)carbonyl](1-methylethyl)amino]-1-{4-[(E)-2-cyclohexylethenyl]phenyl}-1H-pyrazole-4-carboxylic acid;
1-[4-(2-Cyclohexylethyl)phenyl]-3-[[(*trans*-4-methylcyclohexyl)carbonyl](1-methylethyl)amino]-1H-pyrazole-4-carboxylic acid;
3-[[(*trans*-4-Methylcyclohexyl)carbonyl](1-methylethyl)amino]-1-{4-[2-pyridinylethenyl]phenyl}-1H-pyrazole-4-carboxylic; acid;
3-[[(*trans*-4-Methylcyclohexyl)carbonyl](1-methylethyl)amino]-1-{4-[2-pyridinylethyl]phenyl}-1H-pyrazole-4-carboxylic acid;
3-[[(*trans*-4-Methyleyclohexyl)carbonyl](1-methylethyl)amino]-1-{4-[1,3-thiazol-2-ylethyl]phenyl}-1H-pyrazole-4-carboxylic acid;
3-[[(*trans*-4-Methylcyclohexyl)carbonyl](1-methylethyl)amino]-1-{4-[2-(1*H*-pyrazol-3-yl)ethyl]phenyl}-1*H*-pyrazole-4-carboxylic acid;
3-[[(*trans*-4-Methylcyclohexyl)carbonyl](1-methylèthyl)amino]-1-{4-[(phenylamino)carbonyl]phenyl}-1*H*-pyrazole-4-carboxylic acid;
3-[[(*trans*-4-Methylcyclohexyl)carbonyl](1-methylsthyl)amino]-1-{4-[(phenylcarbonyl)amino]phenyl}-1*H*-pyrazole-4-carboxylic acid;
3-[[(*trans*-4-Methylcyclohexyl)carbonyl](1-methylethyl)amino]-1-{4-[(3-methylphenylcarbonyl)amino]phenyl}-1*H*-pyrazole-4-carboxylic acid;
3-[[(*trans*-4-Methylcyclohexyl)carbonyl]{1-[(*tert*-butyloxy)carbonyl]-4-piperidinyl}amino]-1-phenyl-1H-pyrazole-4-carboxylic acid;
3-[[(*trans-*4-Methylcyclohexyl)carbonyl](1-methylethyl)amino]-1-{4-[(4-fluorophenylcarbonyl)amino]phenyl}-1*H*-pyrazole-4-carboxylic acid;
3-[[(*trans-*4-Methylcyclohexyl)carbonyl](1-methylethyl)amino]-1-{4-[(cyclohexylcarbonyl)amino]phenyl}-1*H*-pyrazole-4-carboxylic acid;
1-(4-{[(4-Fluorophenyl)amino]carbonyl}phenyl)-3-[[(*trans*-4-methylcyclohexyl)carbonyl](1-methylethyl)amino]-1H-pyrazole-4-r-arboxylic acid;
3-[[(*trans*-4-Methylcyclohexyl)carbonyl](1-methylethyl)amino]-1-{3-[(chlorophenylcarbonyl)amino]phenyl}-1*H*-pyrazole-4-carboxylic acid;
3-[[(*trans*-4-Methylcyclohexyl)carbonyl](1-methylethyl)amino]-1-{4-[(phenylsulfonyl)amino]phenyl}-1*H*-pyrazole-4-carboxylic acid;
1-(4-Methyl-1-cyclohexen-1-yl)-3-[[(*trans*-4-methylcyclohexyl)carbonyl](1-methylethyl)amino]-1*H*-pyrazole-4-carboxylic acid;
1-(4,4-Dimethyl-1-cyclohexen-1-yl)-3-[[(*tran*s-4-methylcyclohexyl)carbonyl](tetrahydro-3-furanyl)amino]-1*H*-pyrazole-4-carboxylic acid
and salts, solvates and esters, and individual enantiomers thereof where appropriate.

3. At least one chemical entity chosen from compounds of Formula (I) 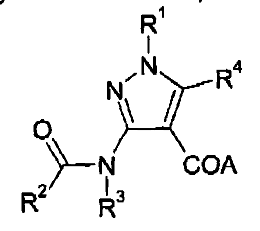 wherein:
A represents hydroxy;
R¹ represents aryl, heteroaryl bonded through a ring carbon atom, or heterocyclyl bonded through a ring carbon atom, each of which may be optionally substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{O}, -NR^{E}CO₂R^{D}, -NR^{E}C(O)NR^{F}R^{G}, -SO₂NR^{F}R^{G}, -SO₂R^{D}, nitro, cyano, -CF₃, -OCF₃, NR^{E}SO₂R^{D}, phenyl and heterocyclyl, wherein the -C₁₋₆alkyl substituent itself may be optionally substituted by one or more substituents selected from -C₅₋₉cycloalkyl, halo, -NR^{B}R^{C}, -C(O)NR^{B}R^{C}, -NR^{E}C(O)R^{O}, -SR^{A}, -SO₂R^{D}, OR^{A}, oxo, phenyl, heteroaryl or heterocyclyl; or R¹ represents -C₁₋₆alkyl or -C₅₋₉cycloalkyl;
R² represents phenyl substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{O}, -NR^{E}CO₂R^{D}, -NR^{E}C(O)NR^{F}R^{G}, -SO₂NR^{F}R^{G}, -SO₂R^{D}, nitro, cyano, and heterocyclyl; or R² represents -(CH₂)ₙC₅₋₇cycloalkyl optionally substituted on the cycloalkyl by one or more substitutents selected from -C₁₋₆alkyl, =CH(CH₂)ₜH, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo, and heterocyclyl, or wherein two substituents may together form a C₁₋₂alkylene bridge substituent;
t represents 0, 1, 2, 3 or 4;
n represents 0 or 1;
R³ represents heterocyclyl or heteroaryl; or phenyl optionally substituted by one or more substituents selected from -C₁₋₆alkyl, halo, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, and heterocyclyl; or R³ represents -C₁₋₆alkyl optionally substituted by one or more substituents selected from -C₁₋₆alkyl, -OR^{A}, -SR^{A}, -C(O)NR^{B}R^{C}, -C(O)R^{D}. -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}. fluoro, nitro, cyano, oxo, phenyl, heteroaryl and heterocyclyl;
R⁴ represents hydrogen;
R^{A} represents hydrogen, -C₁₋₆alkyl, arylalkyl, heteroarylalkyl, aryl, heterocyclyl or heteroaryl;
R^{B} and R^{C} independently represent hydrogen, -C₁₋₆alkyl, aryl, heterocyclyl or heteroaryl; or R^{B} and R^{C} together with the nitrogen atom to which they are attached form a 5 or 6 membered saturated cyclic group;
R^{D} is selected from the group consisting of -C₁₋₆alkyl, aryl, heterocyclyl, heteroaryl, arylalkyl, and heteroarylalkyl;
R^{E} represents hydrogen or -C₁₋₆alkyl;
R^{F} and R^{G} are independently selected from the group consisting of hydrogen, -C₁₋₆alkyl, aryl, heteroaryl, arylalkyl, and heteroarylalkyl; or R^{F} and R^{G} together with the nitrogen atom to which they are attached form a 5 or 6 membered saturated cyclic group;
and salts, solvates and esters thereof
for use in medical therapy.

4. A compound as claimed in claim 3 wherein the medical therapy is the treatment of viral infection.

5. A compound as claimed in claim 4 wherein the viral infection is HCV.

6. A pharmaceutical formulation comprising at least one chemical entity chosen from compounds of Formula (I) and pharmaceutically acceptable salts, solvates and esters thereof as defined in claim 1 in conjunction with at least one pharmaceutically acceptable diluent or carrier.

7. A process for the preparation of a compound of Formula (I) as defined in claim 1, comprising treatment of a compound of Formula (II) 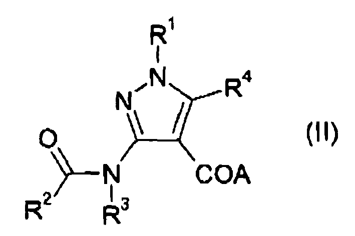 in which A is an alkoxy, benzyloxy or silyloxy group and R¹, R², R³ and R⁴ are as defined above for Formula (I) with a base.

8. A process as claimed in claim 7 in which A is ethoxy.

9. Use of at least one chemical entity chosen from compounds of Formula (I) and pharmaceutically acceptable salts, solvates and esters thereof as claimed in claim 1, in the manufacture of a medicament for the treatment and/or prophylaxis of viral infection.

10. Use as claimed in claim 9 wherein the viral infection is HCV.

## Patentansprüche

1. Mindestens eine chemische Einheit, ausgewählt aus Verbindungen der Formel (I): 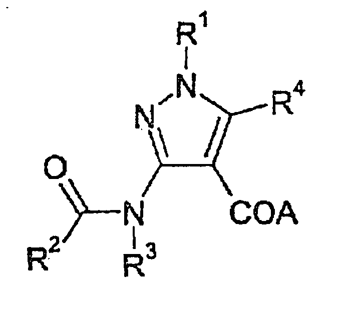 wobei:
A für Hydroxy steht;
R¹ für Aryl, durch ein Ringkohlenstoffatom gebundenes Heteroaryl oder durch ein Ringkohlenstoffatom gebundenes Heterocyclyl steht, wobei jedes davon gegebenenfalls mit einem oder mehreren Substituenten, ausgewählt aus -C₁₋₆-Alkyl, Halogen, -OR^{A}, - SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, Nitro, Cyano, -CF₃, -OCF₃, NR^{E}SO₂R^{D}, Phenyl und Heterocyclyl, substituiert sein kann, wobei der -C₁₋₆-Alkyl-Substituent selbst gegebenenfalls mit einem oder mehreren Substituenten, ausgewählt aus -C₅₋₉-Cycloalkyl, Halogen, -NR^{B}R^{C}, -C(O)NR^{B}R^{C}, -NR^{E}C(O)R^{D}, -SR^{A}, -SO₂R^{D}, OR^{A}, Oxo, Phenyl, Heteroaryl oder Heterocyclyl, substituiert sein kann; oder R¹ für -C₁₋₆-Alkyl oder -C₅₋₉-Cycloalkyl steht;
R² Phenyl, substituiert mit einem oder mehreren Substituenten, ausgewählt aus -C₁₋₆-Alkyl, Halogen, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, Nitro, Cyano und Heterocyclyl, darstellt; oder R² für -(CH₂)ₙC₅₋₇-Cycloalkyl steht, gegebenenfalls an dem Cycloalkyl substituiert mit einem oder mehreren Substituenten, ausgewählt aus -C₁₋₆-Alkyl, =CH(CH₂)ₜH, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, Fluor, Nitro, Cyano, Oxo und Heterocyclyl, oder wobei zwei Substituenten zusammen einen C₁₋₂-Alkylen-Brückensubstituenten bilden können;
t für 0, 1, 2, 3 oder 4 steht;
n für 0 oder 1 steht;
R³ Heterocyclyl oder Heteroaryl darstellt; oder Phenyl, welches gegebenenfalls mit einem oder mehreren Substituenten, ausgewählt aus -C₁₋₆-Alkyl, Halogen, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, - NR^{E}C(O)NR^{F}R^{G}, -SO₂NR^{F}R^{G}, -SO₂R^{D}, Nitro, Cyano und Heterocyclyl, substituiert ist; oder R³ für -C₁₋₆-Alkyl steht, welches gegebenenfalls mit einem oder mehreren Substituenten, ausgewählt aus -C₁₋₆-Alkyl, -OR^{A}, -SR^{A}, -C(O)NR^{B}R^{C}, -C(O)R^{D} , -CO₂H, -CO₂R^{D}, -NR^{B}R^{C}, -NR^{E}C(O)R^{D}, -NR^{E}CO₂R^{D}, NR^{E}C(O)NR^{F}R^{G}, -SO₂NR^{F}R^{G}, -SO₂R^{D}, Fluor, Nitro, Cyano, Oxo, Phenyl, Heteroaryl und Heterocyclyl, substituiert ist;
R⁴ ein Wasserstoffatom darstellt;
R^{A} ein Wasserstoffatom, -C₁₋₆-Alkyl, Arylalkyl, Heteroarylalkyl, Aryl, Heterocyclyl oder Heteroaryl darstellt;
R^{B} und R^{C} unabhängig ein Wasserstoffatom, -C₁₋₆-Alkyl, Aryl, Heterocyclyl oder Heteroaryl darstellen; oder R^{B} und R^{C} zusammen mit dem Stickstoffatom, an welches sie gebunden sind, einen 5- oder 6-gliedrigen gesättigten zyklischen Rest bilden;
R^{D} ausgewählt ist aus -C₁₋₆-Alkyl, Aryl, Heterocyclyl, Heteroaryl, Arylalkyl und Heteroarylalkyl;
R^{E} ein Wasserstoffatom oder -C₁₋₆-Alkyl darstellt;
R^{F} und R^{G} unabhängig ausgewählt sind aus einem Wasserstoffatom, -C₁₋₆-Alkyl, Aryl, Heteroaryl, Arylalkyl und Heteroarylalkyl; oder R^{F} und R^{G} zusammen mit dem Stickstoffatom, an welches sie gebunden sind, einen 5- oder 6-gliedrigen gesättigten zyklischen Rest bilden;
und Salze, Solvate und Ester davon.

2. Mindestens eine chemische Einheit gemäß Anspruch 1, ausgewählt aus Verbindungen der Formel (I), bestehend aus:
3-[[(*trans*-4-Methylcyclohexyl)carbonyl](1-methylethyl)amino]-1-phenyl-1*H*-pyrazol-4-carbonsäure;
3-[[(*trans*-4-Methylcyclohexyl)carbonyl](1-methylethyl)amino]-1-(4-methylphenyl)-1*H* -pyrazol-4-carbonsäure;
1-(1-Cyclohexen-1-yl)-3-[[(*trans*-4-methylcyclohexyl)carbonyl](1-methylethyl)amino]-1*H*-pyrazol-4-carbonsäure;
1-(4-Chlor-3-methylphenyl)-3-[[(*trans*-4-methylcyclohexyl)carbonyl](1-methylethyl)-aminol-1*H*-pyrazol-4-carbonsäure;
1-(4-Fluorphenyl)-3-[[(*trans*-4-methylcydohexyl)carbonyl](1-methylethyl)amino]-1H-pyrazol-4-carbonsäure;
1-(6-Indolyl)-3-[[(*trans*-4-methylcyclohexyl)carbonyl](1-methylethyl)amino]-1H-pyrazol-4-carbonsäure;
1-(4-Hydroxyphenyl)-3-[[(trans-4-methylcyclohexyl)carbonyl](1-methylethyl)amino]-1*H*-pyrazol-4-carbonsäure;
3-[[(*trans*-4-Methylcyclohexyl)carbonyl](1-methylethyl)amino]-1-[4-(trifluormethyl)-phenyl]-1*H*-pyrazol-4-carbonsäure;
1-[4-(Acetylamino)phenyl]-3-[[(*trans*-4-methylcyclohexyl)carbonyl](1-methylethyl)-amino]-1*H*-pyrazol-4-carbonsäure;
1-(4-Biphenylyl)-3-[[(*trans*-4-methylcyclohexyl)carbonyl](1-methylethyl)amino]-1*H-*pyrazol-4-carbonsäure;
1-[4-(Dimethylamino)phenyl]-3-[[(trans-4-methylcyclohexyl)carbonyl](1-methylethyl)-amino]-1H-pyrazol-4-carbonsäure;
3-[[(*trans*-4-Methylcyclohexyl)carbonyl](1-methylethyl)amino]-1-[4-(methyloxy)-phenyl]-1*H*-pyrazol-4-carbonsäure;
1-(4-Acetylphenyl)-3-[[(*trans*-4-methylcyclohexyl)carbonyl](1-methylethyl)amino]-1*H-*pyrazol-4-carbonsäure;
3-[[(*trans*-4-Methylcyclohexyl)carbonyl](1-methylethyl)amino]-1-{4-[(trifluormethyl)-oxy]phenyl}-1*H*-pyrazol-4-carbonsäure;
1-(4-Cyanophenyl)-3-[[(trans-4-methylcyclohexyl)carbonyl](1-methylethyl)amino]-1H-pyrazol-4-carbonsäure;
1-{4-[(Dimethylamino)carbonyl]phenyl}-3-[[(*trans*-4-methylcyclohexyl)carbonyl](1-methylethyl)amino]-1*H*-pyrazol-4-carbonsäure;
3-[[(*trans*-4-Methylcyclohexyl)carbonyl](1-methylethyl)amino]-1-(3-thienyl)-1*H-*pyrazol-4-carbonsäure;
3-[[(*trans*-4-Methylcyclohexyl)carbonyl](1-methylethyl)amino]-1-[3-(trifluormethyl)-phenyl]-1*H*-pyrazol-4-carbonsäure;
1-(3,5-Dimethylphenyl)-3-[[(*trans*-4-methylcyclohexyl)carbonyl](1-methylethyl)amino]-1*H*-pyrazol-4-carbonsäure;
1-(3-Chlor-5-fluorphenyl)-3-[[(*trans*-4-methylcyclohexyl)carbonyl](1-methylethyl)-amino]-1*H*-pyrazol-4-carbonsäure;
1-[3,5-Bis(trifluormethyl)phenyl]-3-[[(*trans*-4-methylcyclohexyl)carbonyl](1-methylethyl)amino]-1*H*-pyrazol-4-carbonsäure;
1-(1,3-Benzodioxol-5-yl)-3-[[(trans-4-methylcyclohexyl)carbonyl](1-methylethyl)-amino]-1*H*-pyrazol-4-carbonsäure;
1-(2,3-Dihydro-1-benzofuran-5-yl)-3-[[(*trans*-4-methylcyclohexyl)carbonyl](1-methylethyl)amino]-1*H*-pyrazol-4-carbonsäure;
1-(2,3-Dihydro-1,4-benzodioxin-6-yl)-3-[[(*trans*-4-methylcyclohexyl)carbonyl](1-methylethyl)amino]-1*H*-pyrazol-4-carbonsäure;
3-[[(trans-4-Methylcyclohexyl)carbonyl](1-methylethyl)amino]-1-(3,4,5-trifluorphenyl)-1*H*-pyrazol-4-carbonsäure;
1-(4-Chlorphenyl)-3-[[(*trans*-4-methylcyclohexyl)carbonyl](1-methylethyl)amino]-1*H-*pyrazol-4-carbonsäure;
3-[[(*trans*-4-Methylcyclohexyl)carbonyl](1-methylethyl)amino]-1-[3-(methyloxy)-phenyl]-1*H*-pyrazol-4-carbonsäure;
3-[[(*trans*-4-Methylcyclohexyl)carbonyl](1-methylethyl)amino]-1-[4-(methylsulfonyl)-phenyl]-1*H*-pyrazol-4-carbonsäure;
1-(2-Fluorphenyl)-3-[[(*trans-*4-methylcyclohexyl)carbonyl](1-methylethyl)amino]-1*H-*pyrazol-4-carbonsäure;
1-(3-Hydroxyphenyl)-3-[[(trans-4-methylcyclohexyl)carbonyl](1-methylethyl)amino]-1*H*-pyrazol-4-carbonsäure;
3-[[(*trans*-4-Methylcyclohexyl)carbonyl](1-methylethyl)amino]-1-(3-methylphenyl)-1*H-*pyrazol-4-carbonsäure;
1-(3-Fluorphenyl)-3-[[(*trans*-4-methylcyclohexyl)carbonyl](1-methylethyl)amino]-1*H-*pyrazol-4-carbonsäure;
1-(4-Aminophenyl)-3-[[(*trans*-4-methylcyclohexyl)carbonyl](1-methylethyl)amino]-1*H-*pyrazol-4-carbonsäure;
1-(3-Chlorphenyl)-3-[[(*trans*-4-methylcyclohexyl)carbonyl](1-methylethyl)amino]-1*H-*pyrazol-4-carbonsäure;
3-[[(*trans*-4-Methylcyclohexyl)carbonyl](1-methylethyl)amino]-1-{3-[(trifluormethyl)-oxy]phenyl}-1*H*-pyrazol-4-carbonsäure;
1-(4-Chlor-3-fluorphenyl)-3-[[(*trans*-4-methylcyclohexyl)carbonyl](1-methylethyl)-amino]-1*H*-pyrazol-4-carbonsäure;
1-(3-Amino-4-methylphenyl)-3-[[(*trans*-4-methylcyclohexyl)carbonyl](1-methylethyl)-amino]-1*H*-pyrazol-4-carbonsäure;
1-(3-Fluor-4-methylphenyl)-3-[[(*trans*-4-methylcyclohexyl)carbonyl](1-methylethyl)-amino]-1*H*-pyrazol-4-carbonsäure ;
1-(3,4-Difluorphenyl)-3-[[(trans-4-methylcyclohexyl)carbonyl](1-methylethyl)amino]-1*H*-pyrazol-4-carbonsäure;
1-[(E)-1-Hexen-1-yl]-3-[[(trans-4-methylcyclohexyl)carbonyl](1-methylethyl)amino]-1*H*-pyrazol-4-carbonsäure;
1-[(E)-2-Cyclohexylethenyl]-3-[[(*trans*-4-methylcyclohexyl)carbonyl](1-methylethyl)-amino]-1*H*-pyrazol-4-carbonsäure ;
3-[[(*trans*-4-Methylcyclohexyl)carbonyl](1-methylethyl)amino]-1-[(E)-4-methyl-1-penten-1-yl]-1*H*-pyrazol-4-carbonsäure;
1-[(E)-2-(4-Fluorphenyl)ethenyl]-3-[[(*trans*-4-methylcyclohexyl)carbonyl](1-methylethyl)amino]-1*H*-pyrazol-4-carbonsäure;
1-(4-Ethenylphenyl)-3-[[(*trans*-4-methylcyclohexyl)carbonyl](1-methylethyl)amino]-1*H*-pyrazol-4-carbonsäure;
1-[4-(Hydroxymethyl)phenyl]-3-[[(*trans*-4-methylcyclohexyl)carbonyl](1-methylethyl)-amino]-1*H*-pyrazol-4-carbonsäure;
1-(4-Ethylphenyl)-3-[[(*trans*-4-methylcyclohexyl)carbonyl](1-methylethyl)amino]-1*H-*pyrazol-4-carbonsäure;
3-[[(*trans*-4-Methylcyclohexyl)carbonyl](1-methylethyl)amino]-1-[4-(1-methylethyl)-phenyl]-1*H*-pyrazol-4-carbonsäure;
1-(5-Acetyl-2-thienyl)-3-[[(*trans*-4-methylcyclohexyl)carbonyl](1-methylethyl)amino]-1*H*-pyrazol-4-carbonsäure;
1-(5-Chlor-2-thienyl)-3-[[(*trans*-4-methylcyclohexyl)carbonyl](1-methylethyl)amino]-1*H*-pyrazol-4-carbonsäure;
3-[[(*trans*-4-Methylcyclohexyl)carbonyl](1-methylethyl)amino]-1-(5-methyl-2-thienyl)-1*H*-pyrazol-4-carbonsäure;
3-[[(*trans-*4-Methylcyclohexyl)carbonyl](1-methylethyl)amino]-1-(5-phenyl-2-thienyl)-1*H*-pyrazol-4-carbonsäure;
1-((4-Methyl)cyclohexen-1-yl)-3-[[(*trans*-4-methylcyclohexyl)carbonyl](tetrahydro-2H-pyran-4-yl)amino]-1*H*-pyrazol-4-carbonsäure;
1-(6-Benzofuranyl)-3-[[(*trans*-4-methylcyclohexyl)carbonyl](1-methylethyl)amino]-1*H-*pyrazol-4-carbonsäure;
1-(Cyclohepten-1-yl)-3-[[(*trans*-4-methylcyclohexyl)carbonyl](tetrahydro-2*H*-pyran-4-yl)amino]-1*H*-pyrazol-4-carbonsäure;
1-((4-Methyl)cyclohexen-1-yl)-3-[[(*trans*-4-methylcyclohexyl)carbonyl](1-(methylsulfonyl)-4-piperidinyl)amino]-1*H*-pyrazol-4-carbonsäure;
1-((4,4-Dimethyl)cyclohexen-1-yl)-3-[[(*trans*-4-methylcyclohexyl)carbonyl](tetrahydro-2H-pyran-4-yl)amino]-1*H*-pyrazol-4-carbonsäure;
1-(3-Chlor-4-benzyloxyphenyl)-3-[[(*trans*-4-methylcyclohexyl)carbonyl](1-methylethyl)-amino]-1*H*-pyrazol-4-carbonsäure;
1-(4-Benzyloxycyclohexen-1-yl)-3-[[(*trans*-4-methylcyclohexyl)carbonyl](1-methylethyl)amino]-1)-1*H*-pyrazol-4-carbonsäure;
1-((4,4-Dimethyl)cyclohexen-1-yl)-3-{[(*trans*-4-methylcyclohexyl)carbonyl](1-methylethyl)amino}-1*H*-pyrazol-4-carbonsäure;
3-[[(*trans*-4-Methylcyclohexyl)carbonyl](tetrahydro-2*H*-pyran-4-yl)amino]-1-{4-[(E)-2-phenylethenyl]phenyl}-1*H*-pyrazol-4-carbonsäure;
3-[[(*trans*-4-Methylcyclohexyl)carbonyl](tetrahydro-2*H*-pyran-4-yl)amino]-1-{4-[(Z)-2-phenylethenyl]phenyl}-1*H*-pyrazol-4-carbonsäure;
3-[[(*trans*-4-Methylcyclohexyl)carbonyl](1-methylethyl)amino]-1-{4-[(Z)-2-(3-pyrazolyl)ethenyl]phenyl}-1*H*-pyrazol-4-carbonsäure;
3-[[(*trans*-4-Methylcyclohexyl)carbonyl](1-methylethyl)amino]-1-{4-[(E)-2-(3-pyrazolyl)ethenyl]phenyl}-1*H*-pyrazol-4-carbonsäure;
3-[[(*trans*-4-Methylcyclohexyl)carbonyl](1-methylethyl)amino]-1-{4-[(E)-2-(tetrahydro-2H-pyran-4-yl)ethenyl]phenyl}-1*H*-pyrazol-4-carbonsäure;
3-[[(*trans*-4-Methylcyclohexyl)carbonyl](tetrahydro-2*H*-pyran-4-yl)amino]-1-{4-[(E)-2-(4-thiazolyl)ethenyl]phenyl}-1*H*-pyrazol-4-carbonsäure;
3-[[(*trans*-4-Methylcyclohexyl)carbonyl](tetrahydro-2*H*-pyran-4-yl)amino]-1-{4-[(Z)-2-(4-thiazolyl)ethenyl]phenyl}-1*H*-pyrazol-4-carbonsäure;
1-((E)-2-*tert*-Butylethenyl)-3-[[(*trans*-4-methylcyclohexyl)carbonyl](1-methylethyl)-amino]-1*H*-pyrazol-4-carbonsäure;
1-((E)-2-Phenylethenyl)-3-[[(*trans*-4-methylcyclohexyl)carbonyl](1-methylethyl)amino]-1*H*-pyrazol-4-carbonsäure;
1-(4-Methyl-1-cyclohexen-1-yl)-3-[[(*trans*-4-methylcyclohexyl)carbonyl](1-methylethyl)-amino]-1*H*-pyrazol-4-carbonsäure;
1-(3-Cyanophenyl)-3-[[(*trans-*4-methylcyclohexyl)carbonyl](1-methylethyl)amino]-1*H-*pyrazol-4-carbonsäure;
3-{(1-Methylethyl)[(4-methylidencyclohexyl)carbonyl]amino}-1-phenyl-1*H*-pyrazol-4-carbonsäure;
1-(4-Trifluormethylcyclohexen-1-yl)-3-[[(*trans*-4-methylcyclohexyl)carbonyl](1-methylethyl)amino]-1*H-*pyrazol-4-carbonsäure;
3-[[(*trans*-4-Methylcyclohexyl)carbonyl](1-methylethyl)amino]-1-{4-[(phenyloxy)-methyl]phenyl}-1*H*-pyrazol-4-carbonsäure;
1-[4-(Phenylsulfonylmethyl)phenyl]-3-[[(*trans*-4-methylcyclohexyl)carbonyl](1-methylethyl)amino]-1*H*-pyrazol-4-carbonsäure;
1-[4-(Phenylthiomethyl)phenyl]-3-[[(*trans*-4-methylcyclohexyl)carbonyl](1-methylethyl)-amino]-1*H*-pyrazol-4-carbonsäure;
1-[4-(Phenoxy)phenyl]-3-[[(*trans*-4-methylcyclohexyl)carbonyl](1-methylethyl)amino]-1*H*-pyrazol-4-carbonsäure;
1-[4-{(1,3-Thiazol-4-yl-methyl)oxy}phenyl]-3-[[(*trans*-4-methylcyclohexyl)carbonyl](1-methylethyl)amino]-1*H*-pyrazol-4-carbonsäure;
1-[4-((E)-Phenylethenyl)phenyl]-3-[[(*trans*-4-methylcyclohexyl)carbonyl](1-methylethyl)amino]-1*H*-pyrazol-4-carbonsäure;
1-[4-((Z)-Phenylethenyl)phenyl]-3-[[(*trans*-4-methylcyclohexyl)carbonyl](1-methylethyl)amino]-1*H*-pyrazol-4-carbonsäure;
1-[4-((E,Z)-(1,3-Thiazol-2-yl)ethenyl)phenyl]-3-[[(*trans-*4-methylcyclohexyl)carbonyl]-(1-methylethyl)amino]-1*H*-pyrazol-4-carbonsäure;
1-[4-((E)-Phenyl-2-methylethenyl)phenyl]-3-[[(*trans*-4-methylcyclohexyl)carbonyl](1-methylethyl)amino]-1*H*-pyrazol-4-carbonsäure;
1-[4-((E)-(Pyridin-4-yl)ethenyl)phenyl]-3-[[(*trans*-4-methylcyclohexyl)carbonyl](1-methylethyl)amino]-1*H*-pyrazol-4-carbonsäure;
1-[4-((E)-(1,3-Thiazol-4-yl)ethenyl)phenyl]-3-[[(*trans*-4-methylcyclohexyl)carbonyl](1-methylethyl)amino]-1*H*-pyrazol-4-carbonsäure;
1-[4-((E)-(Furan-2-yl)ethenyl)phenyl]-3-[[(*trans*-4-methylcyclohexyl)carbonyl](1-methylethyl)amino]-1*H*-pyrazol-4-carbonsäure;
1-[4-((E)-(2-Methyl-1,3-thiazol-4-yl)ethenyl)phenyl]-3-[[(*trans*-4-methylcyclohexyl)-carbonyl](1-methylethyl)amino]-1*H*-pyrazol-4-carbonsäure;
3-[(Cyclohexylacetyl)(1-methylethyl)amino]-1-phenyl-1*H*-pyrazol-4-carbonsäure;
3-{(1-Methylethyl)[(4-methylphenyl)carbonyl]amino}-1-phenyl-1*H*-pyrazol-4-carbonsäure;
3-[[(4-Brom-2-chlorphenyl)carbonyl](1-methylethyl)amino]-1-phenyl-1*H*-pyrazol-4-carbonsäure;
3-[[(*trans*-4-Methylcyclohexyl)carbonyl](phenyl)amino]-1-phenyl-1*H*-pyrazol-4-carbonsäure;
3- {[2-(Dimethylamino)2-oxoethyl][(*trans*-4-methylcyclohexyl)carbonyl]amino}-1-phenyl-1*H*-pyrazol-4-carbonsäure;
3-{[(*trans*-4-Methylcyclohexyl)carbonyl]{1-[(methyloxy)carbonyl]-4-piperidinyl}amino}-1-phenyl-1*H*-pyrazol-4-carbonsäure;
3-{[(*trans*-4-Methylcyclohexyl)carbonyl][1-(methylsulfonyl)-4-piperidinyl]amino}-1-phenyl-1*H*-pyrazol-4-carbonsäure;
3-[[(*trans*-4-Methylcyclohexyl)carbonyl](1-methyl-4-piperidinyl)amino]-1-phenyl-1*H-*pyrazol-4-carbonsäure;
3-{{1-[(Ethylamino)carbonyl]-4-piperidinyl}[(*trans*-4-methylcyclohexyl)carbonyl]-amino}-1-phenyl-1*H*-pyrazol-4-carbonsäure;
3-[[(*trans*-4-Methylcyclohexyl)carbonyl](2-pyrazinylmethyl)amino]-1-phenyl-1*H-*pyrazol-4-carbonsäure;
*rel*-3-[{[(1S,2R,4S)-2-Hydroxy-4-methylcyclohexyl]carbonyl}(1-methylethyl)amino]-1-phenyl-1*H*-pyrazol-4-carbonsäure;
3-[[(*trans*-4-Methylcyclohexyl)carbonyl](1-methylethyl)amino]-1-{4-[(3-methoxyphenyl-carbonyl)amino]phenyl}-1*H*-pyrazol-4-carbonsäure;
3-[[(*trans*-4-Methylcyclohexyl)carbonyl](1-methylethyl)amino]-1-{4-[(phenylmethyl)-oxy]phenyl}-1*H*-pyrazol-4-carbonsäure ;
1-(1H-Indol-5-yl)-3-[[(*trans*-4-methylcyclohexyl)carbonyl](1-methylethyl)amino]-1*H-*pyrazol-4-carbonsäure;
3-[[(*trans-*4-Methylcyclohexyl)carbonyl](1-methylethyl)amino]-1-{4-[(E/Z)-2-phenylethenyl]phenyl}-1*H*-pyrazol-4-carbonsäure;
3-[[(*trans*-4-Methylcyclohexyl)carbonyl](1-methylethyl)amino]-1-[4-(2-phenylethyl)-phenyl]-1*H*-pyrazol-4-carbonsäure;
3-[[(*trans*-4-Methylcyclohexyl)carbonyl](tetrahydro-2*H*-pyran-4-yl)amino]-1-{4-[2-phenylethyl]phenyl}-1*H*-pyrazol-4-carbonsäure;
3-[[(*trans*-4-Methylcyclohexyl)carbonyl](1-methylethyl)amino]-1-[4-[(1,3-thiazol-4-yl)ethyl]phenyl]-1*H*-pyrazol-4-carbonsäure;
3-[[(*trans*-4-Methylcyclohexyl)carbonyl](tetrahydro-2*H*-pyran-4-yl)amino]-1-{4-[(1,3-thiazol-4-yl)ethyl]phenyl}-1*H*-pyrazol-4-carbonsäure;
1-Cyclohexyl-3-[[(*trans*-4-methylcyclohexyl)carbonyl](1-methylethyl)amino]-1*H-*pyrazol-4-carbonsäure;
3-[[(*trans*-4-Methylcyclohexyl)carbonyl](1-methylethyl)amino]-1-[1-(methylsulfonyl)-1,2,3,6-tetrahydro-4-pyridinyl]-1*H*-pyrazol-4-carbonsäure;
3-[[(*trans*-4-Methylcyclohexyl)carbonyl](phenylmethyl)amino]-1-phenyl-1*H*-pyrazol-4-carbonsäure;
3- {Cyclopentyl[(*trans*-4-methylcyclohexyl)carbonyl]amino}-1-phenyl-1*H*-pyrazol-4-carbonsäure;
3-[[(*trans*-4-Methylcyclohexyl)carbonyl](tetrahydro-2*H*-pyran-4-yl)amino]-1-phenyl-1*H*-pyrazol-4-carbonsäure;
3- {(1-Acetyl-4-piperidinyl)[(*trans-*4-methylcyclohexyl)carbonyl]amino}-1-phenyl-1*H-*pyrazol-4-carbonsäure;
3-[[(*trans*-4-Methylcyclohexyl)carbonyl](4-piperidinyl)amino]-1-phenyl-1*H*-pyrazol-4-carbonsäure;
3-[[(*trans*-4-Methylcyclohexyl)carbonyl](1-methylethyl)amino]-1-{4-[(E)-2-cyclohexylethenyl]phenyl}-1*H*-pyrazol-4-carbonsäure;
1-[4-(2-Cyclohexylethyl)phenyl]-3-[[(*trans*-4-methylcyclohexyl)carbonyl](1-methylethyl)amino]-1*H*-pyrazol-4-carbonsäure;
3-[[(*trans*-4-Methylcyclohexyl)carbonyl](1-methylethyl)amino]-1-{4-[2-pyridinylethenyl]phenyl}-1*H*-pyrazol-4-carbonsäure;
3-[[(*trans*-4-Methylcyclohexyl)carbonyl](1-methylethyl)amino]-1-{4-[2-pyridinylethyl]phenyl}-1*H*-pyrazol-4-carbonsäure;
3-[[(*trans*-4-Methylcyclohexyl)carbonyl](1-methylethyl)amino]-1-{4-[1,3-thiazol-2-yl-ethyl]phenyl}-1*H*-pyrazol-4-carbonsäure;
3-[[(*trans*-4-Methylcyclohexyl)carbonyl](1-methylethyl)amino]-1- {4-[2-(1H-pyrazol-3-yl)ethyl]phenyl}-1*H*-pyrazol-4-carbonsäure;
3-[[(*trans*-4-Methylcyclohexyl)carbonyl](1-methylethyl)amino]-1- {4-[(phenylamino)-carbonyl]phenyl}-1*H*-pyrazol-4-carbonsäure;
3-[[(*trans*-4-Methylcyclohexyl)carbonyl](1-methylethyl)amino]-1- {4-[(phenylcarbonyl)-amino]phenyl}-1*H*-pyrazol-4-carbonsäure ;
3-[[(*trans*-4-Methylcyclohexyl)carbonyl](1-methylethyl)amino]-1-{4-[(3-methylphenyl-carbonyl)amino]phenyl}-1*H*-pyrazol-4-carbonsäure ;
3-[[(*trans*-4-Methylcyclohexyl)carbonyl] {1-[(*tert*-butyloxy)carbonyl]-4-piperidinyl}amino]-1-phenyl-1*H*-pyrazol-4-carbonsäure;
3-[[(*trans*-4-Methylcyclohexyl)carbonyl](1-methylethyl)amino]-1-{4-[(4-fluorphenylcarbonyl)amino]phenyl}-1*H*-pyrazol-4-carbonsäure;
3-[[(*trans*-4-Methylcyclohexyl)carbonyl](1-methylethyl)amino]-1-{4-[(cyclohexylcarbonyl)amino]phenyl}-1*H*-pyrazol-4-carbonsäure ;
1-(4-{[(4-Fluorphenyl)amino]carbonyl} phenyl)-3-[[(*trans*-4-methylcyclohexyl)-carbonyl](1-methylethyl)amino]-1*H*-pyrazol-4-carbonsäure;
3-[[(*trans*-4-Methylcyclohexyl)carbonyl](1-methylethyl)amino]-1-{3-[(chlorphenylcarbonyl)amino]phenyl}-1*H*-pyrazol-4-carbonsäure;
3-[[(*trans-*4-Methylcyclohexyl)carbonyl](1-methylethyl)amino]-1-{4-[(phenylsulfonyl)-amino]phenyl}-1*H*-pyrazol-4-carbonsäure ;
1-(4-Methyl-1-cyclohexen-1-yl)-3-[[(*trans*-4-methylcyclohexyl)-carbonyl](1-methylethyl)amino]-1*H*-pyrazol-4-carbonsäure;
1-(4,4-Dimethyl-1-cyclohexen-1-yl)-3-[[(*trans*-4-methylcyclohexyl)-carbonyl](tetrahydro-3-furanyl)amino]-1*H*-pyrazol-4-carbonsäure;
und Salze, Solvate und Ester und einzelne Enantiomere davon, wo zutreffend.

3. Mindestens eine chemische Einheit ausgewählt aus Verbindungen der Formel (I) 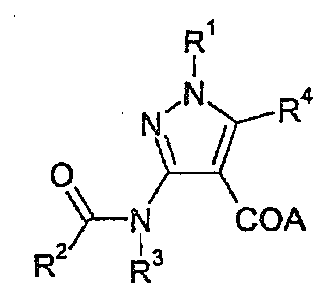 wobei:
A für Hydroxy steht;
R¹ für Aryl, durch ein Ringkohlenstoffatom gebundenes Heteroaryl oder durch ein Ringkohlenstoffatom gebundenes Heterocyclyl steht, wobei jedes davon gegebenenfalls mit einem oder mehreren Substituenten, ausgewählt aus -C₁₋₆-Alkyl, Halogen, -OR^{A}, - SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, Nitro, Cyano, -CF₃, -OCF₃, NR^{E}SO₂R^{D}, Phenyl und Heterocyclyl, substituiert sein kann, wobei der -C₁₋₆-Alkyl-Substituent selbst gegebenenfalls mit einem oder mehreren Substituenten, ausgewählt aus -C₅₋₉-Cycloalkyl, Halogen, -NR^{B}R^{C}, -C(O)NR^{B}R^{C}, -NR^{E}C(O)R^{D}, -SR^{A}, -SO₂R^{D}, OR^{A}, Oxo, Phenyl, Heteroaryl oder Heterocyclyl, substituiert sein kann; oder R¹ für -C₁₋₆-Alkyl oder -C₅₋₉-Cycloalkyl steht;
R² Phenyl, substituiert mit einem oder mehreren Substituenten, ausgewählt aus -C₁₋₆-Alkyl, Halogen, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}; -NR^{E}CO₂R^{D}, -NR^{E}C(O)NR^{F}R^{G}, -SO₂NR^{F}R^{G}, -SO₂R^{D}, Nitro, Cyano und Heterocyclyl, darstellt; oder R² für -(CH₂)ₙC₅₋₇-Cycloalkyl steht, gegebenenfalls an dem Cycloalkyl substituiert mit einem oder mehreren Substituenten, ausgewählt aus -C₁₋₆-Alkyl, =CH(CH₂)ₜH, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, Fluor, Nitro, Cyano, Oxo und Heterocyclyl, oder wobei zwei Substituenten zusammen einen C₁₋₂-Alkylen-Brückensubstituenten bilden können;
t für 0, 1, 2, 3 oder 4 steht;
n für 0 oder 1 steht;
R³ Heterocyclyl oder Heteroaryl darstellt; oder Phenyl, welches gegebenenfalls mit einem oder mehreren Substituenten, ausgewählt aus -C₁₋₆-Alkyl, Halogen, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, - NR^{E}C(O)NR^{F}R^{G}, -SO₂NR^{F}R^{G}, -SO₂R^{D}, Nitro, Cyano und Heterocyclyl, substituiert ist; oder R³ für -C₁₋₆-Alkyl steht, welches gegebenenfalls mit einem oder mehreren Substituenten, ausgewählt aus -C₁₋₆-Alkyl, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, NR^{E}C(O)NR^{F}R^{G}, -SO₂NR^{F}R^{G}, -SO₂R^{D}, Fluor, Nitro, Cyano, Oxo, Phenyl, Heteroaryl und Heterocyclyl, substituiert ist;
R⁴ ein Wasserstoffatom darstellt;
R^{A} ein Wasserstoffatom, -C₁₋₆-Alkyl, Arylalkyl, Heteroarylalkyl, Aryl, Heterocyclyl oder Heteroaryl darstellt;
R^{B} und R^{C} unabhängig ein Wasserstoffatom, -C₁₋₆-Alkyl, Aryl, Heterocyclyl oder Heteroaryl darstellen; oder R^{B} und R^{C} zusammen mit dem Stickstoffatom, an welches sie gebunden sind, einen 5- oder 6-gliedrigen gesättigten zyklischen Rest bilden;
R^{D} ausgewählt ist aus -C₁₋₆-Alkyl, Aryl, Heterocyclyl, Heteroaryl, Arylalkyl und Heteroarylalkyl;
R^{E} ein Wasserstoffatom oder -C₁₋₆-Alkyl darstellt;
R^{F} und R^{G} unabhängig ausgewählt sind aus einem Wasserstoffatom, -C₁₋₆-Alkyl, Aryl, Heteroaryl, Arylalkyl und Heteroarylalkyl; oder R^{F} und R^{G} zusammen mit dem Stickstoffatom, an welches sie gebunden sind, einen 5- oder 6-gliedrigen gesättigten zyklischen Rest bilden;
und Salze, Solvate und Ester davon zur Verwendung in der medizinischen Therapie.

4. Verbindung gemäß Anspruch 3, wobei die medizinische Therapie die Behandlung von Virusinfektionen darstellt.

5. Verbindung gemäß Anspruch 4, wobei die Virusinfektion HCV ist.

6. Pharmazeutische Formulierung, umfassend mindestens eine chemische Einheit, ausgewählt aus Verbindungen der Formel (I) und pharmazeutisch verträglichen Salzen, Solvaten und Estern davon wie in Anspruch 1 definiert, in Verbindung mit mindestens einem pharmazeutisch verträglichen Verdünnungsmittel oder Träger.

7. Verfahren zur Herstellung einer Verbindung der Formel (I) wie in Anspruch 1 definiert, umfassend die Behandlung einer Verbindung der Formel (II) 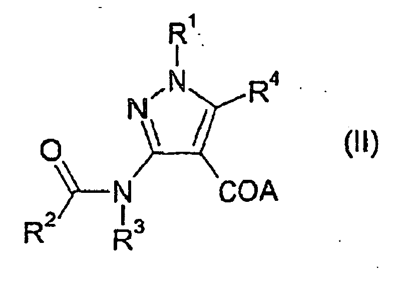 in welcher A für einen Alkoxy-, Benzyloxy- oder Silyloxyrest steht und R¹, R², R³ und
R⁴ wie oben stehend für Formel (I) definiert sind, mit einer Base.

8. Verfahren gemäß Anspruch 7, wobei A für Ethoxy steht.

9. Verwendung von mindestens einer chemischen Einheit, ausgewählt aus Verbindungen der Formel (I) und pharmazeutisch verträglichen Salzen, Solvaten und Estern davon gemäß Anspruch 1, zur Herstellung eines Medikaments zur Behandlung und/oder Prophylaxe einer Virusinfektion.

10. Verwendung gemäß Anspruch 9, wobei die Virusinfektion HCV ist.

## Revendications

1. Au moins une entité chimique choisie parmi des composés de formule (I) : 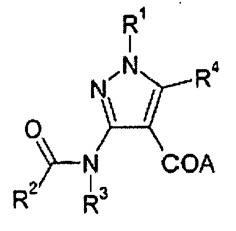 dans laquelle :
A représente un groupe hydroxy ;
R¹ représente un groupe aryle, hétéroaryle lié par un atome carbone du noyau, ou hétérocyclyle lié par un, atome de carbone du noyau, chacun pouvant être facultativement substitué avec un ou plusieurs substituants choisis entre des substituants -alkyle en C₁ à C₆, halogéno, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano, -CF₃, -OCF₃, NR^{E}SO₂R^{D}, phényle et hétérocycle, dans lesquels le substituant -alkyle en C₁ à C₆ proprement dit peut être facultativement substitué avec un ou plusieurs substituants choisis entre des substituants -cycloalkyle en C₅ à C₉, halogéno, -NR^{B}R^{C}, -C(O)NR^{B}R^{C}, -NR^{E}C(O)R^{D}, -SR^{A}, -SO₂R^{D}, OR^{A}, oxo, phényle, hétéroaryle et hétérocyclyle ; ou bien R¹ représente un groupe -alkyle en C₁ à C₆ ou -cycloalkyle en C₅ à C₉ ;
R² représente un groupe phényle substitué avec un ou plusieurs substituants choisis entre des substituants -alkyle en C₁ à C₆, halogéno, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano et hétérocyclyle ; ou bien R² représente un groupe (CH₂)ₙ(cycloalkyle en C₅ à C₇) facultativement substitué sur le groupe cycloalkyle avec un ou plusieurs substituants choisis entre des substituants -alkyle en C₁ à C₆, =CH(CH₂)ₜH, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, NR^{E}CO₂R^{D}, NR^{E}C(O)NR^{F}R^{G},-SO₂NR^{F}R^{G}, -SO₂R^{D}, fluoro, nitro, cyano, oxo et hétérocyclyle, ou bien deux substituants peuvent former conjointement un substituant à pont alkylène en C₁ ou C₂ ;
t est égal à 0, 1, 2, 3 ou 4 ;
n est égal à 0 ou 1 ;
R³ représente un groupe hétérocyclyle ou hétéroaryle ; ou un groupe phényle facultativement substitué avec un ou plusieurs substituants choisis entre des substituants -alkyle en C₁ à C₆, halogéno, -OR^{A}, -SR^{A}, -C(O)NR^{B}R^{C}, -C(O)R^{D}, -CO₂H, -CO₂R^{D}, NR^{B}R^{C}, -NR^{E}C(O)R^{D}, NR^{E}CO₂R^{D} , -NR^{E}C(O)NR^{F}R^{G}, SO₂NR^{F}R^{G} , -SO₂R^{D}, nitro, cyano et hétérocyclyle ; ou bien R³ représente un groupe -alkyle en C₁ à C₆ facultativement substitué avec un ou plusieurs substituants choisis entre des substituants -alkyle en C₁ à C₆, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, NR^{E}C(O)NR^{F}R^{G},- SO₂NR^{F}R^{G}, -SO₂R^{D}, fluoro, nitro, cyano, oxo, phényle, hétéroaryle et hétérocyclyle ;
R⁴ représente un atome d'hydrogène ;
R^{A} représente un atome d'hydrogène, un groupe -alkyle en C₁ à C₆, arylalkyle, hétéroarylalkyle, aryle, hétérocyclyle ou hétéroaryle ;
R^{B} et R^{C} représentent indépendamment un atome d'hydrogène, un groupe -alkyle en C₁ à C₆, aryle, hétérocyclyle ou hétéroaryle ; ou bien R^{B} et R^{C}, conjointement avec l'atome d'azote auquel ils sont fixés, forment un groupe cyclique penta- ou hexagonal saturé;
R^{D} est choisi dans le groupe consistant en des groupes -alkyle en C₁ à C₆, aryle, hétérocyclyle, hétéroaryle, arylalkyle et hétéroarylalkyle ;
R^{E} représente un atome d'hydrogène ou un groupe -alkyle en C₁ à C₆ ;
R^{F} et R^{G} sont choisis indépendamment dans le groupe consistant en un atome d'hydrogène, des groupes -alkyle en C₁ à C₆, aryle, hétéroaryle, arylalkyle et hétéroarylalkyle ; ou bien R^{F} et R^{G}, conjointement avec l'atome d'azote auquel ils sont fixés, forment un groupe cyclique penta- ou hexagonal saturé ;
et leurs sels, produits de solvatation et esters.

2. Au moins une entité chimique suivant la revendication 1, choisie parmi les composés de formule (I) choisis dans le groupe consistant en :
acide 3-[[(*trans*-4-méthylcyclohexyl)carbonyl](1-méthyléthyl)amino]-1-phényl-1*H*-pyrazole-4-carboxylique ;
acide 3-[[(*trans*-4-méthylcyclohexyl)carbonyl](1-méthyléthyl)amino]-1-(4-méthylphényl)-1H-pyrazole-4-carboxylique ;
acide 1-(1-cyclohexène-1-yl)-3-[[(*trans*-4-méthylcyclohexyl)-carbonyl](1-méthyléthyl)amino]-1H-pyrazole-4-carboxylique ;
acide 1-(4-chloro-3-méthylphényl)-3-[[(*trans*-4-méthylcyclohexyl)carbonyl](1-méthyléthyl)amino]-1H-pyrazole-4-carboxylique ;
acide 1-(4-fluorophényl)-3-[[(*trans*-4-méthylcyclohexyl)-carbonyl](1-méthyléthyl)amino]-1H-pyrazole-4-carboxylique ;
acide 1-(6-indolyl)-3-[[(*trans*-4-méthylcyclohexyl)carbonyl]-(1-méthyléthyl)amino]-1H-pyrazole-4-carboxylique ;
acide 1-(4-hydroxyphényl)-3-[[(*trans*-4-méthylcyclohexyl)-carbonyl](1-méthyléthyl)amino]-1H-pyrazole-4-carboxylique ;
acide 3-[[(*trans*-4-méthylcyclohexyl)carbonyl] (1-méthyléthyl)amino]-1-[4-(trifluorométhyl)phényl]-1H-pyrazole-4-carboxylique ;
acide 1-[4-(acétylamino)phényl]-3-[[(*trans*-4-méthylcyclohexyl)-carbonyl](1-méthyléthyl)amino]-1H-pyrazole-4-carboxylique ;
acide 1-(4-biphénylyl)-3-[[(*trans*-4-méthylcyclohexyl)-carbonyl](1-méthyléthyl)amino]-1H-pyrazole-4-carboxylique;
acide 1-[4-(diméthylamino)phényl]-3-[[(*trans*-4-méthylcyclohexyl)carbonyl](1-méthyléthyl)amino]-1H-pyrazole-4-carboxylique ;
acide 3-[[(*trans*-4-méthylcyclohexyl)carbonyl](1-méthyléthyl)aminol-1-[4-(méthyloxy)phényl]-1H-pyrazole-4-carboxylique;
acide 1-(4-acétylphényl)-3-[[(trans-4-méthylcyclohexyl)-carbonyl](1-méthyléthyl)amino]-1H-pyrazole-4-carboxylique ;
acide 3-[[(*trans*-4-méthylcyclohexyl)carbonyl](1-méthyléthyl)amino]-1-{4-[(trifluorométhyl)oxy]phényl}-1H-pyrazole-4-carboxylique ;
acide 1-(4-cyanophényl)-3-[[(*trans*-4-méthylcyclohexyl)-carbonyl](1-méthyléthyl)amino]-1H-pyrazole-4-carboxylique ;
acide 1-{4-[(diméthylamino)carbonyl]phényl}-3-[[(*trans-*4-méthylcyclohexyl)carbonyl](1-méthyléthyl)amino]-1H-pyrazole-4-carboxylique ;
acide 3-[[(*trans*-4-méthylcyclohexyl)carbonyl](1-méthyléthyl)amino]-1-(3-thiényl)-1H-pyrazole-4-carboxylique ;
acide 3-[[(*trans*-4-méthylcyclohexyl)carbonyl](1-méthyléthyl)amino]-1-[3-(trifluorométhyl)phényl]-1H-pyrazole-4-carboxylique ;
acide 1-(3,5-diméthylphényl)-3-[[(*trans*-4-méthylcyclohexyl)-carbonyl](1-méthyléthyl)amino]-1H-pyrazole-4-carboxylique ;
acide 1-(3-chloro-5-fluorophényl)-3-[[(*trans*-4-méthylcyclohexyl)carbonyl](1-méthyléthyl)amino]-1H-pyrazole-4-carboxylique ;
acide 1-[3,5-bis-(trifluorométhyl)phényl]-3-[[(*trans*-4-méthylcyclohexyl)carbonyl](1-méthyléthyl)amino]-1H-pyrazole-4-carboxylique ;
acide 1-(1,3-benzodioxole-5-yl)-3-[[(*trans*-4-méthylcyclohexyl)-carbonyl](1-méthyléthyl)amino]-1H-pyrazole-4-carboxylique ;
acide 1-(2,3-dihydro-1-benzofuranne-5-yl)-3-[[(*trans*-4-méthylcyclohexyl)carbonyl](1-méthyléthyl)amino]-1H-pyrazole-4-carboxylique ;
acide 1-(2,3-dihydro-1,4-benzodioxine-6-yl)-3-[[(*trans-*4-méthylcyclohexyl)carbonyl](1-méthyléthyl)amino]-1H-pyrazole-4-carboxylique ;
acide 3-[[(*trans-*4-méthylcyclohexyl)carbonyl](1-méthyléthyl)amino]-1-(3,4,5-trifluorophényl)-1H-pyrazole-4-carboxylique ;
acide 1-(4-chlorophényl)-3-[[(*trans*-4-méthylcyclohexyl)-carbonyl](1-méthyléthyl)amino]-1H-pyrazole-4-carboxylique ;
acide 3-[[(*trans-*4-méthylcyclohexyl)carbonyl](1-méthyléthyl)amino]-1-[3-(méthyloxy)phényl]-1H-pyrazole-4-carboxylique ;
acide 3-[[(*trans*-4-méthylcyclohexyl)carbonyl](1-méthyléthyl)amino]-1-[4-(méthylsulfonyl)phényl]-1H-pyrazole-4-carboxylique ;
acide 1-(2-fluorophényl)-3-[[(*trans*-4-méthylcyclohexyl)-carbonyl](1-méthyléthyl)amino]-1H-pyrazole-4-carboxylique ;
acide 1-(3-hydroxyphényl)-3-[[(*trans*-4-méthylcyclohexyl)-carbonyl](1-méthyléthyl)amino]-1H-pyrazole-4-carboxylique ;
acide 3-[[(*trans*-4-méthylcyclohexyl)carbonyl](1-méthyléthyl)amino]-1-(3-méthylphényl)-1H-pyrazole-4-carboxylique ;
acide 1-(3-fluorophényl)-3-[[(*trans*-4-méthylcyclohexyl)-carbonyl](1-méthyléthyl)amino]-1H-pyrazole-4-carboxylique ;
acide 1-(4-aminophényl)-3-[[(*trans*-4-méthylcyclohexyl)-carbonyl] (1-méthyléthyl) amino]-1H-pyrazole-4-carboxylique ;
acide 1-(3-chlorophényl)-3-[[(*trans*-4-méthylcyclohexyl)-carbonyl](1-méthyléthyl)amino]-1H-pyrazole-4-carboxylique ;
acide 3-[[(*trans*-4-méthylcyclohexyl)carbonyl](1-méthyléthyl)amino]-1-{3-[(trifluorométhyl)oxy]phényl}-1H-pyrazole-4-carboxylique ;
acide 1-(4-chloro-3-fluorophényl)-3-[[(*trans*-4-méthylcyclohexyl)carbonyl](1-méthyléthyl)amino]-1H-pyrazole-4-carboxylique ;
acide 1-(3-amino-4-méthylphényl)-3-[[(*trans*-4-méthylcyclohexyl)carbonyl](1-méthyléthyl)amino]-1H-pyrazole-4-carboxylique;
acide 1-(3-fluoro-4-méthylphényl)-3-[[(*trans*-4-méthylcyclohexyl)carbonyl](1-méthyléthyl)amino]-1H-pyrazole-4-carboxylique ;
acide 1-(3,4-difluorophényl)-3-[[(*trans*-4-méthylcyclohexyl)-carbonyl](1-méthyléthyl)amino]-1H-pyrazole-4-carboxylique ;
acide 1-[(E)-1-hexène-1-yl]-3-[[(*trans*-4-méthylcyclohexyl)-carbonyl](1-méthyléthyl)amino]-1H-pyrazole-4-carboxylique ;
acide 1-[(E)-2-cyclohexyléthényl]-3-[[(*trans*-4-méthylcyclohexyl)carbonyl](1-méthyléthyl)amino]-1H-pyrazole-4-carboxylique ;
acide 3-[[(*trans*-4-méthylcyclohexyl)carbonyl] (1-méthyléthyl)amino]-1-[(E)-4-méthyl-1-péntène-1-yl]-1H-pyrazole-4-carboxylique ;
acide 1-[(E)-2-(4-fluorophényl)éthényl]-3-[[(*trans*-4-méthylcyclohexyl)carbonyl](1-méthyléthyl)amino]-1H-pyrazole-4-carboxylique ;
acide 1-(4-éthénylphényl)-3-[[(*trans*-4-méthylcyclohexyl)-carbonyl](1-méthyléthyl)amino]-1H-pyrazole-4-carboxylique ;
acide 1-[4-(hydroxyméthyl)phényl]-3-[[(*trans*-4-méthylcyclohexyl)carbonyl](1-méthyléthyl)amino]-1H-pyrazole-4-carboxylique ;
acide 1-(4-éthylphényl)-3-[((*trans-*4-méthylcyclohexyl)-carbonyl](1-méthyléthyl)amino]-1H-pyrazole-4-carboxylique ;
acide 3-[[(*trans*-4-méthylcyclohexyl)carbonyl](1-méthyléthyl)amino]-1-[4-(1-méthyléthyl)phényl]-1H-pyrazole-4-carboxylique ;
acide 1-(5-acétyl-2-thiényl)-3-[[(*trans*-4-méthylcyclohexyl)-carbonyl](1-méthyléthyl)amino]-1H-pyrazole-4-carboxylique ;
acide 1-(5-chloro-2-thiényl)-3-[[(*trans*-4-méthylcyclohexyl)-carbonyl](1-méthyléthyl)amino]-1H-pyrazole-4-carboxylique ;
acide 3-[[(*trans*-4-méthylcyclohexyl)carbonyl](1-méthyléthyl)amino]-1-(5-méthyl-2-thiényl)-1H-pyrazole-4-carboxylique ;
acide 3-[[(*trans*-4-méthylcyclohexyl)carbonyl](1-méthyléthyl)amino]-1-(5-phényl-2-thiényl)-1H-pyrazole-4-carboxylique;
acide 1-((4-méthyl)cyclohexène-1-yl)-3-[[(*trans*-4-méthylcyclohexyl)carbonyl](tétrahydro-2H-pyranne-4-yl)amino]-1H-pyrazole-4-carboxylique ;
acide 1-(6-benzofurannyl)-3-[[(*trans*-4-méthylcyclohexyl)-carbonyl](1-méthyléthyl)amino]-1H-pyrazole-4-carboxylique;
acide 1-(cycloheptène-1-yl)-3-[[(*trans*-4-méthylcyclohexyl)-carbonyl](tétrahydro-2H-pyranne-4-yl)amino]-1H-pyrazole-4-carboxylique ;
acide 1-((4-méthyl)cyclohexène-1-yl)-3-[[(*trans*-4-méthylcyclohexyl)carbonyl](1-(méthylsulfonyl)-4-pipéridinyl)amino]-1H-pyrazole-4-carboxylique ;
acide 1-((4,4-diméthyl)cyclohexène-1-yl)-3-[[(*trans*-4-méthylcyclohexyl)carbonyl](tétrahydro-2H-pyranne-4-yl)amino]-1H-pyrazole-4-carboxylique ;
acide 1-(3-chloro-4-benzyloxyphényl)-3-[[(*trans*-4-méthylcyclohexyl)carbonyl](1-méthyléthyl)amino]-1H-pyrazole-4-carboxylique ;
acide 1-(4-benzyloxy-cyclohexène-1-yl)-3-[[(*trans*-4-méthylcyclohexyl)carbonyl](1-méthyléthyl)amino]-1H-pyrazole-4-carboxylique ;
acide 1-(4,4-diméthyl-cyclohexène-1-yl)-3-{[(*trans*-4-méthylcyclohexyl)carbonyl](1-méthyléthyl)amino]-1H-pyrazole-4-carboxylique ;
acide 3-[[(*trans*-4-méthylcyclohexyl)carbonyl](tétrahydro-2H-pyranne-4-yl)amino]-1-{4-[(E)-2-phényléthényl]phényl}-1H-pyrazole-4-carboxylique ;
acide 3-[[(*trans*-4-méthylcyclohexyl)carbonyl](tétrahydro-2H-pyranne-4-yl)amino]-1-{4-[(Z)-2-phényléthényl]phényl}-1H-pyrazole-4-carboxylique ;
acide 3-[[(*trans*-4-méthylcyclohexyl)carbonyl](1-méthyléthyl)amino]-1-{4-[(Z)-2-(3-pyrazolyl)-éthényl]phényl}-1H-pyrazole-4-carboxylique ;
acide 3-[[(*trans*-4-méthylcyclohexyl)carbonyl](1-méthyléthyl)amino]-1-{4-[(E)-2-(3-pyrazolyl)-éthényl]phényl}-1H-pyrazole-4-carboxylique ;
acide 3-[[(*trans*-4-méthylcyclohexyl)carbonyl](1-méthyléthyl)amino]-1-{4-[(E)-2-(tétrahydro-2H-pyranne-4-yl)-éthényl]-phényl}-1H-pyrazole-4-carboxylique ;
acide 3-[[(*trans*-4-méthylcyclohexyl)carbonyl] (tétrahydro-2H-pyranne-4-yl)amino]-1-{4-[(E)-2-(4-thiazolyl)éthényl]-phényl}-1H-pyrazole-4-carboxylique
acide 3-[[(trans-4-méthylcyclohexyl)carbonyl](tétrahydro-2H-pyranne-4-yl)amino]-1-{4-[(Z)-2-(4-thiazolyl)éthényl]-phényl}-1H-pyrazole-4-carboxylique ;
acide 1-((E)-2-tertiobutyl-éthényl)-3-[[(*trans*-4-méthylcyclohexyl)carbonyl] (1-méthyléthyl)amino]-1H-pyrazole-4-carboxylique ;
acide 1-((E)-2-phényl-éthényl)-3-[[(*trans*-4-méthylcyclohexyl)-carbonyl](1-méthyléthyl)amino]-1H-pyrazole-4-carboxylique ;
acide 1-(4-méthyl-1-cyclohexène-1-yl)-3-[[(*trans*-4-méthylcyclohexyl)carbonyl](1-méthyléthyl)amino]-1H-pyrazole-4-carboxylique ;
acide 1-(3-cyanophényl)-3-[[(*trans*-4-méthylcyclohexyl)-carbonyl](1-méthyléthyl)amino]-1H-pyrazole-4-carboxylique ;
acide 3-{(1-méthyéthyl)[(4-méthylidènecyclohexyl)carbonyl]-amino}-1-phényl-1*H*-pyrazole-4-carboxylique ;
acide 1-(4-trifluorométhyl-cyclohexène-1-yl)-3-[[(*trans-*4-méthylcyclohexyl)carbonyl](1-méthyléthyl)amino]-1H-pyrazole-4-carboxylique ;
acide 3-[[(*trans*-4-méthylcyclohexyl)carbonyl](1-méthyléthyl)amino]-1-{4-[(phényloxy)méthyl]phényl}-1H-pyrazole-4-carboxylique ;
acide 1-[4-(phénylsulfonylméthyl)phényl]-3-[[(*trans*-4-méthylcyclohexyl)carbonyl](1-méthyléthyl)amino]-1H-pyrazole-4-carboxylique ;
acide 1-[4-(phénylthiométhyl)phényl]-3-[[(*trans*-4-méthylcyclohexyl)carbonyl](1-méthyléthyl)amino]-1H-pyrazole-4-carboxylique ;
acide 1-[4-(phénoxy)phényl]-3-[[(*trans*-4-méthylcyclohexyl)-carbonyl](1-méthyléthyl)amino]-1H-pyrazole-4-carboxylique ;
acide 1-[4-{(1,3-thiazole-4-ylméthyl)oxy}phényl]-3-[[(*trans-*4-méthylcyclohexyl)carbonyl](1-méthyléthyl)amino]-1H-pyrazole-4-carboxylique ;
acide 1-[4-((E)-phényléthényl)phényl]-3-[[(*trans*-4-méthylcyclohexyl)carbonyl](1-méthyléthyl)amino]-1H-pyrazole-4-carboxylique ;
acide 1-[4-((Z)-phényléthényl)phényl]-3-[[(*trans*-4-méthylcyclohexyl)carbonyl](1-méthyléthyl)amino]-1H-pyrazole-4-carboxylique ;
acide 1-[4-((E,Z)-(1,3-thiazole-2-yl)éthényl)phényl]-3-[[(*trans*-4-méthylcyclohexyl)carbonyl](1-méthyléthyl)amino]-1H-pyrazole-4-carboxylique ;
acide 1-[4-((E)-phényl-2-méthyléthényl)phényl]-3-[[(*trans-*4-méthylcyclohexyl)carbonyl](1-méthyléthyl)amino]-1H-pyrazole-4-carboxylique ;
acide 1-[4-((E)-(pyridine-4yl)éthényl)phényl]-3-[[(*trans-*4-méthylcyclohexyl)carbonyl](1-méthyléthyl)amino]-1H-pyrazole-4 -carboxylique ;
acide 1-[4-((E)-(1,3-thiazole-4-yl)éthényl)phényl]-3-[[(*trans*-4-méthylcyclohexyl)carbonyl](1-méthyléthyl)amino]-1H-pyrazole-4-carboxylique ;
acide 1-[4-((E)-(furanne-2-yl)éthényl)phényl]-3-[[(*trans-*4-méthylcyclohexyl)carbonyl](1-méthyléthyl)amino]-1H-pyrazole-4-carboxylique ;
acide 1-[4-((E)-(2-méthyl-1,3-thiazole-4-yl)éthényl)phényl]-3-[[(*trans*-4-méthylcyclohexyl)carbonyl](1-méthyléthyl)amino]-1H-pyrazole-4-carboxylique ;
acide 3-[(cyclohexylacétyl)(1-méthyléthyl)amino]-1-phényl-1H-pyrazole-4-carboxylique ;
acide 3-{(1-méthyléthyl)[(4-méthylphényl)carbonyl]amino}-1-phényl-1H-pyrazole-4-carboxylique ;
acide -[[(4-bromo-2-chlorophényl)carbonyl](1-méthyléthyl)-amino]-1-phényl-1H-pyrazole-4-carboxylique ;
acide 3-[[(*trans*-4-méthylcyclohexyl)carbonyl](phényl)-amino]-1-phényl-1H-pyrazole-4-carboxylique;
acide 3-{[2-diméthylamino)-2-oxoéthyl][(*trans*-4-méthylcyclohexyl)carbonyl]amino}-1-phényl-1H-pyrazole-4-carboxylique ;
acide 3-{[(*trans*-4-méthylcyclohexyl)carbonyl]{1-[(méthyloxy)-carbonyl]-4-pipéridinyl}amino}-1-phényl-1H-pyrazole-4-carboxylique;
acide 3- [[(*trans*-4-méthylcyclohexyl)carbonyl][1-(méthylsulfonyl)-4-pipéridinyl]amino}-1-phényl-1H-pyrazole-4-carboxylique ;
acide 3-[[(*trans*-4-méthylcyclohexyl) carbonyl] (1-méthyl-4-pipéridinyl)amino]-1-phényl-1H-pyrazole-4-carboxylique ;
acide 3-{{1-[(éthylamino)carbonyl]-4-pipéridinyl][(*trans-*4-méthylcyclohexyl)carbonyl]amino}-1-phényl-1H-pyrazole-4-carboxylique ;
acide 3-[[(*trans*-4-méthylcyclohexyl)carbonyl](2-pyrazinylméthyl)amino]-1-phényl-1H-pyrazole-4-carboxylique ;
acide *rel*-3-[{[(1S,2R,4S)-2-hydroxy-4-méthylcyclohexyl]carbonyl}(1-méthyléthyl)amino]-1-phényl-1H-pyrazole-4-carboxylique ;
acide 3-[[(*trans*-4-méthylcyclohexyl)carbonyl](1-méthyléthyl)amino]-1-{4-[(3-méthoxyphénylcarbonyl)amino]phényl}-1*H*-pyrazole-4-carboxylique ;
acide 3-[[(*trans*-4-méthylcyclohexyl)carbonyl](1-méthyléthyl)amino]-1-{4-[(phénylméthyl)oxy]phényl}-1H-pyrazole-4-carboxylique ;
acide 1-(1H-indol-5-yl)-3-[[(*trans*-4-méthylcyclohexyl)-carbonyl](1-méthyléthyl)amino]-1H-pyrazole-4-carboxylique ;
acide 3-[[(*trans-*4-méthylcyclohexyl)carbonyl] (1-méthyléthyl)amino]-1-{4-[(E/Z)-2-phényléthényl]phényl}-1H-pyrazole-4-carboxylique ;
acide 3-[[(*trans*-4-méthylcyclohexyl)carbonyl](1-méthyléthyl)amino]-1-[4-(2-phényléthyl)phényl]-1H-pyrazole-4-carboxylique ;
acide 3-[[(*trans*-4-méthylcyclohexyl)carbonyl](tétrahydro-2H-pyranne-4-yl)amino]-1-{4-[2-phényléthyl]phényl}-1H-pyrazole-4-carboxylique ;
acide 3-[[(*trans*-4-méthylcyclohexyl)carbonyl](1-méthyléthyl)amino]-1-[4-[(1,3-thiazole-4-yl)-éthyl]phényl]-1H-pyrazole-4-carboxylique ;
acide 3-[[(*trans*-4-méthylcyclohexyl)carbonyl](tétrahydro-2H-pyranne-4-yl)amino]-1-{4-[(1,3-thiazole-4-yl)-éthyl]phényl}-1H-pyrazole-4-carboxylique ;
acide 1-cyclohexyl-3-[[(*trans*-4-méthylcyclohexyl)carbonyl]-(1-méthyléthyl)amino]-1H-pyrazole-4-carboxylique ;
acide 3-[[(*trans*-4-méthylcyclohexyl)carbonyl] (1-méthyléthyl)amino]-1-[1-(méthylsulfonyl)-1,2,3,6-tétrahydro-4-pyridinyl]-1H-pyrazole-4-carboxylique ;
acide 3-[[(*trans*-4-méthylcyclohexyl)carbonyl](phénylméthyl)-amino]-1H-pyrazole-4-carboxylique ;
acide 3-{cyclopentyl[(*trans*-4-méthylcyclohexyl)carbonyl]-amino}-1-phényl-1H-pyrazole-4-carboxylique ;
acide 3-[[(*trans*-4-méthylcyclohexyl)carbonyl](tétrahydro-2H-pyranne-4-yl)amino]-1-phényl-1H-pyrazole-4-carboxylique ;
acide 3-{(1-acétyl-4-pipéridinyl)[(*tran*s-4-méthylcyclohexyl)-carbonyl]amino}-1-phényl-1H-pyrazole-4-carboxylique ;
acide 3-[[(*trans*-4-méthylcyclohexyl)carbonyl](4-pipéridinyl)-amino]-1-phényl-1*H*-pyrazole-4-carboxylique ;
acide 3-[[(*trans*-4-méthylcyclohexyl)carbonyl] (1-méthyléthyl)amino]-{4-[(E)-2-cyclohexyléthényl]phényl}-1H-pyrazole-4-carboxylique ;
acide 1-[4-(2-cyclohexyléthyl)phényl]-3-[[(*trans*-4-méthylcyclohexyl)carbonyl](1-méthyléthyl)amino]-1*H*-pyrazole-4-carboxylique ;
acide 3-[[(*trans*-4-méthylcyclohexyl)carbonyl] (1-méthyléthyl)amino]-1-{4-[2-pyridinyléthényl]phényl}-1H-pyrazole-4-carboxylique ;
acide 3-[[(*trans*-4-méthylcyclohexyl) carbonyl] (1-méthyléthyl)amino]-1-{4-[2-pyridinyléthyl]phényl}-1H-pyrazole-4-carboxylique ;
acide 3-[[(*trans*-4-méthylcyclohexyl) carbonyl] (1-méthyléthyl)amino]-1-{4-[1,3-thiazole-2-yléthyl]phényl}-1H-pyrazole-4-carboxylique ;
acide 3-[[(*trans*-4-méthylcyclohexyl)carbonyl] (1-méthyléthyl) amino] -1-{4- [2- (1H-pyrazole-3-yl) éthyl] phényl}-1H-pyrazole-4-carboxylique ;
acide 3-[[(*trans*-4-méthylcyclohexyl) carbonyl](1-méthyléthyl)amino]-1-{4-[(phénylamino)carbonyl]phényl}-1H-pyrazole- - 4-carboxylique ;
acide 3-[[(*trans*-4-méthylcyclohexyl)carbonyl] (1-méthyléthyl)amino]-1-{4-[(phénylcarbonyl)amino]phényl}-1*H*-pyrazole-4-carboxylique ;
acide 3-[[(*trans*-4-méthylcyclohexyl)carbonyl] (1-méthyléthyl)amino]-1-{4-[(3-méthylphénylcarbonyl)amino]phényl}-1*H-*pyrazole-4-carboxylique ;
acide 3-[[(*trans*-4-méthylcyclohexyl)carbonyl]{1-[(tertiobutyloxy)carbonyl]-4-pipéridinyl}amino]-1-phényl-1H-pyrazole-4-carboxylique ;
acide 3-[[(*trans*-4-méthylcyclohexyl)carbonyl] (1-méthyléthyl)amino]-1-{4-[(4-fluorophénylcarbonyl)amino]phényl}-1*H-*pyrazole-4-carboxylique ;
acide 3-[[(*trans*-4-méthylcyclohexyl)carbonyl] (1-méthyléthyl)amino]-1-{4-[(cyclohexylcarbonyl)amino]phényl}-1*H-*pyrazole-4-carboxylique ;
acide 1-(4-{[(4-fluorophényl)amino]carbonyl}phényl)-3-[[(*trans*-4-méthylcyclohexyl)carbonyl](1-méthyléthyl)amino]-1*H*-pyrazole-4-carboxylique ;
acide 3-[[(*trans*-4-méthylcyclohexyl)carbonyl](1-méthyléthyl)amino]-1-{3-[(chlorophénylcarbonyl)amino]phényl}-1*H-*pyrazole-4-carboxylique ;
acide 3-[[(*trans*-4-méthylcyclohexyl)carbonyl](1-méthyléthyl)amino]-1-{4-[(phénylsulfonyl)amino]phényl}-1*H*-pyrazole-4-carboxylique ;
acide 1-(4-méthyl-1-cyclohexène-1-yl)-3-[[(*trans*-4-méthylcyclohexyl)carbonyl](1-méthyléthyl)amino]-1*H*-pyrazole-4-carboxylique ;
acide 1-(4,4-diméthyl-1-cyclohexène-1-yl)-3-[[(*trans*-4-méthylcyclohexyl)carbonyl](tétrahydro-3-furannyl)amino]-1*H-*pyrazole-4-carboxylique ;
et leurs sels, produits de solvatation et esters, et leurs énantiomères individuels lorsque cela est approprié.

3. Au moins une entité chimique choisie parmi des composés de formule (I) : 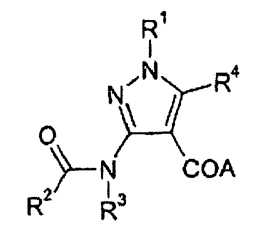 dans laquelle :
A représente un groupe hydroxy ;
R¹ représente un groupe aryle, hétéroaryle lié par un atome carbone du noyau, ou hétérocyclyle lié par un atome de carbone du noyau, chacun d'eux pouvant être facultativement substitué avec un ou plusieurs substituants choisis entre des substituants -alkyle en C₁ à C₆, halogéno, -OR^{A}, -SR^{A}, *-*C(O)NR^{B}R^{C}, -C(O)R^{D}, -CO₂H, -CO₂R^{D}, -NR^{B}R^{C}, NR^{E}C(O)R^{D}, - NR^{E}CO₂R^{D}, -NR^{E}C(O)NR^{F}R^{G}, -SO₂NR^{F}R^{G}, -SO₂R^{D}, nitro, cyano, -CF₃, -OCF₃, NR^{E}SO₂R^{D}, phényle et hétérocycle, dans lesquels le substituant -alkyle en C₁ à C₆ proprement dit peut être facultativement substitué avec un ou plusieurs substituants choisis entre des substituants -cycloalkyle en C₅ à C₉, halogéno, -NR^{B}R^{C}, -C(O)NR^{B}R^{C}, -NR^{E}C(O)R^{D}, -SR^{A}, -SO₂R^{D}, OR^{A}, oxo, phényle, hétéroaryle et hétérocyclyle ; ou bien R¹ représente un groupe -alkyle en C₁ à C₆ ou -cycloalkyle en C₅ à C₉ ;
R² représente un groupe phényle substitué avec un ou plusieurs substituants choisis entre des substituants -alkyle en C₁ à C₆, halogéno, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O) NR^{F}R^{G}, -SO₂NR^{F}R^{G}, -SO₂R^{D}, nitro, cyano et hétérocyclyle ; ou bien R² représente un groupe -(CH₂)ₙ(cycloalkyle en C₅ à C₇) facultativement substitué sur le groupe cycloalkyle avec un ou plusieurs substituants choisis entre des substituants -alkyle en C₁ à C₆, =CH(CH₂)ₜH; -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, fluoro, nitro, cyano, oxo et hétérocyclyle, ou bien deux substituants peuvent former conjointement un substituant à pont alkylène en C₁ ou C₂ ;
t est égal à 0, 1, 2, 3 ou 4 ;
n est égal à 0 ou 1 ;
R³ représente un groupe hétérocyclyle ou hétéroaryle ; ou un groupe phényle facultativement substitué avec un ou plusieurs substituants choisis entre des substituants -alkyle en C₁ à C₆, halogéno, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, -NR^{E}C(O)NR^{F}R^{G}, -SO₂NR^{F}R^{G}, -SO₂R^{D}, nitro, cyano et hétérocyclyle ; ou bien R³ représente un groupe -alkyle en C₁ à C₆ facultativement substitué avec un ou plusieurs substituants choisis entre des substituants -alkyle en C₁ à C₆, -OR^{A}, -SR^{A}, -C(O)NR^{B}R^{C}, -C(O)R^{D}, -CO₂H, -CO₂R^{D}, -NR^{B}R^{C}, -NR^{E}C(O)R^{D}, -NR^{E}CO₂R^{D}, NR^{E}C(O)NR^{F}R^{G}, -SO₂NR^{F}R^{G}, -SO₂R^{D}, fluoro, nitro, cyano, oxo, phényle, hétéroaryle et hétérocyclyle ;
R⁴ représente un atome d'hydrogène ;
R^{A} représente un atome d'hydrogène, un groupe -alkyle en C₁ à C₆, arylalkyle, hétéroarylalkyle, aryle, hétérocyclyle ou hétéroaryle ;
R^{B} et R^{C} représentent indépendamment un atome d'hydrogène, un groupe -alkyle en C₁ à C₆, aryle, hétérocyclyle ou hétéroaryle ; ou bien R^{B} et R^{C}, conjointement avec l'atome d'azote auquel ils sont fixés, forment un groupe cyclique penta- ou hexagonal saturé ;
R^{D} est choisi dans le groupe consistant en des groupes -alkyle en C₁ à C₆, aryle, hétérocyclyle, hétéroaryle, arylalkyle et hétéroarylalkyle ;
R^{E} représente un atome d'hydrogène ou un groupe -alkyle en C₁ à C₆ ;
R^{F} et R^{G} sont choisis indépendamment dans le groupe consistant en un atome d'hydrogène, des groupes -alkyle en C₁ à C₆, aryle, hétéroaryle, arylalkyle et hétéroarylalkyle ; ou bien R^{F} et R^{G}, conjointement avec l'atome d'azote auquel ils sont fixés, forment un groupe cyclique penta- ou hexagonal saturé ;
et leurs sels, produits de solvatation et esters ;
pour une utilisation en thérapie médicale.

4. Composé suivant la revendication 3, dans lequel la thérapie médicale est le traitement d'une infection virale.

5. Composé suivant la revendication 4, dans lequel l'infection virale est une infection par le HCV.

6. Formulation pharmaceutique comprenant au moins une entité chimique choisie parmi des composés de formule (I) et leurs sels, produits de solvatation et esters pharmaceutiquement acceptables tels que définis dans la revendication 1, conjointement avec au moins un diluant ou support pharmaceutiquement acceptable.

7. Procédé pour la préparation d'un composé de formule (I) répondant à la définition figurant dans la revendication 1, comprenant le traitement d'un composé de formule (II) 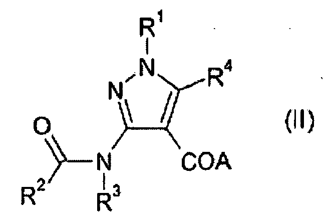 dans laquelle A représente un groupe alkoxy, benzyloxy ou silyloxy et R¹, R², R³ et R⁴ répondent aux définitions précitées pour la formule (I), avec une base.

8. Procédé suivant la revendication 7, dans lequel A représente un groupe éthoxy.

9. Utilisation d'au moins une entité chimique choisie parmi des composés de formule (I) et leurs sels, produits de solvatation et esters pharmaceutiquement acceptables suivant la revendication 1, dans la production d'un médicament destiné au traitement et/ou à la prophylaxie d'une infection virale.

10. Utilisation suivant la revendication 9, dans laquelle l'infection virale est une infection par le HCV.
